# EUROPEAN PATENT APPLICATION

(11) **EP 4 610 728 A2**
(43) Date of publication of application: **03.09.2025**
(21) Application number: 25160214.0
(22) Date of filing: 26.02.2025
(51) Int. Cl.: G03F 7/004, G03F 7/039

(54) **SULFONIUM SALT MONOMER, POLYMER, CHEMICALLY AMPLIFIED POSITIVE RESIST COMPOSITION, AND RESIST PATTERN FORMING PROCESS**

(30) Priority: 28.02.2024 JP 2024028224
(71) Applicant: SHIN-ETSU CHEMICAL CO., LTD., Tokyo 1000005 (JP)
(72) Inventor: MASUNAGA, Keiichi, NIIGATA-KEN, 9428601 (JP); WATANABE, Satoshi, NIIGATA-KEN, 9428601 (JP); FUNATSU, Kenji, NIIGATA-KEN, 9428601 (JP); FUKUSHIMA, Masahiro, NIIGATA-KEN, 9428601 (JP); KOTAKE, Masaaki, NIIGATA-KEN, 9428601 (JP); MATSUZAWA, Yuta, NIIGATA-KEN, 9428601 (JP)
(74) Representative: Ter Meer Steinmeister & Partner

(57) **Abstract**

A sulfonium salt monomer containing an aromatic sulfonic acid anion having a vinyl-substituted aromatic ring generates an acid having an adequate acid strength and reduced diffusion distance. A chemically amplified positive resist composition comprising a polymer comprising repeat units derived from the monomer exhibits etch resistance and organic solvent solubility.

## Description

### TECHNICAL FIELD

This invention relates to a sulfonium salt monomer, a polymer, a chemically amplified positive resist composition, and a resist pattern forming process.

### BACKGROUND ART

To meet the recent demand for higher integration density and operating speed of LSIs, the effort to reduce the pattern rule is in rapid progress. Acid-catalyzed chemically amplified resist compositions are most often used in forming resist patterns with a feature size of 0.2 µm or less. High-energy radiation such as UV, deep-UV or EB is used as the light source for exposure of these resist compositions. In particular, while EB lithography is utilized as the ultra-fine microfabrication technique, it is also indispensable in processing photomask blanks to form photomasks for use in semiconductor device fabrication.

Polymers comprising a major proportion of aromatic structure having an acidic side chain, for example, polyhydroxystyrene are useful in resist materials for the KrF excimer laser lithography. These polymers are not used in resist materials for the ArF excimer laser lithography because they exhibit strong absorption at a wavelength of around 200 nm. These polymers, however, are expected to form useful resist materials for the EB and EUV lithography for forming patterns of smaller size than the processing limit of ArF excimer laser because they offer high etching resistance.

Often used as the base polymer in positive resist compositions for EB and EUV lithography is a polymer having an acidic functional group on phenol side chain masked with an acid-decomposable protective group (acid labile group). Upon exposure to high-energy radiation, the acid-decomposable protective group is deprotected by the catalysis of an acid generated from a photoacid generator so that the polymer may turn soluble in alkaline developer.

Typical of the acid-decomposable protective group are tertiary alkyl, tert-butoxycarbonyl, and acetal groups. The use of protective groups requiring a relatively low level of activation energy for deprotection such as acetal groups offers the advantage that a resist film having a high sensitivity is obtainable. However, if the diffusion of generated acid is not fully controlled, deprotection reaction can occur even in the unexposed region of the resist film, giving rise to problems like a lowering of line edge roughness (LER) and degradation of critical dimension uniformity (CDU) of pattern line width.

One of the important applications of chemically amplified resist material resides in processing of photomask blanks. Some photomask blanks have a surface material that can have an impact on the pattern profile of the overlying chemically amplified resist film, for example, a layer of a chromium compound, typically chromium oxide deposited on a photomask substrate. For high resolution and profile retention after etching, it is one important performance factor to maintain the profile of a resist film pattern rectangular independent of the type of substrate. In recent years, the multibeam mask writing (MBMW) process is used in the processing of mask blanks to achieve further miniaturization. The resist used in the MBMW process is a low-sensitivity resist (or high-dose region) which is advantageous in roughness while a spotlight is brought to the optimization of the resist composition in the high-dose region.

Attempts were made to ameliorate resist sensitivity and pattern profile in a controlled way by properly selecting and combining components used in resist compositions and adjusting processing conditions. One outstanding problem is the diffusion of acid.

Since acid diffusion has a significant impact on the sensitivity and resolution of a chemically amplified resist composition, many studies are made on the acid diffusion problem.

Patent Documents 1 and 2 describe photoacid generators capable of generating bulky acids like benzenesulfonic acid upon exposure, for thereby controlling acid diffusion and reducing roughness. Since these acid generators are still insufficient to control acid diffusion, it is desired to have an acid generator with more controlled diffusion.

Patent Document 3 describes a sulfonium salt capable of generating an acid having a high acid strength such as fluorinated alkane sulfonic acid and a polymer comprising repeat units having an acetal group. This resist has the problem that the pattern has a large LER. Since the acetal group requires a relatively low level of activation energy for deprotection, the acid strength of the fluorinated alkane sulfonic acid is excessively high for the deprotection of acetal group. Although acid diffusion is controlled, deprotection reaction can take place even in the unexposed region with a minor amount of acid having diffused into the unexposed region. The same applies to the sulfonium salt capable of generating benzene sulfonic acid as described in Patent Documents 1 and 2. It is desired to have an acid generator capable of generating an acid having more appropriate strength for the deprotection of acetal group.

With the aim to control acid diffusion, Patent Documents 4 to 6 describe resist compositions based on a polymer comprising repeat units derived from an onium salt of sulfonic acid having a polymerizable unsaturated bond. Since the so-called polymer-bound acid generator generates a polymeric sulfonic acid upon exposure, it is characterized by quite short acid diffusion. Also, sensitivity can be enhanced by increasing the content of the acid generator. In the case of an acid generator of addition type, as the amount of the acid generator added is increased, a higher sensitivity is obtained, but the acid diffusion distance is also increased. Since the acid diffuses non-uniformly, the increased acid diffusion invites degradation of LER or CDU. Since the polymer-bound acid generator has a potentially high capability with respect to the balance of sensitivity and LER or CDU, it is desired to have an acid generator capable of generating an acid having more appropriate strength.

Patent Document 7 discloses a resist composition comprising a polymer to which an acid generator capable of generating a sulfonic acid is bound as the acid generator capable of generating an acid having more appropriate strength, whereby acid diffusion is controlled. The method of controlling acid diffusion by incorporating repeat units capable of generating an acid upon light exposure into a base polymer is effective for forming a pattern with reduced LER. However, the base polymer having repeat units capable of generating an acid upon light exposure bound thereto sometimes gives rise to the problem of organic solvent solubility, depending on the structure and incorporation rate of the repeat units.

With the recent advance of the miniaturization technology, resist patterns suffer from collapse in the development step and poor resistance in the etching step. Although the diffusion of the generated acid is controlled to some extent by incorporating repeat units derived from an onium salt of sulfonic acid having a polymerizable unsaturated bond, there still remains room for improvements in lithography performance, resist pattern collapse, and etch resistance. To meet the future demand for further miniaturization, it is quite important to have a polymeric acid generator capable of satisfying all of lithography performance, resist pattern collapse, and etch resistance.

### Citation List

Patent Document 1: JP-A 2009-053518
Patent Document 2: JP-A 2010-100604
Patent Document 3: JP 5083528
Patent Document 4: JP 4425776
Patent Document 5: JP 5201363 (USP 8,057,985)
Patent Document 6: JP 5231357
Patent Document 7: JP-A 2011-022564 (USP 8,361,693, EP 2264525B1)

### DISCLOSURE OF INVENTION

An object of the invention is to provide a sulfonium salt monomer capable of generating an acid having an adequate acid strength and reduced diffusion, a polymer comprising repeat units derived from the monomer, and a chemically amplified resist composition comprising the polymer, the composition having the advantages of etch resistance and organic solvent solubility. Another object is to provide a pattern forming process using the resist composition.

The inventors have found that when a polymer comprising repeat units derived from a sulfonium salt monomer containing an aromatic sulfonic acid anion having a vinyl-substituted aromatic ring is used as a polymer-bound acid generator, there is obtained a chemically amplified resist composition having high contrast, high resolution, reduced LER, and improved etch resistance.

Accordingly, the invention provides a sulfonium salt monomer, polymer, chemically amplified positive resist composition, and resist pattern forming process, as defined below.
1. A sulfonium salt monomer having the formula (A1): 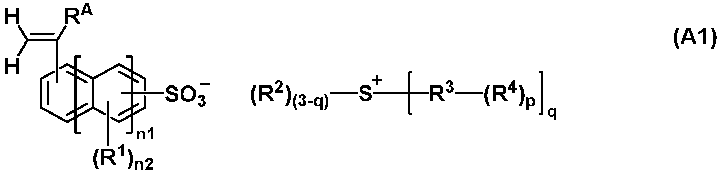 wherein n1 is an integer of 0 to 2, n2 is an integer meeting 0 ≤ n2 ≤ 5+2(n1)-1, p is an integer of 1 to 5, q is an integer of 1 to 3,
   R^{A} is hydrogen, fluorine, methyl or trifluoromethyl,
   R¹ is halogen, nitro, cyano, an optionally halogenated C₁-C₁₀ saturated hydrocarbyl group, optionally halogenated C₁-C₁₀ saturated hydrocarbyloxy group, optionally halogenated C₂-C₁₀ saturated hydrocarbylcarbonyloxy group, or C₁-C₁₀ fluorinated saturated hydrocarbylthio group,
   R² is a C₁-C₃₀ hydrocarbyl group which may contain a heteroatom,
   R³ is a C₁-C₃₀ (p+1)-valent hydrocarbon group which may contain a heteroatom,
   R⁴ is fluorine, a C₁-C₅ fluorinated saturated hydrocarbyl group, C₁-C₅ fluorinated saturated hydrocarbyloxy group, C₂-C₅ fluorinated saturated hydrocarbylcarbonyloxy group, C₂-C₅ fluorinated saturated hydrocarbyloxycarbonyl group, or C₁-C₅ fluorinated saturated hydrocarbylthio group, in which some hydrogen may be substituted by at least one moiety selected from hydroxy, chlorine, bromine, iodine, nitro and cyano, and at least one moiety selected from ester bond, ether bond, sulfonate ester bond, carbonate bond and carbamate bond may intervene in a carbon-carbon bond,
   when q is 1, any two of two R² and R³ may bond together to form a ring with the sulfur atom to which they are attached; when q is 2, any two of R² and two R³ may bond together to form a ring with the sulfur atom to which they are attached; when q is 3, any two of three R³ may bond together to form a ring with the sulfur atom to which they are attached.
2. The sulfonium salt monomer of 1, having the formula (A1-1): 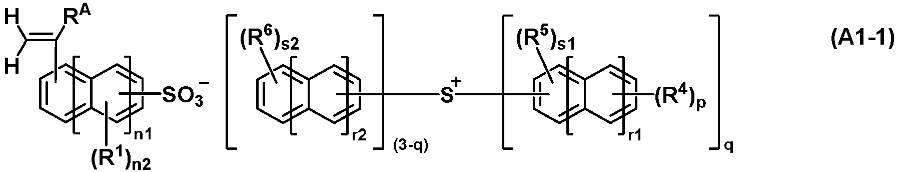 wherein n1, n2, p, q, R^{A}, R¹ and R⁴ are as defined above,
   R⁵ and R⁶ are each independently halogen exclusive of fluorine, nitro, cyano, or a C₁-C₂₀ hydrocarbyl group which may contain a heteroatom,
   r1 and r2 are each independently an integer of 0 to 2, s1 is an integer meeting 0 ≤ s1 ≤ 2(r1)+4, s2 is an integer meeting 0 ≤ s2 ≤ 2(r2)+4.
3. The sulfonium salt monomer of 2 wherein R⁴ is fluorine, trifluoromethyl, trifluoromethoxy or trifluoromethylthio.
4. A polymer comprising repeat units derived from the sulfonium salt monomer of any one of 1 to 3 and adapted to increase its solubility in alkaline aqueous solution under the action of acid.
5. The polymer of 4, further comprising repeat units having the formula (A2): 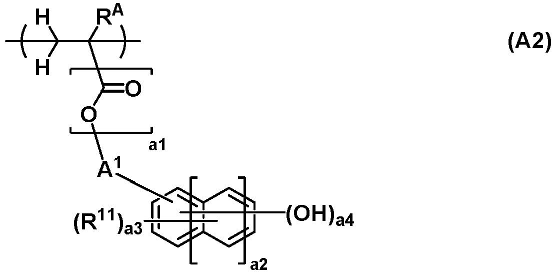 wherein a1 is 0 or 1, a2 is an integer of 0 to 2, a3 is an integer meeting 0 ≤ a3 ≤ 5+2(a2)-a4, a4 is an integer of 1 to 3,
   R^{A} is hydrogen, fluorine, methyl or trifluoromethyl,
   R¹¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, and
   A¹ is a single bond or C₁-C₁₀ saturated hydrocarbylene group in which some -CH₂-may be replaced by -O-.
6. The polymer of 4 or 5, further comprising repeat units of at least one type selected from repeat units having the formula (A3-1) and repeat units having the formula (A3-2): 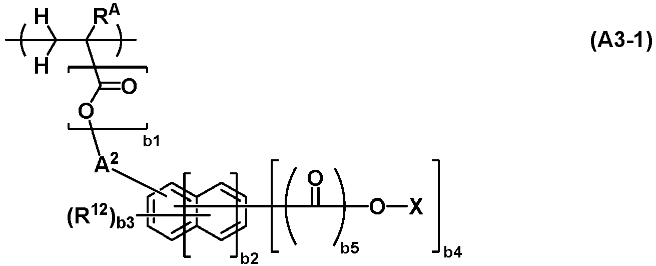 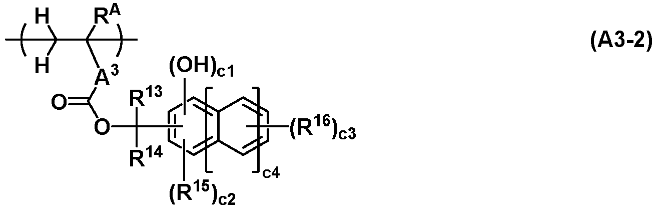 wherein b1 is 0 or 1, b2 is an integer of 0 to 2, b3 is an integer meeting 0 ≤ b3 ≤ 5+2(b2)-b4, b4 is an integer of 1 to 3, b5 is 0 or 1,
   c1 is an integer of 0 to 2, c2 is an integer of 0 to 2, c3 is an integer of 0 to 5, c4 is an integer of 0 to 2,
   R^{A} is hydrogen, fluorine, methyl or trifluoromethyl,
   R¹² is nitro, cyano, halogen, an optionally halogenated C₁-C₆ saturated hydrocarbyl group, optionally halogenated C₁-C₆ saturated hydrocarbyloxy group, optionally halogenated C₁-C₆ saturated hydrocarbylthio group, C₂-C₈ saturated hydrocarbylcarbonyl group, or optionally halogenated C₂-C₈ saturated hydrocarbylcarbonyloxy group,
   R¹³ and R¹⁴ are each independently a C₁-C₁₀ hydrocarbyl group which may contain a heteroatom, R¹³ and R¹⁴ may bond together to form a ring with the carbon atom to which they are attached,
   R¹⁵ is each independently fluorine, a C₁-C₅ fluorinated alkyl group or C₁-C₅ fluorinated alkoxy group,
   R¹⁶ is each independently a C₁-C₁₀ hydrocarbyl group which may contain a heteroatom,
   A² is a single bond or C₁-C₁₀ saturated hydrocarbylene group in which some -CH₂-may be replaced by -O-,
   X is an acid labile group when b4 is 1, and X is each independently hydrogen or an acid labile group, at least one X being an acid labile group, when b4 is 2 or 3,
   A³ is a single bond, phenylene, naphthylene, or *-C(=O)-O-A³¹-, A³¹ is a C₁-C₂₀ aliphatic hydrocarbylene group which may contain at least one moiety selected from hydroxy, ether bond, ester bond and lactone ring, phenylene group, or naphthylene group, * designates a point of attachment to the carbon atom in the backbone.
7. The polymer of any one of 4 to 6, further comprising repeat units of at least one type selected from repeat units having the formula (A4), repeat units having the formula (A5), and repeat units having the formula (A6): 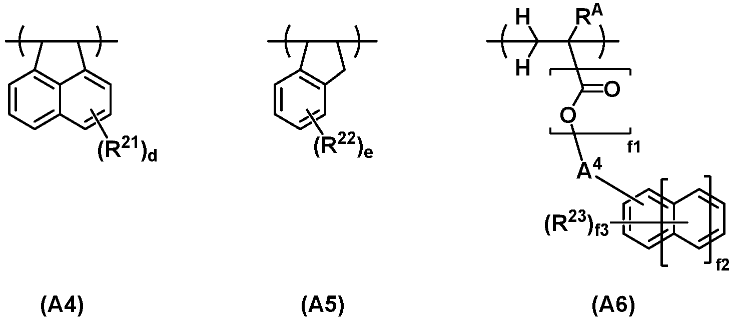 wherein d and e are each independently an integer of 0 to 4, f1 is 0 or 1, f2 is an integer of 0 to 2, f3 is an integer of 0 to 5,
   R^{A} is hydrogen, fluorine, methyl or trifluoromethyl,
   R²¹ and R²² are each independently hydroxy, halogen, an optionally halogenated C₁-C₈ saturated hydrocarbyl group, optionally halogenated C₁-C₈ saturated hydrocarbyloxy group, or optionally halogenated C₂-C₈ saturated hydrocarbylcarbonyloxy group,
   R²³ is halogen, trifluoromethyl, trifluoromethoxy, nitro, cyano, a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, C₁-C₂₀ saturated hydrocarbylsulfinyl group, or C₁-C₂₀ saturated hydrocarbylsulfonyl group, and
   A⁴ is a single bond or C₁-C₁₀ saturated hydrocarbylene group in which some -CH₂-may be replaced by -O-.
8. A chemically amplified positive resist composition comprising (A) a base polymer containing the polymer of any one of 4 to 7.
9. The resist composition of 8 wherein repeat units having an aromatic ring structure account for at least 60 mol% of the overall repeat units of the polymer in the base polymer.
10. The resist composition of 8 or 9, further comprising (B) a quencher.
11. The resist composition of any one of 8 to 10, further comprising (C) a photoacid generator.
12. The resist composition of any one of 8 to 11 wherein the photoacid generator (C) and the quencher (B) are present in a weight ratio of less than 6/1.
13. The resist composition of any one of 8 to 12, further comprising (D) a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6): 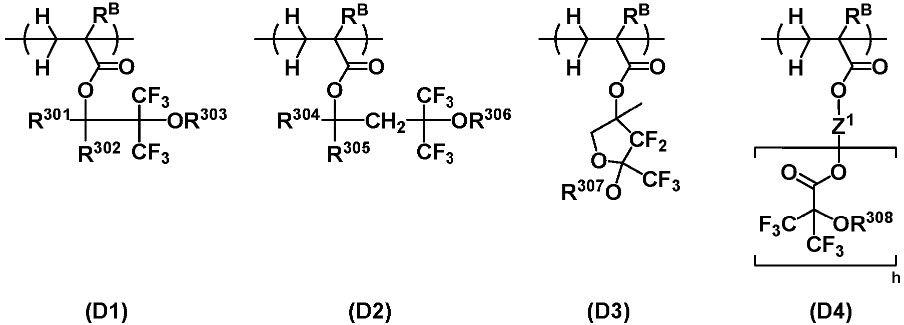 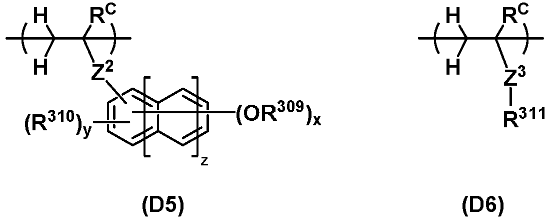 wherein x is an integer of 1 to 3, y is an integer meeting 0 ≤ y ≤ 5+2z-x, z is 0 or 1, h is an integer of 1 to 3,
   R^{B} is each independently hydrogen, fluorine, methyl or trifluoromethyl,
   R^{C} is each independently hydrogen or methyl,
   R³⁰¹, R³⁰², R³⁰⁴ and R³⁰⁵ are each independently hydrogen or a C₁-C₁₀ saturated hydrocarbyl group,
   R³⁰³, R³⁰⁶, R³⁰⁷ and R³⁰⁸ are each independently hydrogen, a C₁-C₁₅ hydrocarbyl group, C₁-C₁₅ fluorinated hydrocarbyl group, or acid labile group, and when R³⁰³, R³⁰⁶, R³⁰⁷ and R³⁰⁸ each are a hydrocarbyl or fluorinated hydrocarbyl group, an ether bond or carbonyl moiety may intervene in a carbon-carbon bond,
   R³⁰⁹ is hydrogen or a C₁-C₅ straight or branched hydrocarbyl group in which a heteroatom-containing moiety may intervene in a carbon-carbon bond,
   R³¹⁰ is a C₁-C₅ straight or branched hydrocarbyl group in which a heteroatom-containing moiety may intervene in a carbon-carbon bond,
   R³¹¹ is a C₁-C₂₀ saturated hydrocarbyl group in which at least one hydrogen is substituted by fluorine, and in which some constituent -CH₂- may be replaced by an ester bond or ether bond,
   Z¹ is a C₁-C₂₀ (h+1)-valent hydrocarbon group or C₁-C₂₀ (h+1)-valent fluorinated hydrocarbon group,
   Z² is a single bond, *-C(=O)-O- or *-C(=O)-NH-, * designates a point of attachment to the carbon atom in the backbone,
   Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.
14. The resist composition of any one of 8 to 13, further comprising (E) an organic solvent.
15. A resist pattern forming process comprising the steps of:
   applying the chemically amplified positive resist composition of any one of 8 to 14 onto a substrate to form a resist film thereon,
   exposing the resist film patternwise to high-energy radiation, and
   developing the exposed resist film in an alkaline developer.
16. The process of 15 wherein the high-energy radiation is EUV or EB.
17. The process of 15 or 16 wherein the substrate has the outermost surface of a material containing at least one element selected from chromium, silicon, tantalum, molybdenum, cobalt, nickel, tungsten, and tin.
18. The process of any one of 15 to 17 wherein the substrate is a mask blank of transmission or reflection type.
19. A mask blank of transmission or reflection type which is coated with the chemically amplified positive resist composition of any one of 8 to 14.

### ADVANTAGEOUS EFFECTS OF INVENTION

A polymer comprising repeat units derived from a sulfonium salt monomer represented by formula (A1) is characterized by high solvent solubility and short acid diffusion because of the robust structure of aromatic sulfonic acid. A positive resist composition comprising the polymer prevents resolution from declining due to blur by acid diffusion and enables to improve LER. The aromatic ring serves as an etch resistant group, which is advantageous in forming small-size patterns.

### BRIEF DESCRIPTION OF DRAWINGS

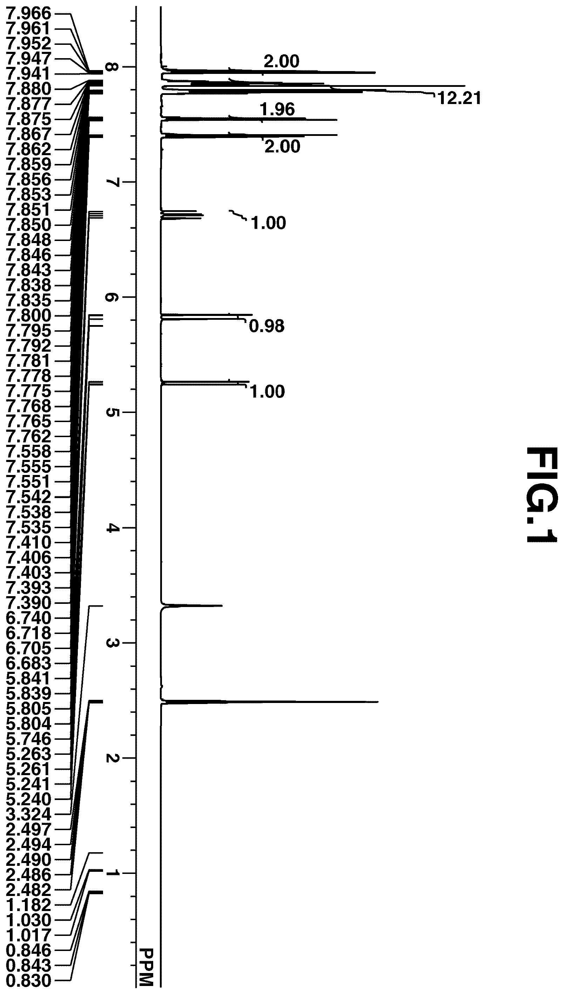

The only figure, FIG. 1 is a diagram showing NMR spectrum (¹H-NMR/DMSO-d₆) of Monomer PM-1 synthesized in Example 1-1.

### DESCRIPTION OF EMBODIMENTS

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group. In chemical formulae, Me stands for methyl, Ac for acetyl. Both the broken line (---) and the asterisk (*) designate a point of attachment or valence bond. As used herein, the term "fluorinated" refers to a fluorine-substituted or fluorine-containing compound or group. The terms "group" and "moiety" are interchangeable.

The abbreviations and acronyms have the following meaning.
- EB:: electron beam
- EUV:: extreme ultraviolet
- Mw:: weight average molecular weight
- Mn:: number average molecular weight
- Mw/Mn:: molecular weight distribution or dispersity
- GPC:: gel permeation chromatography
- PEB:: post-exposure bake
- PAG:: photoacid generator
- LER:: line edge roughness
- LWR:: line width roughness
- CDU:: critical dimension uniformity

It is understood that for some structures represented by chemical formulae, there can exist enantiomers and diastereomers because of the presence of asymmetric carbon atoms. In such a case, a single formula collectively represents all such isomers. The isomers may be used alone or in admixture.

### Sulfonium salt monomer

One embodiment of the invention is a sulfonium salt monomer having the formula (A1).

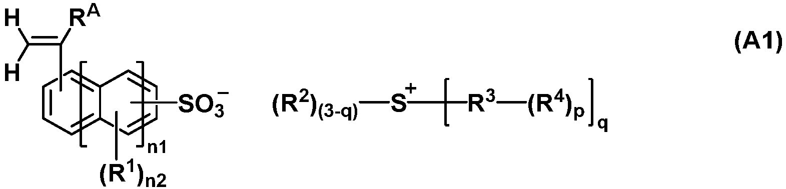

In formula (A1), n1 is an integer of 0 to 2. The structure with n1 encompasses a benzene ring and fused polycyclic aromatic groups composed of three or less aromatic rings such as naphthalene, anthracene and phenanthrene rings. From the aspect of solvent solubility, the benzene ring corresponding to n1=0 is preferred.

In formula (A1), n2 is an integer meeting 0 ≤ n2 ≤ 5+2(n1)-1. When n1=0, n2 is preferably 0, 1, 2 or 3, more preferably 0. When n1=1 or 2, n2 is preferably 0, 1, 2, 3 or 4.

In formula (A1), p is an integer of 1 to 5. From the aspect of reactant availability, p is preferably 1, 2 or 3, more preferably 1 or 2. The subscript q is an integer of 1 to 3, preferably 1 or 2, more preferably 1.

In formula (A1), R^{A} is hydrogen, fluorine, methyl or trifluoromethyl, preferably hydrogen or methyl, most preferably hydrogen.

In formula (A1), R¹ is halogen, nitro, cyano, an optionally halogenated C₁-C₁₀ saturated hydrocarbyl group, an optionally halogenated C₁-C₁₀ saturated hydrocarbyloxy group, an optionally halogenated C₂-C₁₀ saturated hydrocarbylcarbonyloxy group, or a C₁-C₁₀ fluorinated saturated hydrocarbylthio group. The saturated hydrocarbyl group and saturated hydrocarbyl moiety in the saturated hydrocarbyloxy group, saturated hydrocarbylcarbonyloxy group, and saturated hydrocarbylthio group may be straight, branched or cyclic. Examples thereof include C₁-C₁₀ alkyl groups such as methyl, ethyl, n-propyl, isopropyl, butyl, pentyl, and hexyl; C₃-C₁₀ cycloalkyl groups such as cyclopropyl, cyclobutyl, cyclopentyl, and cyclohexyl; and combinations thereof. A carbon count within the upper limit ensures good solubility in alkaline developer. When n2 is 2 or more, a plurality of R¹ may be identical or different.

In formula (A1), R² is a C₁-C₃₀ hydrocarbyl group which may contain a heteroatom. The hydrocarbyl group may be saturated or unsaturated and straight, branched or cyclic. Examples thereof include C₁-C₃₀ alkyl groups such as methyl, ethyl, n-propyl, isopropyl, n-butyl, and tert-butyl; C₃-C₃₀ cyclic saturated hydrocarbyl groups such as cyclopropyl, cyclopentyl, cyclohexyl, cylopropylmethyl, 4-methylcyclohexyl, cyclohexylmethyl, norbornyl, and adamantyl; C₂-C₃₀ alkenyl groups such as vinyl, allyl, propenyl, butenyl, and hexenyl; C₃-C₃₀ cyclic unsaturated hydrocarbyl groups such as cyclohexenyl; C₆-C₃₀ aryl groups such as phenyl and naphthyl; C₇-C₃₀ aralkyl groups such as benzyl, 1-phenylethyl, and 2-phenylethyl, and combinations thereof. Of these, aryl groups are preferred. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, fluorine, chlorine, bromine, iodine, carbonyl moiety, ether bond, ester bond, sulfonate ester bond, carbonate bond, lactone ring, sultone ring, carboxylic anhydride (-C(=O)-O-C(=O)-) or haloalkyl moiety.

In formula (A1), R³ is a C₁-C₃₀ (p+1)-valent hydrocarbon group which may contain a heteroatom. The (p+1)-valent hydrocarbon group may be saturated or unsaturated and straight, branched or cyclic. Examples thereof include C₁-C₃₀ hydrocarbylene groups and groups obtained by eliminating (p-1) number of hydrogen atoms from the hydrocarbylene groups. Examples of the hydrocarbylene group include C₁-C₃₀ alkanediyl groups such as methanediyl, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl, dodecane-1,12-diyl, tridecane-1,13-diyl, tetradecane-1,14-diyl, pentadecane-1,15-diyl, hexadecane-1,16-diyl, and heptadecane-1,17-diyl; C₃-C₃₀ cyclic saturated hydrocarbylene groups such as cyclopentanediyl, cyclohexanediyl, norbornanediyl and adamantanediyl; C₆-C₃₀ arylene groups such as phenylene, methylphenylene, ethylphenylene, n-propylphenylene, isopropylphenylene, n-butylphenylene, isobutylphenylene, sec-butylphenylene, tert-butylphenylene, naphthylene, methylnaphthylene, ethylnaphthylene, n-propylnaphthylene, isopropylnaphthylene, n-butylnaphthylene, isobutylnaphthylene, sec-butylnaphthylene and tert-butylnaphthylene; and combinations thereof.

In the (p+1)-valent hydrocarbon group, some or all of the hydrogen atoms may be substituted by a moiety containing a heteroatom such as oxygen, sulfur, nitrogen or halogen, or some constituent -CH₂- may be replaced by a moiety containing a heteroatom such as oxygen, sulfur or nitrogen, so that the group may contain a hydroxy, cyano, fluorine, chlorine, bromine, iodine, carbonyl, ether bond, ester bond, sulfonic ester bond, carbonate bond, lactone ring, sultone ring, carboxylic anhydride (-C(=O)-O-C(=O)-) or haloalkyl moiety.

In formula (A1), R⁴ is fluorine, a C₁-C₅ fluorinated saturated hydrocarbyl group, C₁-C₅ fluorinated saturated hydrocarbyloxy group, C₂-C₅ fluorinated saturated hydrocarbylcarbonyloxy group, C₂-C₅ fluorinated saturated hydrocarbyloxycarbonyl group, or C₁-C₅ fluorinated saturated hydrocarbylthio group, in which some hydrogen may be substituted by at least one moiety selected from hydroxy, chlorine, bromine, iodine, nitro and cyano, and at least one moiety selected from ester bond, ether bond, sulfonate ester bond, carbonate bond and carbamate bond may intervene in a carbon-carbon bond. Inter alia, R⁴ is preferably fluorine, trifluoromethyl, difluoromethyl, trifluoromethoxy, difluoromethoxy, trifluoromethylthio or difluoromethylthio, more preferably fluorine, trifluoromethyl or trifluoromethoxy, most preferably trifluoromethoxy. Particularly when R⁴ is trifluoromethoxy, an outstanding improvement in solvent solubility is achieved, and the limit on the incorporation rate of repeat units A1 is mitigated, and the sulfonium salt is uniformly dissolved in the solvent without agglomeration and thus uniformly dispersed in a resist film. Since the uniformly dispersed sulfonium salt generates an acid upon exposure to high-energy radiation, high resolution and improved LER are achievable. Since the sulfonium cation site contains fluorine, the solubility of the resist film in unexposed region in alkaline developer is controlled, which contributes to improvements in pattern contrast and resolution.

When q is 1, any two of two R² and R³ may bond together to form a ring with the sulfur atom to which they are attached. When q is 2, any two of R² and two R³ may bond together to form a ring with the sulfur atom to which they are attached. When q is 3, any two of three R³ may bond together to form a ring with the sulfur atom to which they are attached. Exemplary rings are shown below.

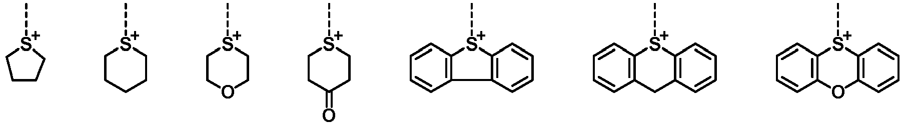

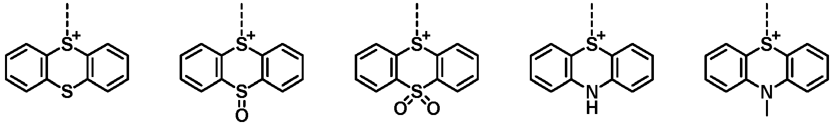

Of the sulfonium salts having formula (A1), those having the formula (A1-1) are preferred. 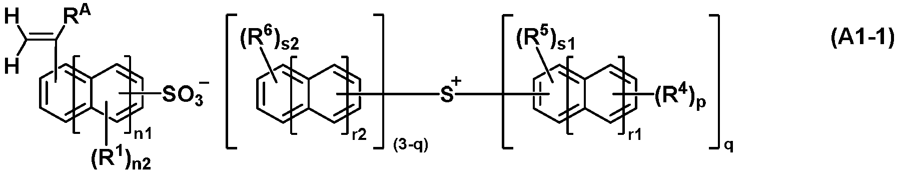 Herein n1, n2, p, q, R^{A}, R¹ and R⁴ are as defined above.

In formula (A1-1), R⁵ and R⁶ are each independently halogen exclusive of fluorine, nitro, cyano, or a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, propyl, isopropyl, n-butyl, sec-butyl, tert-butyl, n-pentyl, tert-pentyl, n-hexyl, n-octyl, 2-ethylhexyl, n-nonyl, and n-decyl; C₃-C₂₀ cyclic saturated hydrocarbyl groups such as cyclopentyl, cyclohexyl, cylopentylmethyl, cylopentylethyl, cylopentylbutyl, cyclohexylmethyl, cyclohexylethyl, cyclohexylbutyl, norbornyl, tricyclo[5.2.1.0^{2,6}]decanyl, adamantyl and adamantylmethyl; C₆-C₂₀ aryl groups such as phenyl, naphthyl and anthracenyl; and combinations thereof.

In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, fluorine, chlorine, bromine, iodine, carbonyl moiety, ether bond, ester bond, sulfonate ester bond, carbonate bond, carbamate bond, amide bond, imide bond, lactone ring, sultone ring, thiolactone ring, lactam ring, sultam ring, carboxylic anhydride (-C(=O)-O-C(=O)-) or haloalkyl moiety.

In formula (A1-1), r1 and r2 are each independently an integer of 0 to 2. The relevant structure is a benzene ring in case of r1 or r2 is 0, a naphthalene ring in case of r1 or r2 is 1, and an anthracene ring in case of r1 or r2 is 2. From the aspect of solvent solubility, the benzene ring corresponding to r1 or r2=0 is preferred.

In formula (A1-1), s1 is an integer meeting 0 ≤ s1 ≤ 2(r1)+4, preferably 0, and s2 is an integer meeting 0 ≤ s2 ≤ 2(r2)+4, preferably 0. When s1 is 2 or more, a plurality of R⁵ may be identical or different and a plurality of R⁵ may bond together to form a ring. When s2 is 2 or more, a plurality of R⁶ may be identical or different and a plurality of R⁶ may bond together to form a ring. Examples of the ring include cyclopropane, cyclobutane, cyclopentane, cyclohexane, norbornane, and adamantane rings.

Examples of the sulfonate anion in the sulfonium salt monomer having formula (A1) are shown below, but not limited thereto. Herein R^{A} is as defined above.

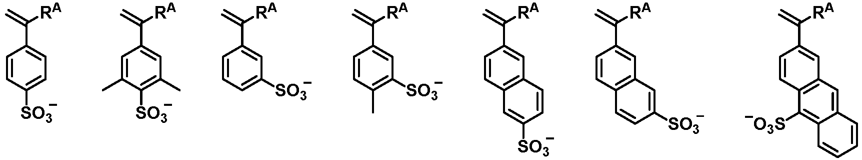

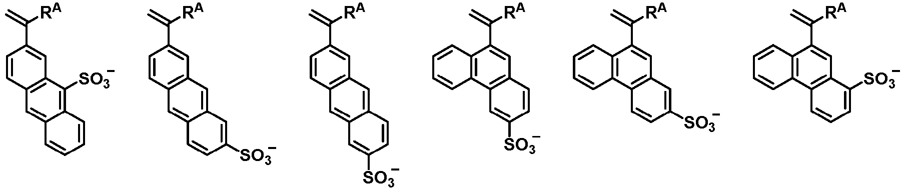

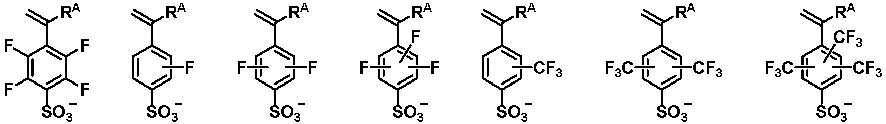

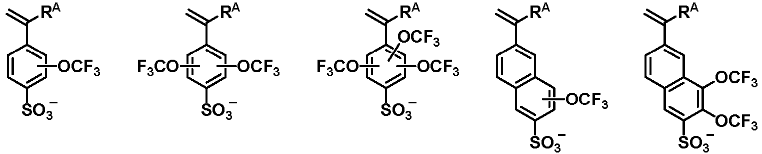

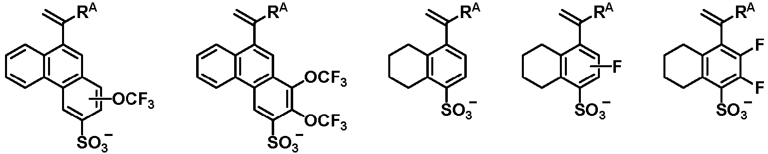

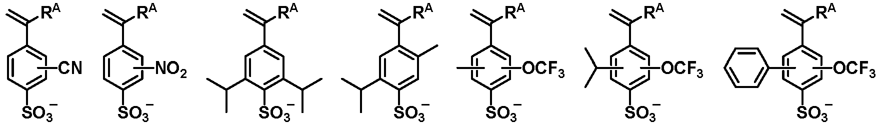

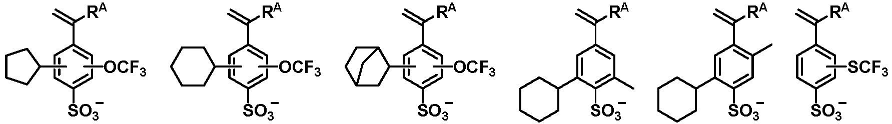

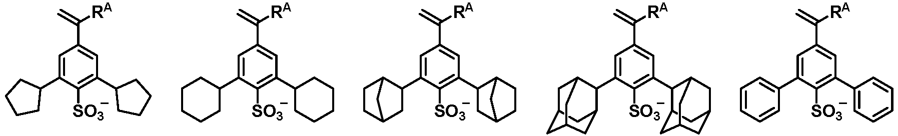

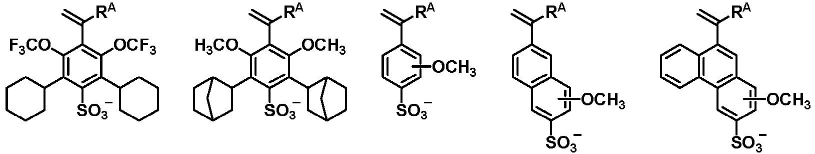

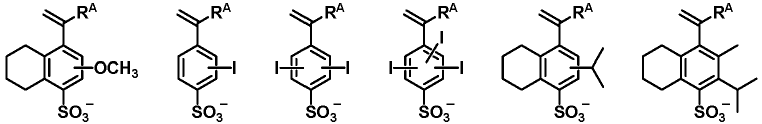

Examples of the sulfonium cation in the sulfonium salt monomer having formula (A1) are shown below, but not limited thereto.

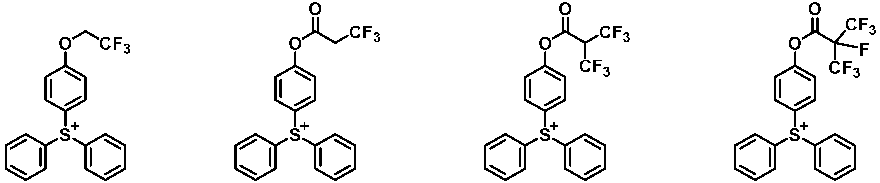

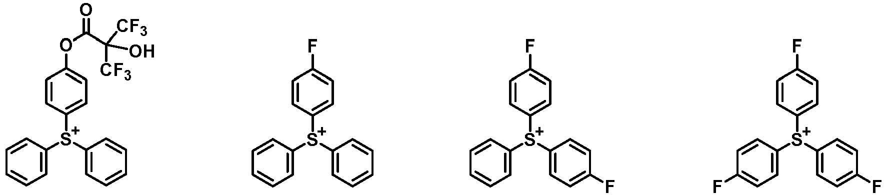

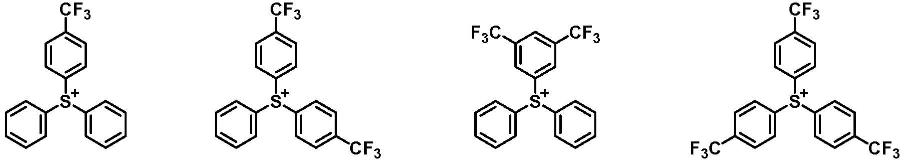

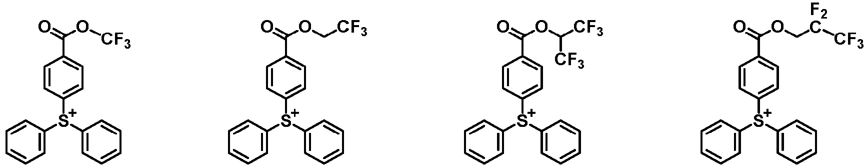

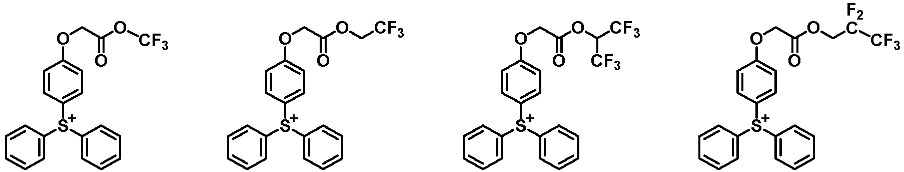

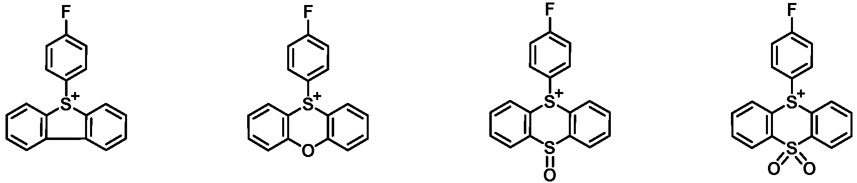

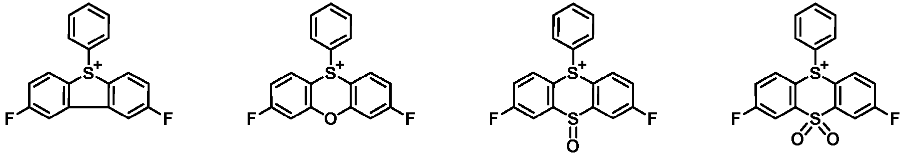

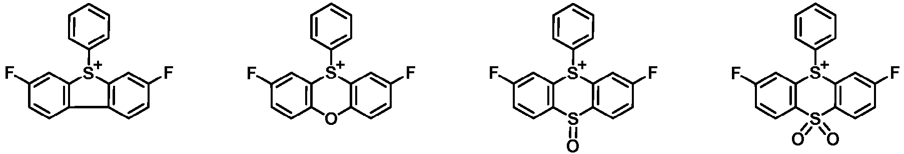

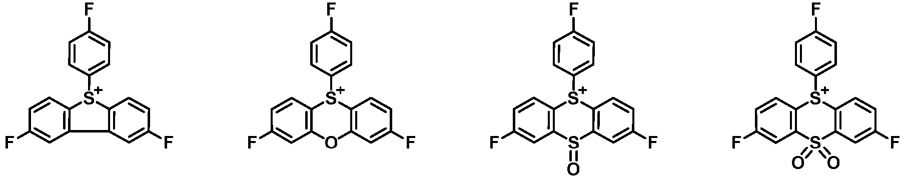

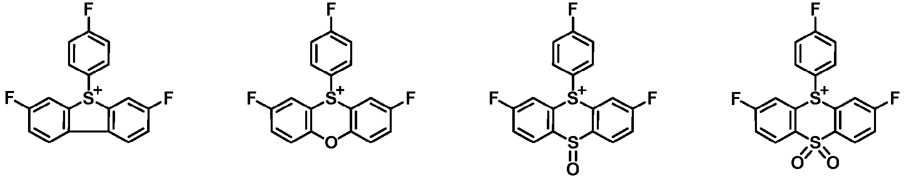

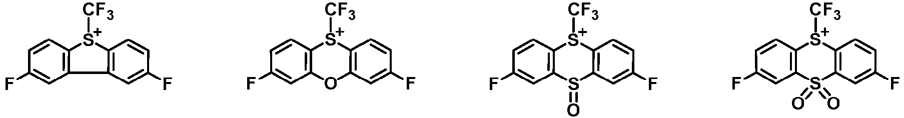

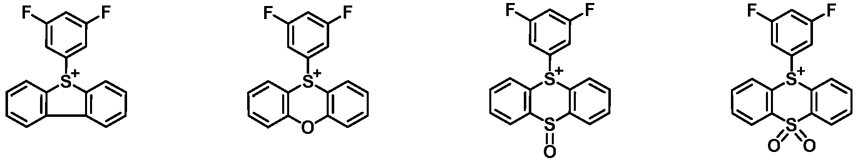

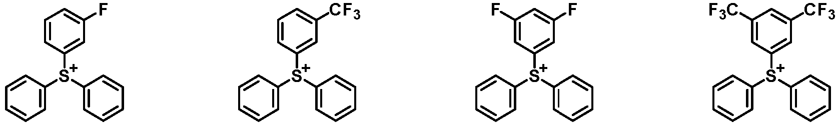

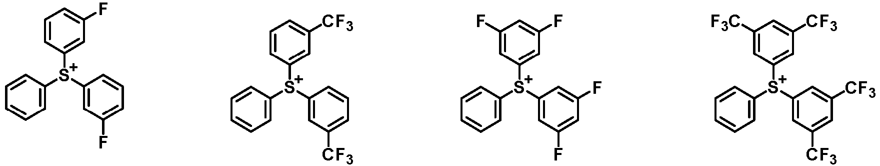

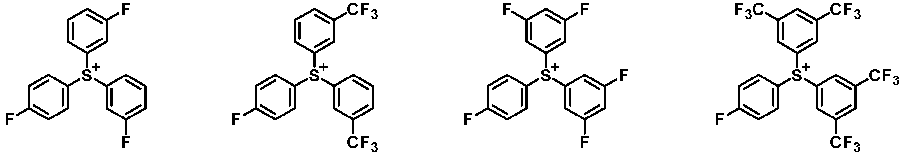

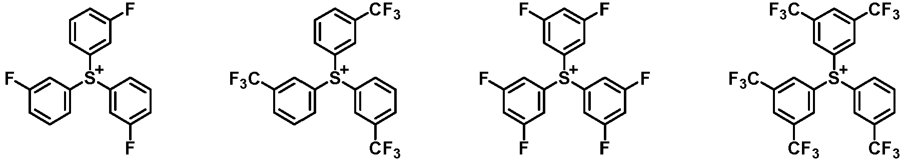

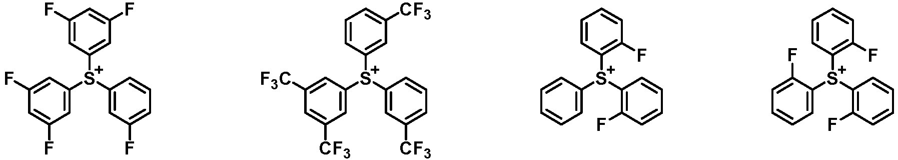

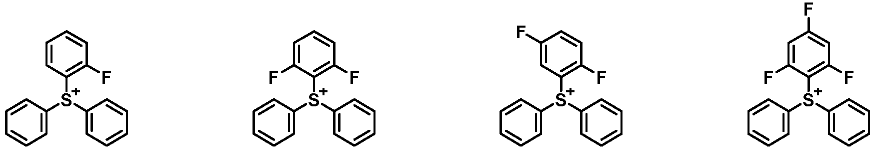

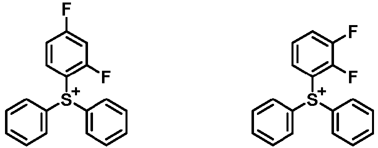

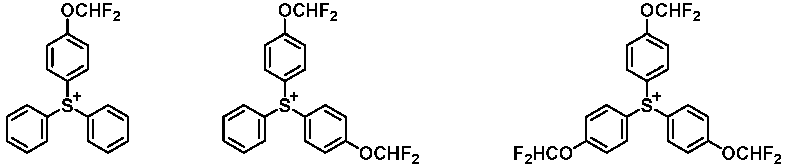

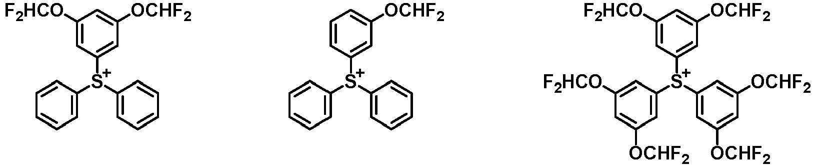

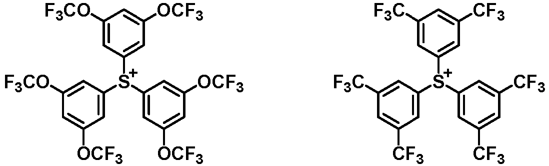

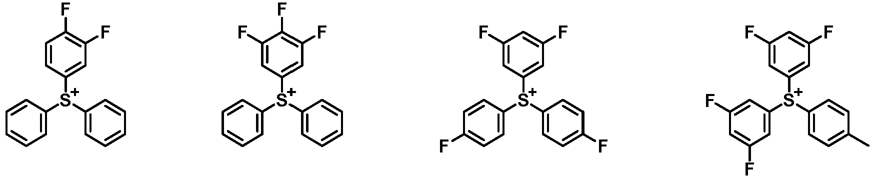

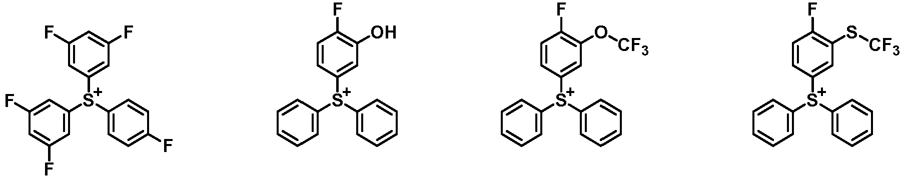

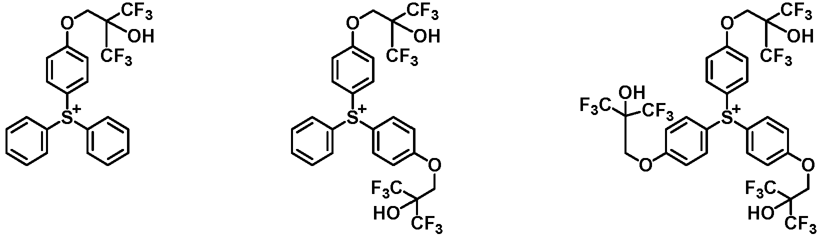

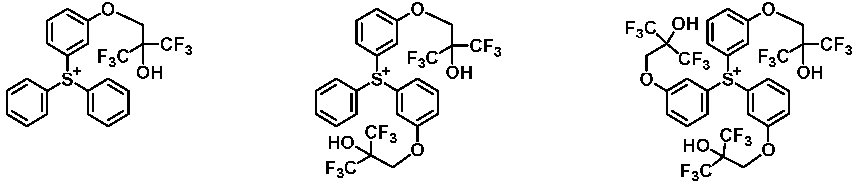

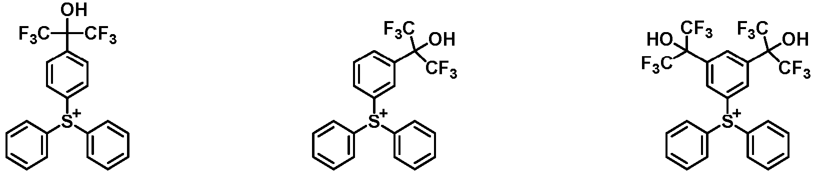

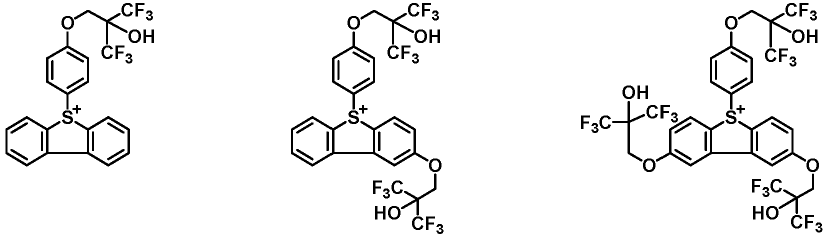

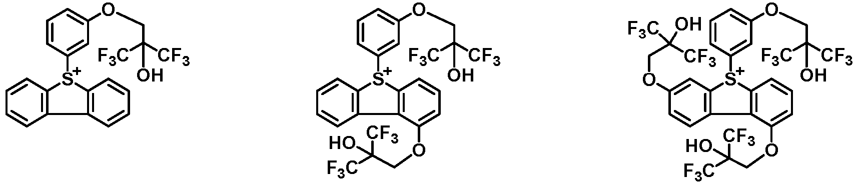

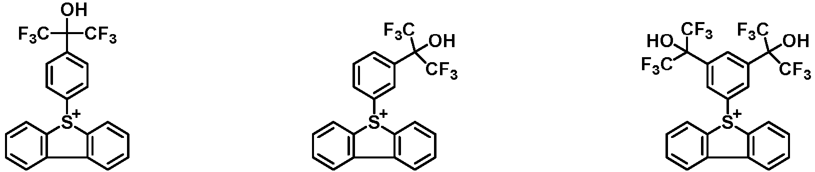

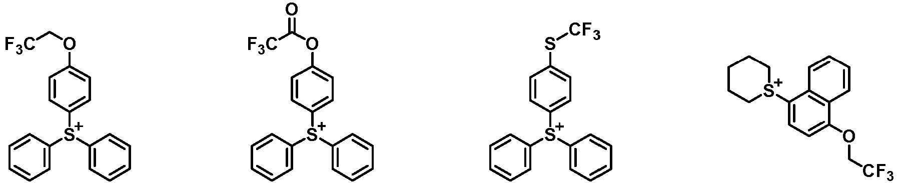

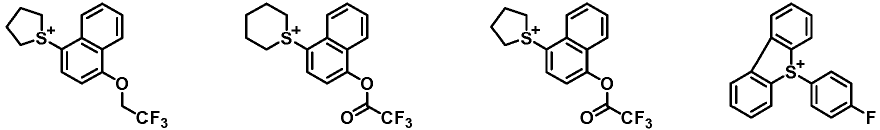

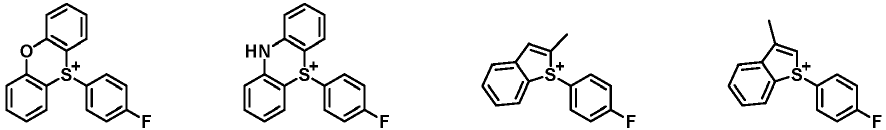

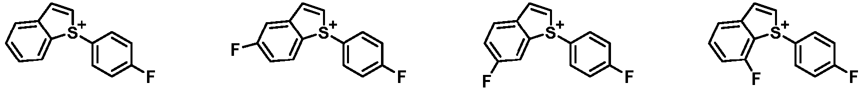

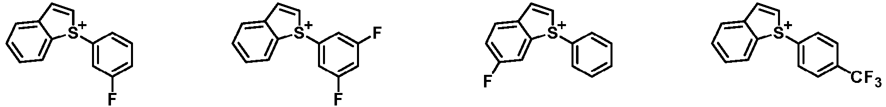

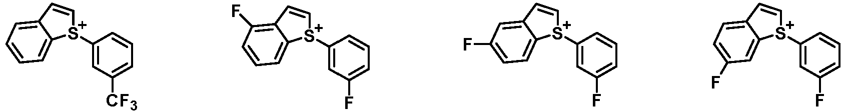

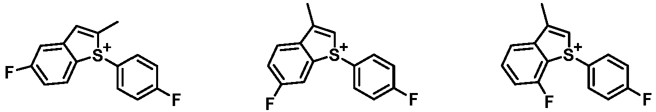

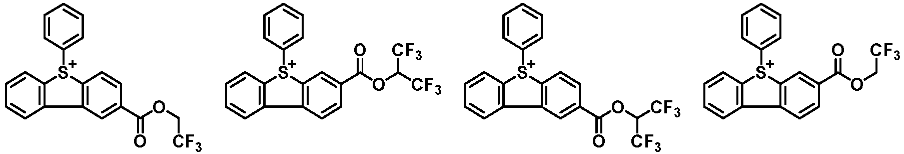

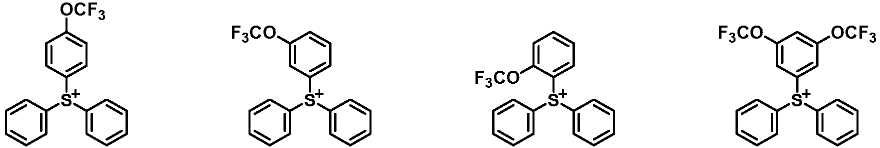

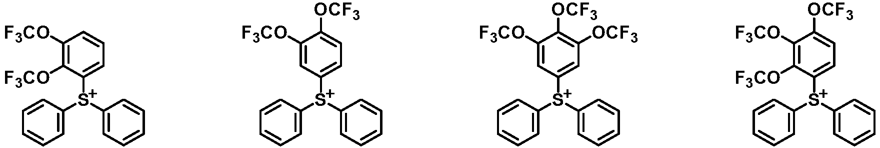

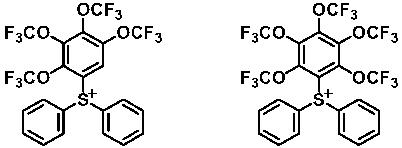

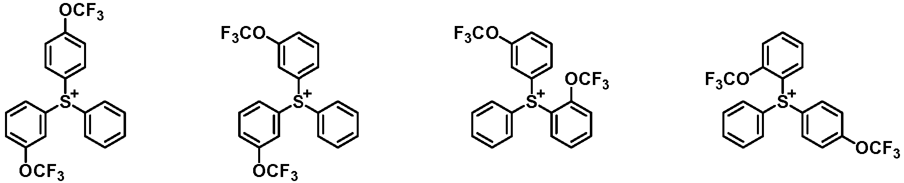

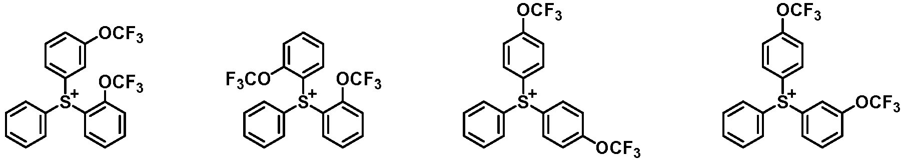

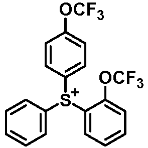

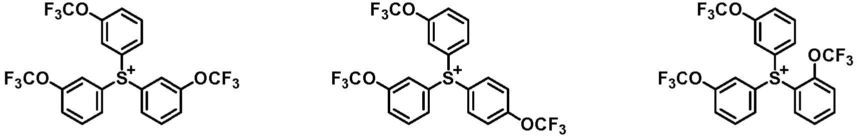

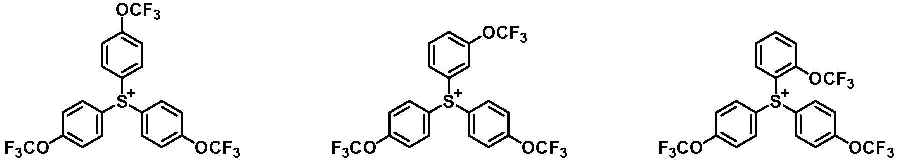

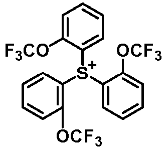

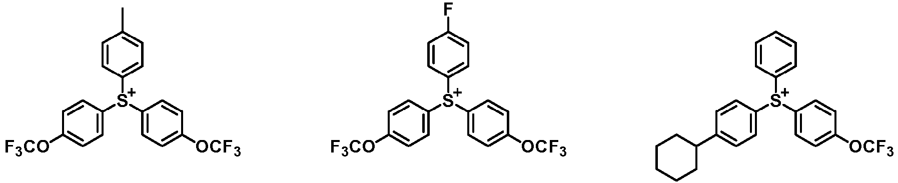

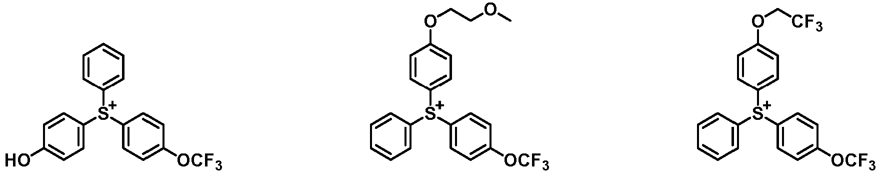

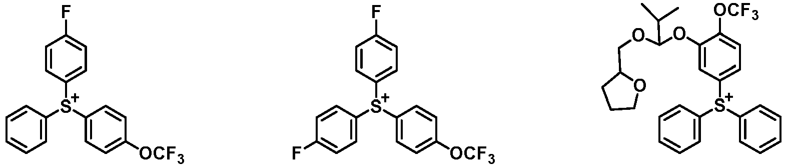

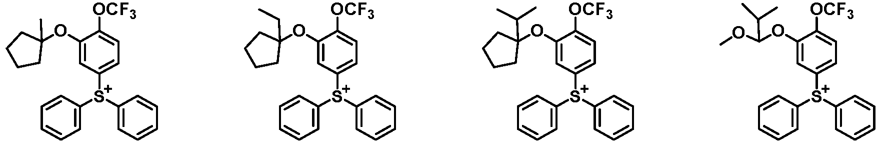

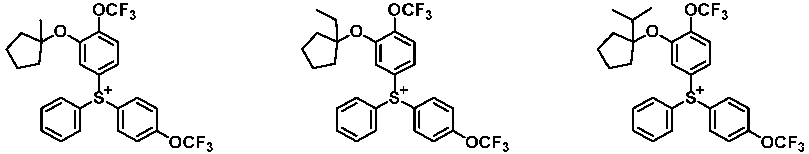

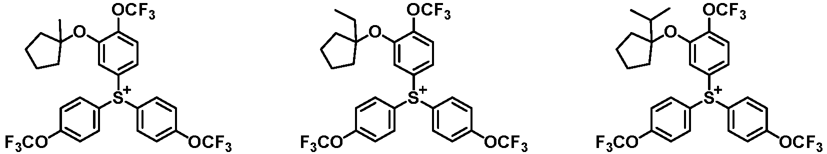

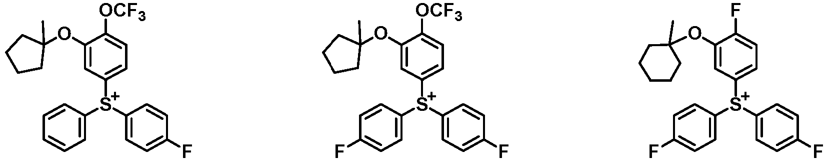

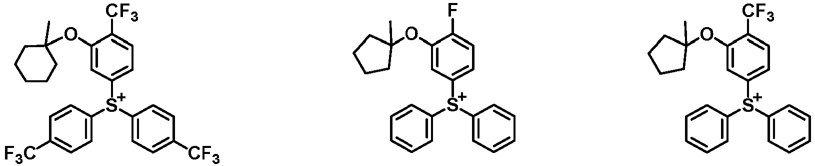

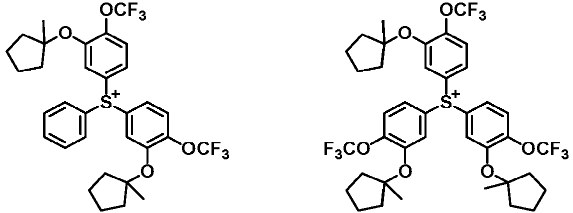

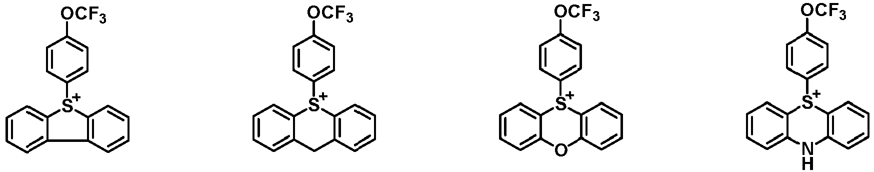

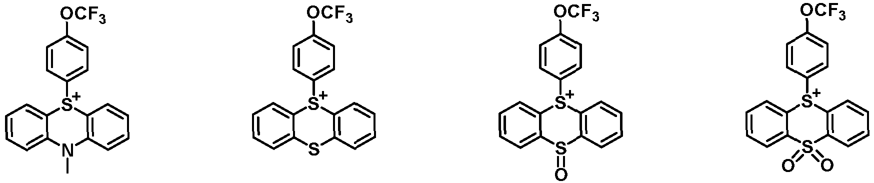

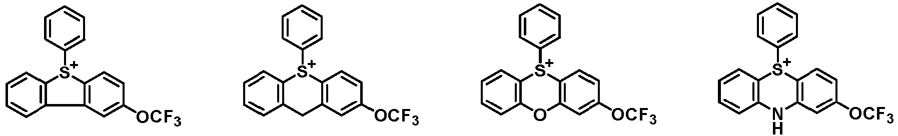

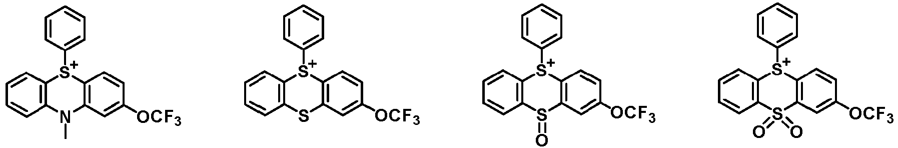

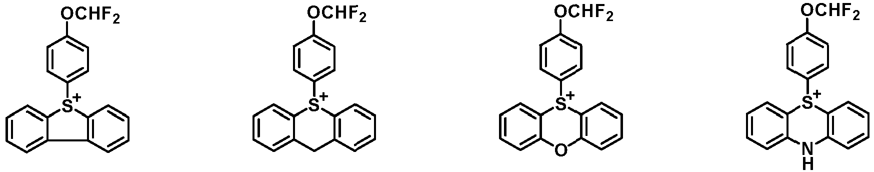

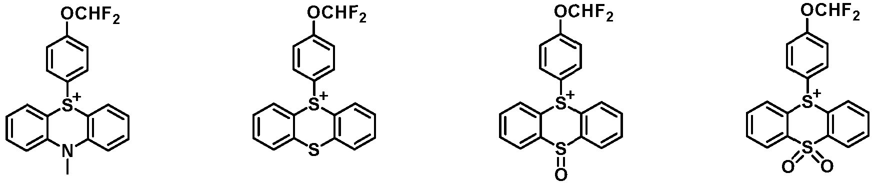

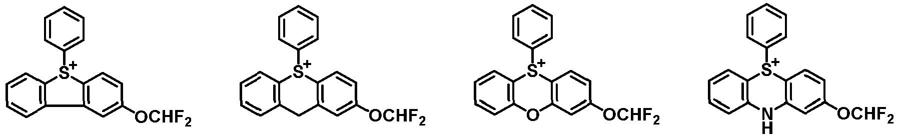

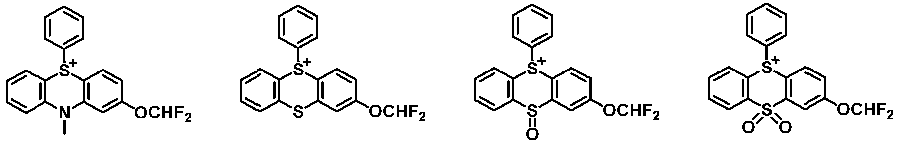

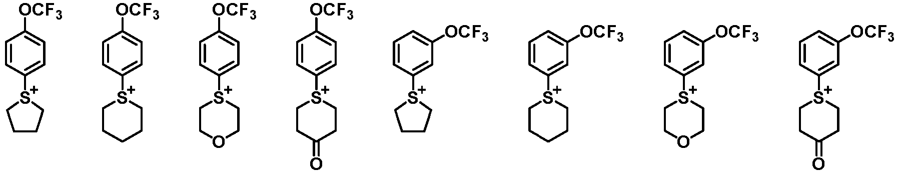

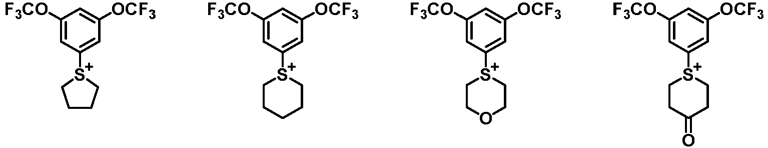

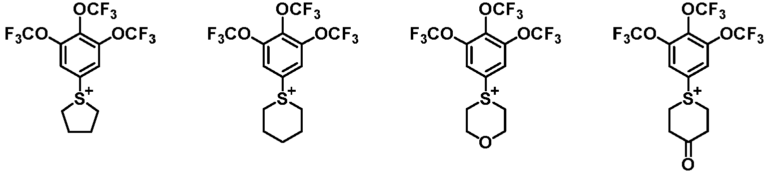

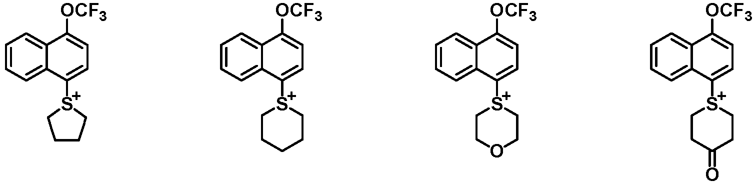

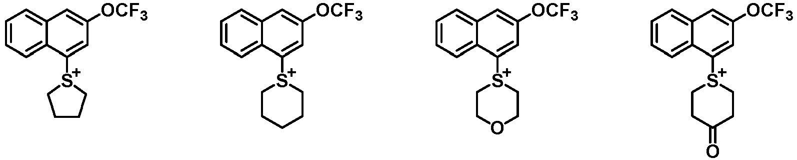

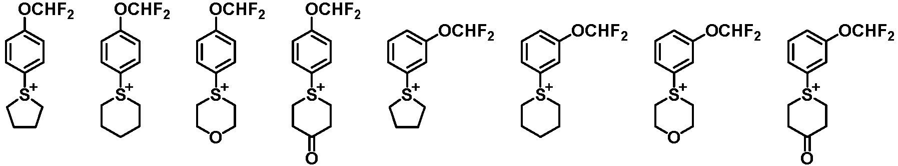

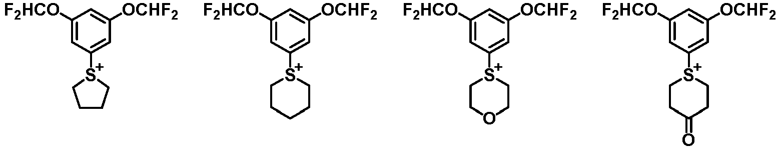

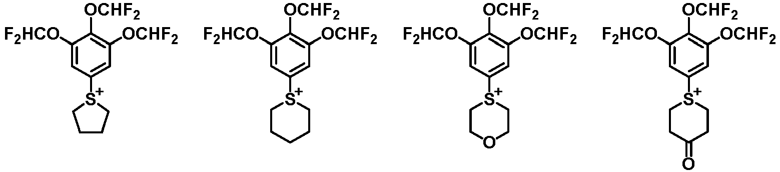

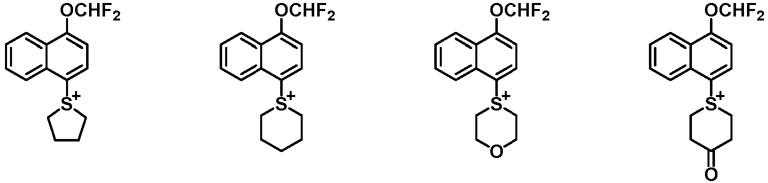

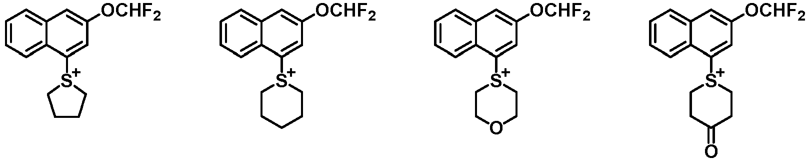

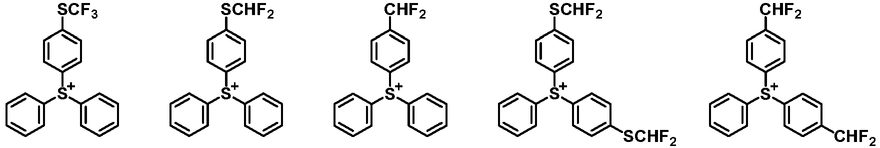

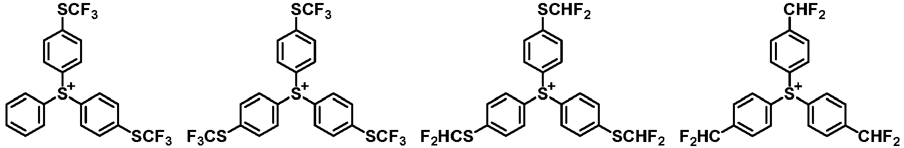

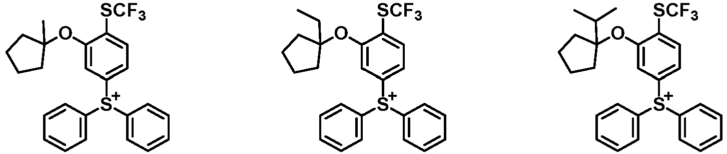

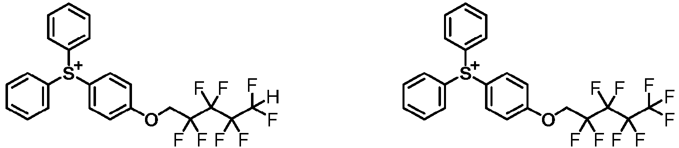

The sulfonium salt monomer having formula (A1) may be synthesized, for example, by the same method as the synthesis of the sulfonium salt having a polymerizable anion described in USP 8,057,985 (JP 5201363). The synthesis method is not limited thereto.

### Polymer

Another embodiment of the invention is a polymer comprising repeat units derived from the sulfonium salt monomer having formula (A1), which are also referred to as repeat units A1, and adapted to increase its solubility in alkaline aqueous solution under the action of acid.

In a chemically amplified resist composition, the polymer is a polymer-bound photoacid generator which functions as both a photoacid generator and a base polymer. The sulfonium salt monomer is structurally characterized by having an aromatic vinyl structure as a polymerizable group and an aromatic sulfonic acid anion. The aromatic ring directly bonded to the backbone makes the polymer backbone rigid so that the polymer may have a higher glass transition temperature (Tg). Owing to the interaction of aromatic rings within or between the polymer (π-π stacking effect), the polymer is arranged in order. This ensures that when a resist film is developed in a developer to form a small-size pattern, the resist pattern is resistant to collapse. Also, in the etching step after the small-size pattern formation, the aromatic ring directly bonded to the backbone contributes to better etching resistance. The aromatic sulfonic acid site of the generated acid has a more rigid structure than prior art partially fluorinated alkanesulfonic acid anions, which is effective for restraining excessive acid diffusion. Further, the inclusion of fluorine in the sulfonium cation structure insures organic solvent solubility. Due to the synergy of these effects, the inventive polymer enables to form patterns having collapse resistance, reduced LER and etch resistance. The inventive polymer is thus useful as one component of a chemically amplified positive resist composition.

The content of repeat units A1 is preferably 1 to 30 mol%, more preferably 2 to 15 mol%, even more preferably 3 to 10 mol% of the overall repeat units of the polymer. A content in excess of 30 mol% is undesirable in that organic solvent solubility becomes poor, and synthesis of the desired polymer is prohibited, or even when a polymer can be synthesized, a resist composition comprising the polymer suffers from possible shortcomings of precipitation, coating defects, and development defects. The repeat units A1 may be used alone or in admixture of two or more types.

The polymer may further comprise repeat units having the formula (A2), which are also referred to as repeat units A2.

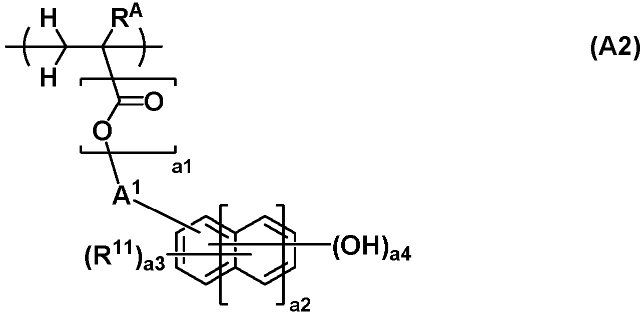

In formula (A2), a1 is 0 or 1. The subscript a2 is an integer of 0 to 2. The relevant structure is a benzene ring when a2=0, a naphthalene ring when a2=1, and an anthracene ring when a2=2. The subscript a3 is an integer meeting 0 ≤ a3 ≤ 5+2(a2)-a4. The subscript a4 is an integer of 1 to 3. When a2=0, preferably a3 is 0, 1, 2 or 3, and a4 is 1, 2 or 3. When a2=1 or 2, preferably a3 is 0, 1, 2, 3 or 4, and a4 is 1, 2 or 3.

In formula (A2), R^{A} is hydrogen, fluorine, methyl or trifluoromethyl.

In formula (A2), R¹¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group. The saturated hydrocarbyl group and saturated hydrocarbyl moiety in the saturated hydrocarbyloxy group and saturated hydrocarbylcarbonyloxy group may be straight, branched or cyclic. Examples thereof include alkyl groups such as methyl, ethyl, n-propyl, isopropyl, butyl, pentyl, hexyl and structural isomers thereof; cycloalkyl groups such as cyclopropyl, cyclobutyl, cyclopentyl, and cyclohexyl; and combinations thereof. A carbon count within the upper limit ensures good solubility in alkaline developer. When a3 is 2 or more, a plurality of R¹¹ may be identical or different.

In formula (A2), A¹ is a single bond or C₁-C₁₀ saturated hydrocarbylene group in which some -CH₂- may be replaced by -O-. The saturated hydrocarbylene group may be straight, branched or cyclic. Examples of the hydrocarbylene group include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of a1=1 in formula (A2), the ether bond may be incorporated at any position excluding the position between the α- and β-carbons relative to the ester oxygen. In case of a1=0, the atom bonding to the backbone becomes an ether oxygen atom, and a second ether bond may be incorporated at any position excluding the position between the α- and β-carbons relative to the ether oxygen. Saturated hydrocarbylene groups having no more than 10 carbon atoms are desirable because of a sufficient solubility in alkaline developer.

Preferred examples of the repeat units A2 wherein a1=0 and A¹ is a single bond (meaning that the aromatic ring is directly bonded to the backbone of the polymer), that is, repeat units free of a linker: -C(=O)-O-A¹- include units derived from 3-hydroxystyrene, 4-hydroxystyrene, 5-hydroxy-2-vinylnaphthalene, and 6-hydroxy-2-vinylnaphthalene. Repeat units having the formula (A2-1) are especially preferred. 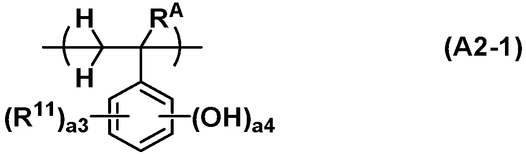 Herein R^{A}, R¹¹, a3 and a4 are as defined above.

Examples of the repeat units A2 wherein a1=0 and A¹ is a single bond, that is, repeat units free of a linker: -C(=O)-O-A¹- are shown below, but not limited thereto. Herein R^{A} is as defined above.

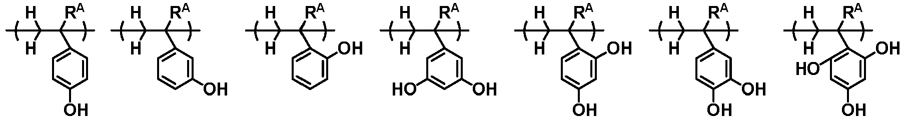

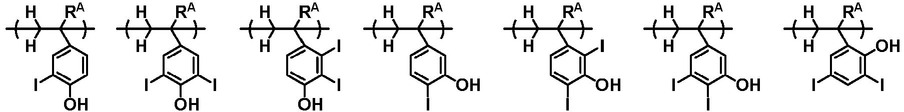

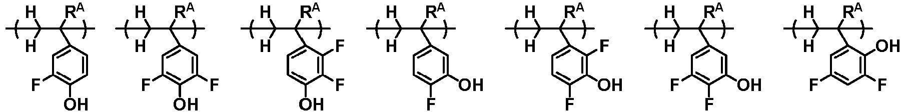

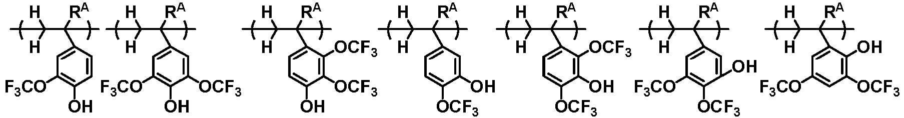

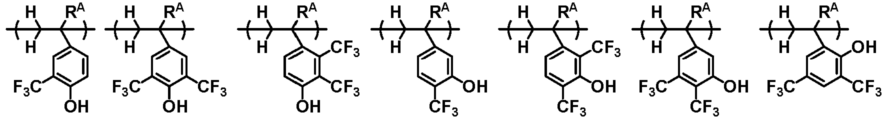

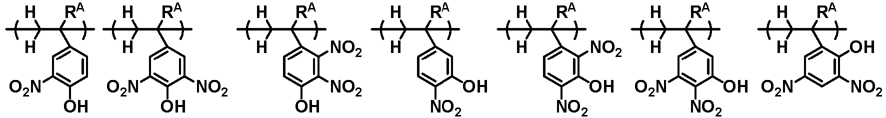

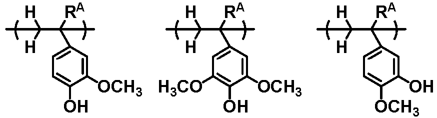

Preferred examples of the repeat units A2 wherein a1=1, that is, having a linker: -C(=O)-O-A¹- are shown below, but not limited thereto. Herein R^{A} is as defined above.

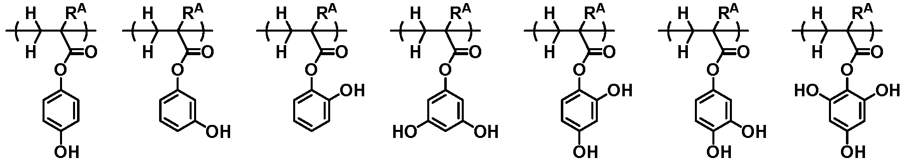

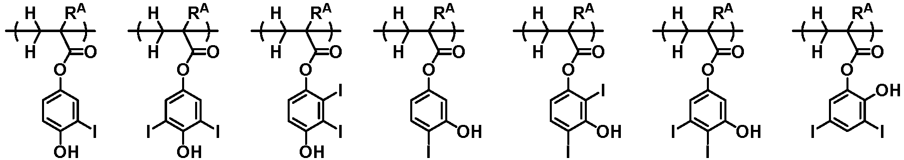

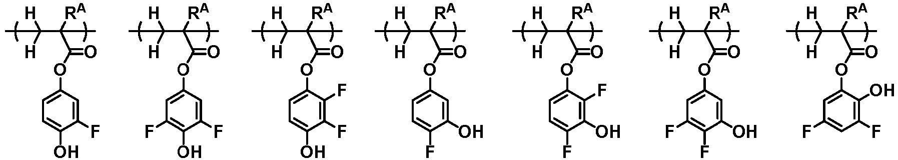

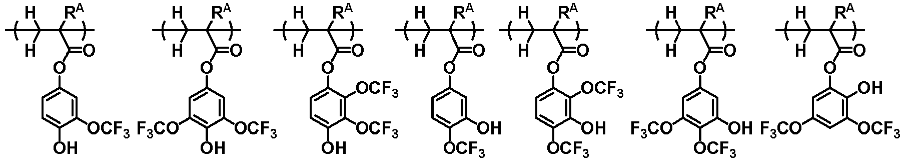

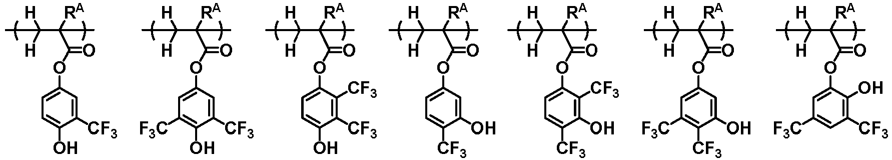

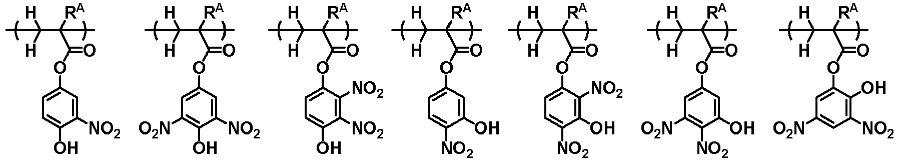

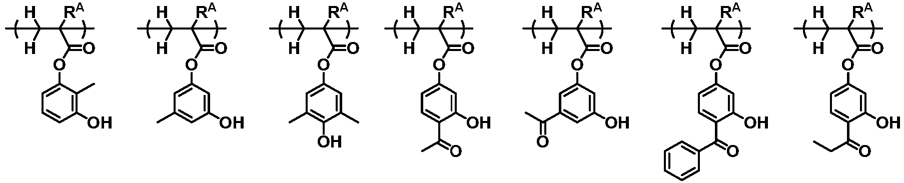

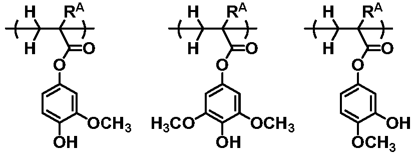

The content of repeat units A2 is preferably 10 to 95 mol%, more preferably 50 to 90 mol%, even more preferably 55 to 85 mol% of the overall repeat units of the polymer. When the polymer further contains repeat units having formula (A4) and/or repeat units having formula (A5), which provide the polymer with higher etch resistance, the repeat units having a phenolic hydroxy group as a substituent, the total content of repeat units A2 and repeat units A4 and/or A5 is preferably in the range. The repeat units A2 may be used alone or in admixture of two or more.

In a preferred embodiment, the polymer further contains repeat units A3 having an acidic functional group protected with an acid labile group (i.e., repeat units protected with an acid labile group and adapted to turn alkali soluble under the action of acid) so that the positive resist composition in exposed areas may be dissolved in alkaline developer. In this embodiment wherein the acid labile group (or protective group) in the repeat unit undergoes deprotection reaction under the action of acid, the polymer turns more soluble in alkaline developer.

Typical of the repeat unit A3 is a unit having the formula (A3-1), also referred to as repeat unit A3-1.

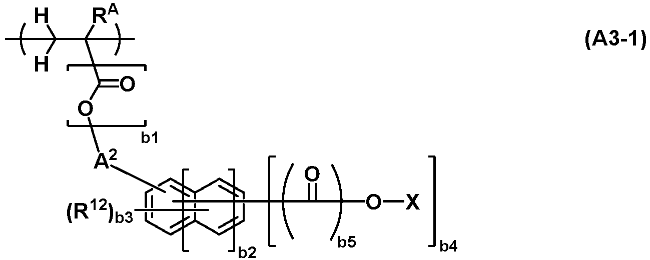

In formula (A3-1), b1 is 0 or 1. The subscript b2 is an integer of 0 to 2. The structure represents a benzene skeleton when b2=0, a naphthalene skeleton when b2=1, and an anthracene skeleton when b2=2. The subscript b3 is an integer meeting 0 ≤ b3 ≤ 5+2(b2)-b4. The subscript b4 is an integer of 1 to 3, and b5 is 0 or 1. When b2=0, preferably b3 is 0, 1, 2 or 3 and b4 is 1, 2 or 3. When b2=1 or 2, preferably b3 is 0, 1, 2, 3 or 4 and b4 is 1, 2 or 3.

In formula (A3-1), R^{A} is hydrogen, fluorine, methyl or trifluoromethyl.

In formula (A3-1), R¹² is nitro, cyano, halogen, an optionally halogenated C₁-C₆ saturated hydrocarbyl group, optionally halogenated C₁-C₆ saturated hydrocarbyloxy group, optionally halogenated C₁-C₆ saturated hydrocarbylthio group, C₂-C₈ saturated hydrocarbylcarbonyl group or optionally halogenated C₂-C₈ saturated hydrocarbylcarbonyloxy group. Suitable halogen atoms include fluorine, chlorine, bromine and iodine. The saturated hydrocarbyl group and saturated hydrocarbyl moiety in the saturated hydrocarbyloxy group and saturated hydrocarbylcarbonyloxy group may be straight, branched or cyclic, and examples thereof include alkyl groups such as methyl, ethyl, n-propyl, isopropyl, butyl, pentyl, hexyl, and structural isomers thereof, cycloalkyl groups such as cyclopropyl, cyclobutyl, cyclopentyl and cyclohexyl, and combinations thereof. Examples of the halogenated C₁-C₆ saturated hydrocarbyl group include trifluoromethyl and difluoromethyl. Examples of the halogenated C₁-C₆ saturated hydrocarbyloxy group include trifluoromethoxy and difluoromethoxy. Examples of the halogenated C₁-C₆ saturated hydrocarbylthio group include trifluoromethylthio and difluoromethylthio. A carbon count within the upper limit ensures good solubility in alkaline developer. A plurality of R¹² may be identical or different when b3 is 2 or more.

In formula (A3-1), A² is a single bond or C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of b1=1 in formula (A3-1), the ether bond may be incorporated at any position excluding the position between the α-carbon and β-carbon relative to the ester oxygen. In case of b1=0, the atom that bonds with the backbone becomes an ethereal oxygen, and a second ether bond may be incorporated at any position excluding the position between the α-carbon and β-carbon relative to that ethereal oxygen. Saturated hydrocarbylene groups having no more than 10 carbon atoms are desirable because of a sufficient solubility in alkaline developer.

In formula (A3-1), X is an acid labile group when b4=1, and hydrogen or an acid labile group, at least one X being an acid labile group, when b4=2 or 3. That is, repeat units A3-1 have phenolic hydroxy groups bonded to an aromatic ring, at least one of which is protected with an acid labile group, or repeat units A3-1 have a carboxy group bonded to an aromatic ring, which is protected with an acid labile group. The acid labile group used herein is not particularly limited as long as it is commonly used in a number of well-known chemically amplified resist compositions and eliminated under the action of acid to provide an acidic group.

Preferred as the acid labile group X is a group having the formula (X-1).

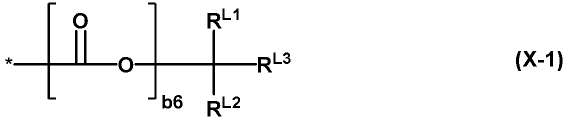

The asterisk (*) designates a point of attachment to the adjacent -O-.

In formula (X-₁), R^{L1}, R^{L2} and R^{L3} are each independently a C₁-C₁₀ hydrocarbyl group. Any two of R^{L1}, R^{L2} and R^{L3} may bond together to form a ring with the carbon atom to which they are attached. Examples of the ring include cyclopropane, cyclobutane, cyclopentane, cyclohexane, norbornane and adamantane rings. The subscript b6 is 0 or 1, preferably 0.

Examples of the group having formula (X-1) are shown below, but not limited thereto. The asterisk (*) designates a point of attachment to the adjacent -O-.

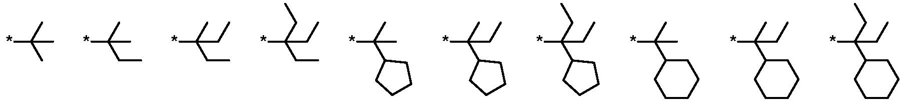

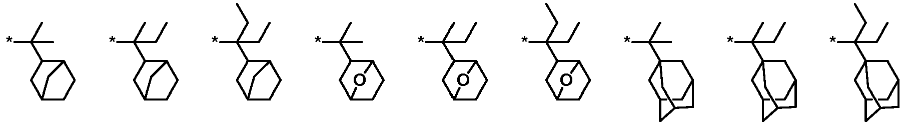

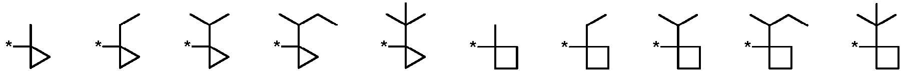

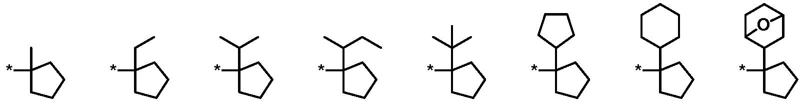

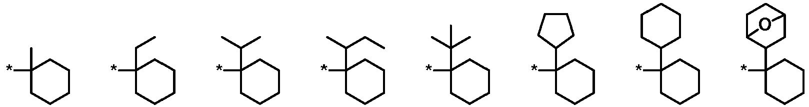

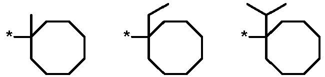

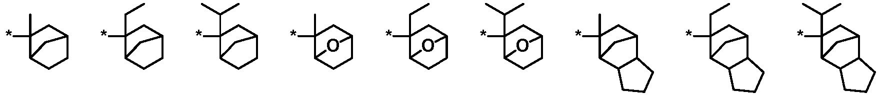

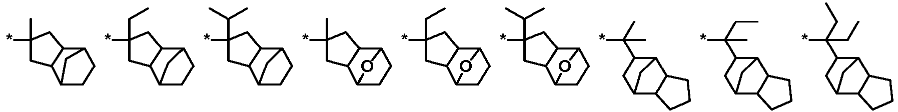

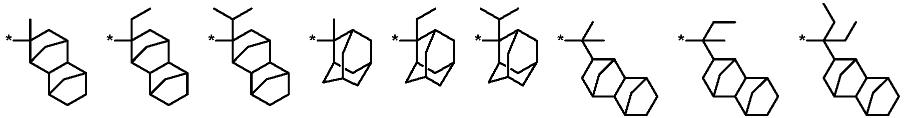

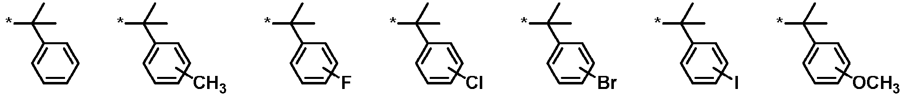

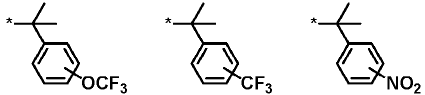

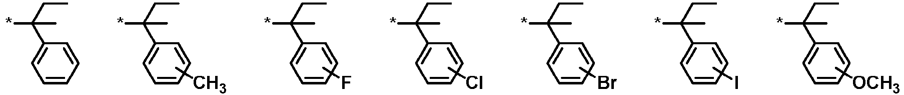

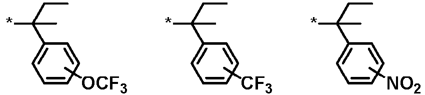

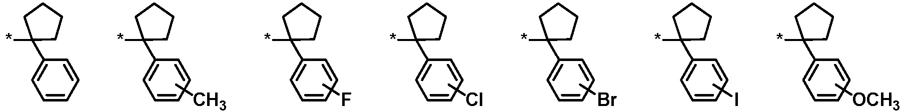

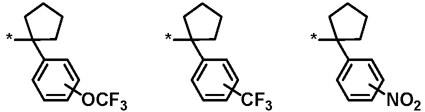

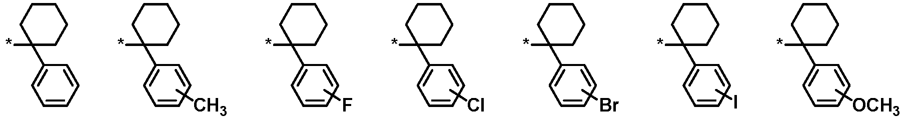

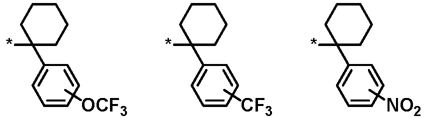

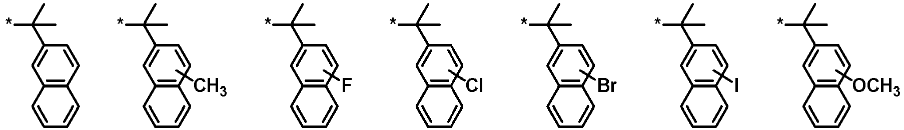

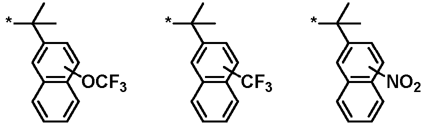

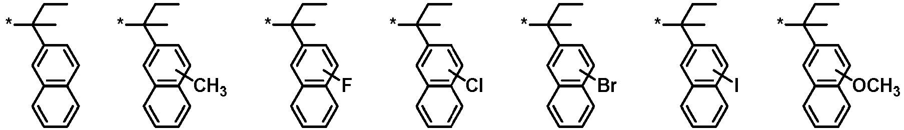

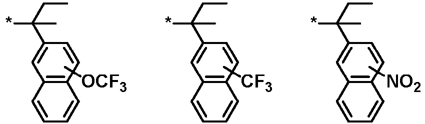

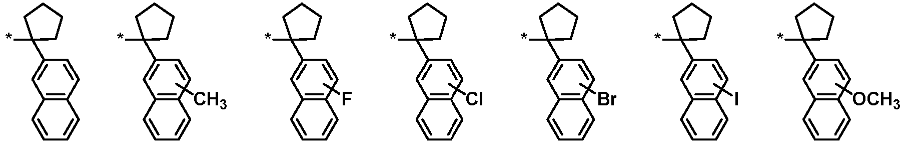

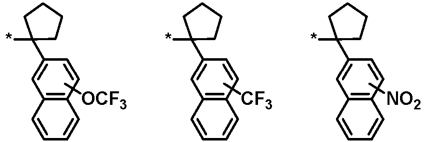

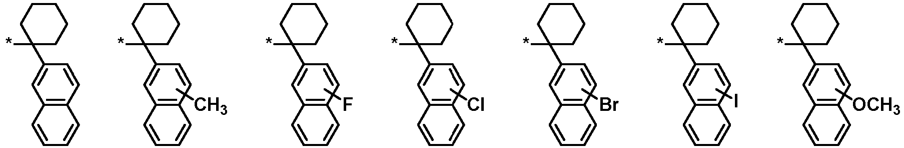

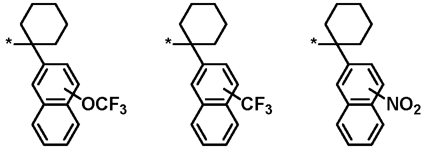

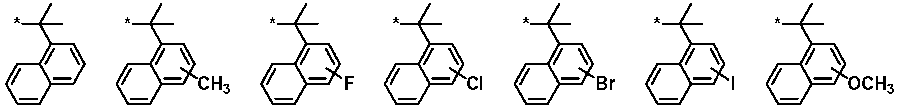

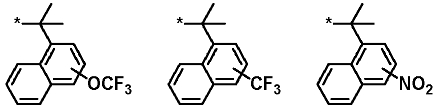

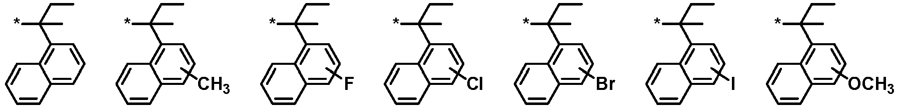

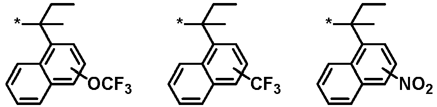

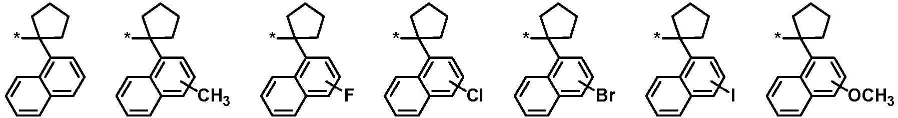

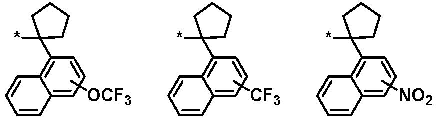

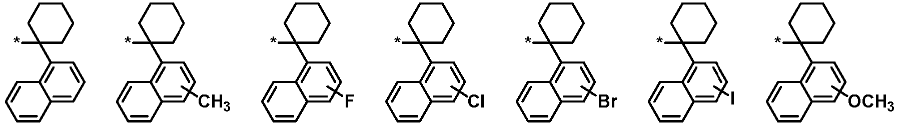

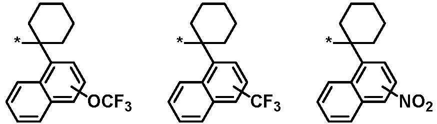

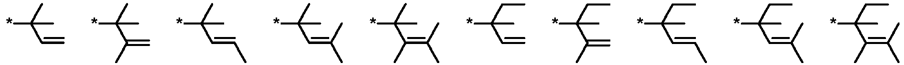

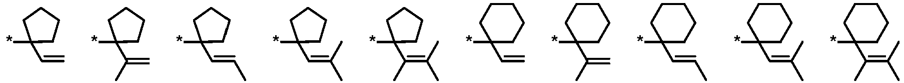

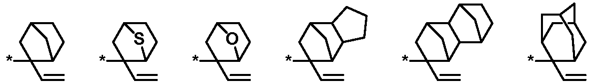

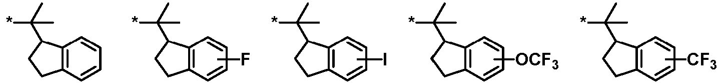

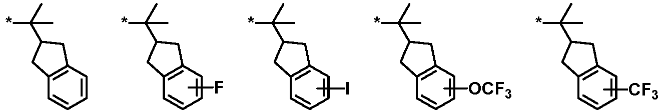

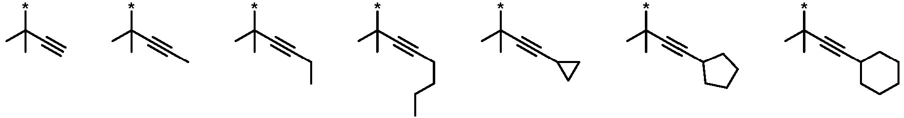

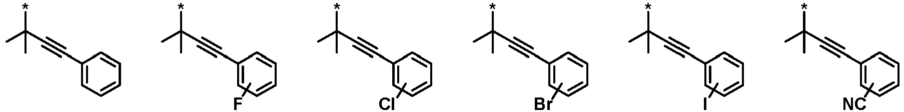

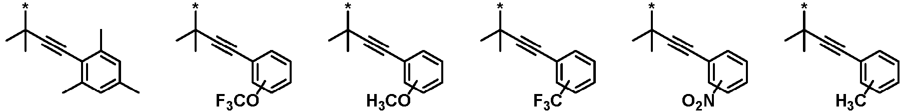

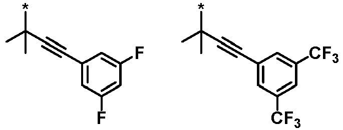

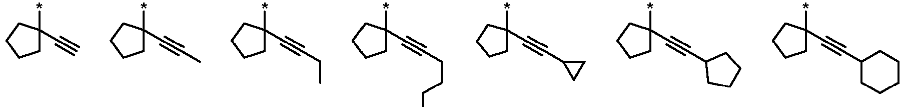

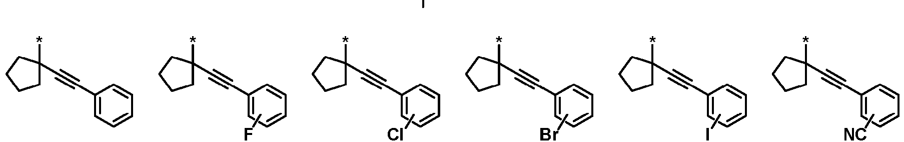

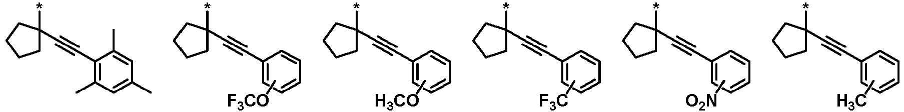

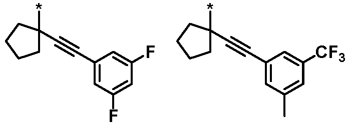

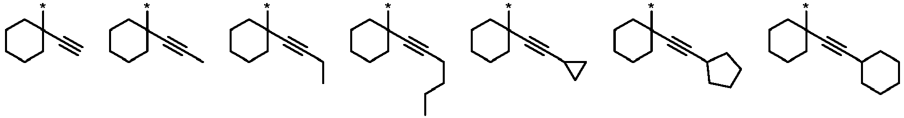

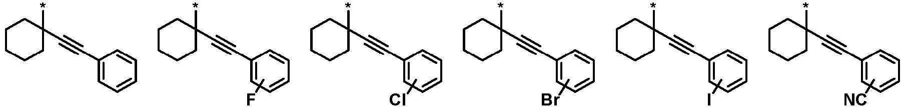

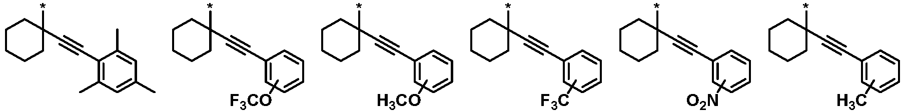

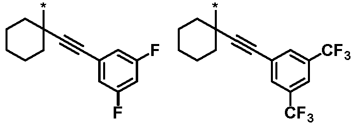

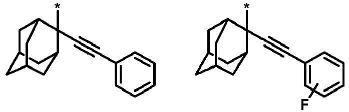

A choice of a tertiary saturated hydrocarbyl group as the acid labile group is preferred for the reason that even when a resist film is formed to a thickness of 10 to 100 nm and processed to form a fine pattern having a line width of up to 45 nm, the pattern has reduced LER. Of the tertiary saturated hydrocarbyl groups, tertiary alkyl groups of 4 to 18 carbon atoms are preferred because the corresponding monomer for use in polymerization is available through distillation. The group attached to the tertiary carbon atom in the tertiary saturated hydrocarbyl group is typically a C₁-C₁₅ saturated hydrocarbyl group which may contain an oxygen-containing functional group such as ether bond or carbonyl group. The groups attached to the tertiary carbon atom may bond together to form a ring.

A group having the following formula (X-2) is also suitable as the acid labile group. The group having formula (X-2) is often used as the acid labile group. It is a good choice of the acid labile group that ensures to form a pattern having a relatively rectangular pattern-substrate interface in a consistent manner. An acetal structure is formed when X is a group having formula (X-2).

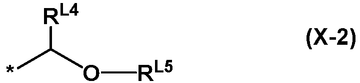

In formula (X-2), R^{L4} is hydrogen or a C₁-C₁₀ saturated hydrocarbyl group. R^{L5} is a C₁-C₃₀ saturated hydrocarbyl group. The saturated hydrocarbyl group may be straight, branched or cyclic. The asterisk (*) designates a point of attachment to the adjacent -O-.

A choice of R^{L4} may depend on the designed sensitivity of decomposable group to acid. For example, hydrogen is selected when the acid decomposable group is designed to ensure relatively high stability and to be decomposed with strong acid. A straight alkyl group is selected when the acid decomposable group is designed to have relatively high reactivity and high sensitivity to pH changes. Although the choice varies with a particular combination of acid generator and basic compound in the resist composition, R^{L4} is preferably a group in which the carbon in bond with acetal carbon is secondary, when R^{L5} is a relatively large alkyl group substituted at the end and the acid decomposable group is designed to undergo a substantial change of solubility by decomposition. Preferred examples of R^{L4} include methyl, ethyl, isopropyl, sec-butyl, tert-butyl, cyclopentyl, cyclohexyl, phenyl, 2,6-difluorophenyl, 4-fluorophenyl, 4-chlorophenyl, 4-bromophenyl, 4-iodophenyl, 4-trifluoromethylphenyl, 4-trifluoromethoxyphenyl, 4-methylphenyl, 4-methoxyphenyl, adamantyl and tricyclodecyl.

Examples of the group R^{L5} are shown below, but not limited thereto. The asterisk (*) designates a point of attachment to the adjacent -O-.

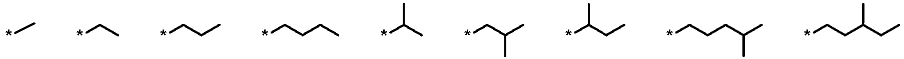

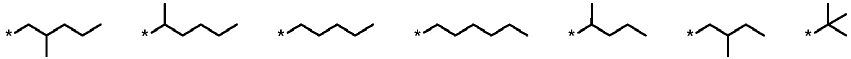

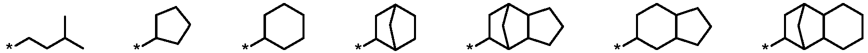

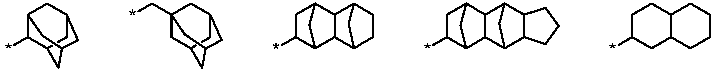

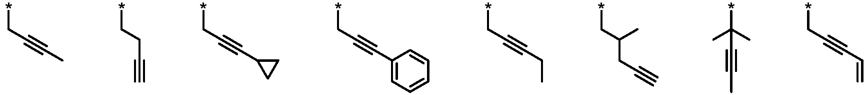

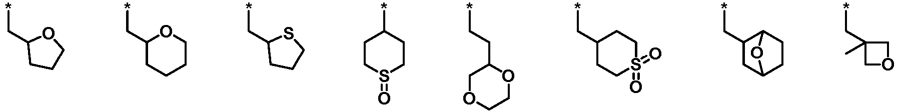

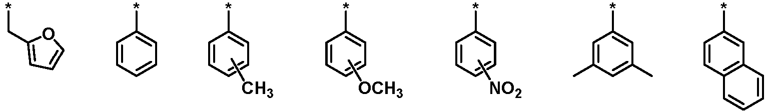

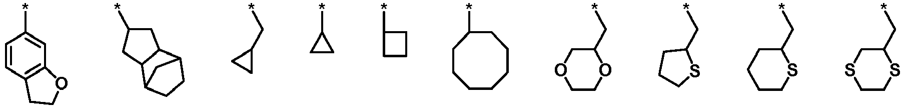

Of the repeat units A3-1, units having the formula (A3-1-1) are preferred. 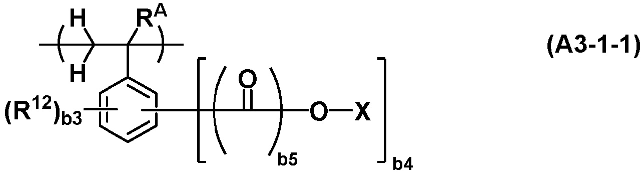 Herein, R^{A}, R¹², b3, b4, b5 and X are as defined above.

Another example of repeat unit A3 is a repeat unit having the following formula (A3-2), referred to as repeat unit A3-2. The repeat unit A3-2 which enables to increase the dissolution rate in the exposed region is a useful choice of the acid labile group-containing unit which affords satisfactory performance against line width variations during develop loading.

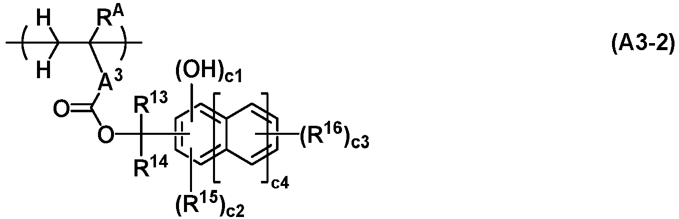

In formula (A3-2), c1 is an integer of 0 to 2, c2 is an integer of 0 to 2, c3 is an integer of 0 to 5, and c4 is an integer of 0 to 2.

In formula (A3-2), R^{A} is hydrogen, fluorine, methyl or trifluoromethyl.

In formula (A3-2), R¹³ and R¹⁴ are each independently a C₁-C₁₀ hydrocarbyl group which may contain a heteroatom. R¹³ and R¹⁴ may bond together to form a ring with the carbon atom to which they are attached.

In formula (A3-2), R¹⁵ is each independently fluorine, C₁-C₅ fluorinated alkyl group or C₁-C₅ fluorinated alkoxy group.

In formula (A3-2), R¹⁶ is each independently a C₁-C₁₀ hydrocarbyl group which may contain a heteroatom.

In formula (A3-2), A³ is a single bond, phenylene group, naphthylene group, or *-C(=O)-O-A³¹-. A³¹ is a C₁-C₂₀ aliphatic hydrocarbylene group which may contain hydroxy, ether bond, ester bond or lactone ring, or a phenylene or naphthylene group, and * is a point of attachment to the carbon atom in the backbone.

Preferred examples of the repeat unit A3-2 are shown below, but not limited thereto. Herein R^{A} is as defined above.

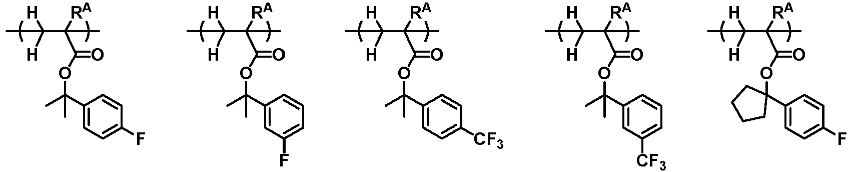

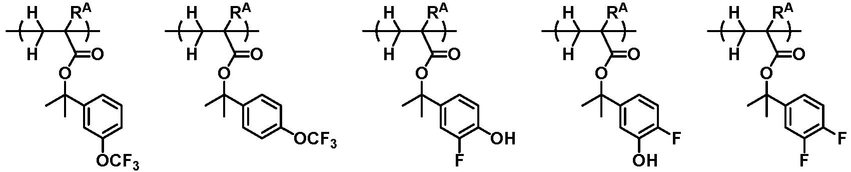

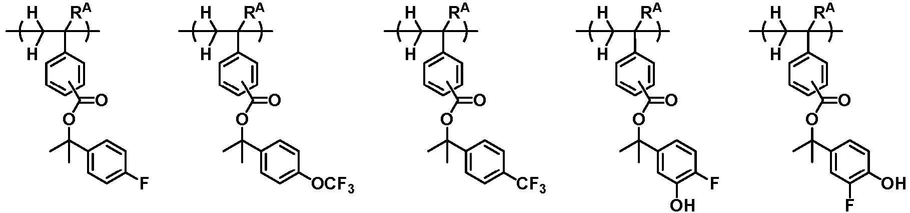

A further useful acid labile group is a phenolic hydroxy group whose hydrogen is substituted by -CH₂COO-(tertiary saturated hydrocarbyl group). The tertiary saturated hydrocarbyl group used herein is the same as the aforementioned tertiary saturated hydrocarbyl group used for the protection of phenolic hydroxy group.

The content of repeat units A3 is preferably 5 to 50 mol%, more preferably 10 to 30 mol% based on the overall repeat units of the polymer. Each of repeat units A3 may be of one type or a mixture of two or more types.

In a preferred embodiment, the polymer further comprises repeat units of at least one type selected from units having the formulae (A4), (A5) and (A6). These repeat units are simply referred to as repeat units A4, A5 and A6, respectively.

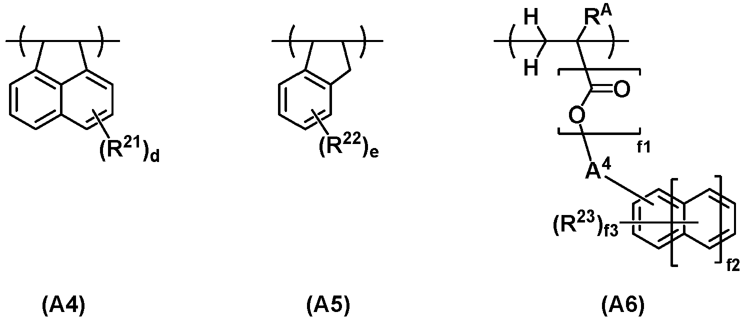

In formulae (A4) and (A5), d and e are each independently an integer of 0 to 4.

In formulae (A4) and (A5), R²¹ and R²² are each independently hydroxy, halogen, an optionally halogenated C₁-C₈ saturated hydrocarbyl group, optionally halogenated C₁-C₈ saturated hydrocarbyloxy group, or optionally halogenated C₂-C₈ saturated hydrocarbylcarbonyloxy group. The saturated hydrocarbyl group, saturated hydrocarbyloxy group and saturated hydrocarbylcarbonyloxy group may be straight, branched or cyclic. When d is 2 or more, a plurality of groups R²¹ may be identical or different. When e is 2 or more, a plurality of groups R²² may be identical or different.

In formula (A6), f1 is 0 or 1. The subscript f2 is an integer of 0 to 2. The corresponding structure represents a benzene skeleton when f2=0, a naphthalene skeleton when f2=1, and an anthracene skeleton when f2=2. The subscript f3 is an integer of 0 to 5. In case of f2=0, preferably f3 is 0, 1, 2 or 3. In case of f2=1 or 2, preferably f3 is 0, 1, 2, 3 or 4.

In formula (A6), R^{A} is hydrogen, fluorine, methyl or trifluoromethyl.

In formula (A6), R²³ is halogen, trifluoromethyl, trifluoromethoxy, nitro, cyano, a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, C₁-C₂₀ saturated hydrocarbylsulfinyl group, or C₁-C₂₀ saturated hydrocarbylsulfonyl group. The saturated hydrocarbyl group, saturated hydrocarbyloxy group, saturated hydrocarbylcarbonyloxy group, saturated hydrocarbyloxyhydrocarbyl group, saturated hydrocarbylthiohydrocarbyl group, saturated hydrocarbylsulfinyl group and saturated hydrocarbylsulfonyl group may be straight, branched or cyclic. When f3 is 2 or more, a plurality of groups R²³ may be identical or different.

R²³ is preferably selected from halogen atoms such as fluorine, chlorine, bromine and iodine; trifluoromethyl, trifluoromethoxy, nitro, cyano; saturated hydrocarbyl groups such as methyl, ethyl, propyl, butyl, tert-butyl, pentyl, hexyl, cyclopentyl, cyclohexyl, and structural isomers thereof; and saturated hydrocarbyloxy groups such as methoxy, ethoxy, propoxy, butoxy, tert-butoxy, pentyloxy, hexyloxy, cyclopentyloxy, cyclohexyloxy, and structural isomers of their hydrocarbon moiety.

The saturated hydrocarbylcarbonyloxy group may be readily introduced into a polymer even after polymerization, by a chemical modification method and is advantageously utilized for fine adjustment of the solubility of the polymer in alkaline developer. Examples of the saturated hydrocarbylcarbonyloxy group include methylcarbonyloxy, ethylcarbonyloxy, propylcarbonyloxy, butylcarbonyloxy, pentylcarbonyloxy, hexylcarbonyloxy, cyclopentylcarbonyloxy, cyclohexylcarbonyloxy, benzoyloxy, and structural isomers of their hydrocarbon moiety. As long as the carbon count is equal to or less than 20, an appropriate effect of controlling or adjusting (typically reducing) the solubility of the polymer in alkaline developer is obtainable, and the generation of scum or development defects may be suppressed.

Of the foregoing preferred substituent groups, such substituent groups as fluorine, chlorine, bromine, iodine, methyl, ethyl, methoxy, trifluoromethyl, trifluoromethoxy, nitro and cyano are useful because the corresponding monomers may be readily prepared.

In formula (A6), A⁴ is a single bond or C₁-C₁₀ saturated hydrocarbylene group in which some constituent -CH₂- may be replaced by -O-. The saturated hydrocarbylene group may be straight, branched or cyclic. Examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of f1=1 in formula (A6), the ether bond may be incorporated at any position excluding the position between the α- and β-carbons relative to the ester oxygen. In case of f1=0, the atom bonding to the backbone becomes an ether oxygen atom, and a second ether bond may be incorporated at any position excluding the position between the α- and β-carbons relative to the ether oxygen. Saturated hydrocarbylene groups having no more than 10 carbon atoms are desirable because of a sufficient solubility in alkaline developer.

Preferred examples of the repeat units A6 wherein f1 is 0 and A⁴ is a single bond (meaning that the aromatic ring is directly bonded to the backbone of the polymer), that is, repeat units free of the linker: -C(=O)-O-A⁴- include units derived from styrene, 4-chlorostyrene, 4-bromostyrene, 4-methylstyrene, 4-methoxystyrene, 4-acetoxystyrene, 2-hydroxypropylstyrene, 2-vinylnaphthalene, and 3-vinylnaphthalene.

Preferred examples of the repeat units A6 wherein f1 is 1, that is, having the linker: -C(=O)-O-A⁴- are shown below, but not limited thereto. R^{A} is as defined above.

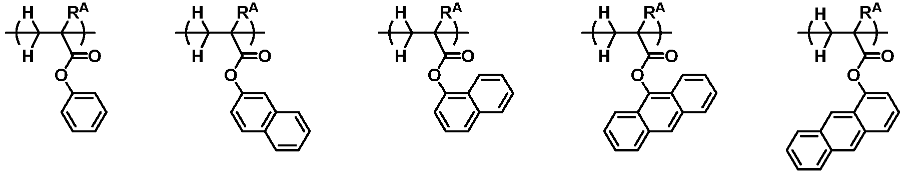

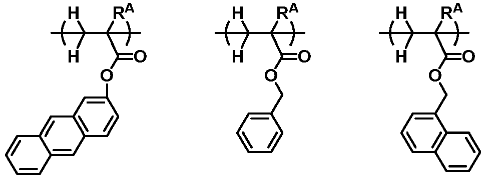

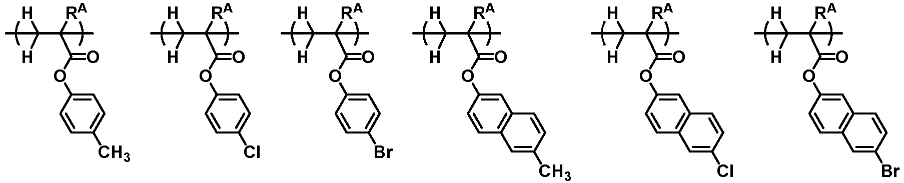

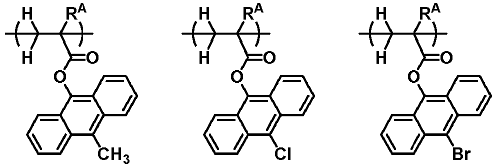

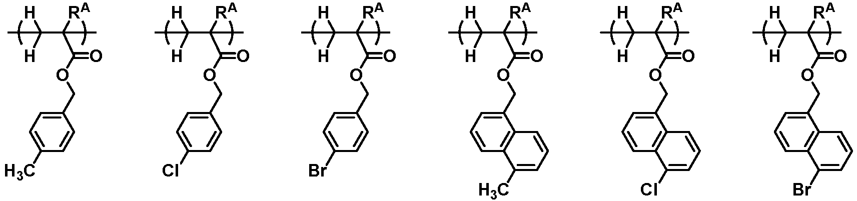

When repeat units of at least one type selected from repeat units A4 to A6 are incorporated, better performance is obtained because not only the aromatic ring possesses etch resistance, but the cyclic structure incorporated into the backbone also exerts the effect of improving etch resistance and resistance to EB irradiation during pattern inspection step.

The content of repeat units A4 to A6 is preferably at least 5 mol% based on the overall repeat units of the polymer for obtaining the effect of improving etch resistance. Also, the content of repeat units A4 to A6 is preferably up to 35 mol%, more preferably up to 30 mol% based on the overall repeat units of the polymer. When the relevant units are free of functional groups or have a functional group other than hydroxy, their content of up to 35 mol% is preferred because the risk of forming development defects is eliminated. Each of the repeat units A4 to A6 may be of one type or a combination of plural types.

It is preferred that the polymer comprise repeat units A1, repeat units A2, repeat units A3-1 and/or A3-2, and repeat units of at least one type selected from repeat units A4 to A6, because both etch resistance and high resolution are achievable. The total content of these repeat units is preferably at least 60 mol%, more preferably at least 80 mol%, even more preferably 100 mol% based on the overall repeat units of the polymer.

From the aspect of etch resistance, repeat units having an aromatic ring structure account for preferably at least 60 mol%, more preferably at least 80 mol% of the overall repeat units of the polymer. Most preferably, all repeat units have an aromatic ring structure.

The polymer may further comprise (meth)acrylate units protected with an acid labile group or (meth)acrylate units having an adhesive group such as lactone structure or hydroxy group other than phenolic hydroxy as commonly used in the art. These repeat units are effective for fine adjustment of properties of a resist film, but not essential.

Examples of the (meth)acrylate unit having an adhesive group include repeat units having the following formulae (A7) to (A9), which are also referred to as repeat units A7 to A9. While these units do not exhibit acidity, they may be used as auxiliary units for providing adhesion to substrates or adjusting solubility.

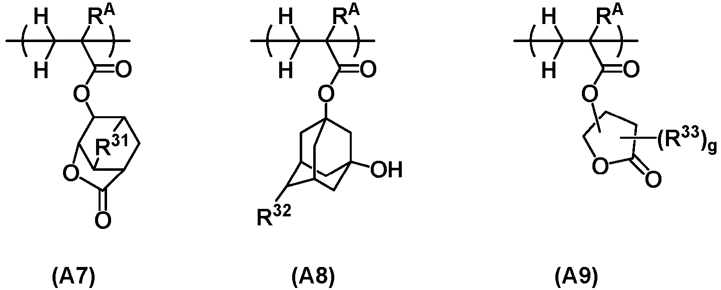

In formulae (A7) to (A9), R^{A} is each independently hydrogen, fluorine, methyl or trifluoromethyl. R³¹ is -O- or methylene. R³² is hydrogen or hydroxy. R³³ is a C₁-C₄ saturated hydrocarbyl group, and g is an integer of 0 to 3.

When repeat units A7 to A9 are included, their content is preferably 0 to 30 mol%, more preferably 0 to 20 mol% based on the overall repeat units of the polymer. Each of repeat units A7 to A9 may be of one type or a combination of plural types.

The polymer should preferably have a Mw of 1,000 to 50,000, and more preferably 2,000 to 20,000. A Mw of at least 1,000 eliminates the risk that pattern features are rounded at their top, inviting degradations of resolution, LER and CDU. A Mw of up to 50,000 eliminates the risk that LER and CDU are degraded when a pattern with a line width of up to 100 nm is formed. As used herein, Mw is measured by GPC versus polystyrene standards using tetrahydrofuran (THF) or dimethylformamide (DMF) solvent.

The polymer preferably has a narrow molecular weight distribution or dispersity (Mw/Mn) of 1.0 to 2.2, more preferably 1.0 to 2.0. A polymer with such a narrow dispersity eliminates the risk that foreign particles are left on the pattern after development and the pattern profile is aggravated.

The polymer may be synthesized, for example, by dissolving a monomer or monomers corresponding to the above-mentioned repeat units in an organic solvent, adding a radical polymerization initiator, and heating for polymerization.

Examples of the organic solvent which can be used for polymerization include toluene, benzene, THF, diethyl ether, dioxane, cyclohexane, cyclopentane, methyl ethyl ketone (MEK), propylene glycol monomethyl ether acetate (PGMEA), and γ-butyrolactone (GBL). Examples of the polymerization initiator used herein include 2,2'-azobisisobutyronitrile (AIBN), 2,2'-azobis(2,4-dimethylvaleronitrile), dimethyl 2,2-azobis(2-methylpropionate), 1,1'-azobis(1-acetoxy-1-phenylethane), benzoyl peroxide, and lauroyl peroxide. The initiator is preferably added in an amount of 0.01 to 25 mol% based on the total of monomers to be polymerized. The reaction temperature is preferably 50 to 150°C, more preferably 60 to 100°C. The reaction time is preferably 2 to 24 hours, more preferably 2 to 12 hours in view of production efficiency.

The polymerization initiator may be fed to the reactor either by adding the initiator to the monomer solution and feeding the solution to the reactor, or by dissolving the initiator in a solvent to form an initiator solution and feeding the initiator solution and the monomer solution independently to the reactor. Because of a possibility that in the standby duration, the initiator generates a radical which triggers polymerization reaction to form a ultra-high-molecular-weight polymer, it is preferred from the standpoint of quality control to prepare the monomer solution and the initiator solution separately and add them dropwise. The acid labile group that has been incorporated in the monomer may be kept as such, or polymerization may be followed by protection or partial protection. During the polymer synthesis, any known chain transfer agent such as dodecyl mercaptan or 2-mercaptoethanol may be added for molecular weight control purpose. The amount of chain transfer agent added is preferably 0.01 to 20 mol% based on the total of monomers.

When a hydroxy-containing monomer is copolymerized, the hydroxy group is substituted by an acetal group which is susceptible to deprotection with acid, typically ethoxyethoxy, prior to polymerization, and the polymerization is followed by deprotection with weak acid and water. Alternatively, the hydroxy group is substituted by an acetyl, formyl or pivaloyl group prior to polymerization, and the polymerization is followed by alkaline hydrolysis.

When hydroxystyrene or hydroxyvinylnaphthalene is copolymerized, one method is dissolving hydroxystyrene or hydroxyvinylnaphthalene and other monomers in an organic solvent, adding a radical polymerization initiator thereto, and heating the solution for polymerization. In an alternative method, acetoxystyrene or acetoxyvinylnaphthalene is used instead, and after polymerization, the acetoxy group is deprotected by alkaline hydrolysis, for thereby converting the polymer product to polyhydroxystyrene or polyhydroxyvinylnaphthalene.

For alkaline hydrolysis, a base such as aqueous ammonia or triethylamine may be used. Preferably the reaction temperature is -20°C to 100°C, more preferably 0°C to 60°C, and the reaction time is 0.2 to 100 hours, more preferably 0.5 to 20 hours.

The amounts of monomers in the monomer solution may be determined appropriate so as to provide the preferred fractions of repeat units.

It is now described how to use the polymer obtained by the above preparation method. The reaction solution resulting from polymerization reaction may be used as the final product. Alternatively, the polymer may be recovered in powder form through a purifying step such as re-precipitation step of adding the polymerization solution to a poor solvent and letting the polymer precipitate as powder, after which the polymer powder is used as the final product. It is preferred from the standpoints of operation efficiency and consistent quality to handle a polymer solution which is obtained by dissolving the powder polymer resulting from the purifying step in a solvent, as the final product.

The solvents which can be used herein are described in JP-A 2008-111103, paragraphs [0144]-[0145] (USP 7,537,880). Exemplary solvents include ketones such as cyclohexanone and methyl-2-n-pentyl ketone; alcohols such as 3-methoxybutanol, 3-methyl-3-methoxybutanol, 1-methoxy-2-propanol, and 1-ethoxy-2-propanol; keto-alcohols such as diacetone alcohol (DAA); ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactones such as γ-butyrolactone (GBL); and high-boiling alcohols such as diethylene glycol, propylene glycol, glycerol, 1,4-butanediol, and 1,3-butanediol, which may be used alone or in admixture.

The polymer solution preferably has a polymer concentration of 0.01 to 30% by weight, more preferably 0.1 to 20% by weight.

Prior to use, the reaction solution or polymer solution is preferably filtered through a filter. Filtration is effective for consistent quality because foreign particles and gel which can cause defects are removed.

Suitable materials of which the filter is made include fluorocarbon, cellulose, nylon, polyester, and hydrocarbon base materials. Preferred for the filtration of a resist composition are filters made of fluorocarbons commonly known as Teflon^{®}, hydrocarbons such as polyethylene and polypropylene, and nylon. While the pore size of the filter may be selected appropriate to comply with the desired cleanness, the filter preferably has a pore size of up to 100 nm, more preferably up to 20 nm. A single filter may be used or a plurality of filters may be used in combination. Although the filtering method may be single pass of the solution, preferably the filtering step is repeated by flowing the solution in a circulating manner. In the polymer preparation process, the filtering step may be carried out any times, in any order and in any stage. The reaction solution as polymerized or the polymer solution may be filtered, preferably both are filtered.

### Chemically amplified resist composition

A further embodiment of the invention is a chemically amplified resist composition comprising (A) a base polymer containing the polymer defined above.

### (A) Base polymer

The polymer defined above may be used alone or as a mixture of two or more polymers which are different in compositional ratio, Mw and/or Mw/Mn. When two or more polymers are combined, a polymer comprising repeat units A1 may be combined with a polymer comprising at least one of repeat units A2, A3-1, A3-2, A4, A5, and A6, but not repeat units A1. In this mixture, the amount of the polymer free of repeat units A1 is preferably 2 to 5,000 parts by weight, more preferably 10 to 1,000 parts by weight per 100 parts by weight of the polymer comprising repeat units A1.

It is preferred from the aspect of etch resistance of the base polymer that repeat units having an aromatic ring skeleton account for at least 60 mol%, more preferably at least 80 mol% of the overall repeat units of the polymer in the base polymer. Most preferably, all repeat units have an aromatic ring skeleton.

### (B) Quencher

The chemically amplified positive resist composition preferably comprises a quencher as component (B). As used herein, the quencher refers to a compound capable of trapping an acid generated from the PAG upon exposure to prevent the acid from diffusing into the unexposed region, thus enabling to form the desired pattern.

The quencher is typically selected from conventional basic compounds. Conventional basic compounds include primary, secondary, and tertiary aliphatic amines, mixed amines, aromatic amines, heterocyclic amines, nitrogen-containing compounds with carboxy group, nitrogen-containing compounds with sulfonyl group, nitrogen-containing compounds with hydroxy group, nitrogen-containing compounds with hydroxyphenyl group, alcoholic nitrogen-containing compounds, amide derivatives, imide derivatives, and carbamate derivatives. Also included are primary, secondary, and tertiary amine compounds, specifically amine compounds having a hydroxy, ether bond, ester bond, lactone ring, cyano, or sulfonate ester bond as described in JP-A 2008-111103, paragraphs [0146]-[0164], and compounds having a carbamate group as described in JP 3790649. Inter alia, tris[2-(methoxymethoxy)ethyl]amine, tris[2-(methoxymethoxy)ethyl]amine-N-oxide, dibutylaminobenzoic acid, morpholine derivatives, and imidazole derivatives are preferred. Addition of a basic compound may be effective for further suppressing the diffusion rate of acid in the resist film or correcting the pattern profile.

A betaine compound having the formula (B1) is preferred as the quencher.

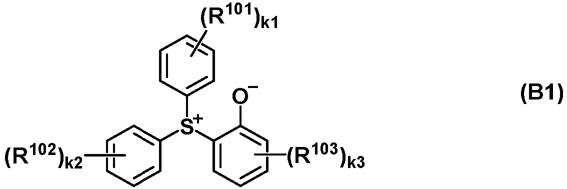

In formula (B1), R¹⁰¹, R¹⁰² and R¹⁰³ are each independently a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, n-propyl, isopropyl, n-butyl, sec-butyl, tert-butyl, n-pentyl, tert-pentyl, n-hexyl, n-octyl, 2-ethylhexyl, n-nonyl, and n-decyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; and C₆-C₂₀ aryl groups such as phenyl, naphthyl, and anthracenyl. In the hydrocarbyl group, some hydrogen may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, fluorine, chlorine, bromine, iodine, cyano moiety, carbonyl moiety, ether bond, thioether bond, ester bond, sulfonate ester bond, carbonate bond, carbamate bond, lactone ring, sultone ring, carboxylic anhydride (-C(=O)-O-C(=O)-) or haloalkyl moiety.

In formula (B1), k1 and k2 are each independently an integer of 0 to 5, and k3 is an integer of 0 to 4. From the standpoints of ease of synthesis and availability of reactants, k1, k2 and k3 each are preferably 0, 1 or 2.

When k1 is 2 to 5, two adjoining R¹⁰¹ may bond together to form a ring with the carbon atoms to which they are attached. When k2 is 2 to 5, two adjoining R¹⁰² may bond together to form a ring with the carbon atoms to which they are attached. When k3 is 2 to 4, two adjoining R¹⁰³ may bond together to form a ring with the carbon atoms to which they are attached.

Examples of the betaine compound having formula (B 1) are shown below, but not limited thereto.

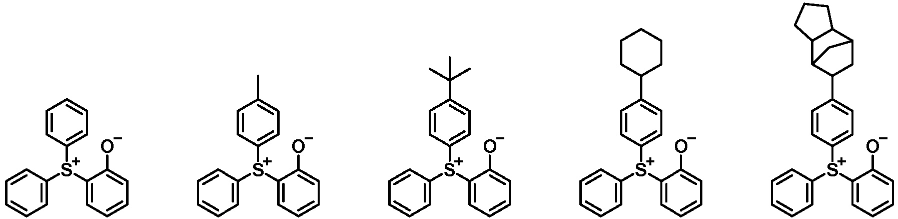

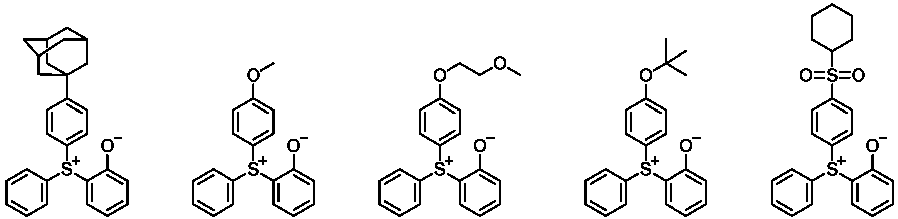

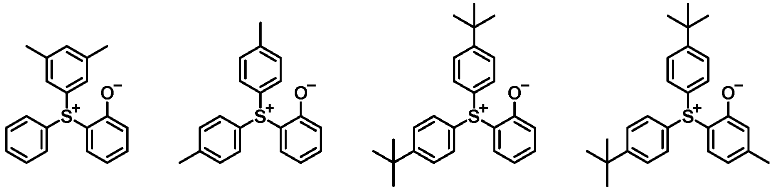

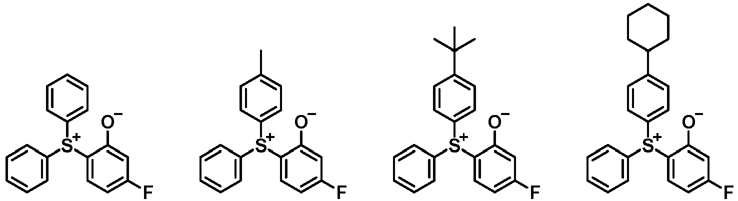

The betaine compound whose counter anion is a strongly basic phenoxide has a superior acid diffusion controlling ability and contributes to a contrast improvement. When blended in a resist composition, the betaine compound does not incur a salt exchange with the sulfonium salt in repeat units A1. Then, the base polymer containing repeat units A1 maintains its solvent solubility and remains in the resist composition without forming agglomerates. The betaine compound exerts superior performance in terms of development defects after resist patterning and defects after etching. Even small-size patterns can be formed with few or no agglomerate defects. A resist composition of next generation featuring high resolution and superior defect control is available.

A betaine compound of weak acid can also be used as the quencher. Examples of the betaine compound of weak acid are shown below, but not limited thereto.

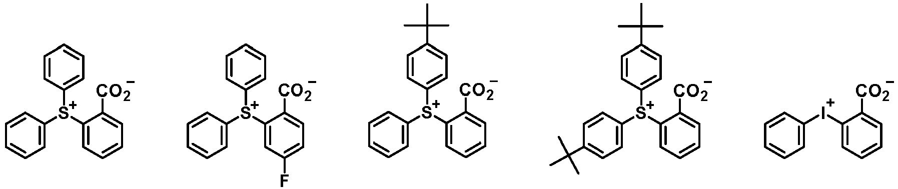

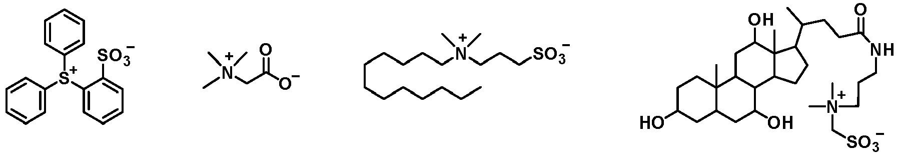

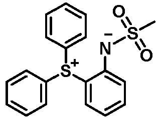

Onium salts such as sulfonium, iodonium and ammonium salts of carboxylic acids which are not fluorinated at α-position as described in USP 8,795,942 (JP-A 2008-158339) may also be used as the quencher. While an α-fluorinated sulfonic acid, imide acid, and methide acid are necessary to deprotect the acid labile group, an α-non-fluorinated carboxylic acid is released by salt exchange with an α-non-fluorinated onium salt. The α-non-fluorinated carboxylic acid functions as a quencher because it does not induce substantial deprotection reaction.

Examples of the onium salt of α-non-fluorinated carboxylic acid include compounds having the formula (B2).

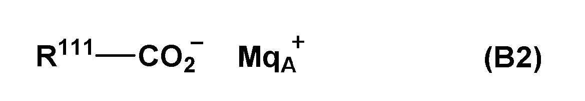

In formula (B2), R¹¹¹ is hydrogen or a C₁-C₄₀ hydrocarbyl group which may contain a heteroatom, exclusive of the hydrocarbyl group in which the hydrogen bonded to the carbon atom at α-position of the carboxy group is substituted by fluorine or fluoroalkyl.

The hydrocarbyl group R¹¹¹ may be saturated or unsaturated and straight, branched or cyclic. Examples thereof include C₁-C₄₀ alkyl groups such as methyl, ethyl, n-propyl, isopropyl, n-butyl, isobutyl, sec-butyl, tert-butyl, n-pentyl, tert-pentyl, n-hexyl, n-octyl, 2-ethylhexyl, n-nonyl, n-decyl; C₃-C₄₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; C₂-C₄₀ alkenyl groups such as vinyl, allyl, propenyl, butenyl and hexenyl; C₃-C₄₀ cyclic unsaturated aliphatic hydrocarbyl groups such as cyclohexenyl; C₆-C₄₀ aryl groups such as phenyl, naphthyl, alkylphenyl groups (e.g., 2-methylphenyl, 3-methylphenyl, 4-methylphenyl, 4-ethylphenyl, 4-tert-butylphenyl, 4-n-butylphenyl), di- or trialkylphenyl groups (e.g., 2,4-dimethylphenyl and 2,4,6-triisopropylphenyl), alkylnaphthyl groups (e.g., methylnaphthyl and ethylnaphthyl), dialkylnaphthyl groups (e.g., dimethylnaphthyl and diethylnaphthyl); and C₇-C₄₀ aralkyl groups such as benzyl, 1-phenylethyl and 2-phenylethyl.

In the hydrocarbyl groups, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy moiety, cyano moiety, fluorine, chlorine, bromine, iodine, carbonyl moiety, ether bond, thioether bond, ester bond, sulfonate ester bond, carbonate bond, lactone ring, sultone ring, carboxylic anhydride (-C(=O)-O-C(=O)-), or haloalkyl moiety. Suitable heteroatom-containing hydrocarbyl groups include heteroaryl groups such as thienyl; alkoxyphenyl groups such as 4-hydroxyphenyl, 4-methoxyphenyl, 3-methoxyphenyl, 2-methoxyphenyl, 4-ethoxyphenyl, 4-tert-butoxyphenyl, 3-tert-butoxyphenyl; alkoxynaphthyl groups such as methoxynaphthyl, ethoxynaphthyl, n-propoxynaphthyl and n-butoxynaphthyl; dialkoxynaphthyl groups such as dimethoxynaphthyl and diethoxynaphthyl; and aryloxoalkyl groups, typically 2-aryl-2-oxoethyl groups such as 2-phenyl-2-oxoethyl, 2-(1-naphthyl)-2-oxoethyl and 2-(2-naphthyl)-2-oxoethyl.

In formula (B2), Mq_{A}⁺ is an onium cation. The onium cation is preferably selected from sulfonium, iodonium and ammonium cations, more preferably sulfonium and iodonium cations. Preferred cations include those described in JP-A 2023-091749, paragraphs [0154]-[0182]. The cations of the sulfonium salt having formula (A1) are more preferred.

Examples of the anion in the onium salt having formula (B2) are shown below, but not limited thereto.

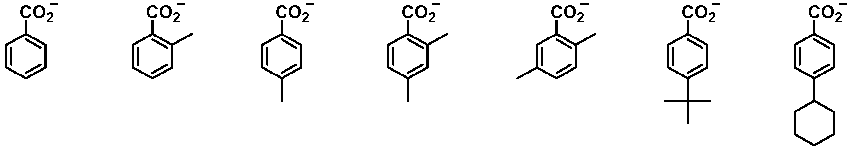

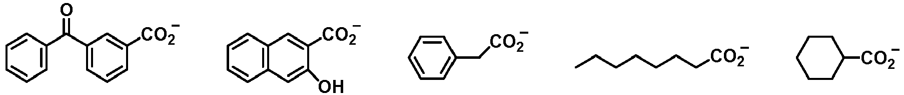

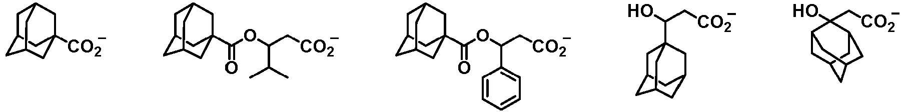

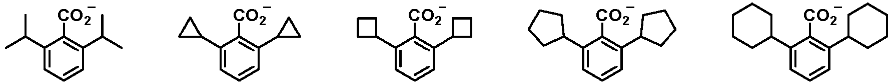

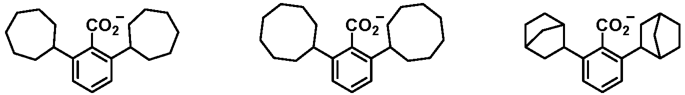

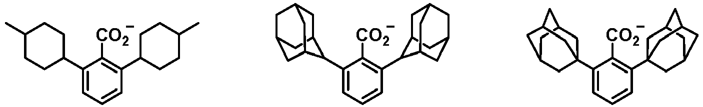

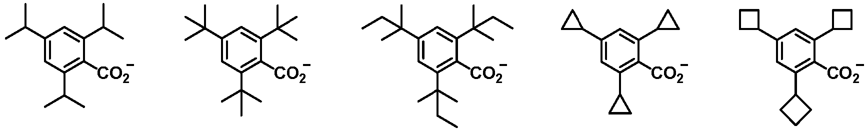

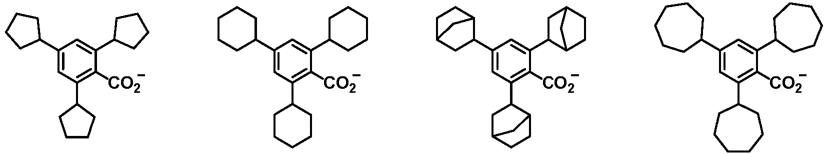

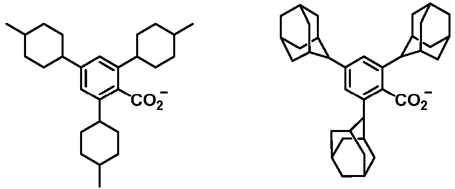

A sulfonium salt of iodized benzene ring-containing carboxylic acid having the formula (B3) is also useful as the quencher.

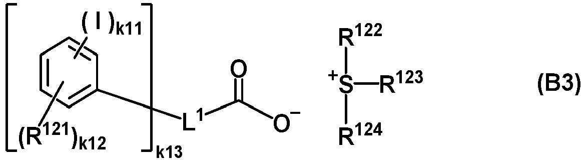

In formula (B3), k11 is an integer of 1 to 5, k12 is an integer of 0 to 3, and k13 is an integer of 1 to 3.

In formula (B3), R¹²¹ is hydroxy, fluorine, chlorine, bromine, amino, nitro, cyano, or a C₁-C₆ saturated hydrocarbyl group which may contain halogen, C₁-C₆ saturated hydrocarbyloxy group which may contain halogen, C₂-C₆ saturated hydrocarbylcarbonyloxy group which may contain halogen or C₁-C₄ saturated hydrocarbylsulfonyloxy group which may contain halogen, -N(R^{121A})-C(=O)-R^{121B}, or -N(R^{121A})-C(=O)-O-R^{121B}. R^{121A} is hydrogen or a C₁-C₆ saturated hydrocarbyl group. R^{121B} is a C₁-C₆ saturated hydrocarbyl or C₂-C₈ unsaturated aliphatic hydrocarbyl group. A plurality of R¹²¹ may be the same or different when k11 and/or k13 is 2 or more.

In formula (B3), L¹ is a single bond, or a C₁-C₂₀ (k13+1)-valent linking group which may contain at least one moiety selected from ether bond, carbonyl moiety, ester bond, amide bond, sultone ring, lactam ring, carbonate bond, halogen, hydroxy moiety, and carboxy moiety. The saturated hydrocarbyl, saturated hydrocarbyloxy, saturated hydrocarbylcarbonyloxy, and saturated hydrocarbylsulfonyloxy groups may be straight, branched or cyclic.

In formula (B3), R¹²², R¹²³ and R¹²⁴ are each independently halogen, or a C₁-C₂₀ hydrocarbyl group which may contain a heteroatom. The hydrocarbyl group may be saturated or unsaturated and straight, branched or cyclic. Examples thereof include C₁-C₂₀ alkyl, C₂-C₂₀ alkenyl, C₆-C₂₀ aryl, and C₇-C₂₀ aralkyl groups. In these groups, some or all hydrogen may be substituted by hydroxy, carboxy, halogen, oxo, cyano, nitro, sultone ring, sulfo, or sulfonium salt-containing moiety, or some -CH₂- may be replaced by an ether bond, ester bond, carbonyl moiety, amide bond, carbonate bond or sulfonate ester bond. Also, R¹²² and R¹²³ may bond together to form a ring with the sulfur atom to which they are attached. Exemplary structures of the sulfonium cation in the sulfonium salt having formula (B3) are described in JP-A 2023-091749, paragraphs [0154]-[0180]. The cations of the sulfonium salts having formula (A1) are preferred.

Examples of the compound having formula (B3) include those described in USP 10,295,904 (JP-A 2017-219836). These compounds exert a sensitizing effect due to remarkable absorption and an acid diffusion-controlling effect.

A nitrogen-containing carboxylic acid salt compound having the formula (B4) is also useful as the quencher.

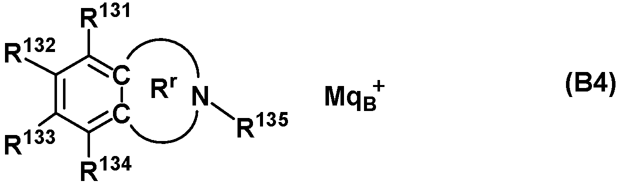

In formula (B4), R¹³¹ to R¹³⁴ are each independently hydrogen, -L²-CO₂⁻, or a C₁-C₂₀ hydrocarbyl group which may contain a heteroatom. R¹³¹ and R¹³², R¹³² and R¹³³, or R¹³³ and R¹³⁴ may bond together to form a ring with the carbon atom to which they are attached. L² is a single bond or a C₁-C₂₀ hydrocarbylene group which may contain a heteroatom. R¹³⁵ is hydrogen or a C₁-C₂₀ hydrocarbyl group which may contain a heteroatom.

In formula (B4), the ring R^{r} is a C₂-C₆ ring containing the carbon and nitrogen atoms in the formula, in which some or all of the carbon-bonded hydrogen atoms may be substituted by a C₁-C₂₀ hydrocarbyl group or -L²-CO₂⁻ and in which some carbon may be replaced by sulfur, oxygen or nitrogen. The ring may be alicyclic or aromatic and is preferably a 5- or 6-membered ring. Suitable rings include pyridine, pyrrole, pyrrolidine, piperidine, pyrazole, imidazoline, pyridazine, pyrimidine, pyrazine, imidazoline, oxazole, thiazole, morpholine, thiazine, and triazole rings.

The carboxylic onium salt having formula (B4) has at least one -L²-CO₂⁻. That is, at least one of R¹³¹ to R¹³⁴ is -L²-CO₂⁻, and/or at least one of hydrogen atoms bonded to carbon atoms in the ring R^{r} is substituted by -L²-CO₂⁻.

In formula (B4), Mq_{B}⁺ is a sulfonium, iodonium or ammonium cation, with the sulfonium cation being preferred. Examples of the sulfonium cation include those described in JP-A 2023-091749, paragraphs [0154]-[0180]. The cations of the sulfonium salt having formula (A1) are preferred.

Examples of the anion in the compound having formula (B4) are shown below, but not limited thereto.

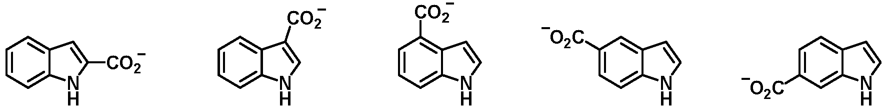

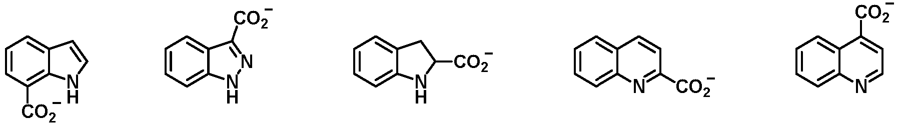

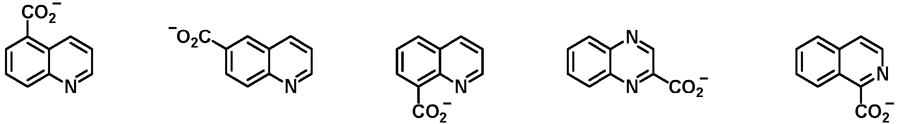

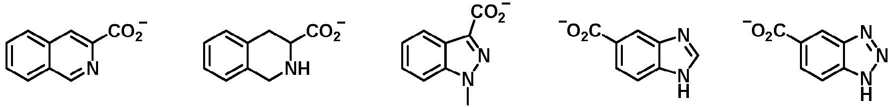

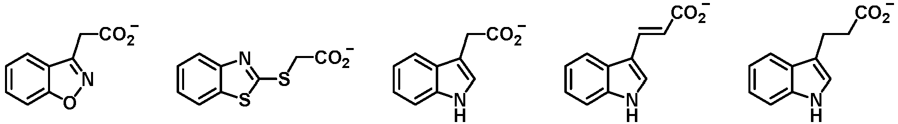

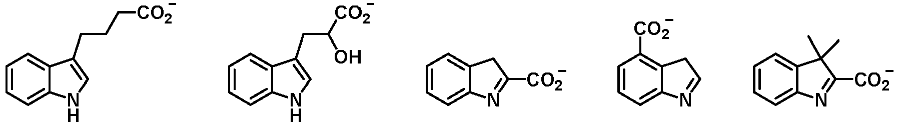

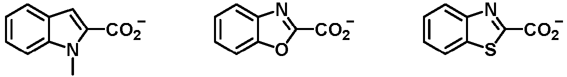

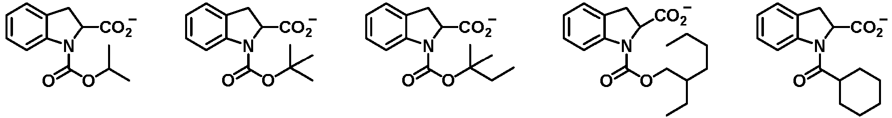

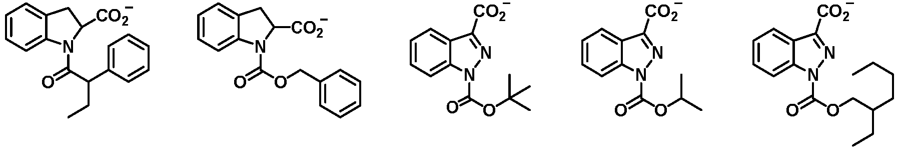

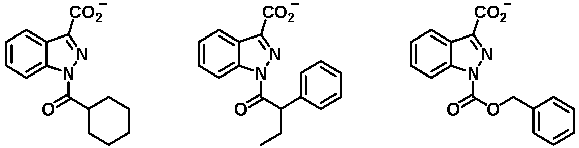

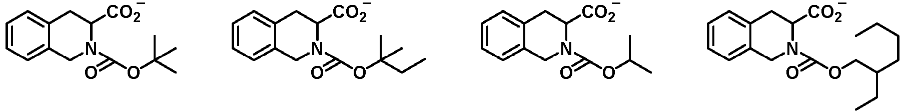

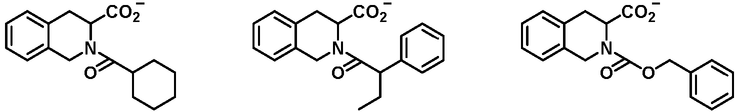

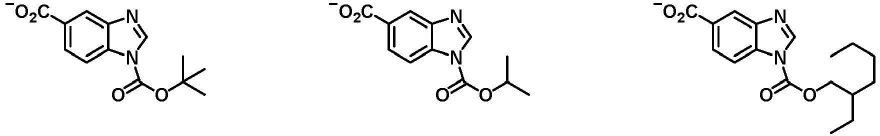

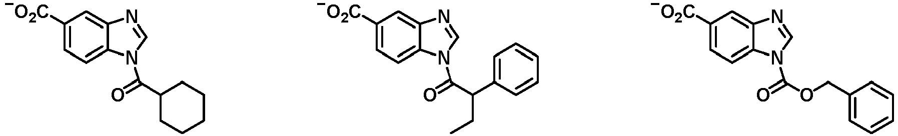

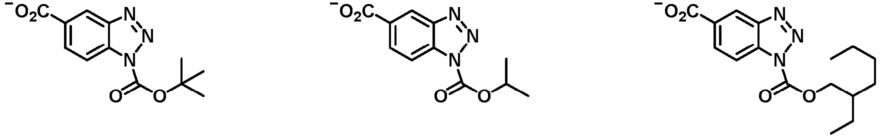

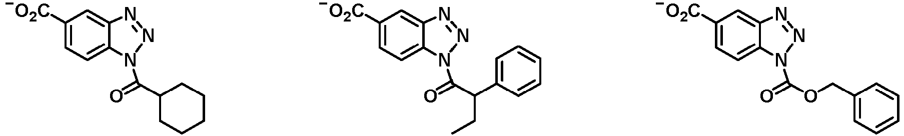

Also useful are quenchers of polymer type as described in USP 7,598,016 (JP-A 2008-239918). The polymeric quencher segregates at the resist surface after coating and thus enhances the rectangularity of resist pattern. When a protective film is applied as is often the case in the immersion lithography, the polymeric quencher is also effective for preventing a film thickness loss of resist pattern or rounding of pattern top.

When the resist composition contains the quencher (B), it is preferably added in an amount of 0.01 to 50 parts, more preferably 0.1 to 30 parts by weight per 80 parts by weight of the base polymer (A). For the acid diffusion control purpose, the quencher (B) is used in an amount of preferably at least 5 parts, more preferably at least 6 parts, even more preferably at least 7 parts by weight per 80 parts by weight of the base polymer (A) whereby patterns with satisfactory resolution and LER are formed. In particular, the quenchers of betaine type and sulfonium salt type are preferred. The quencher may be used alone or in admixture.

### (C) Photoacid generator

The chemically amplified positive resist composition may further comprise a photoacid generator (PAG) as component (C). The PAG used herein may be any compound capable of generating an acid upon exposure to high-energy radiation. Suitable PAGs include sulfonium salts, iodonium salts, sulfonyldiazomethane, N-sulfonyloxyimide, and oxime-O-sulfonate acid generators.

Suitable PAGs include nonafluorobutane sulfonate, partially fluorinated sulfonates described in JP-A 2012-189977, paragraphs [0247]-[0251], partially fluorinated sulfonates described in JP-A 2013-101271, paragraphs [0261]-[0265], and fluorobenzenesulfonates as described in JP-A 2008-111103, paragraphs [0122]-[0142] and JP-A 2010-215608, paragraphs [0080]-[0081]. Among others, arylsulfonate and alkanesulfonate type PAGs are preferred because they generate acids having an appropriate strength to deprotect the acid labile group in repeat unit having formula (A5).

The preferred PAGs are onium salt compounds having anions of the structure shown below.

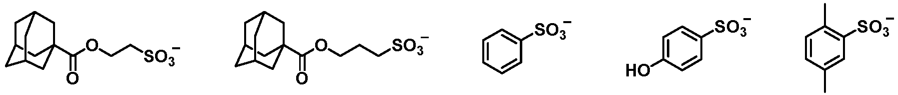

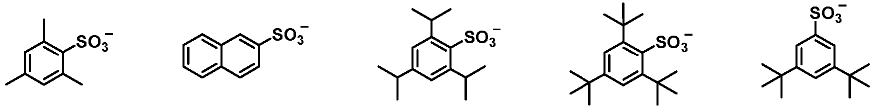

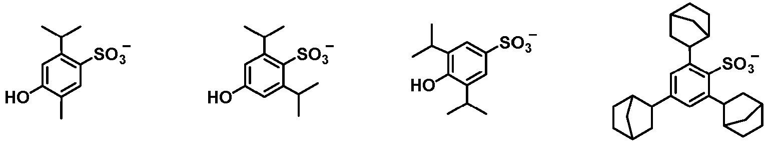

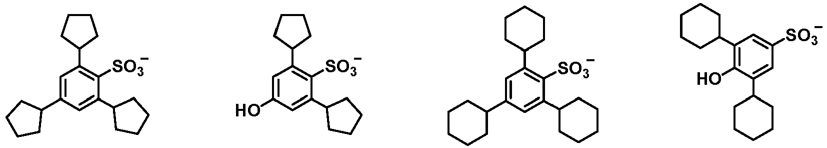

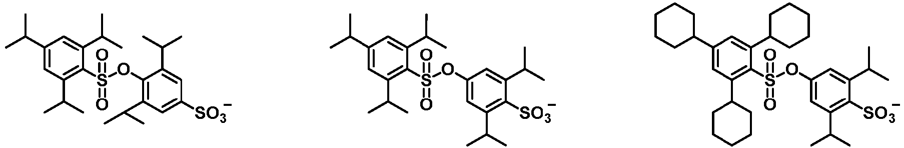

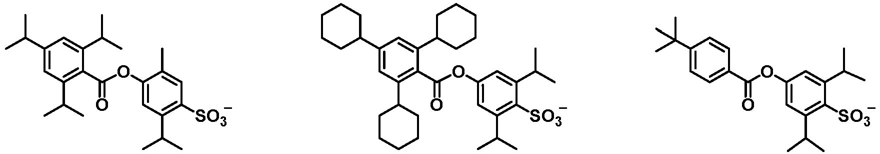

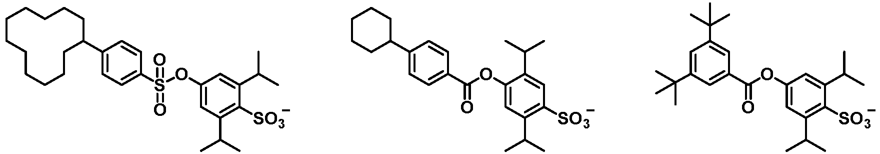

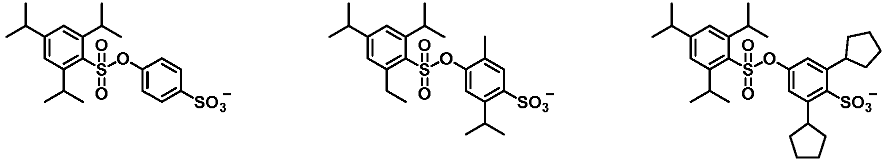

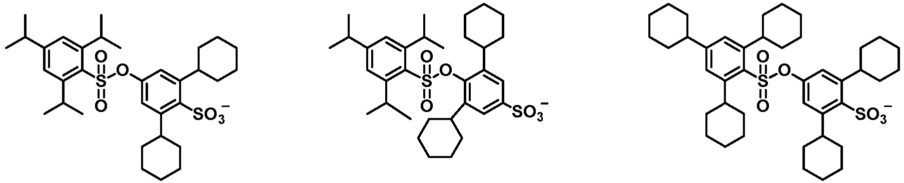

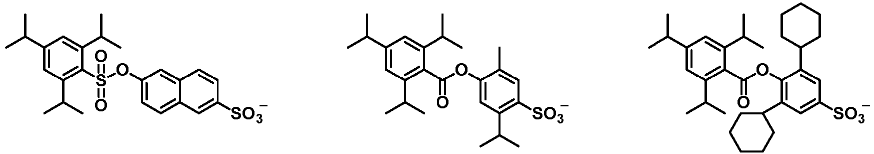

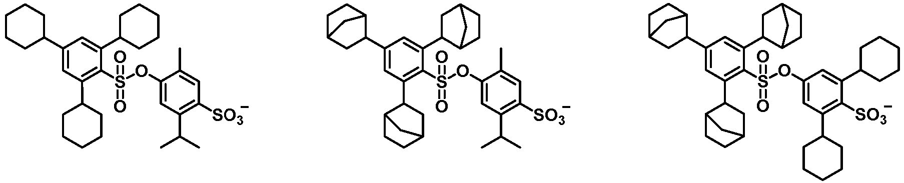

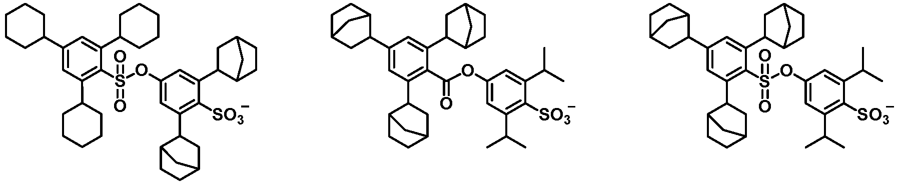

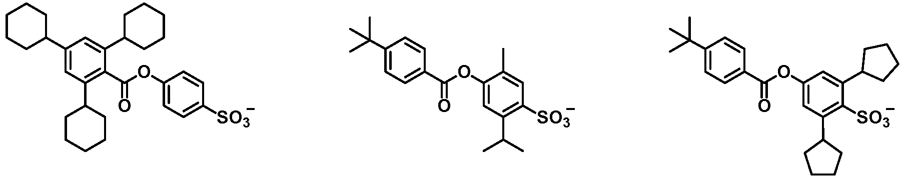

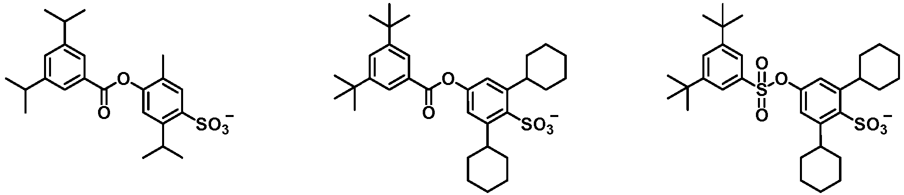

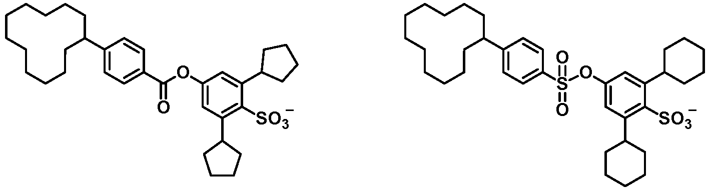

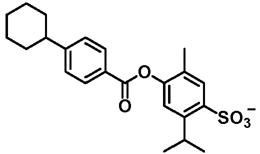

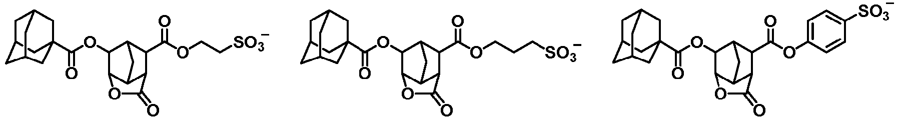

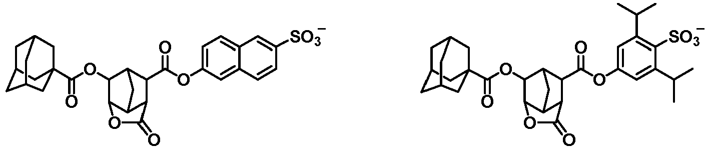

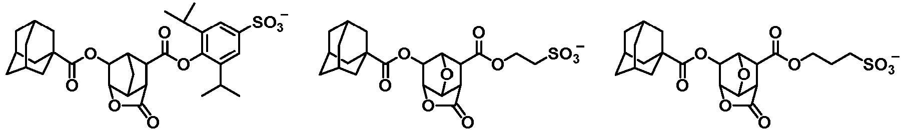

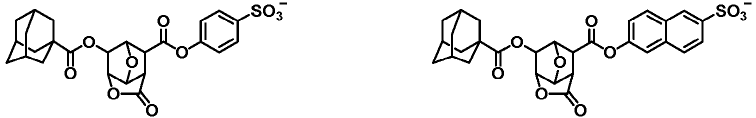

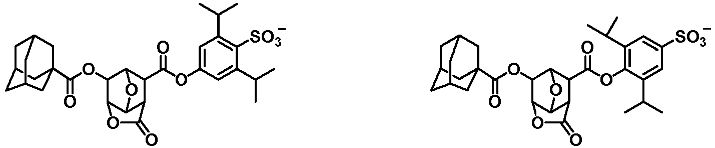

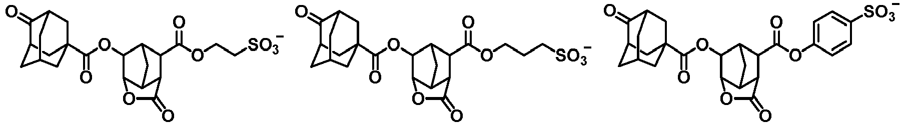

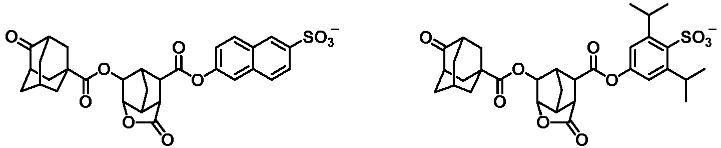

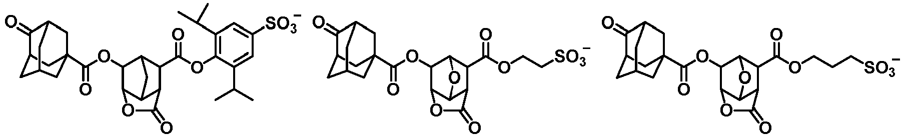

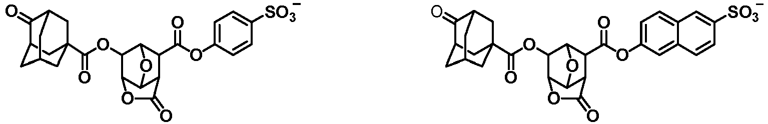

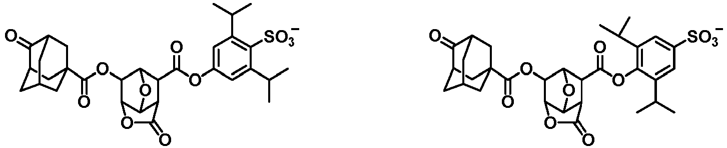

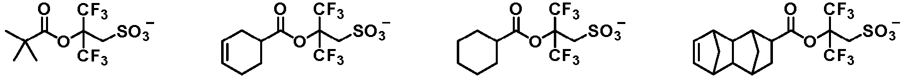

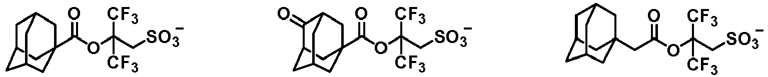

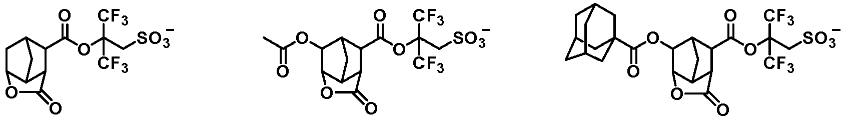

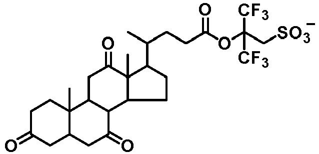

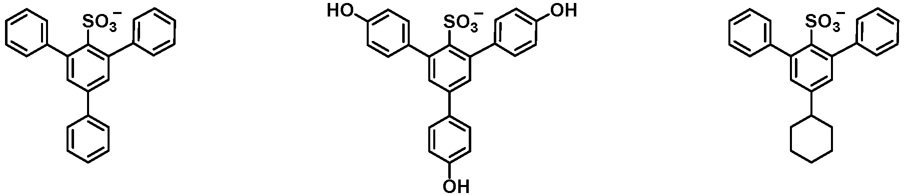

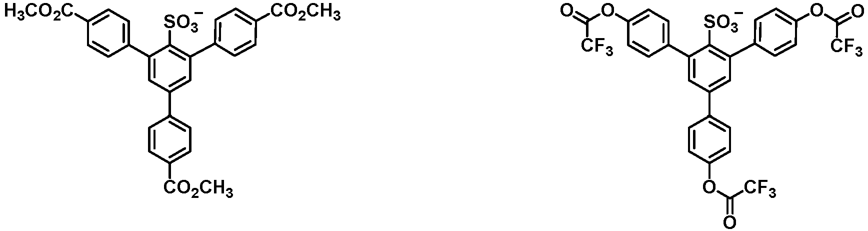

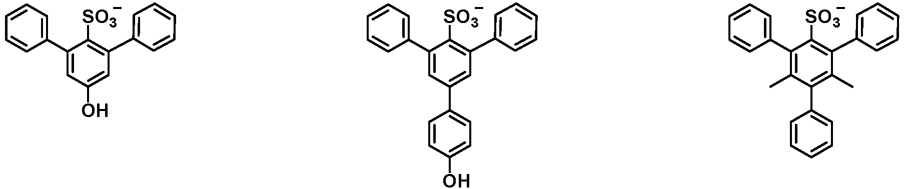

Onium salt compounds having an anion of the formula (C-1) are also preferred as the PAG (C).

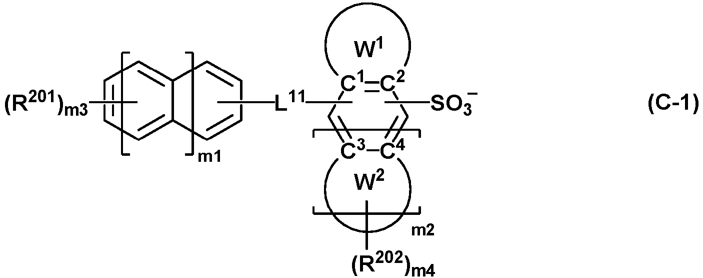

In formula (C-1), m1 and m2 are each independently an integer of 0 to 2. The relevant structure represents a benzene ring when m1=0, a naphthalene ring when m1=1, and an anthracene ring when m1=2. From the aspect of solvent solubility, the benzene ring corresponding to m1=0 is preferred. From the aspect of solvent solubility, m2=0 is preferred.

In formula (C-1), m3 is an integer of 1 to 5 in case of m1=0, an integer of 1 to 7 in case of m1=1, and an integer of 1 to 9 in case of m1=2. The subscript m4 is an integer of 0 to 5 in case of m2=0, an integer of 0 to 7 in case of m2=1, and an integer of 0 to 9 in case of m2=2.

In formula (C-1), L¹¹ is a single bond, ether bond, ester bond, sulfonate ester bond, amide bond, carbonate bond or carbamate bond. Inter alia, an ether bond, ester bond, and sulfonate ester bond are preferred, with sulfonate ester bond being more preferred.

In formula (C-1), R²⁰¹ is each independently iodine or a C₃-C₂₀ branched or cyclic hydrocarbyl group which may contain a heteroatom, at least one R²⁰¹ is attached to a carbon atom adjoining the carbon atom to which L¹¹ is attached. Examples of the hydrocarbyl group include, but are not limited to, C₃-C₂₀ aliphatic cyclic hydrocarbon groups such as isopropyl, sec-butyl, tert-butyl, tert-pentyl, cyclopentyl, cyclohexyl, 2-ethylhexyl, cyclopentylmethyl, cyclopentylethyl, cyclopentylbutyl, cyclohexylmethyl, cyclohexylethyl, cyclohexylbutyl, norbornyl, oxanorbornyl, tricyclo[5.2.1.0^{2,6}]decyl, and adamantyl; aryl groups such as phenyl, naphthyl and anthracenyl; and combinations thereof. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, halogen, carbonyl moiety, ether bond, ester bond, sulfonate ester bond, carbonate bond, lactone ring, sultone ring, carboxylic anhydride (-C(=O)-O-C(=O)-) or haloalkyl moiety. Suitable halogen atoms include fluorine, chlorine, bromine and iodine, with fluorine and iodine being preferred. When m3 is 2 or more, a plurality of R²⁰¹ may be identical or different.

When m3 is 2 or more, a plurality of R²⁰¹ may bond together to form a ring with the carbon atoms to which they are attached. Preferred is a 5 to 8-membered ring.

In formula (C-1), R²⁰² is each independently a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, tert-butyl, n-pentyl, tert-pentyl, n-hexyl, n-octyl, 2-ethylhexyl, n-nonyl, and n-decyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; and C₆-C₂₀ aryl groups such as phenyl, naphthyl and anthracenyl. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, halogen, carbonyl moiety, ether bond, ester bond, sulfonate ester bond, carbonate bond, lactone ring, sultone ring, carboxylic anhydride (-C(=O)-O-C(=O)-) or haloalkyl moiety. Suitable halogen atoms include fluorine, chlorine, bromine, and iodine, with fluorine and iodine being preferred. When m4 is 2 or more, a plurality of R²⁰² may be identical or different.

When m4 is 2 or more, a plurality of R²⁰² may bond together to form a ring with the carbon atoms to which they are attached. Preferred is a 5 to 8-membered ring.

In formula (C-1), W¹ forms a C₃-C₁₅ ring with the carbon atoms C¹ and C² on the adjoining aromatic ring, some carbon on the ring may be replaced by a heteroatom-containing group. The ring may be mono or polycyclic. W² forms a C₃-C₁₅ ring with the carbon atoms C³ and C⁴ on the adjoining aromatic ring, some carbon on the ring may be replaced by a heteroatom-containing group. The ring may be mono or polycyclic.

Exemplary structures of the ring that W¹ or W² in formula (B-1) forms by fusing to the adjoining aromatic ring are shown below, but not limited thereto. Herein, the broken line designates a point of attachment to L¹¹.

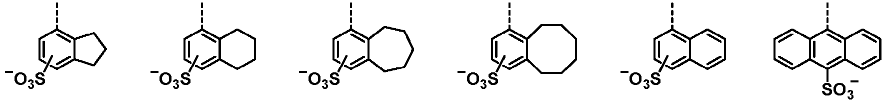

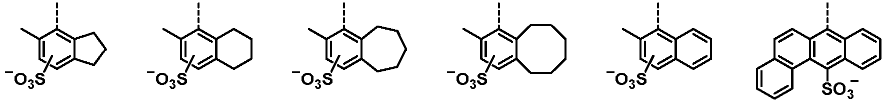

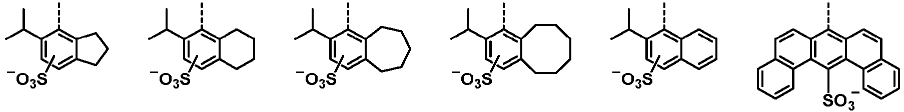

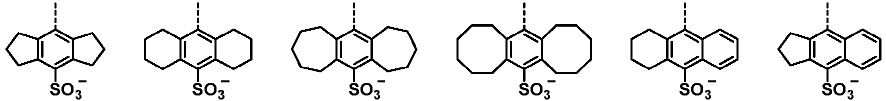

Examples of the structure that R²⁰¹ forms with the aromatic ring in formula (C-1) are shown below, but not limited thereto. Herein, the broken line designates a point of attachment to L¹¹.

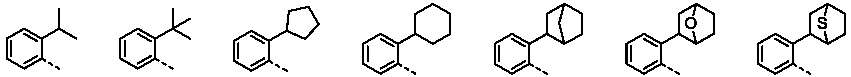

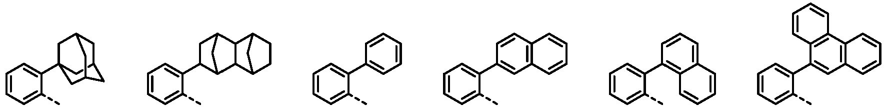

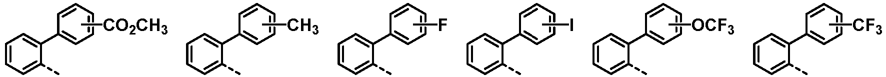

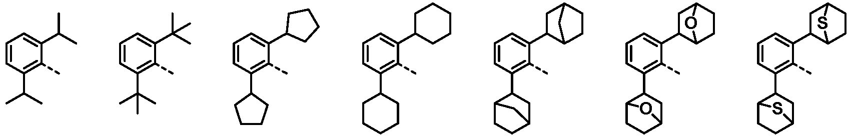

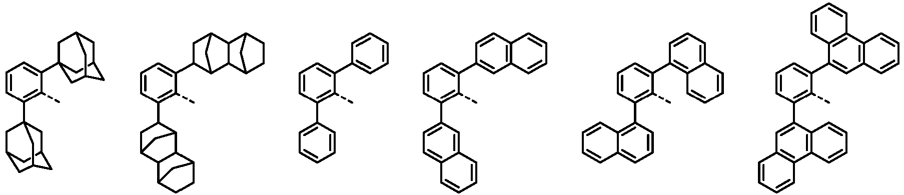

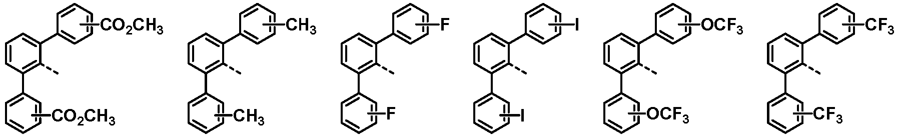

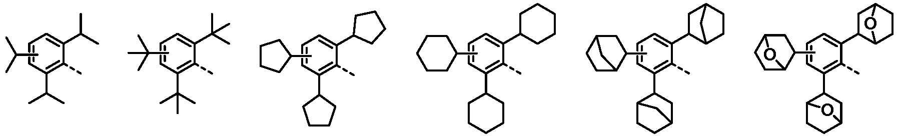

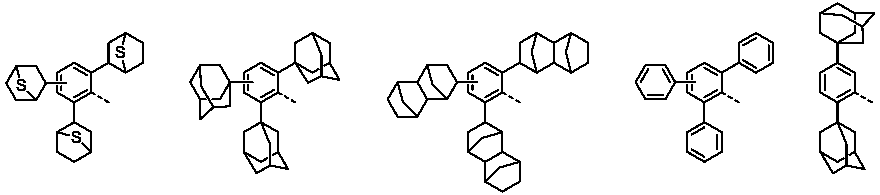

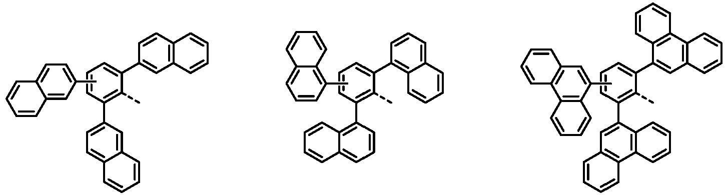

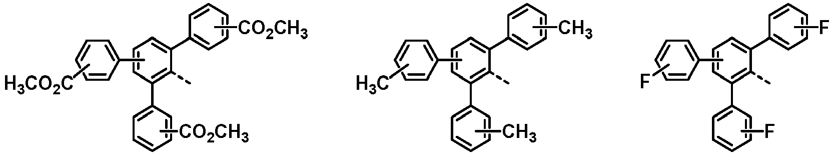

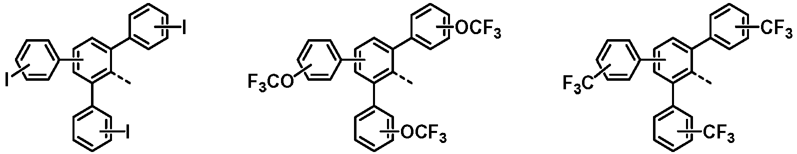

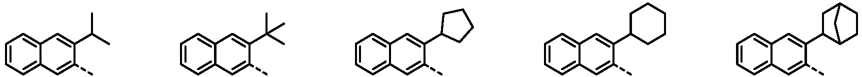

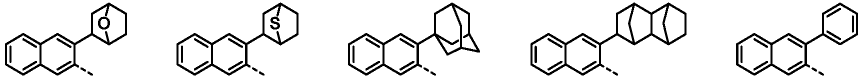

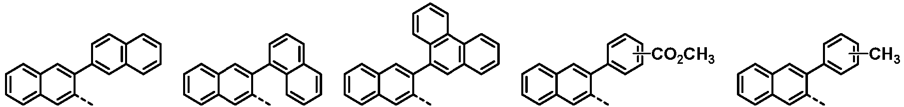

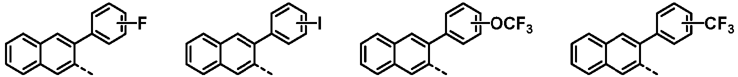

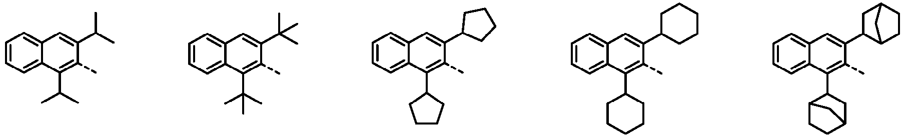

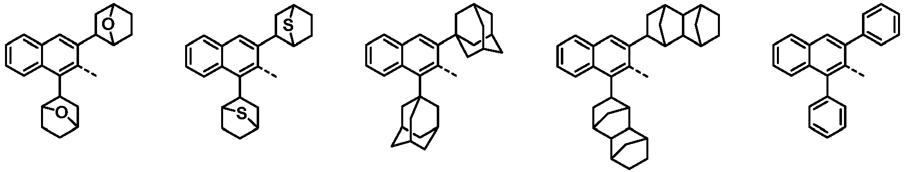

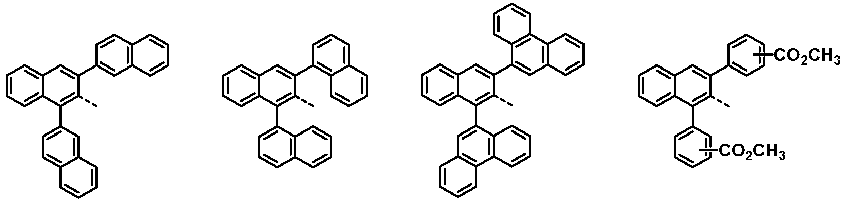

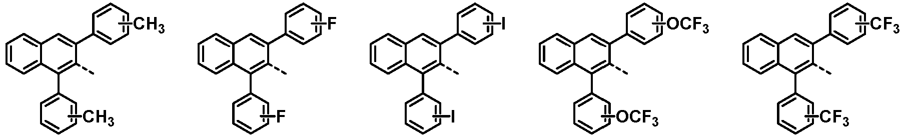

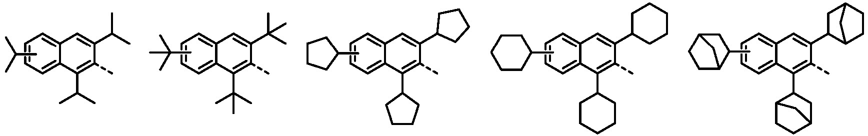

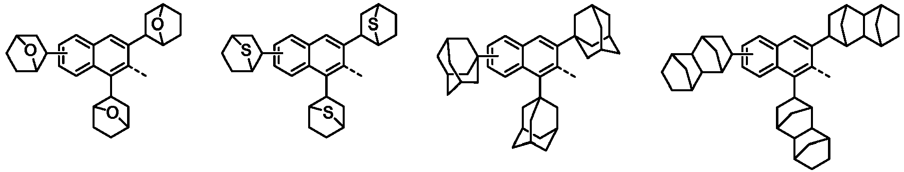

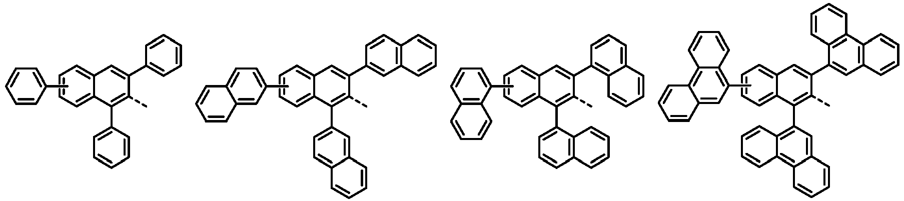

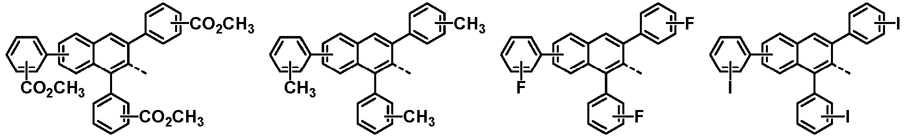

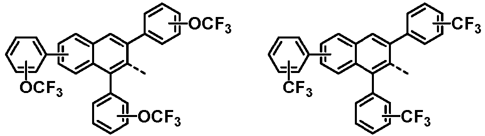

Of the anions having formula (C-1), anions having the formula (C-1-1) are preferred. 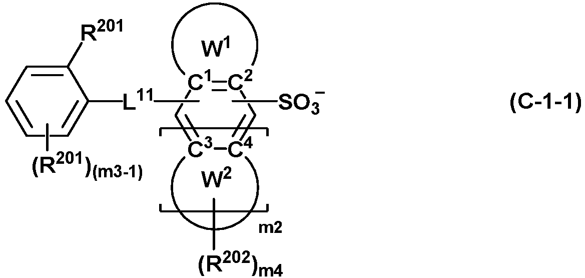 Herein m2, m3, m4, L¹¹, R²⁰¹, R²⁰², W¹, and W² are as defined above.

Of the anions having formula (C-1-1), anions having the formula (C-1-2) are more preferred. 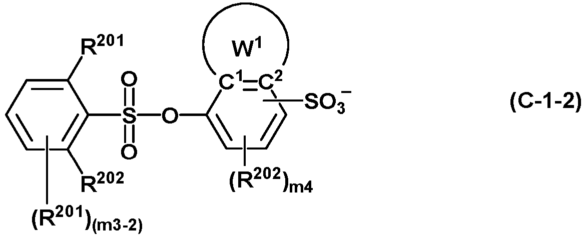 Herein m3, m4, R²⁰¹, R²⁰², and W¹ are as defined above.

Preferred examples of the anion having formula (C-1) are shown below, but not limited thereto.

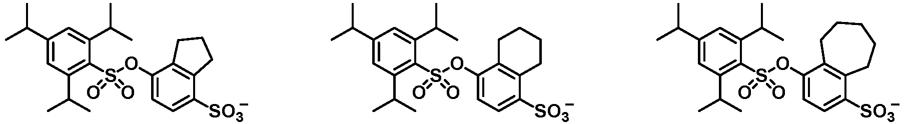

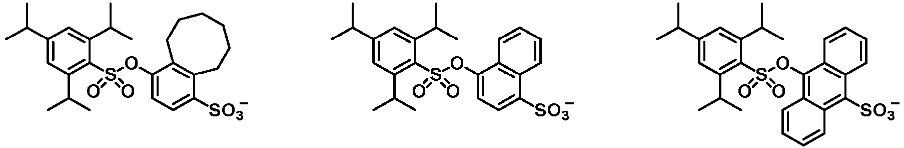

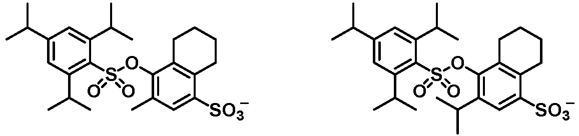

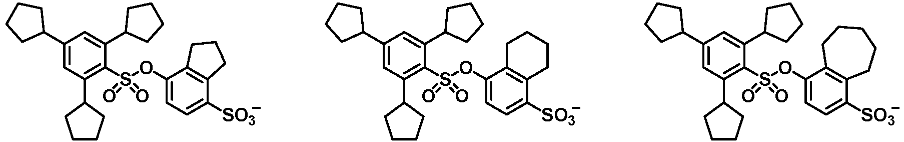

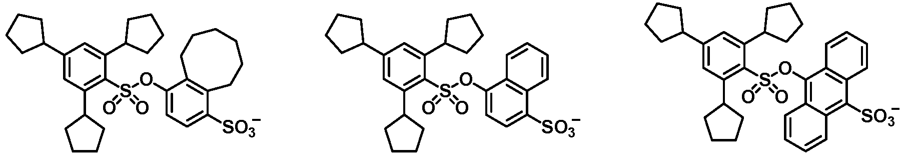

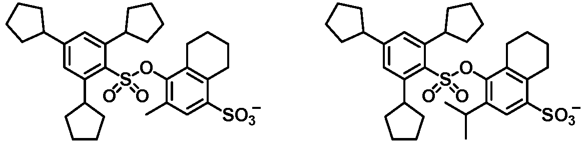

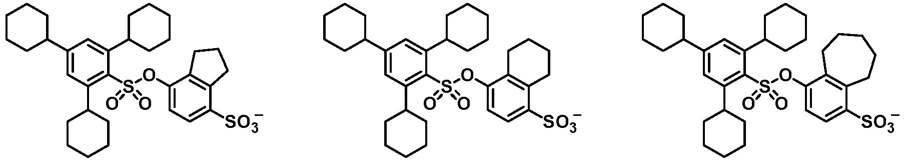

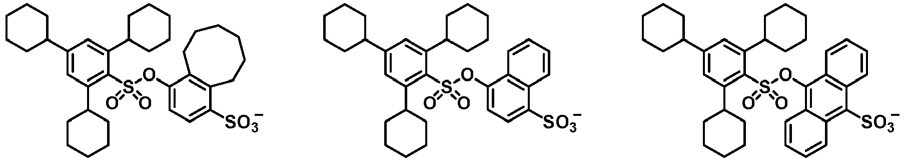

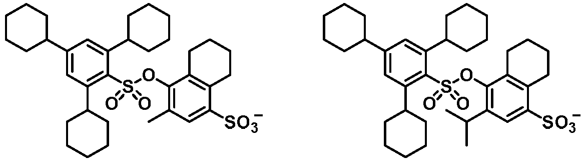

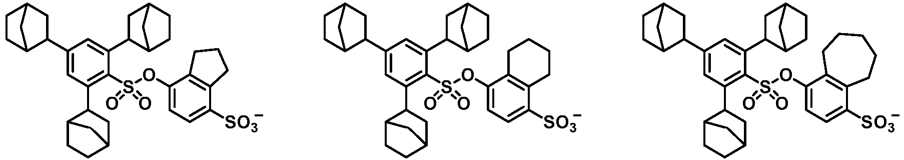

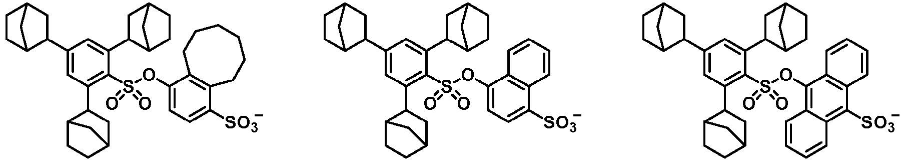

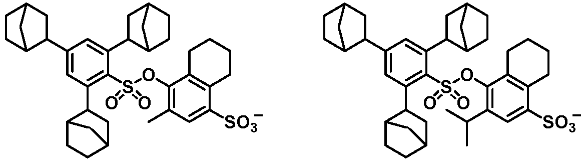

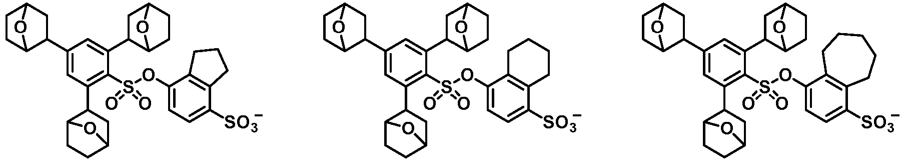

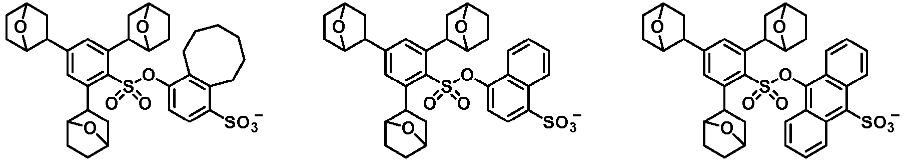

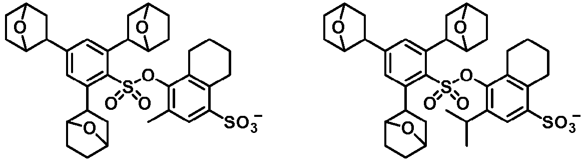

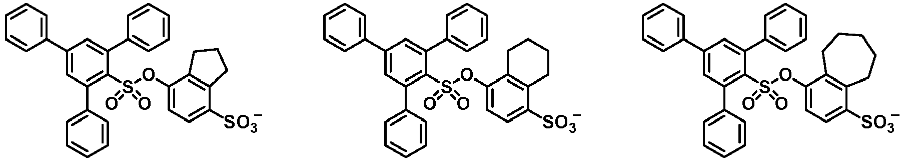

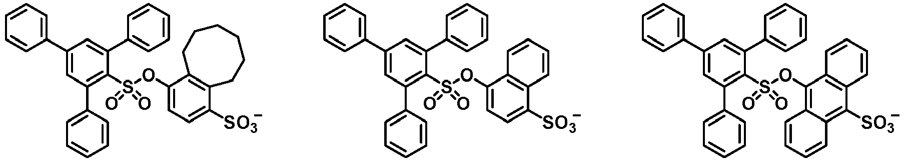

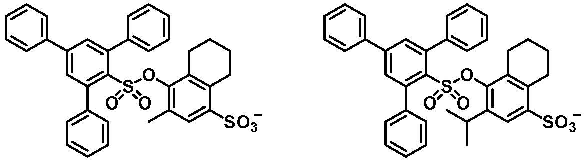

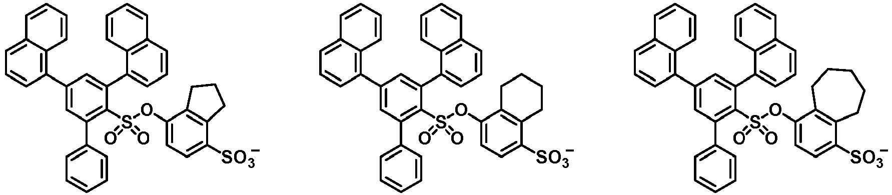

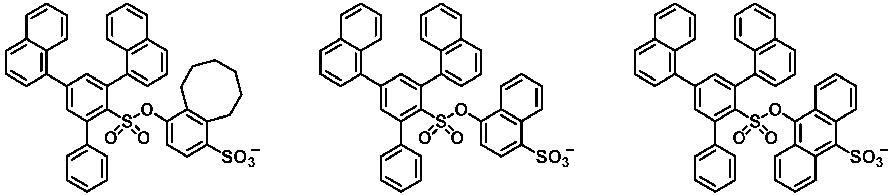

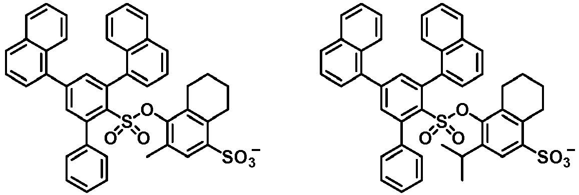

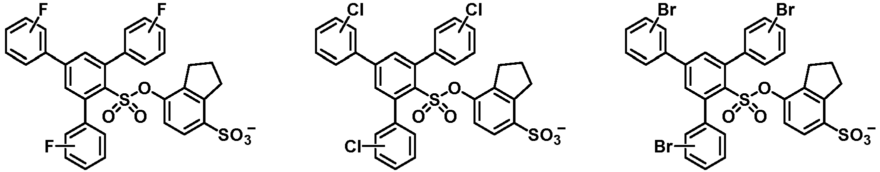

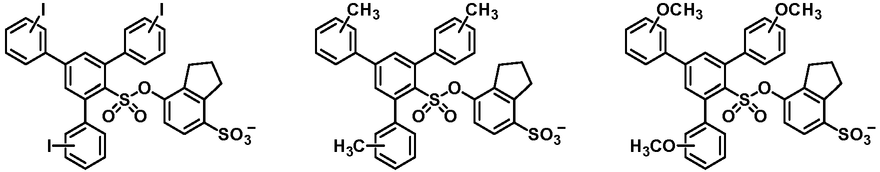

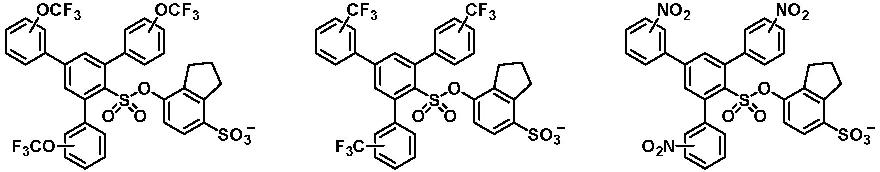

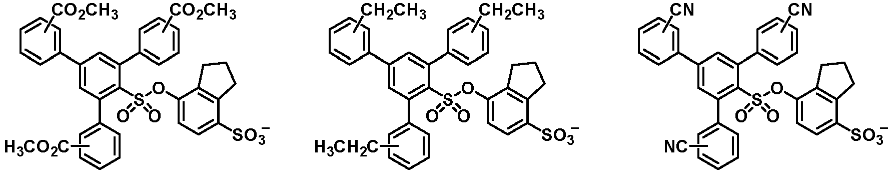

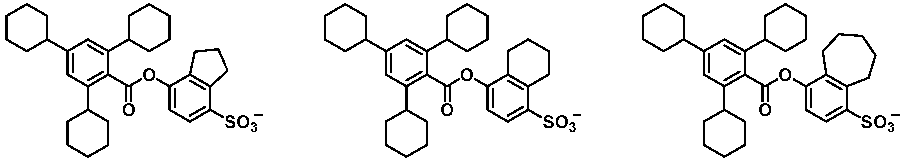

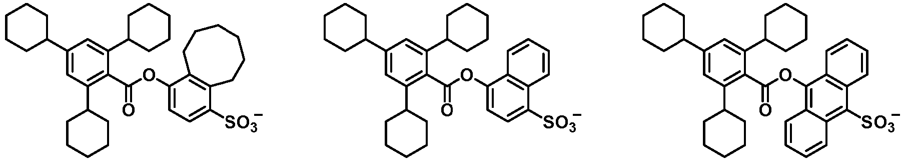

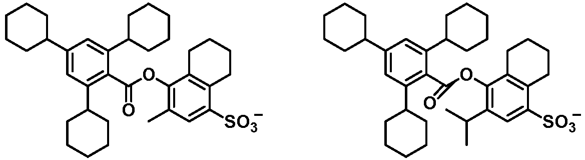

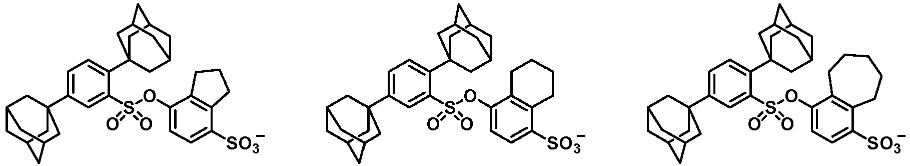

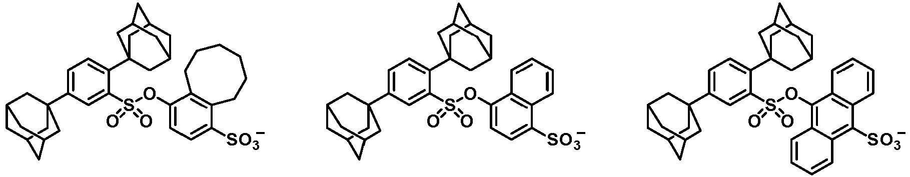

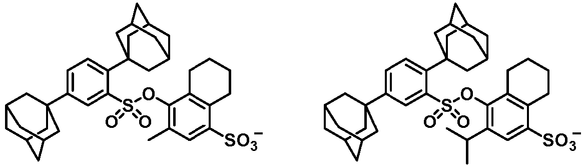

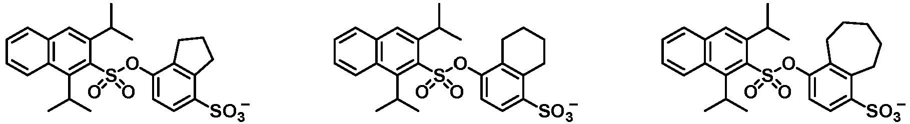

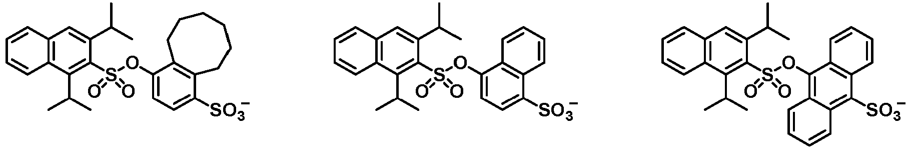

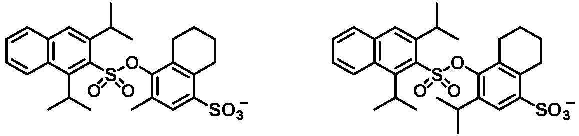

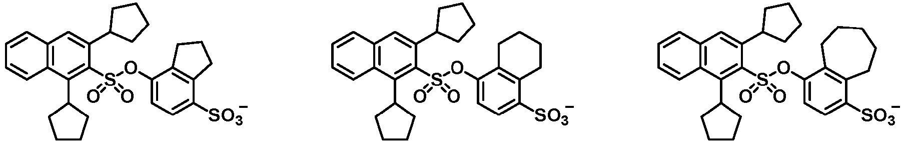

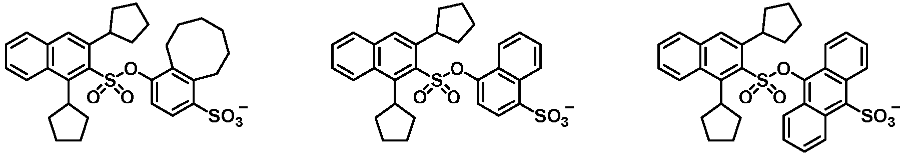

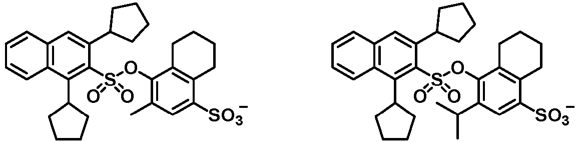

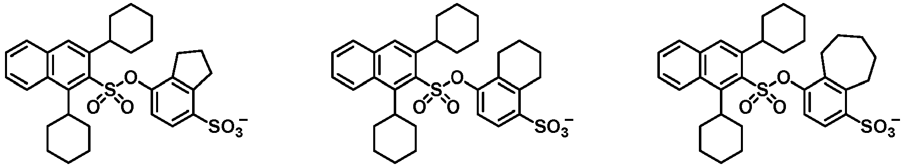

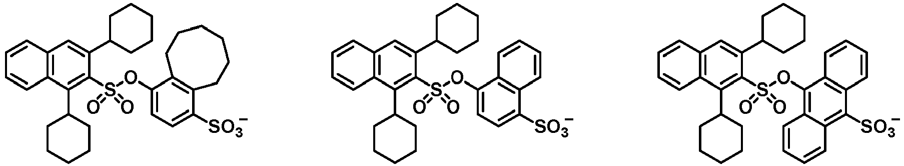

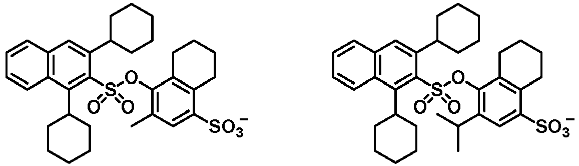

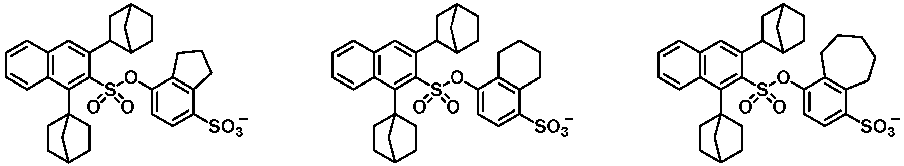

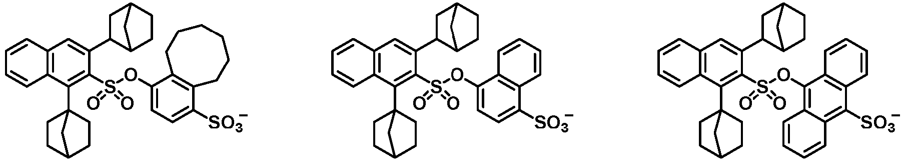

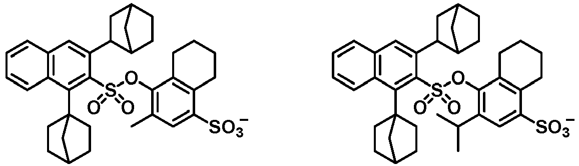

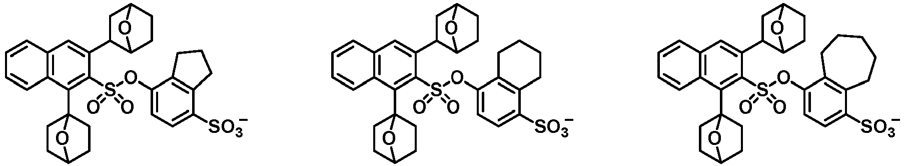

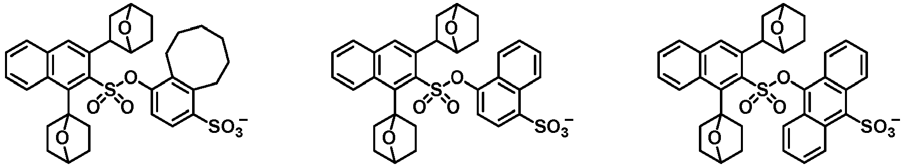

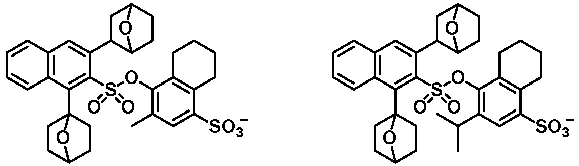

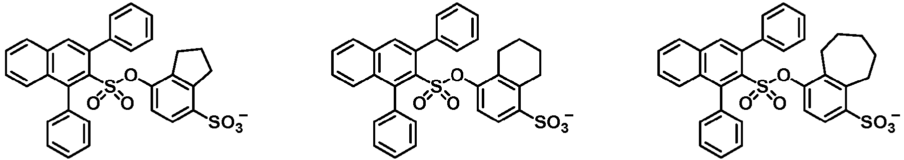

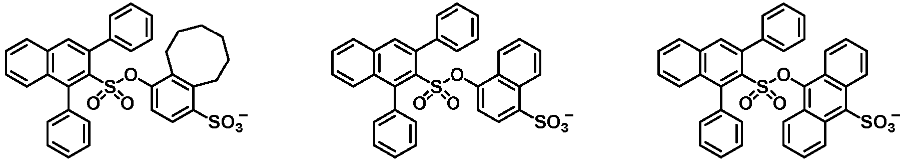

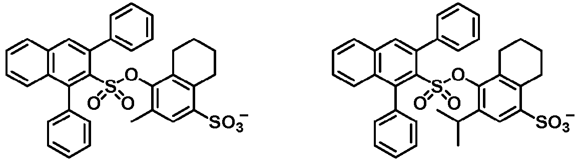

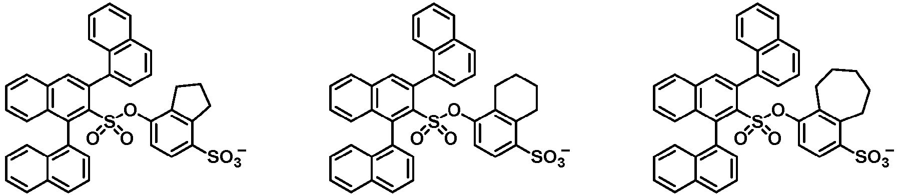

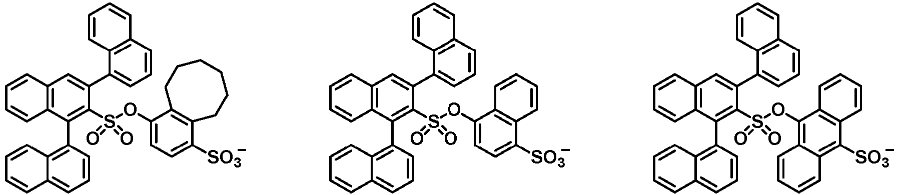

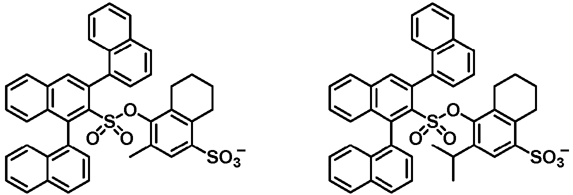

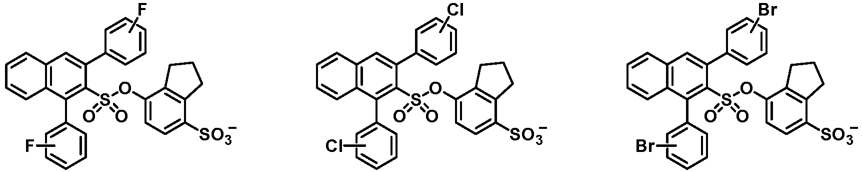

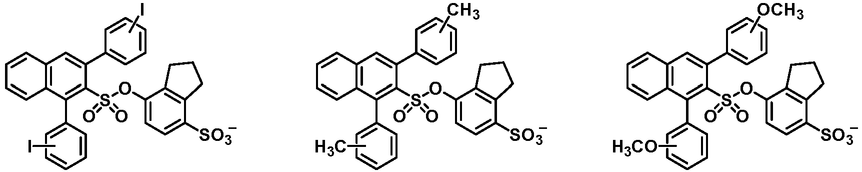

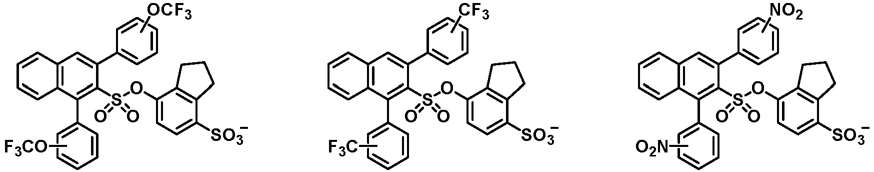

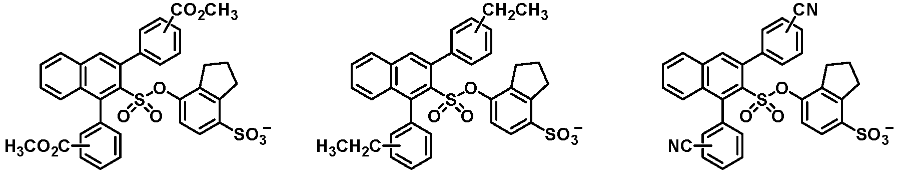

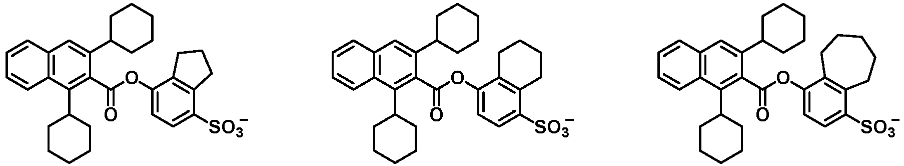

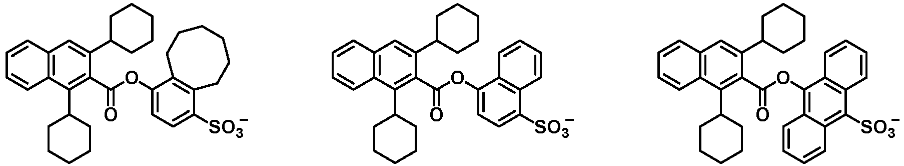

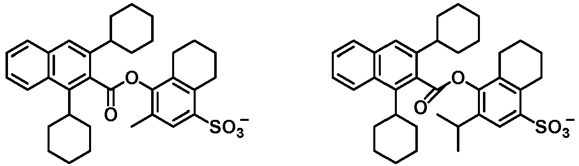

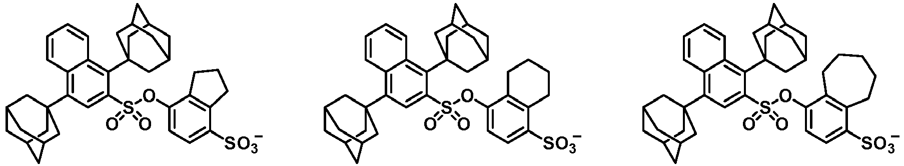

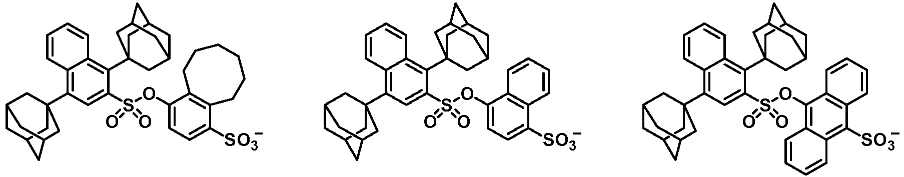

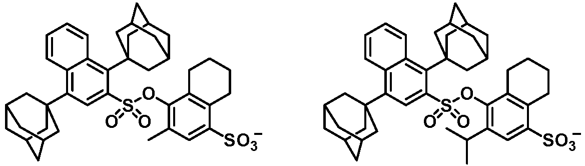

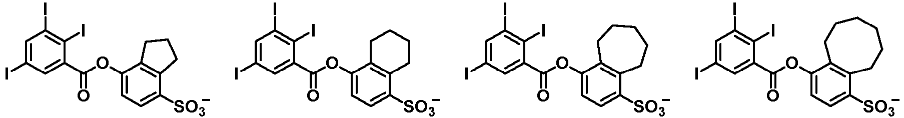

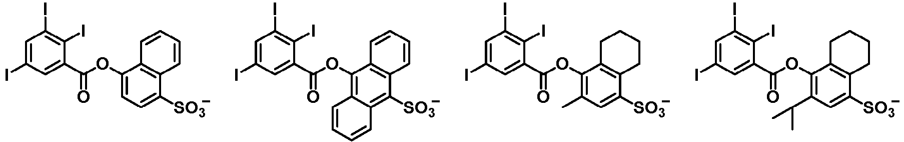

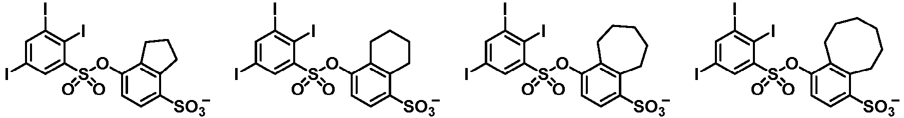

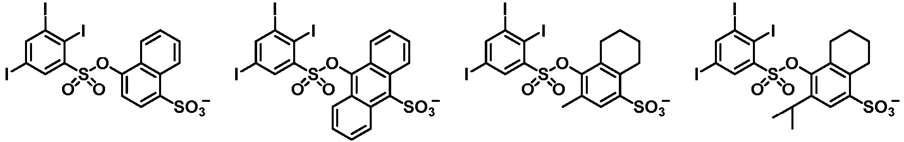

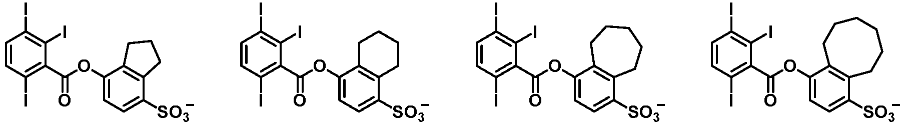

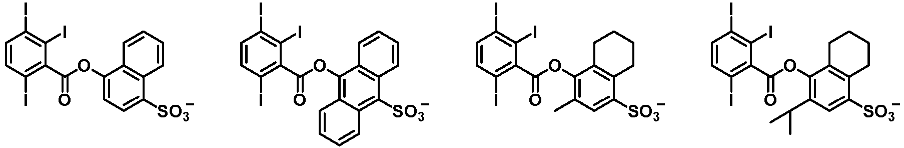

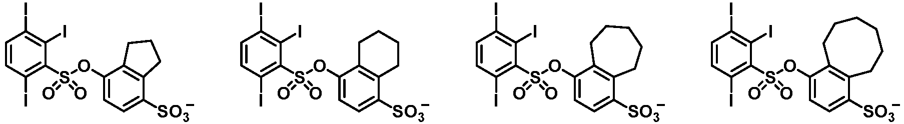

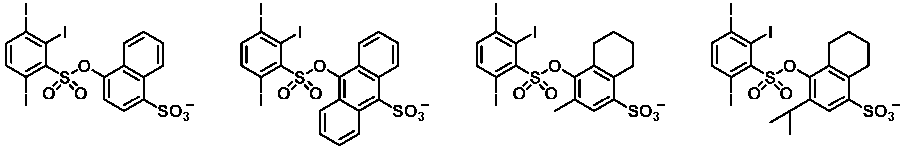

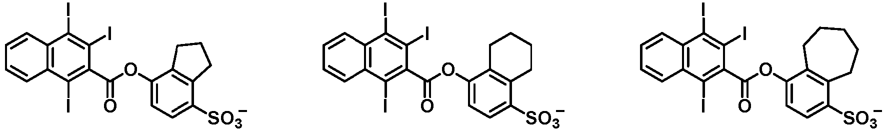

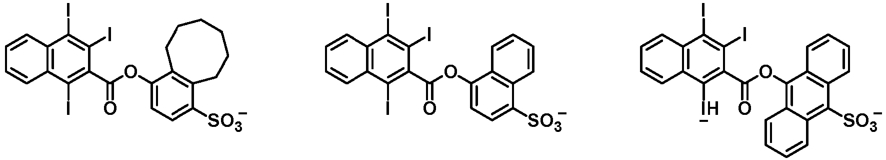

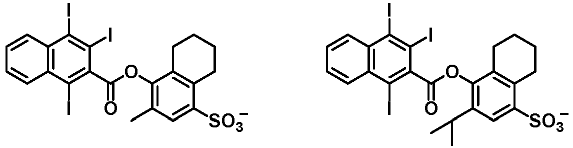

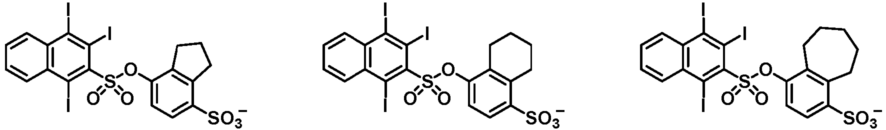

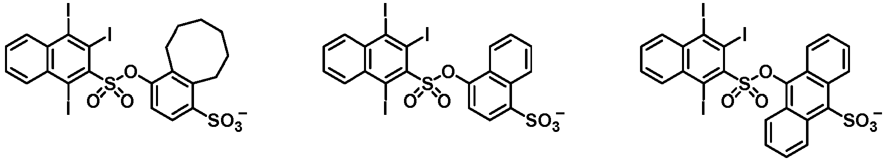

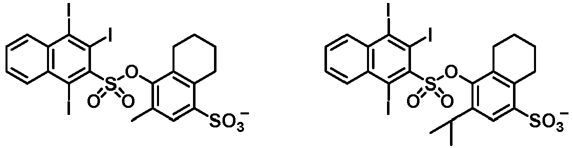

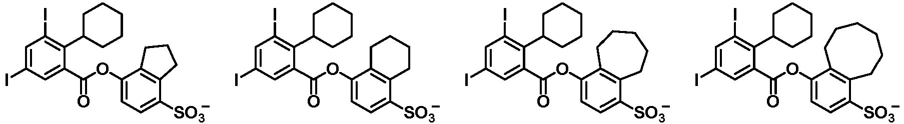

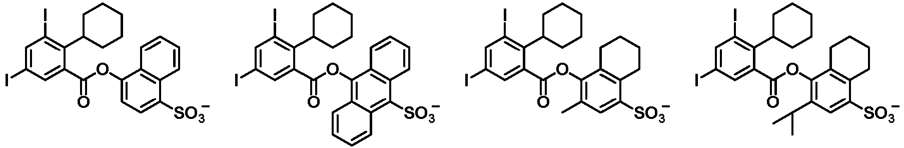

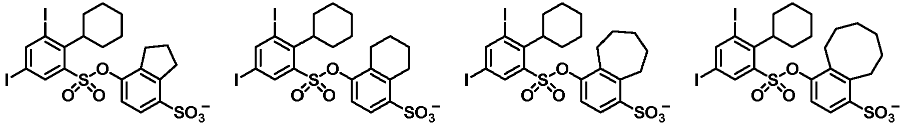

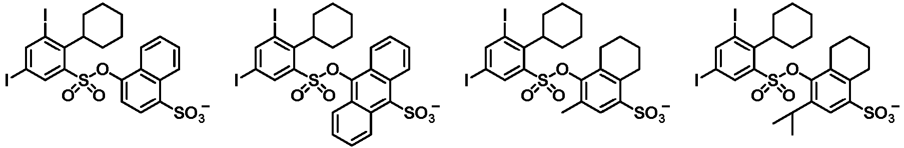

Since the onium salt containing an anion of formula (C-1) is an onium salt of a non-fluorinated sulfonic acid, it generates an acid having an adequate strength upon exposure to high-energy radiation. The onium salt is structurally characterized by having one ring structure fused to an aromatic ring attached to a sulfo group of an anion and another aromatic ring structure containing a bulky substituent. The steric hindrance of these two aromatic rings restrains the degree of freedom of rotation of the bond linking the aromatic rings, for thereby limiting excessive diffusion of the generated acid. In addition, since the onium salt is fully lipophilic, its preparation and handling are easy. Due to these effects, the acid featuring an adequate acid strength and controlled diffusion is generated in the resist film. Then a small-size pattern is formed with satisfactory resolution and reduced LER. When the positive resist composition is developed in an alkaline developer, a pattern of satisfactory rectangularity is obtainable by virtue of properly inhibited dissolution.

An onium salt compound containing an anion having the formula (C-2) is also preferred as the PAG (C).

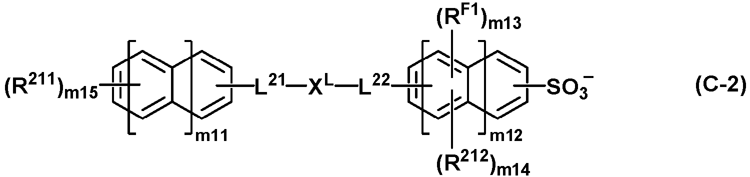

In formula (C-2), m11 and m12 are each independently an integer of 0 to 2, m13 is an integer of 1 to 4 in case of m12=0, an integer of 1 to 6 in case of m12=1, and an integer of 1 to 8 in case of m12=2.

In formula (C-2), m14 is an integer of 0 to 3 in case of m12=0, an integer of 0 to 5 in case of m12=1, and an integer of 0 to 7 in case of m12=2, m13+m14 is from 1 to 4 in case of m12=0, from 1 to 6 in case of m12=1, and from 1 to 8 in case of m12=2.

In formula (C-2), m15 is an integer of 1 to 5 in case of m11=0, an integer of 1 to 7 in case of m11=1, and an integer of 1 to 9 in case of m11=2.

In formula (C-2), R²¹¹ is each independently iodine or a C₃-C₂₀ branched or cyclic hydrocarbyl group which may contain a heteroatom, at least one R²¹¹ is attached to a carbon atom adjoining the carbon atom to which L²¹ is attached. Examples of the hydrocarbyl group include, but are not limited to, branched alkyl groups such as isopropyl, sec-butyl, tert-butyl, tert-pentyl, and 2-ethylhexyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, oxanorbornyl, tricyclo[5.2.1.0^{2,6}]decyl, and adamantyl; aryl groups such as phenyl, naphthyl and anthracenyl; and combinations thereof. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, halogen, carbonyl moiety, ether bond, ester bond, sulfonate ester bond, carbonate bond, lactone ring, sultone ring, carboxylic anhydride

(-C(=O)-O-C(=O)-) or haloalkyl moiety. Suitable halogen atoms include fluorine, chlorine, bromine and iodine, with fluorine and iodine being preferred. A plurality of R²¹¹ may be identical or different when m15 is 2 or more.

A plurality of R²¹¹ may bond together to form a ring with the carbon atoms to which they are attached when m15 is 2 or more. Preferred is a 5 to 8-membered ring.

Examples of the structure formed by bonding R²¹¹ to the aromatic ring in formula (C-2) are shown below, but not limited thereto. The broken line designates a point of attachment to L²¹.

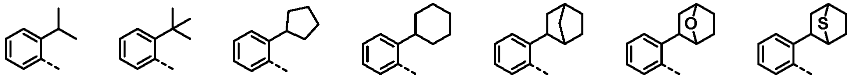

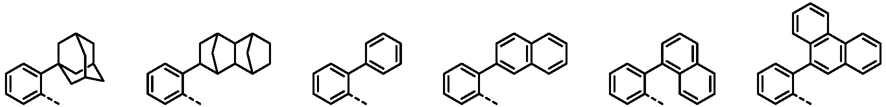

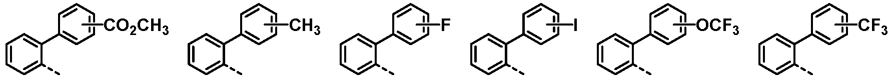

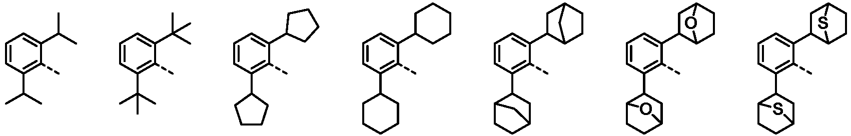

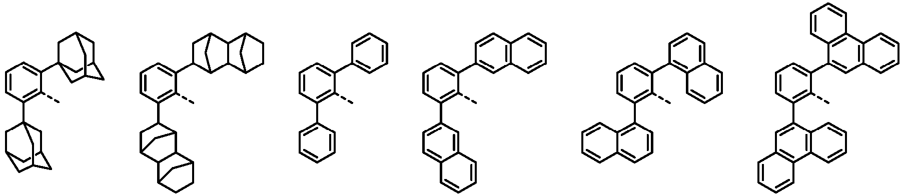

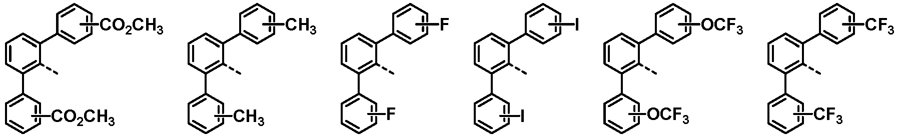

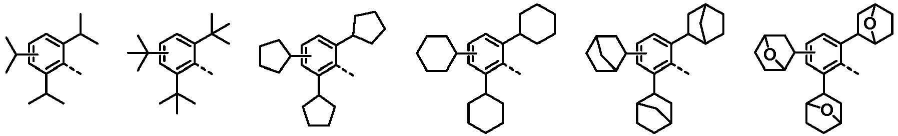

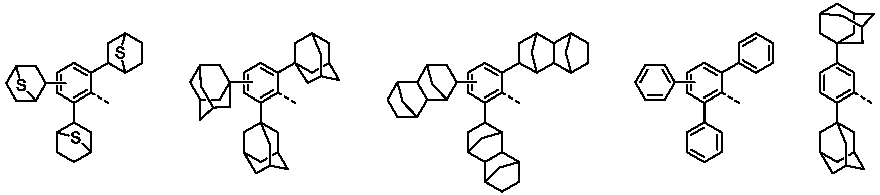

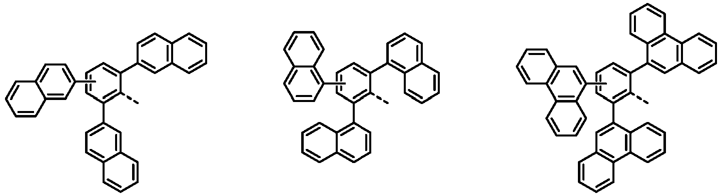

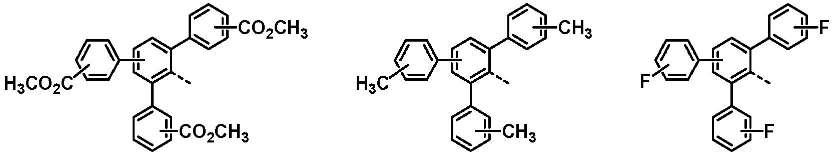

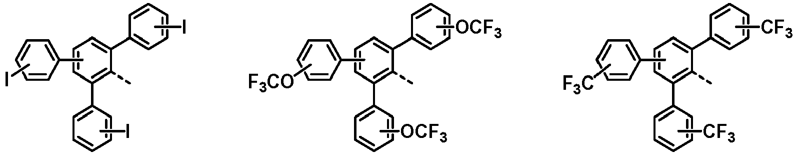

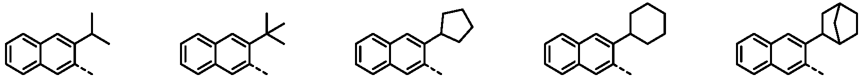

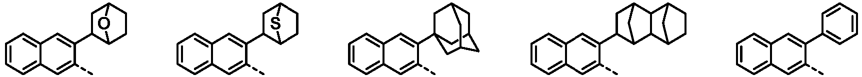

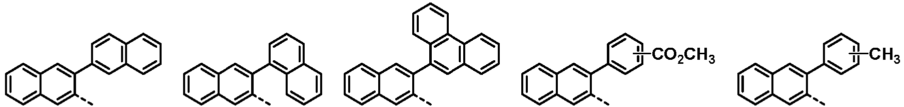

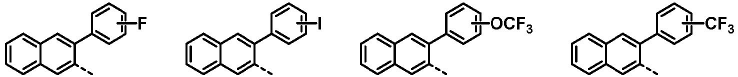

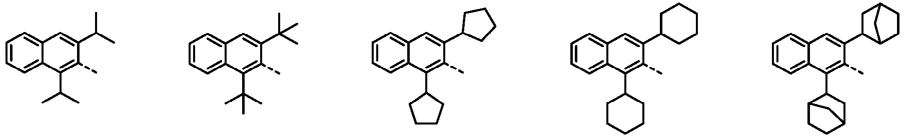

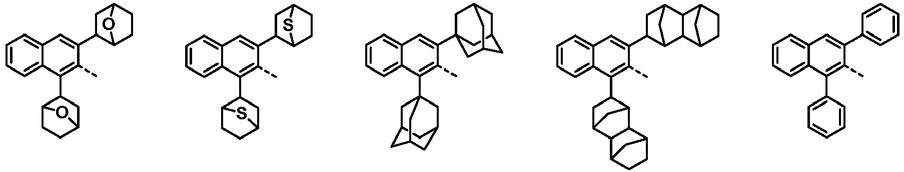

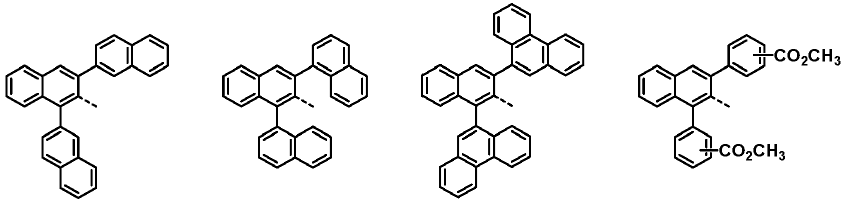

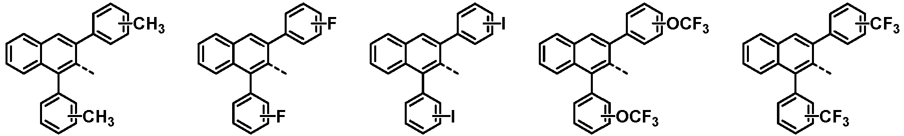

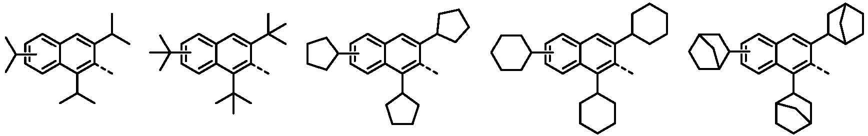

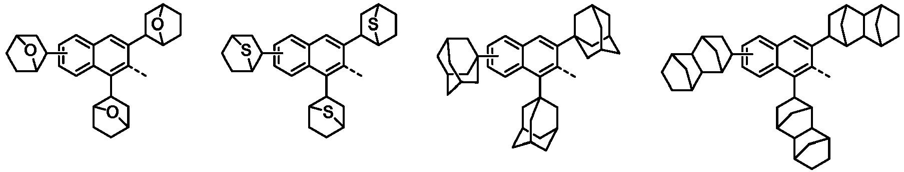

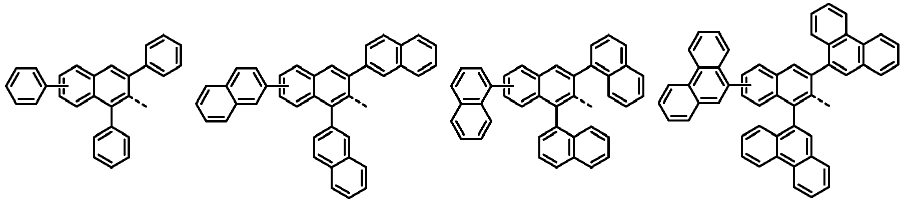

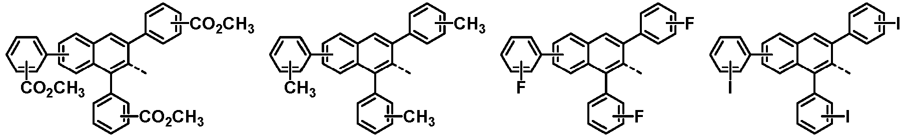

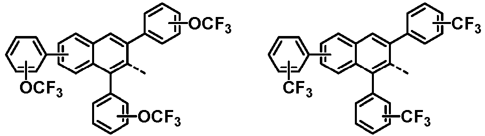

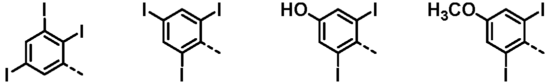

In formula (C-2), R²⁰² is each independently a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, tert-butyl, n-pentyl, tert-pentyl, n-hexyl, n-octyl, 2-ethylhexyl, n-nonyl, and n-decyl; C₃-C₂₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; and C₆-C₂₀ aryl groups such as phenyl, naphthyl and anthracenyl. In the hydrocarbyl group, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, halogen, carbonyl moiety, ether bond, ester bond, sulfonate ester bond, carbonate bond, lactone ring, sultone ring, carboxylic anhydride (-C(=O)-O-C(=O)-) or haloalkyl moiety. Suitable halogen atoms include fluorine, chlorine, bromine, and iodine, with fluorine and iodine being preferred. A plurality of R²⁰² may be identical or different when m14 is 2 or more.

A plurality of R²⁰² may bond together to form a ring with the carbon atoms to which they are attached when m14 is 2 or more. Preferred is a 5 to 8-membered ring.

In formula (C-2), R^{F1} is each independently fluorine, a C₁-C₆ fluorinated alkyl group, C₁-C₆ fluorinated alkoxy group, or C₁-C₆ fluorinated thioalkoxy group. R^{F1} is preferably fluorine, trifluoromethyl, difluoromethyl, trifluoromethoxy, difluoromethoxy, trifluoromethylthio or difluoromethylthio, more preferably fluorine, trifluoromethyl or trifluoromethoxy. Because of fluorine included, the generated acid has a sufficiently high acid strength for deprotection reaction to take place smoothly. A plurality of R^{F1} may be identical or different when m13 is 2 or more.

In formula (C-2), L²¹ and L²² are each independently a single bond, ether bond, ester bond, amide bond, sulfonate ester bond, carbonate bond or carbamate bond. Inter alia, ether bond, ester bond or sulfonate ester bond is preferred, with ether bond or sulfonate ester bond being more preferred.

In formula (C-2), X^{L} is a single bond or a C₁-C₄₀ hydrocarbylene group which may contain a heteroatom. The hydrocarbylene group may be straight, branched or cyclic and examples thereof include alkanediyl and cyclic saturated hydrocarbylene groups. Suitable heteroatoms include oxygen, nitrogen and sulfur atoms.

Examples of the optionally heteroatom-containing C₁-C₄₀ hydrocarbylene group X^{L} are shown below, but not limited thereto. Herein, * designates a point of attachment to L²¹ or L²².

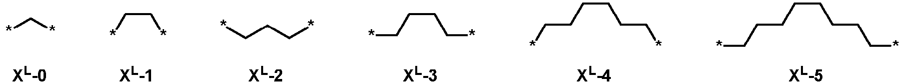

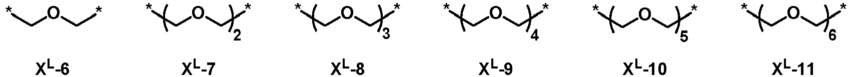

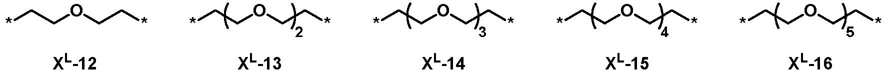

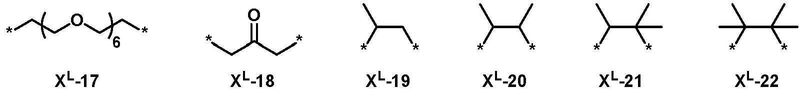

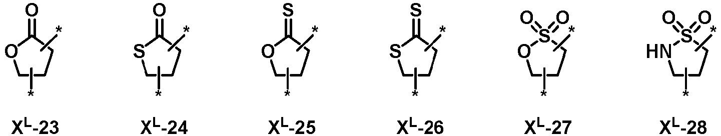

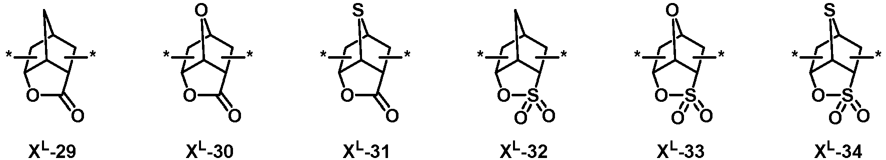

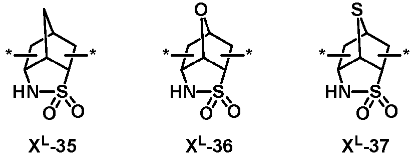

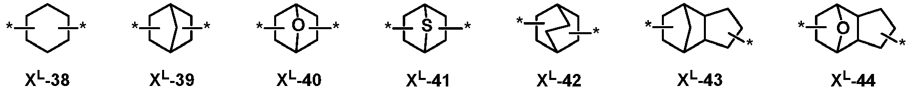

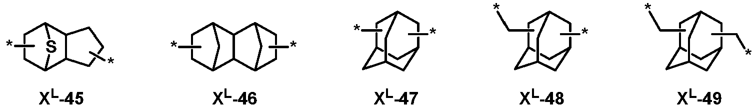

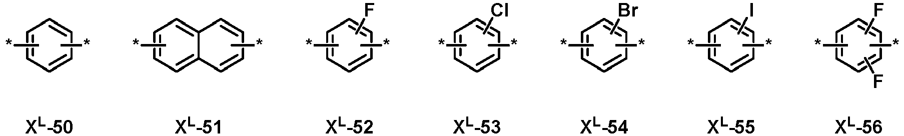

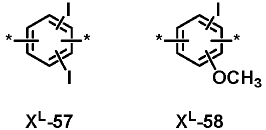

Of these, X^{L}-0 to X^{L}-22 and X^{L}-47 to X^{L}-58 are preferred.

Of the acid generators having formula (C-2), compounds having the formula (C-2-1) are preferred. 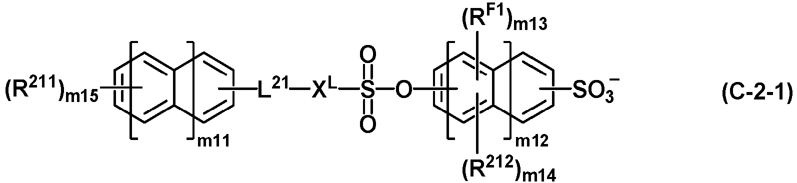 Herein m11 to m15, R²¹¹, R²¹², R^{F1}, L²¹, and X^{L} are as defined above.

Examples of the anion having formula (C-2) are shown below, but not limited thereto.

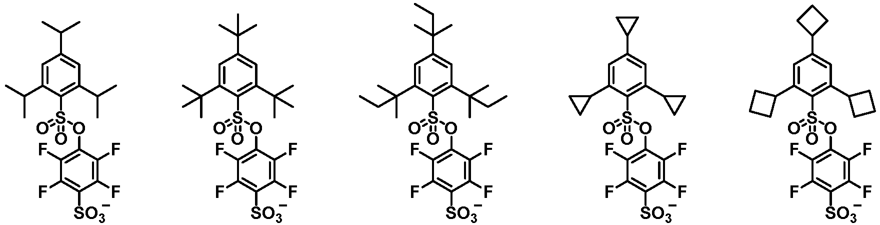

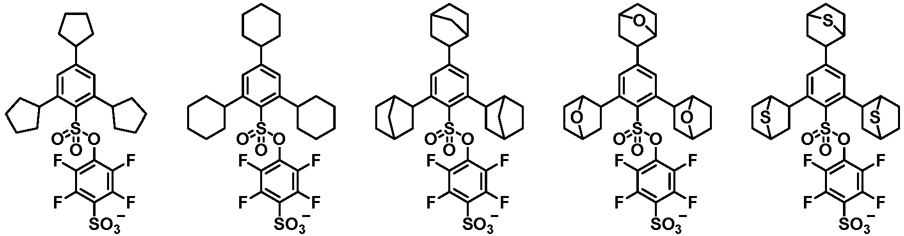

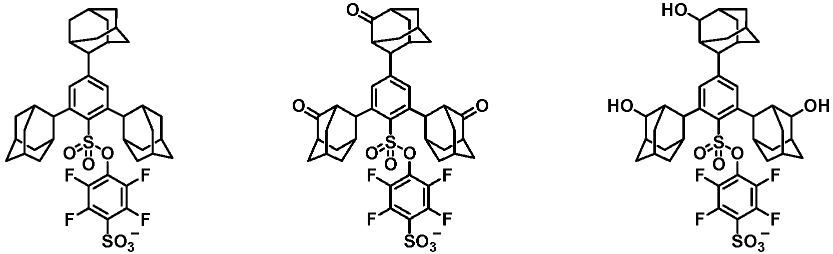

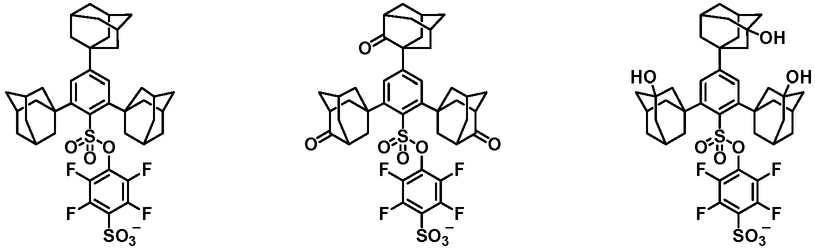

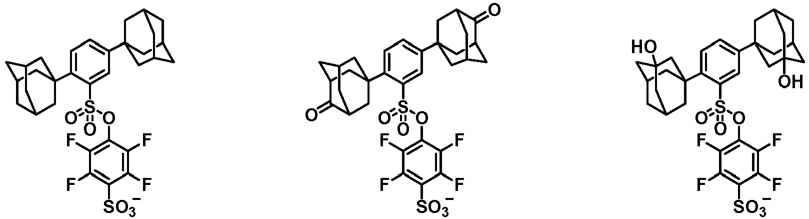

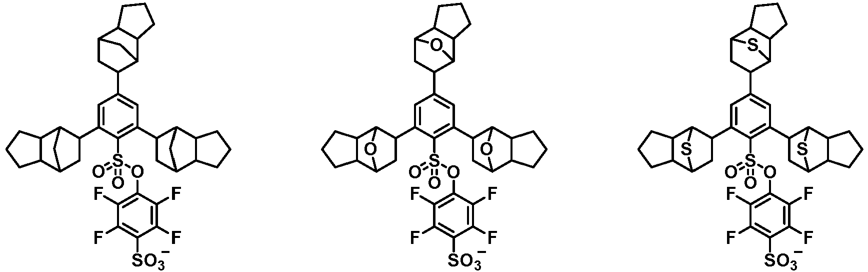

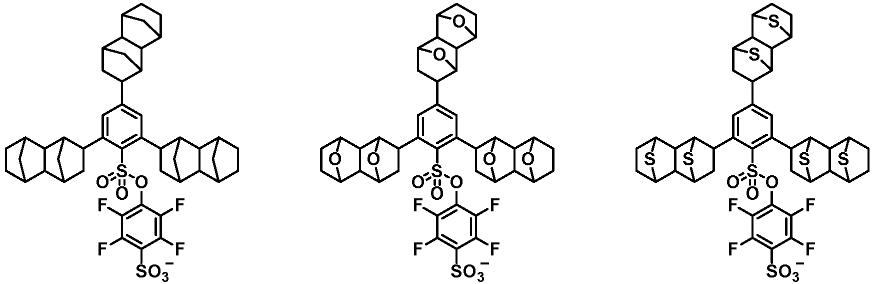

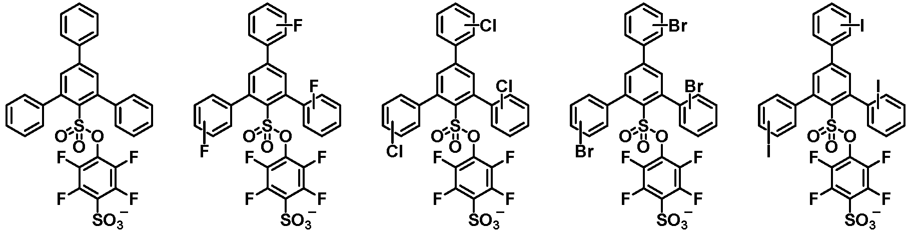

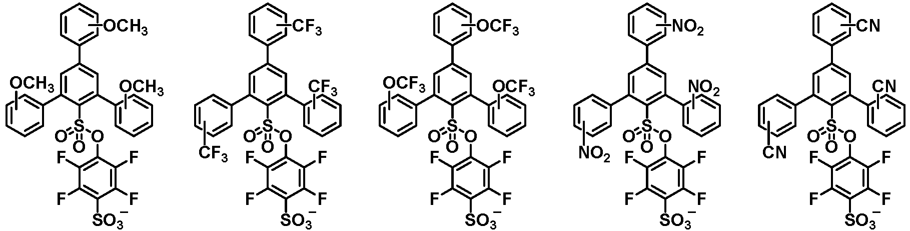

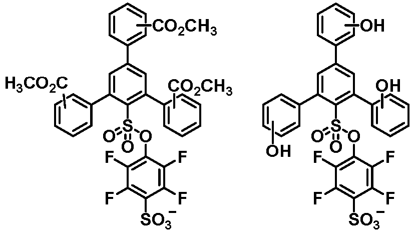

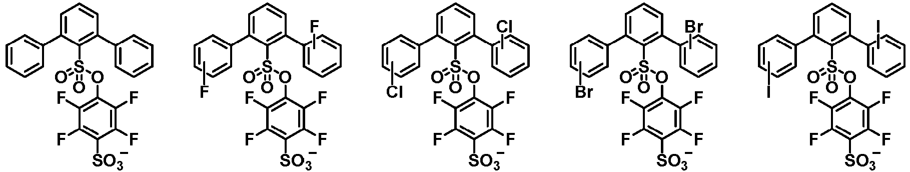

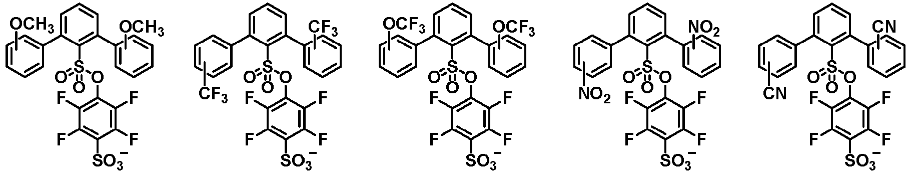

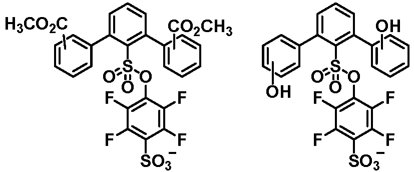

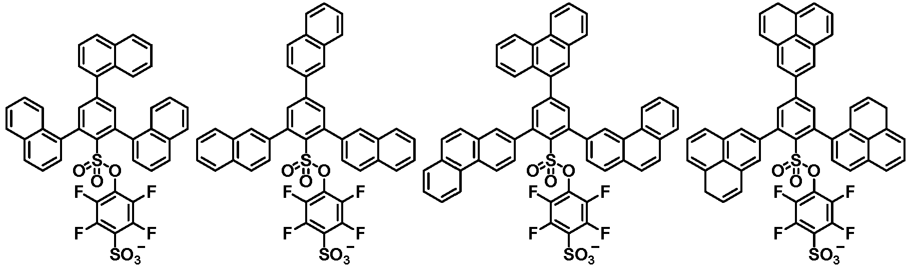

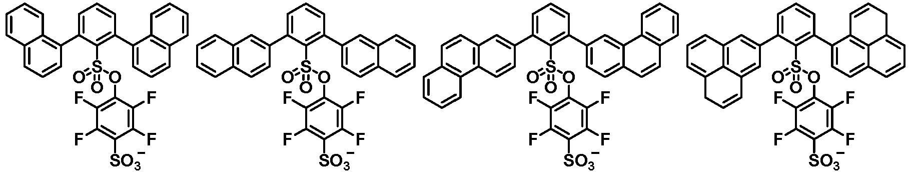

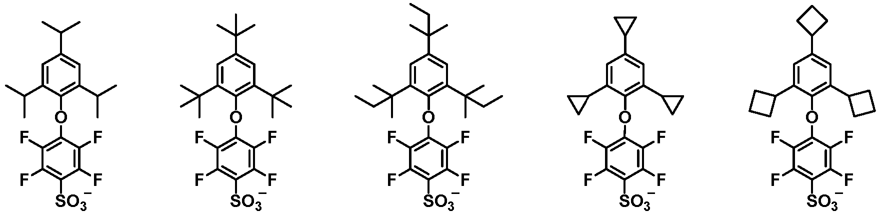

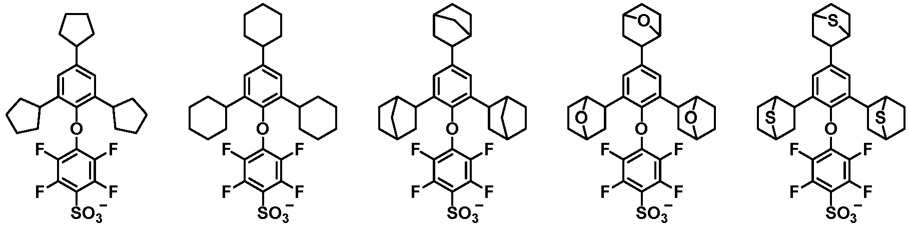

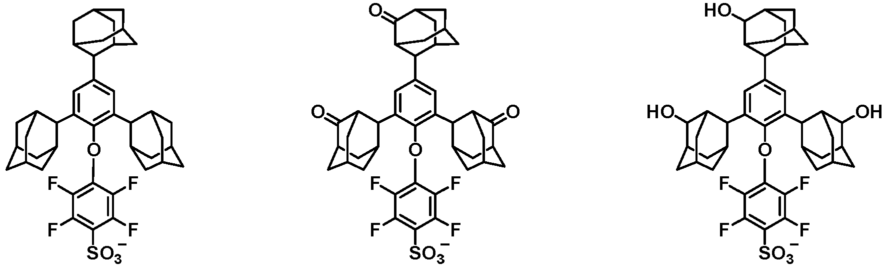

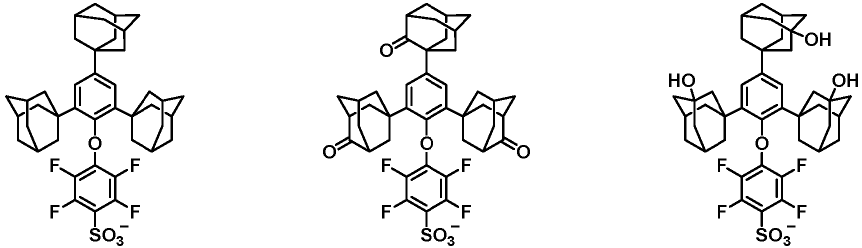

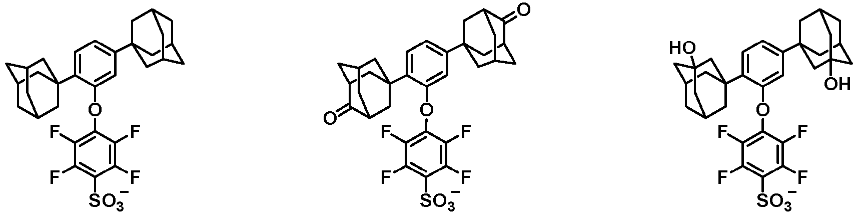

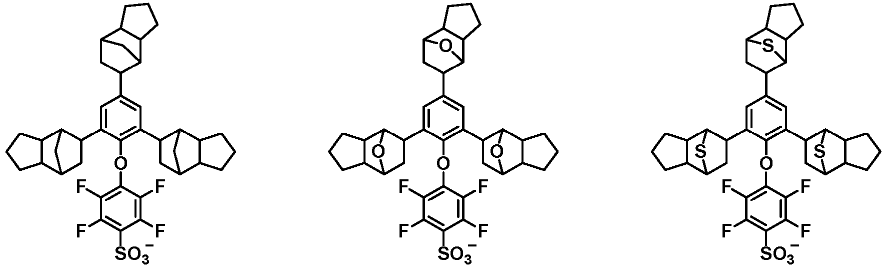

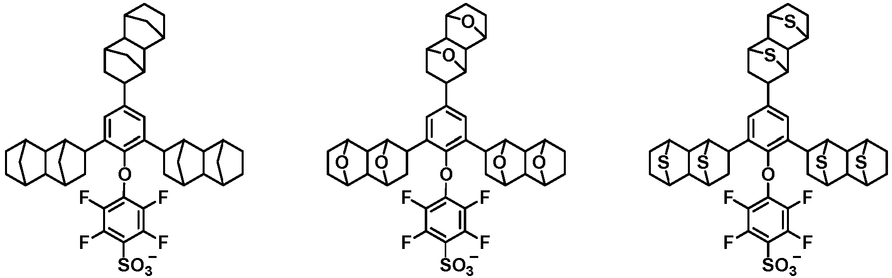

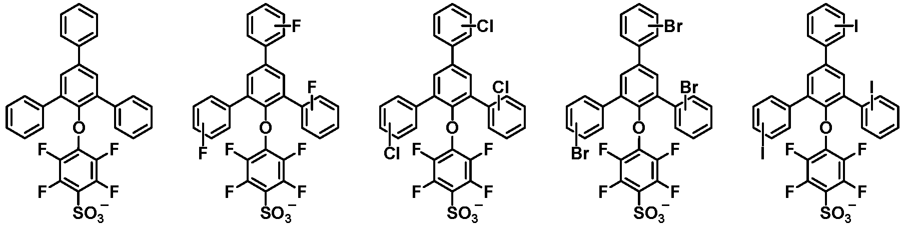

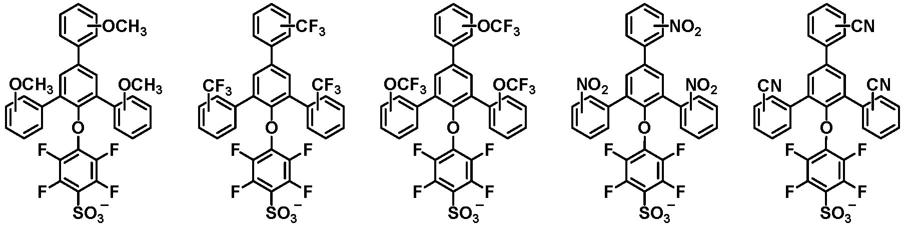

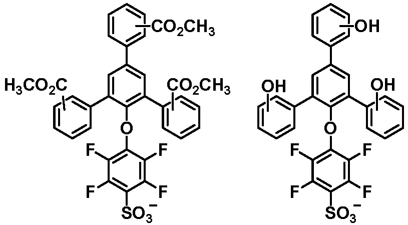

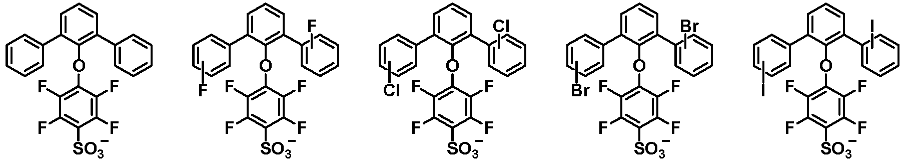

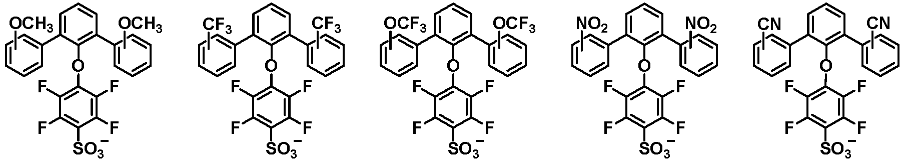

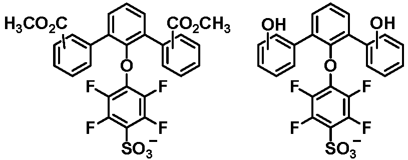

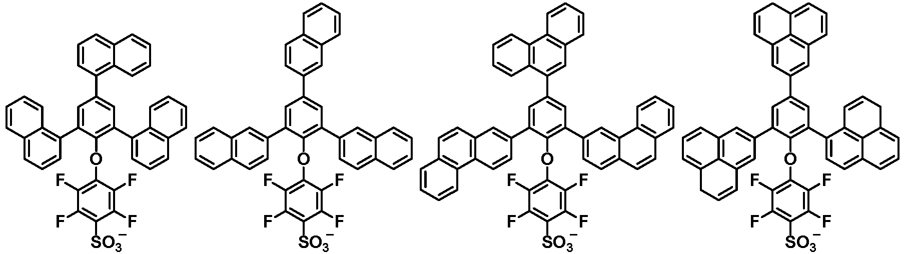

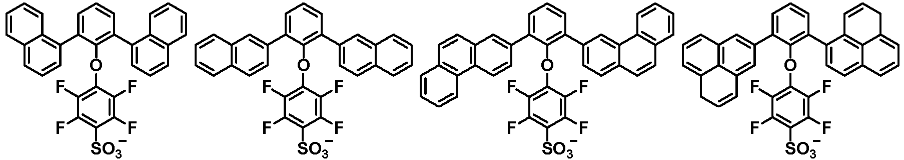

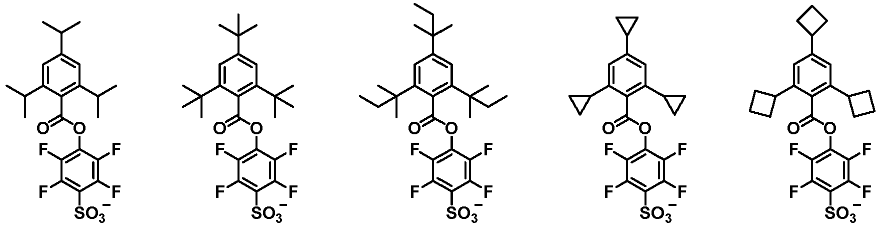

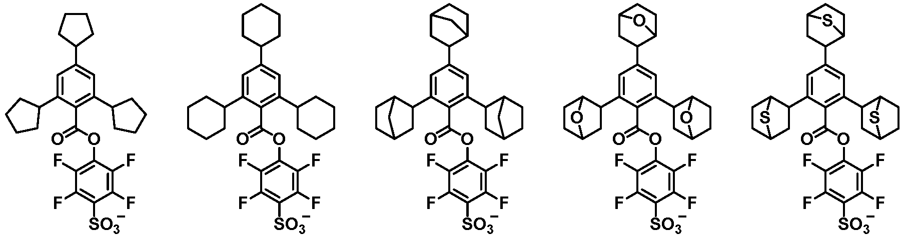

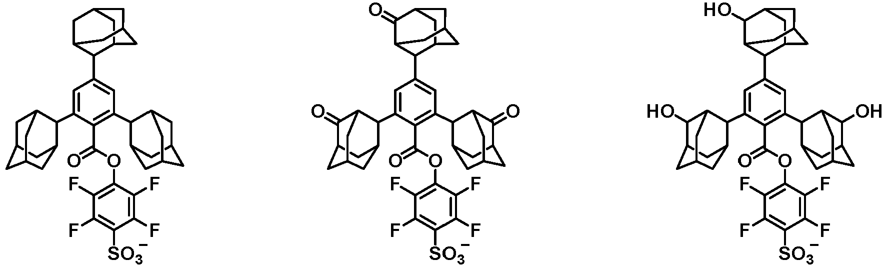

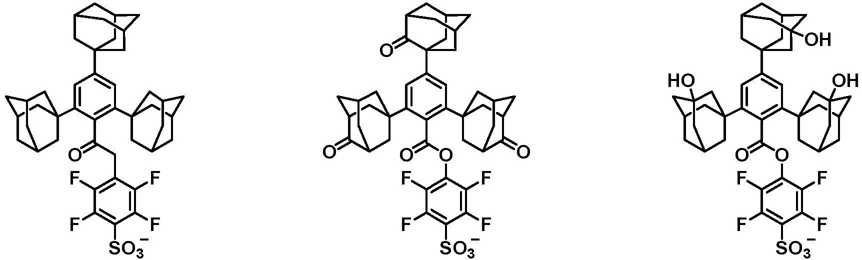

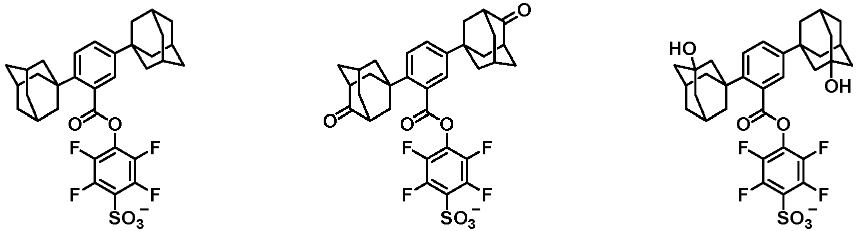

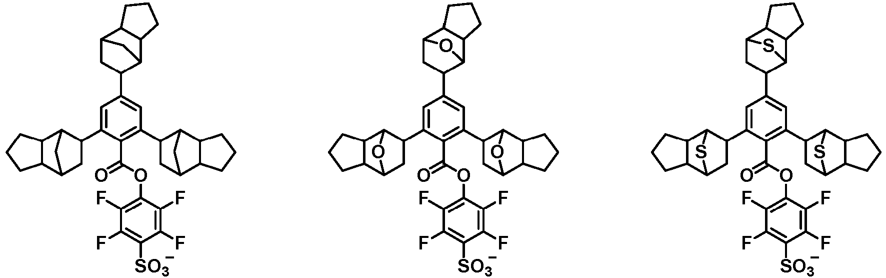

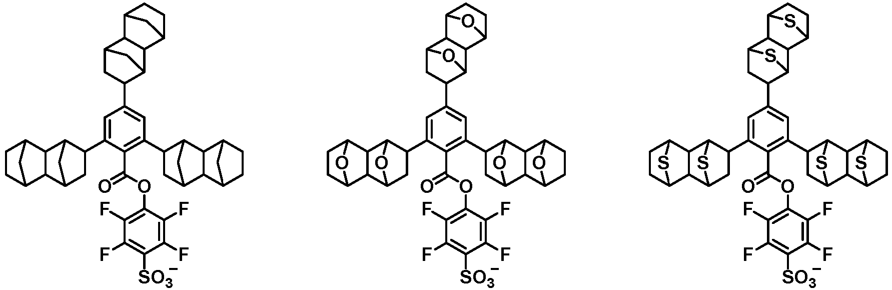

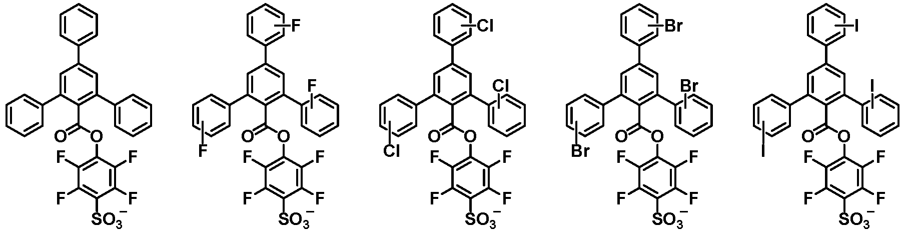

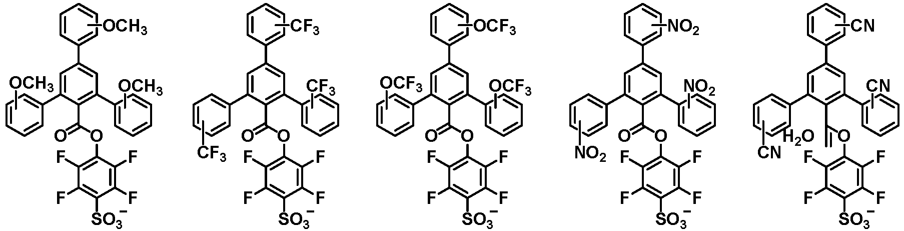

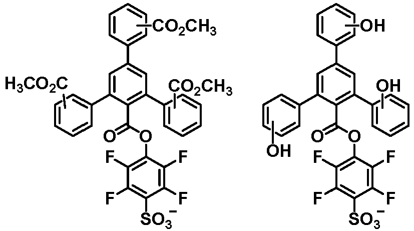

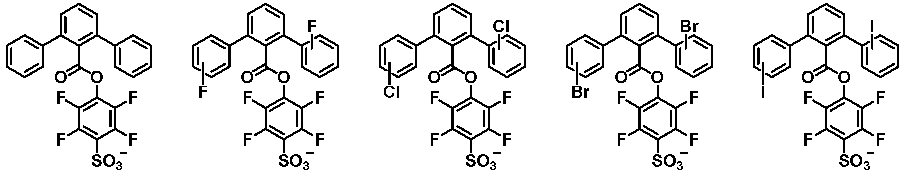

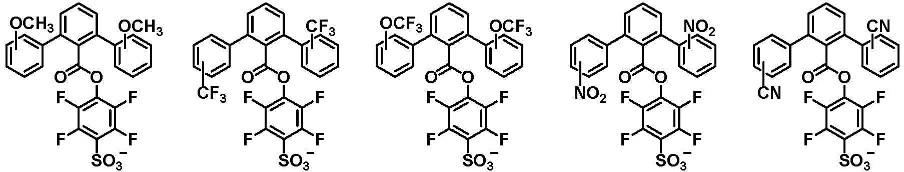

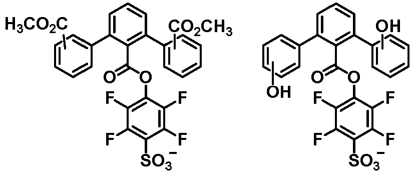

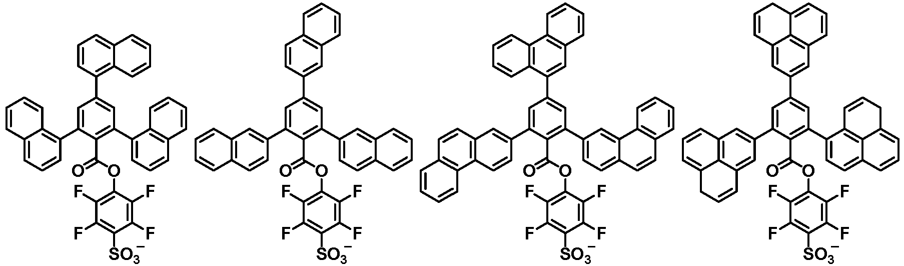

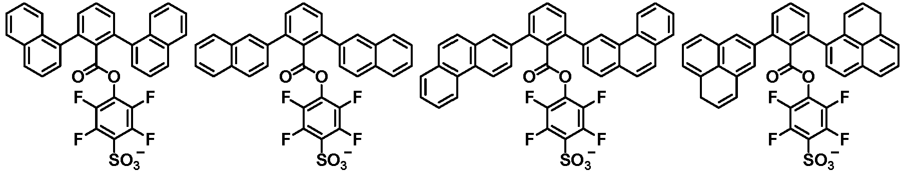

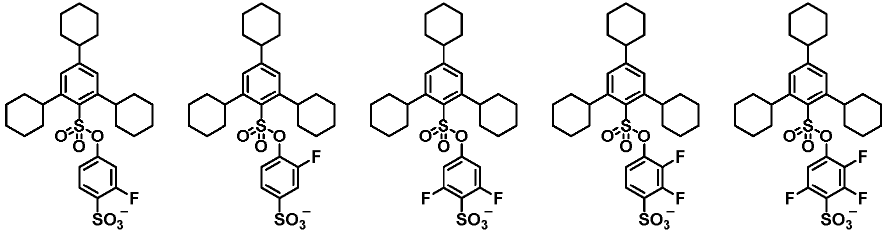

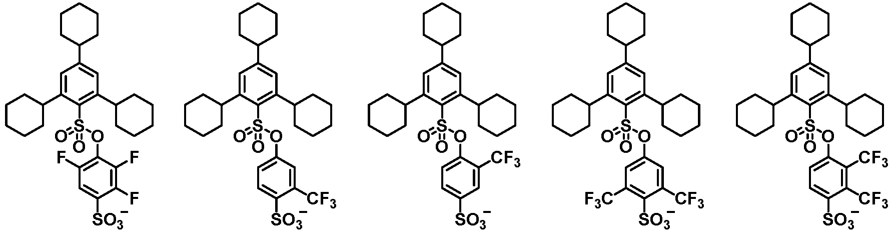

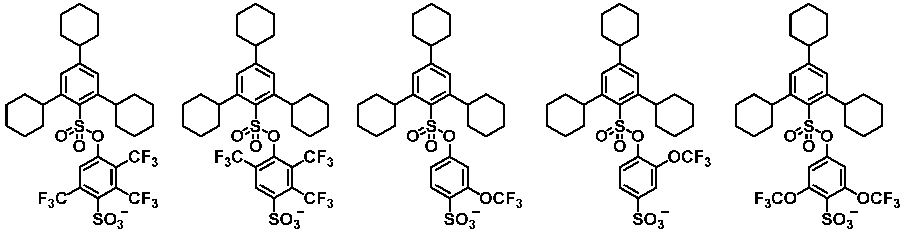

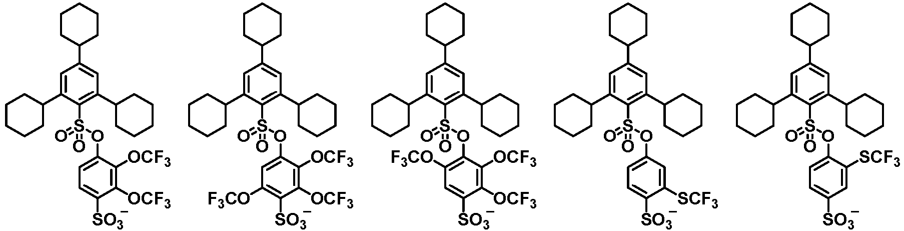

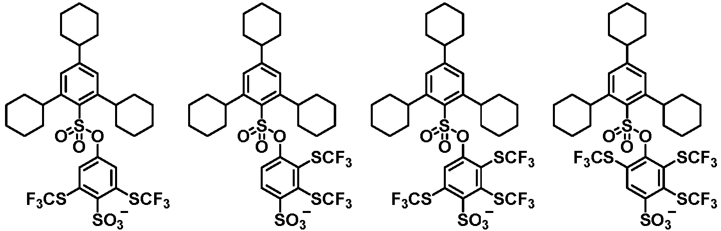

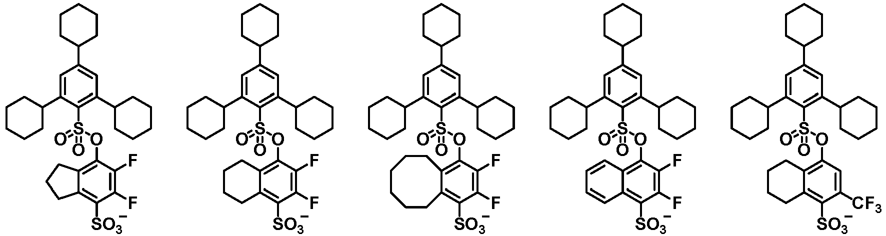

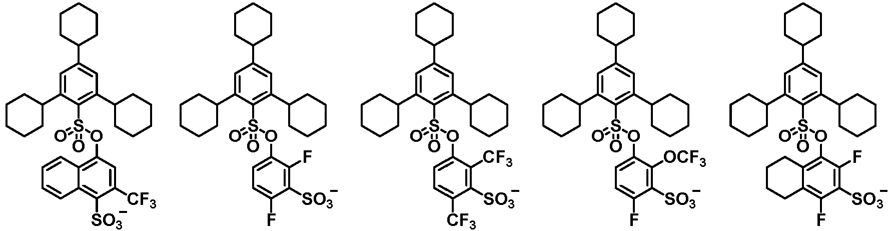

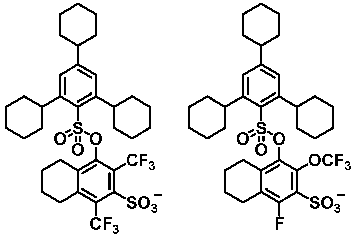

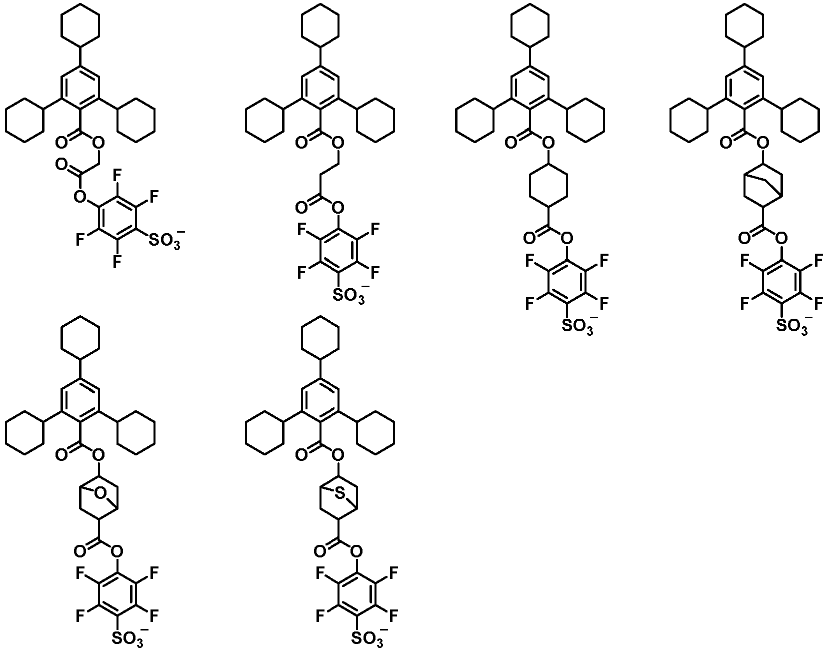

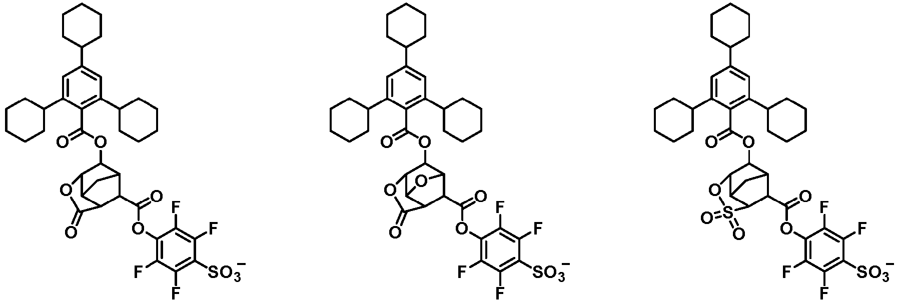

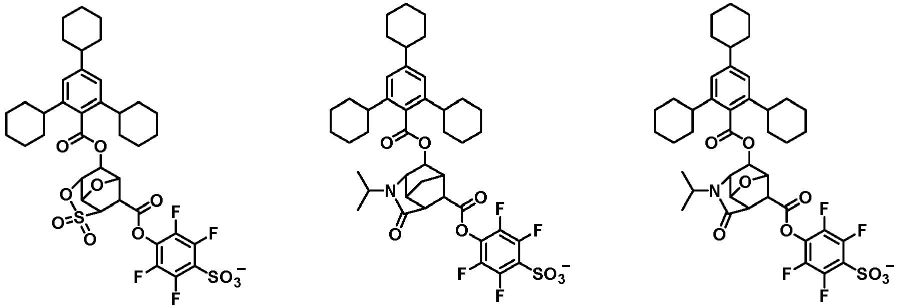

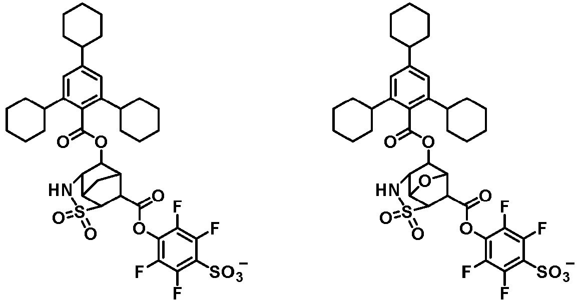

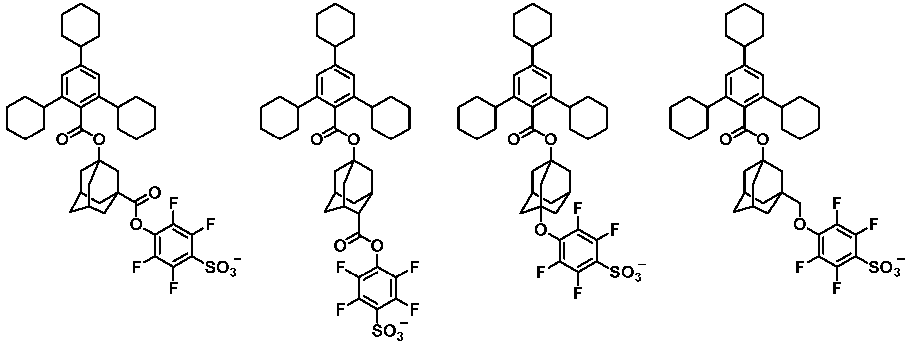

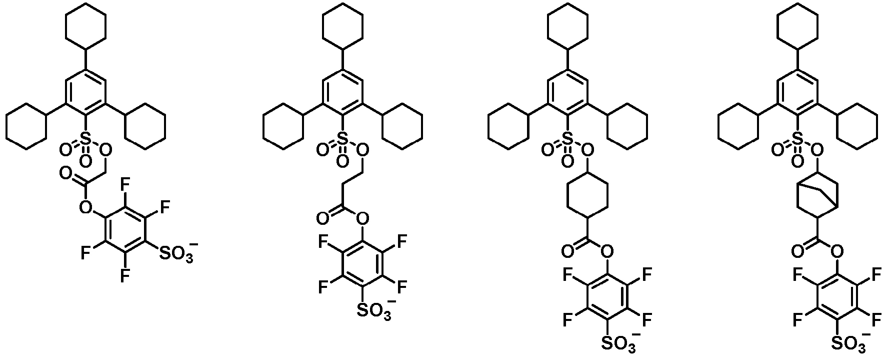

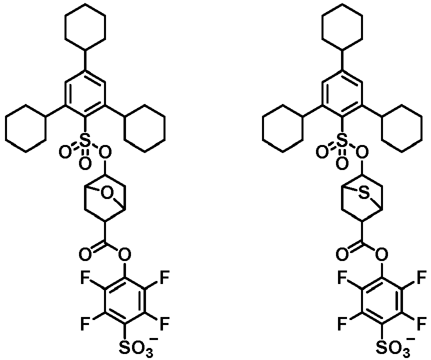

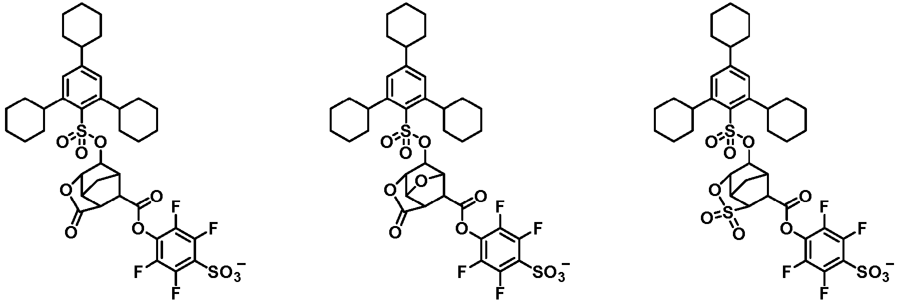

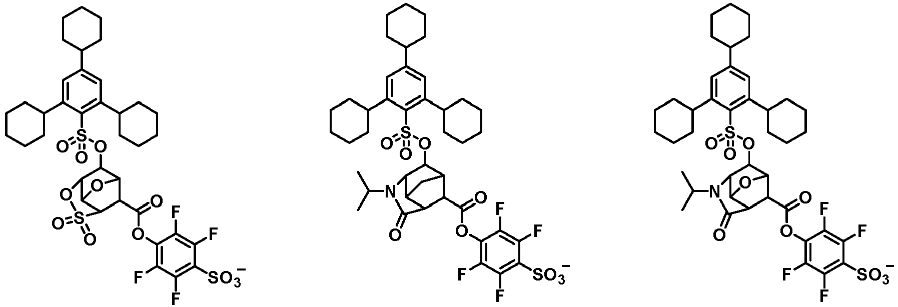

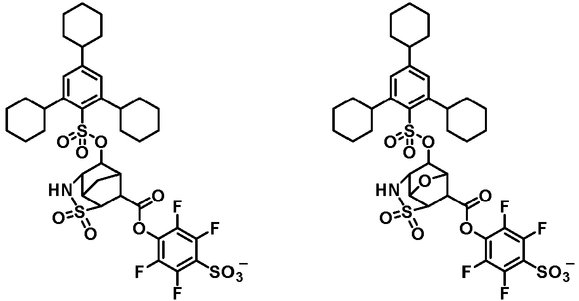

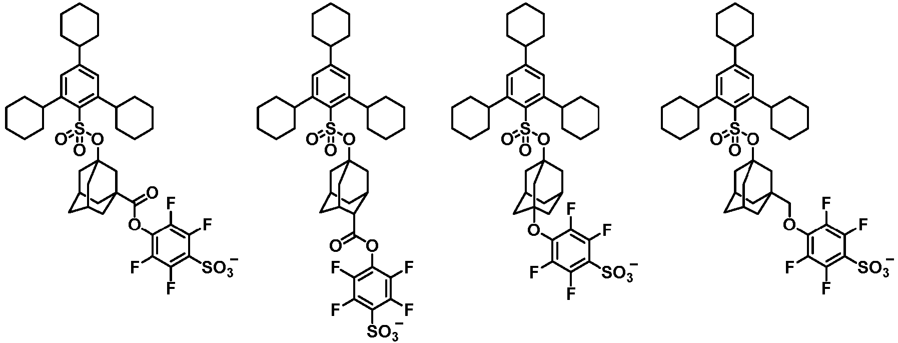

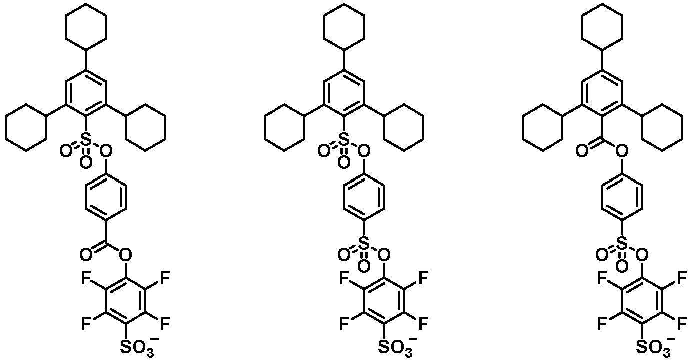

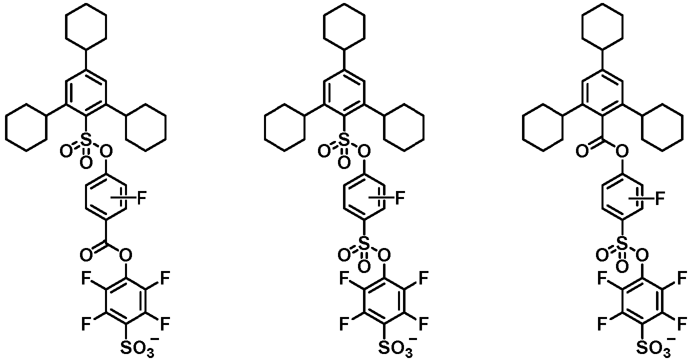

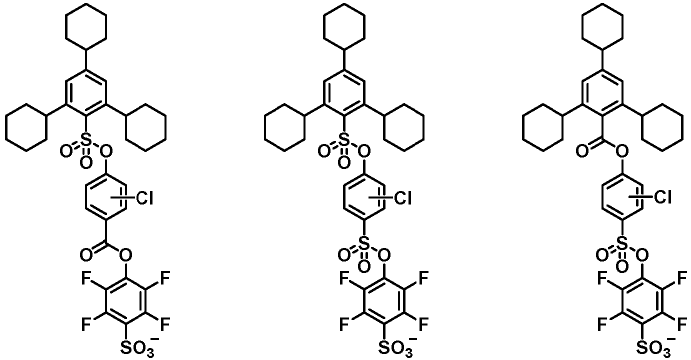

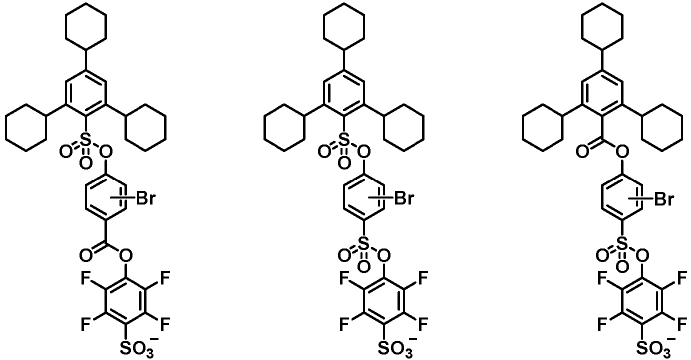

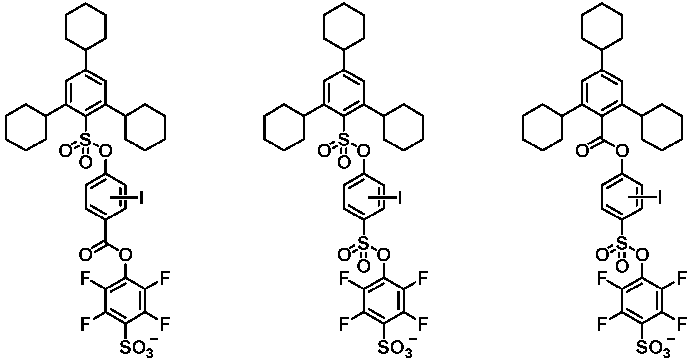

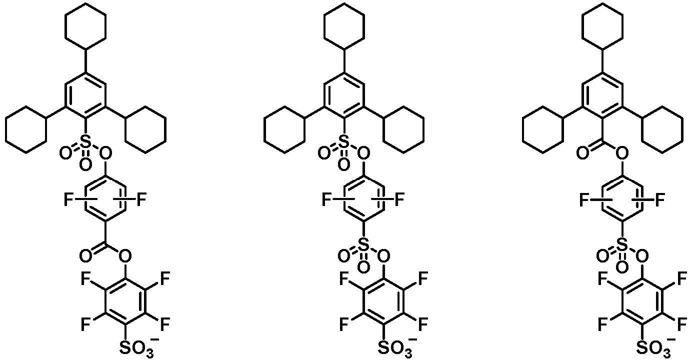

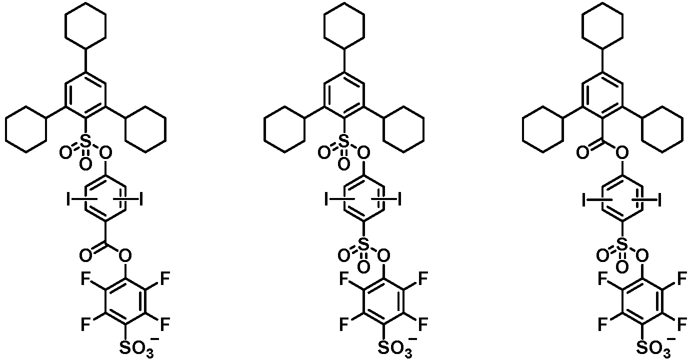

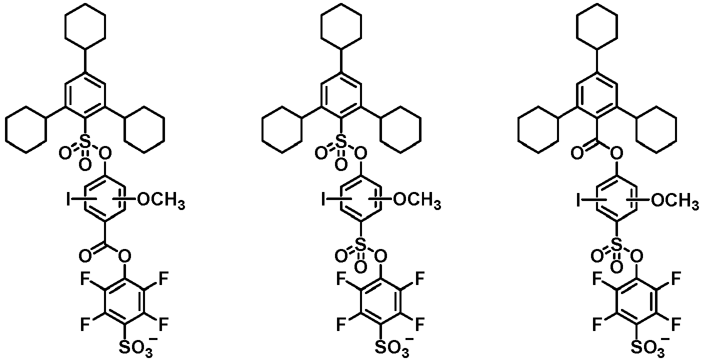

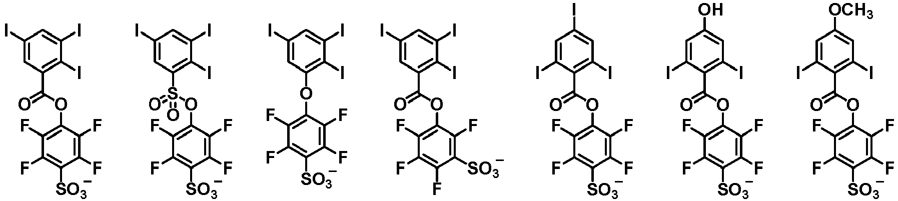

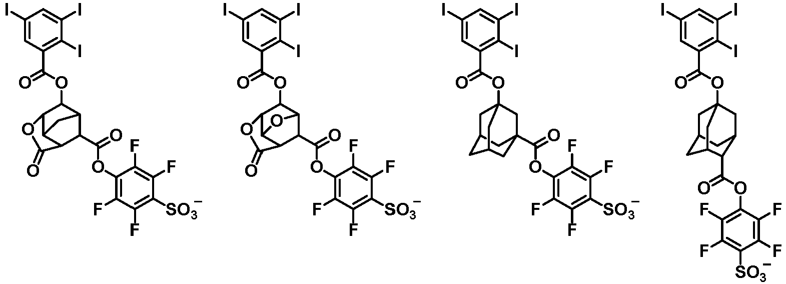

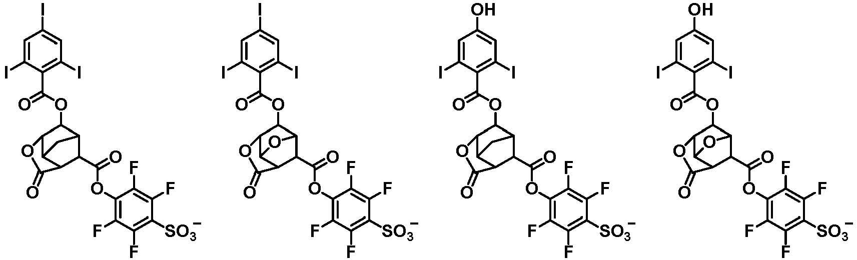

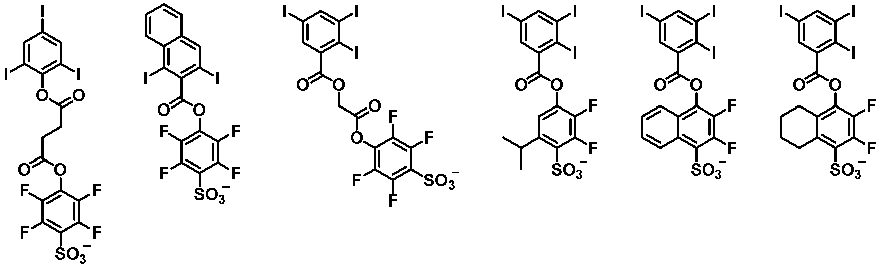

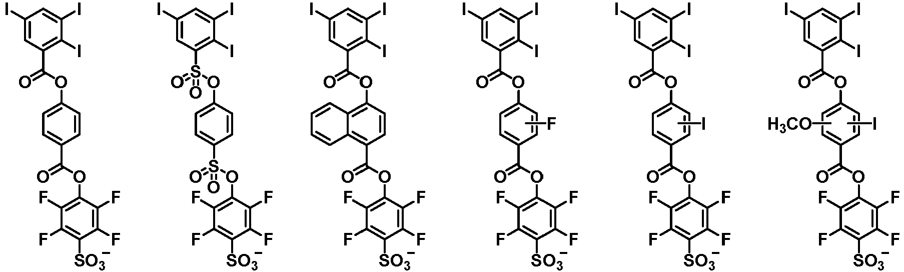

An onium salt compound containing an anion having the formula (C-3) is also preferred as the PAG (C).

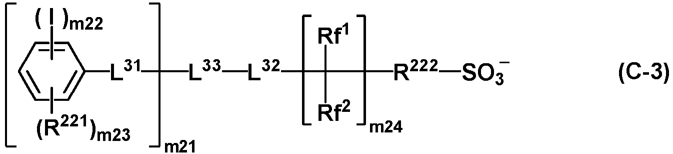

In formula (C-3), m21 is 1, 2 or 3, m22 is an integer of 1 to 5, m23 is an integer of 0 to 3, and m24 is 0 or 1.

In formula (C-3), L³¹ is a single bond, ether bond, ester bond, sulfonate ester bond, carbonate bond or carbamate bond.

In formula (C-3), L³² is an ether bond, ester bond, sulfonate ester bond, carbonate bond or carbamate bond.

In formula (C-3), L³³ is a single bond or C₁-C₂₀ hydrocarbylene group when m21=1, and a C₁-C₂₀ (m21+1)-valent hydrocarbon group when m21=2 or 3. The hydrocarbylene group and (m21+1)-valent hydrocarbon group may contain at least one moiety selected from an ether bond, carbonyl moiety, ester bond, amide bond, sultone ring, lactam ring, carbonate bond, halogen, hydroxy moiety, and carboxy moiety.

The C₁-C₂₀ hydrocarbylene group L³³ may be saturated or unsaturated and straight, branched or cyclic. Examples thereof include C₁-C₂₀ alkanediyl groups such as methanediyl, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl, dodecane-1,12-diyl; C₃-C₂₀ cyclic saturated hydrocarbylene groups such as cyclopentanediyl, cyclohexanediyl, norbornanediyl and adamantanediyl; C₂-C₂₀ unsaturated aliphatic hydrocarbylene groups such as vinylene and propene-1,3-diyl; C₆-C₂₀ arylene groups such as phenylene and naphthylene; and combinations thereof. The C₁-C₂₀ (m21+1)-valent hydrocarbon group L³³ may be saturated or unsaturated and straight, branched or cyclic. Examples thereof include those exemplified above for the C₁-C₂₀ hydrocarbylene group, with one or two hydrogen atoms being eliminated.

In formula (C-3), Rf¹ and Rf² are each independently hydrogen, fluorine or trifluoromethyl, at least one being fluorine or trifluoromethyl.

In formula (C-3), R²²¹ is hydroxy, carboxy, a C₁-C₆ saturated hydrocarbyl group, C₁-C₆ saturated hydrocarbyloxy group, C₂-C₆ saturated hydrocarbylcarbonyloxy group, fluorine, chlorine, bromine, -N(R^{221A})(R^{221B})-, -N(R^{221C})-C(=O)-R^{221D} or -N(R^{221C})-C(=O)-O-R^{221D}. R^{221A} and R^{221B} are each independently hydrogen or a C₁-C₆ saturated hydrocarbyl group. R^{221C} is hydrogen or a C₁-C₆ saturated hydrocarbyl group. R^{221D} is a C₁-C₆ saturated hydrocarbyl group or C₂-C₈ unsaturated aliphatic hydrocarbyl group.

The C₁-C₆ saturated hydrocarbyl group represented by R²²¹, R^{221A}, R^{221B} and R^{221C} may be straight, branched or cyclic. Examples thereof include C₁-C₆ alkyl groups such as methyl, ethyl, n-propyl, isopropyl, n-butyl, isobutyl, sec-butyl, tert-butyl, n-pentyl, and n-hexyl; and C₃-C₆ cyclic saturated hydrocarbyl groups such as cyclopropyl, cyclobutyl, cyclopentyl, and cyclohexyl. Examples of the saturated hydrocarbyl moiety in the C₁-C₆ saturated hydrocarbyloxy group represented by R²²¹ are as exemplified above for the saturated hydrocarbyl group. Examples of the saturated hydrocarbyl moiety in the C₂-C₆ saturated hydrocarbylcarbonyloxy group represented by R²²¹ are as exemplified above for the C₁-C₆ saturated hydrocarbyl group, but of 1 to 5 carbon atoms.

The C₂-C₈ unsaturated aliphatic hydrocarbyl group represented by R^{221D} may be straight, branched or cyclic and examples thereof include C₂-C₈ alkenyl groups such as vinyl, propenyl, butenyl, and hexenyl; C₂-C₈ alkynyl groups such as ethynyl, propynyl, and butynyl; and C₃-C₈ cyclic unsaturated aliphatic hydrocarbyl groups such as cyclohexenyl and norbornenyl.

In formula (C-3), R²²² is a C₁-C₂₀ saturated hydrocarbylene group or C₆-C₂₀ arylene group. Some or all of the hydrogen atoms in the saturated hydrocarbylene group may be substituted by halogen exclusive of fluorine. Some or all of the hydrogen atoms in the arylene group may be substituted by a substituent selected from C₁-C₂₀ saturated hydrocarbyl groups, C₁-C₂₀ saturated hydrocarbyloxy groups, C₆-C₂₀ aryl groups, halogen, and hydroxy.

The C₁-C₂₀ hydrocarbylene group represented by R²²² may be saturated or unsaturated and straight, branched or cyclic. Examples thereof are as exemplified above for the C₁-C₂₀ hydrocarbylene group L³³.

Examples of the C₆-C₂₀ arylene group represented by R²²² include phenylene, naphthylene, phenanthrenediyl, and anthracenediyl. The C₁-C₂₀ saturated hydrocarbyl moiety and hydrocarbyl moiety in the C₁-C₂₀ hydrocarbyloxy moiety, which are substituents on the arylene group, may be straight, branched or cyclic and examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl, and icosyl; and C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl. Examples of the C₆-C₁₄ arylene moiety which is a substituent on the arylene group include phenylene, naphthylene, phenanthrenediyl and anthracenediyl.

Of the anions having formula (C-3), anions having the formula (C-3-1) are preferred.

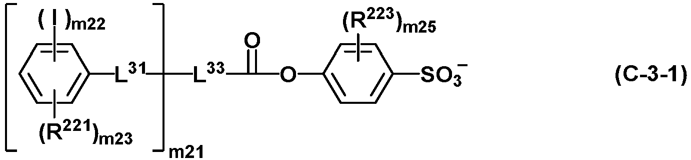

In formula (C-3-1), m21, m22, m23, L³¹, L³³, and R²²¹ are as defined above. The subscript m25 is an integer of 1 to 4. R²²³ is a C₁-C₂₀ saturated hydrocarbyl group, C₁-C₂₀ saturated hydrocarbyloxy group, C₆-C₁₄ aryl group, halogen or hydroxy group. When m25 is 2, 3 or 4, a plurality of R²²³ may be identical or different.

Examples of the anion having formula (C-3) are shown below, but not limited thereto.

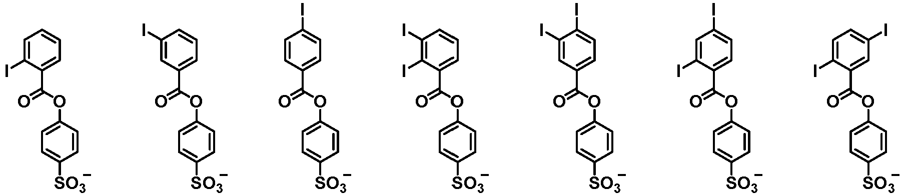

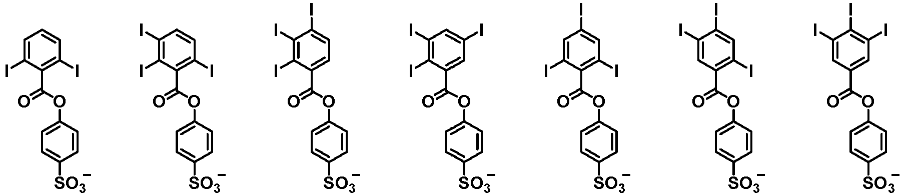

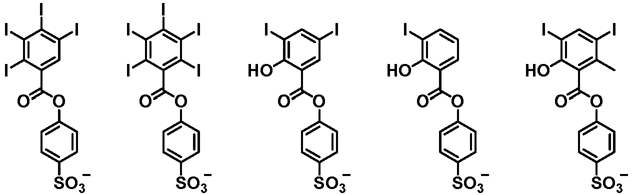

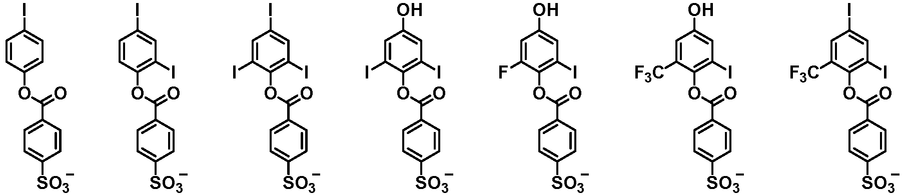

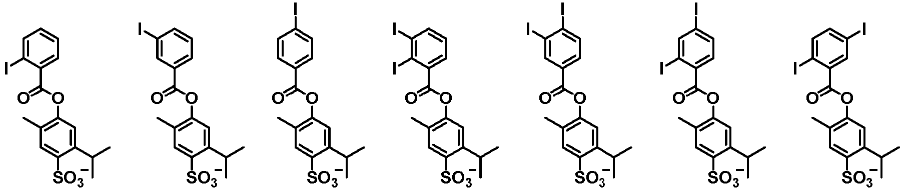

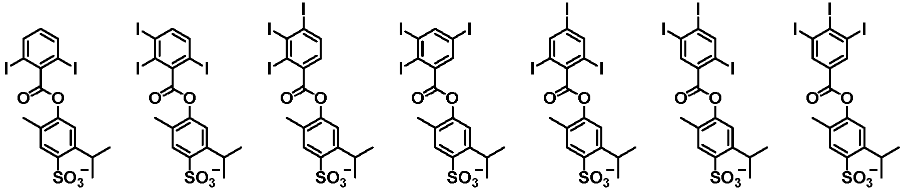

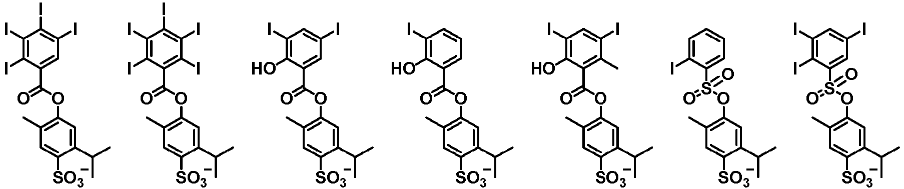

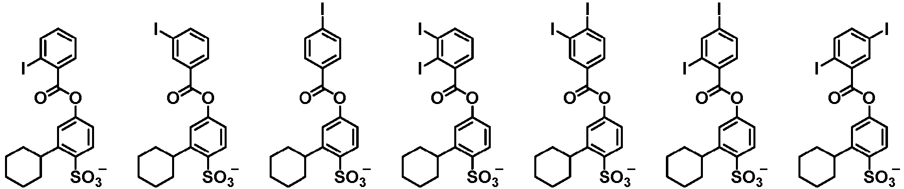

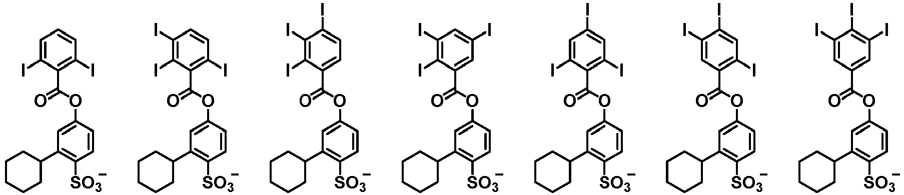

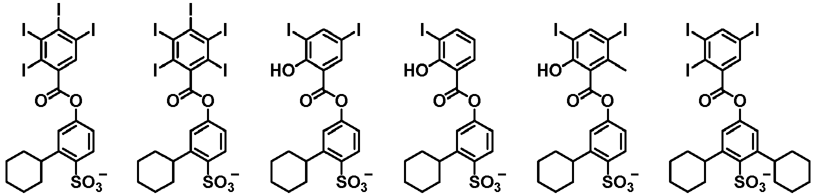

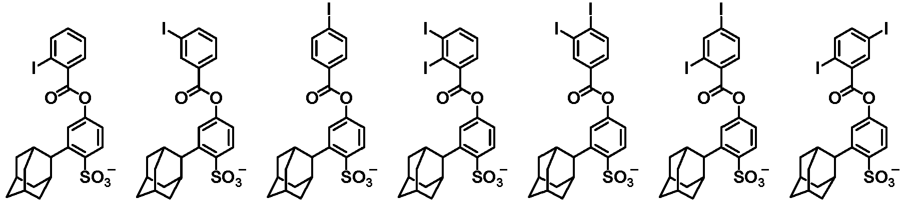

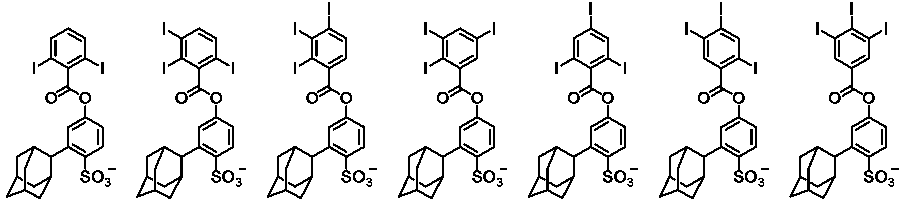

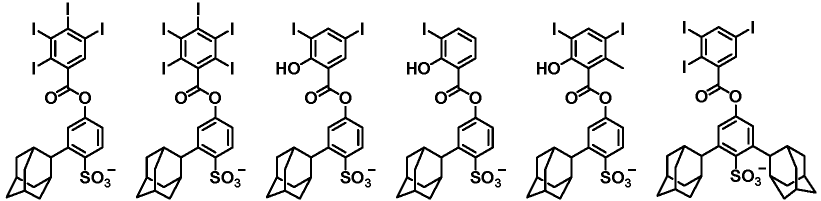

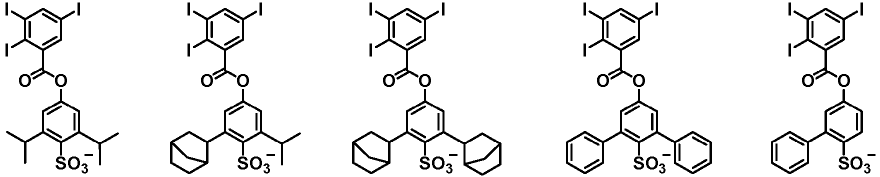

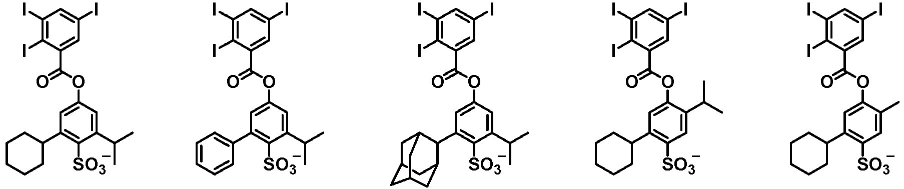

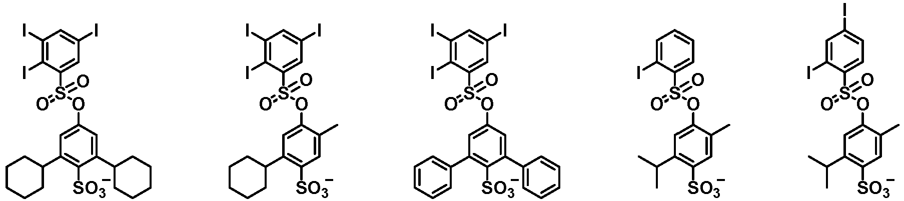

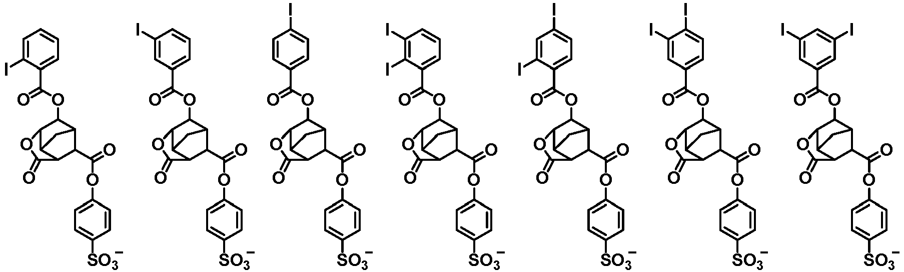

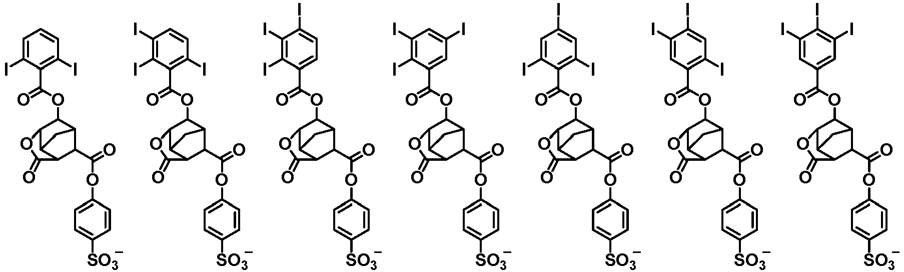

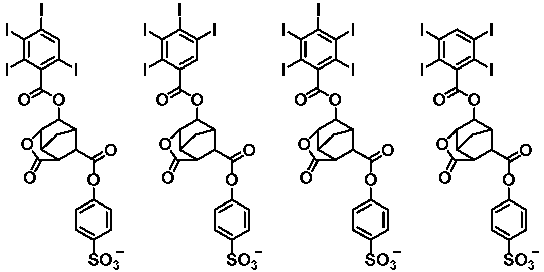

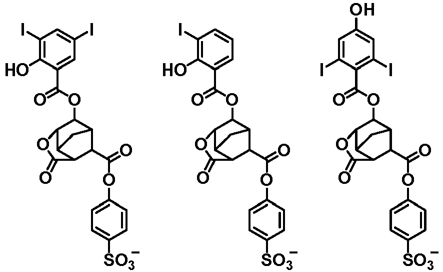

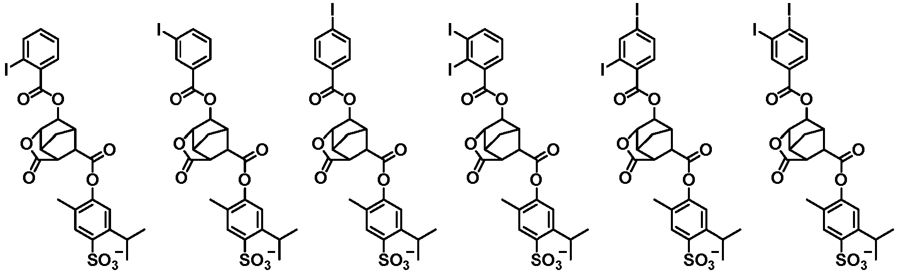

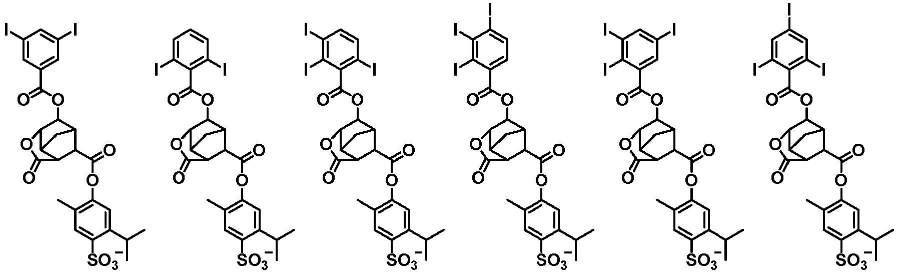

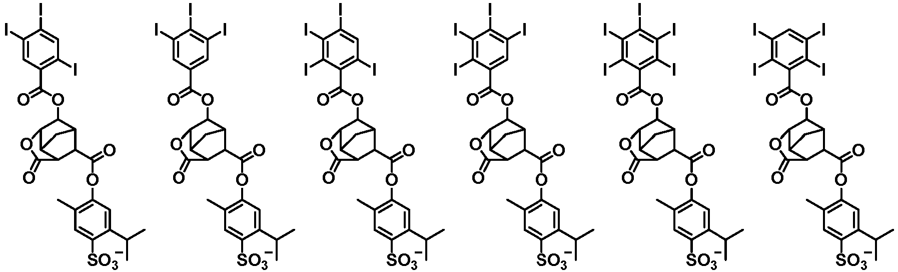

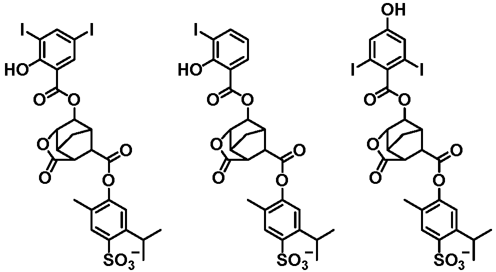

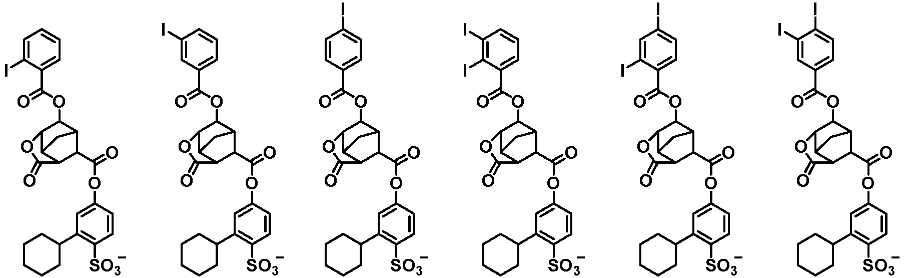

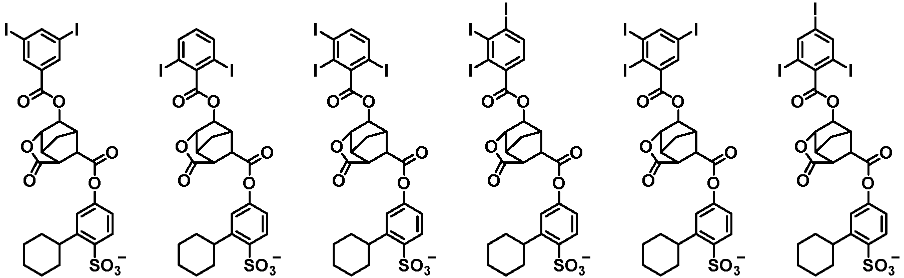

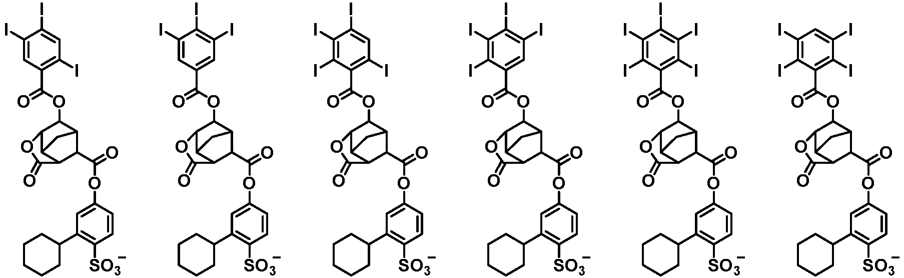

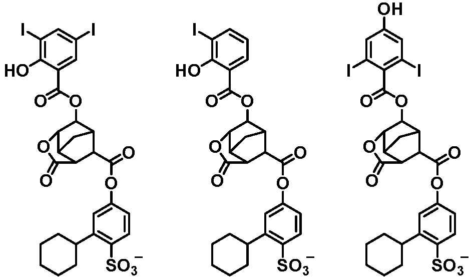

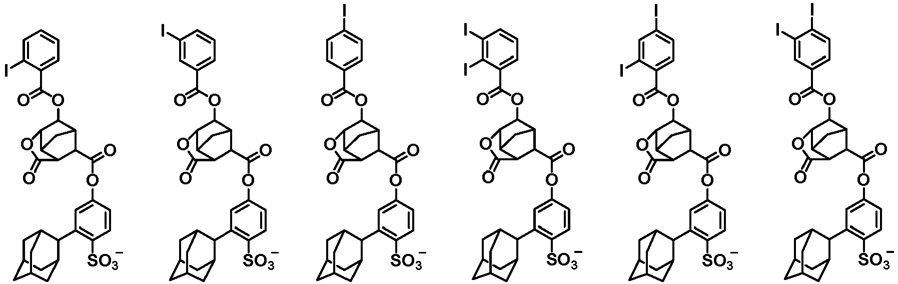

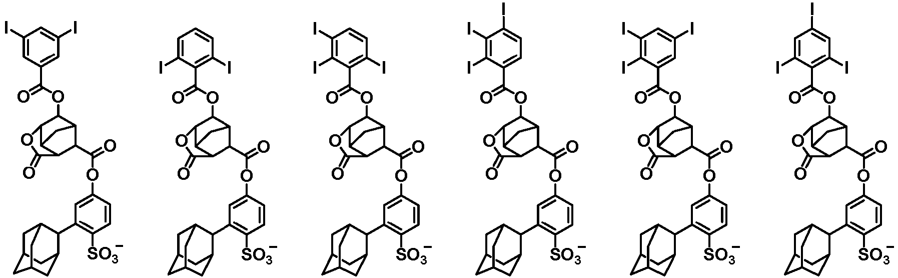

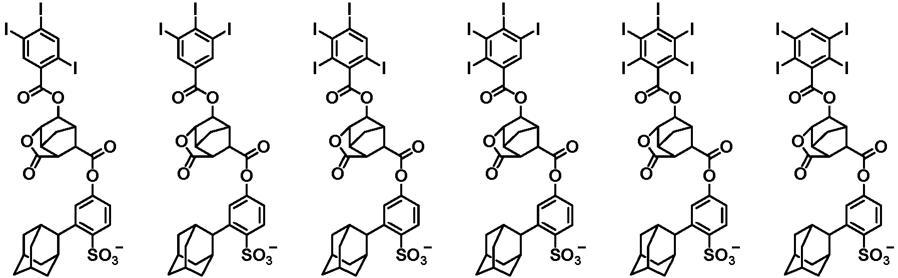

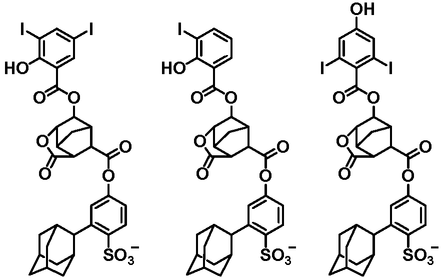

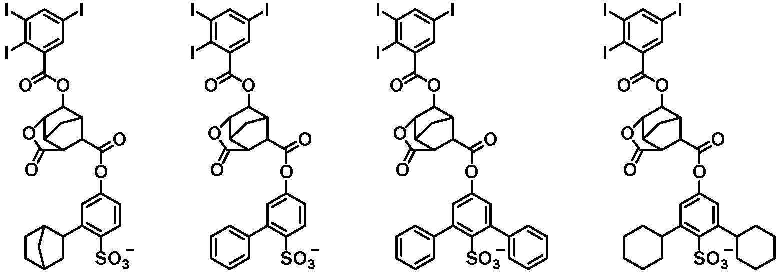

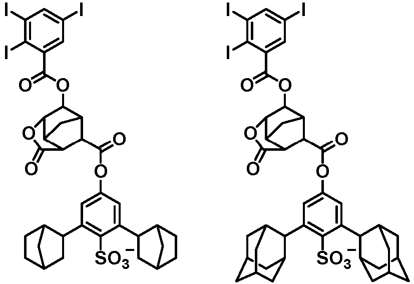

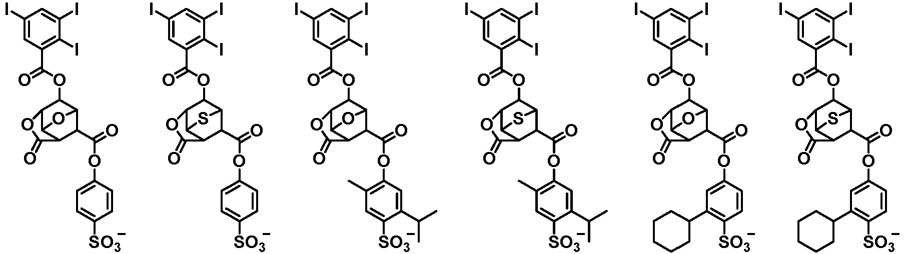

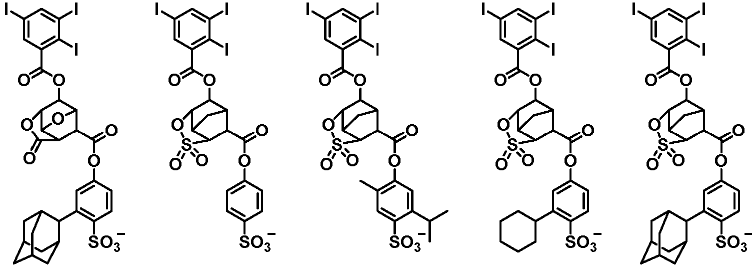

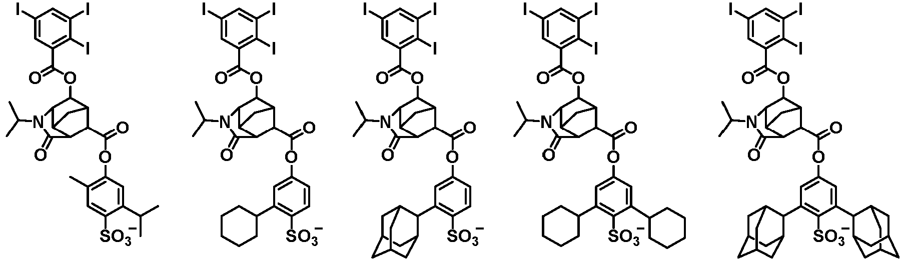

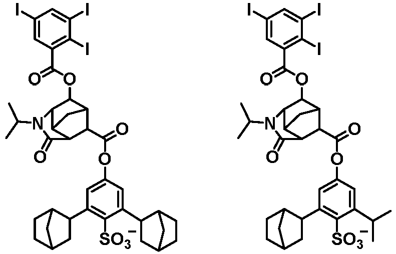

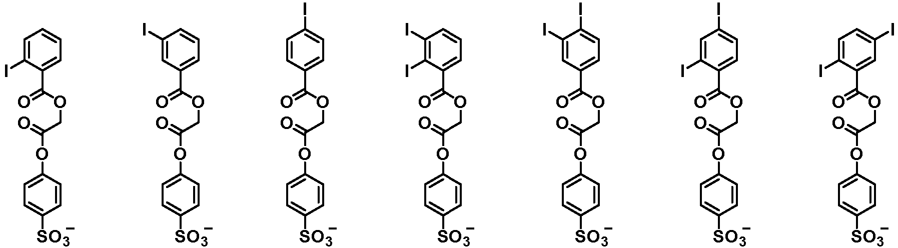

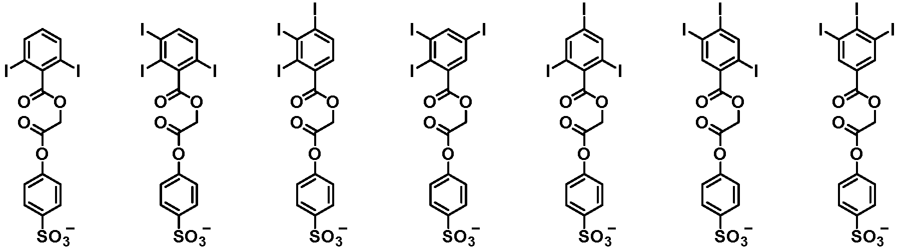

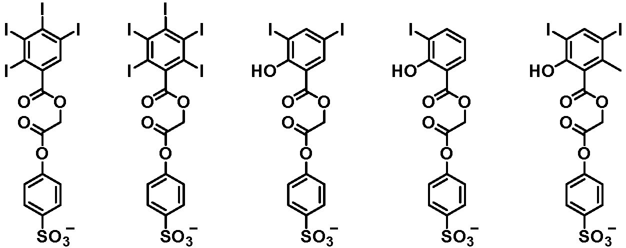

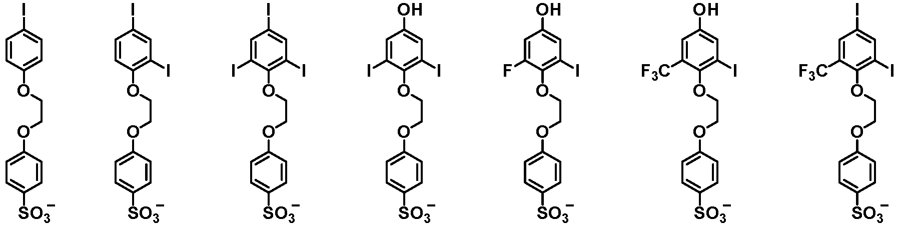

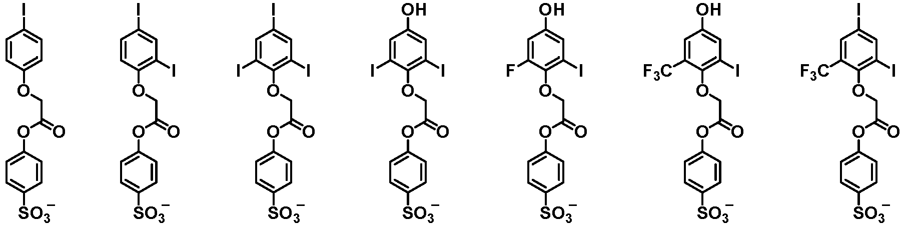

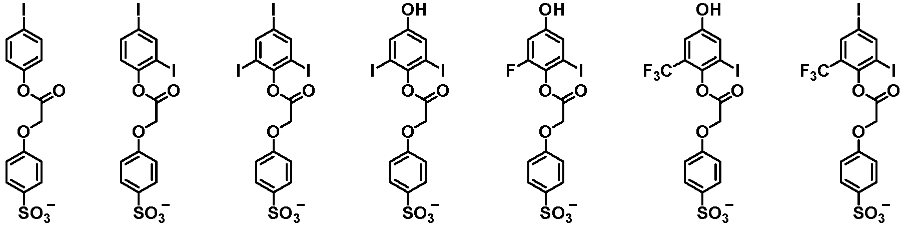

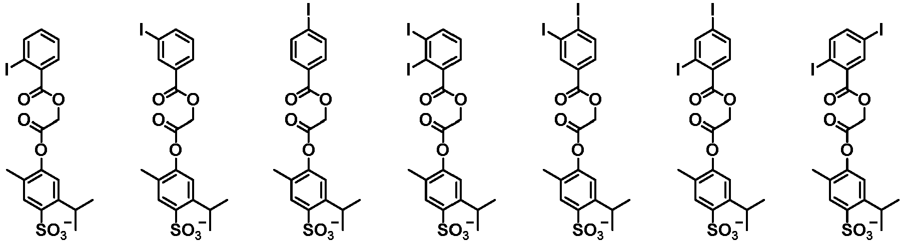

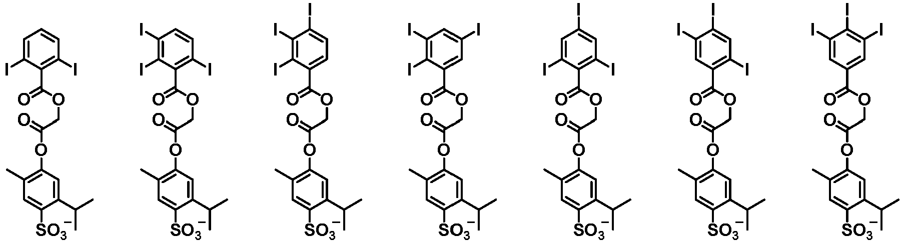

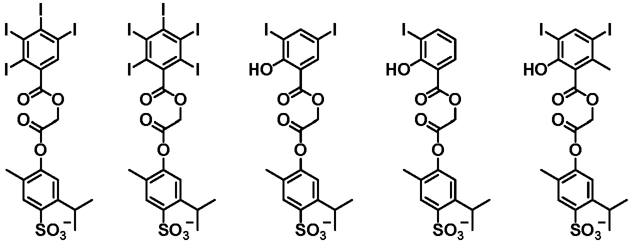

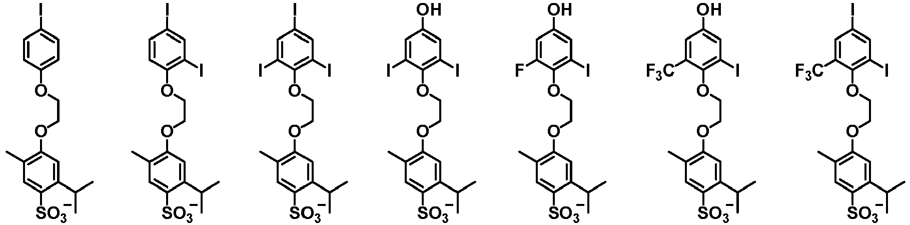

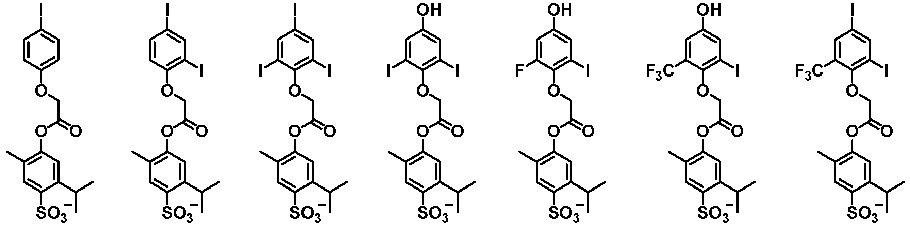

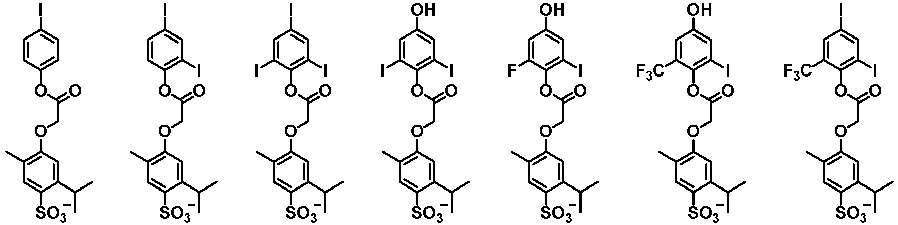

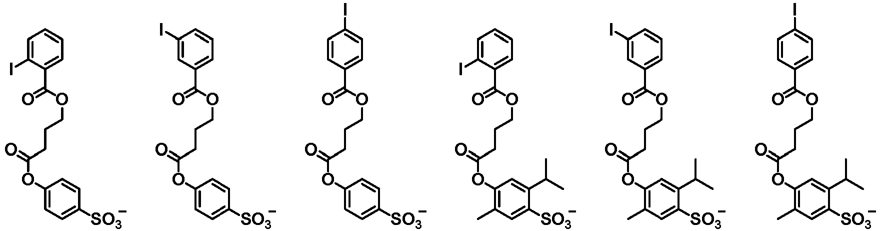

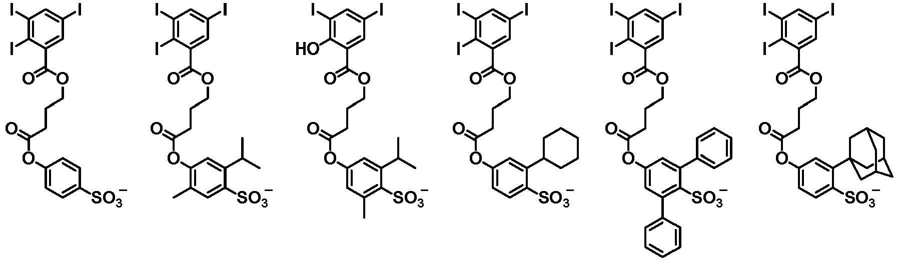

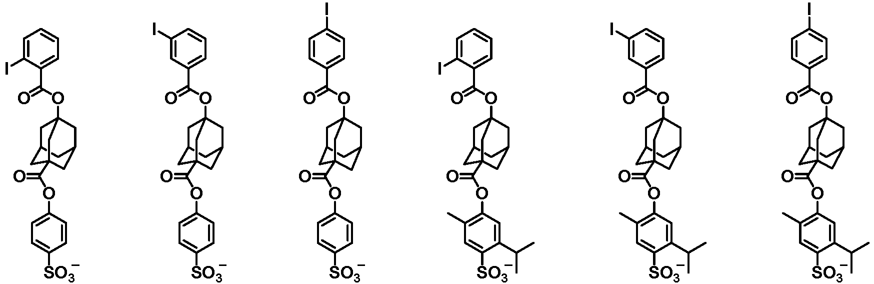

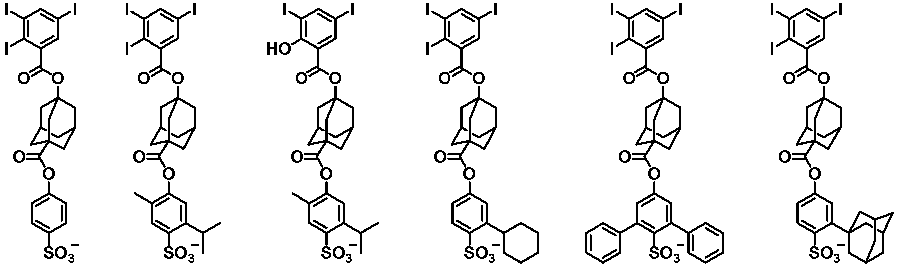

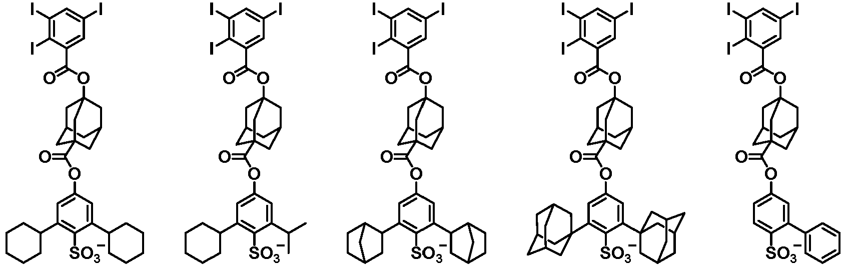

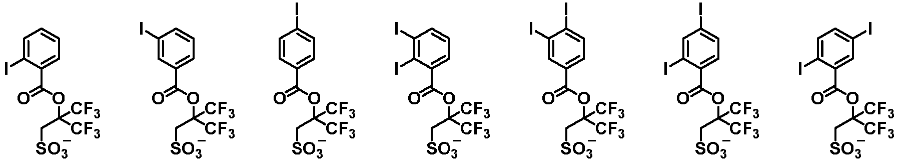

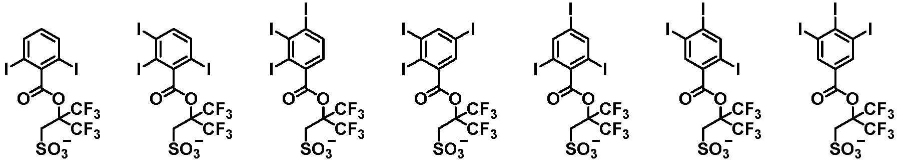

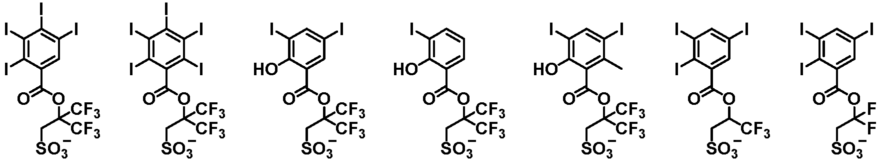

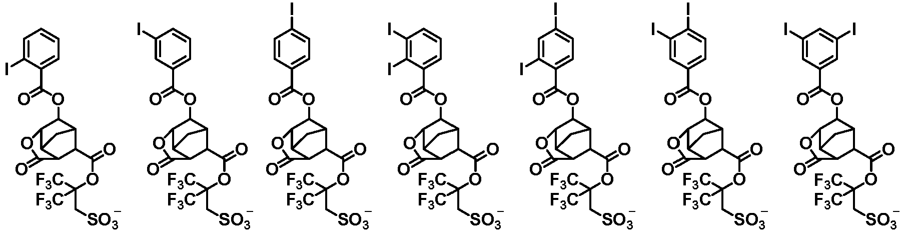

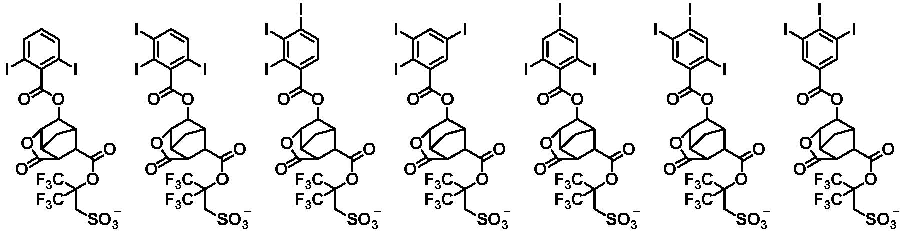

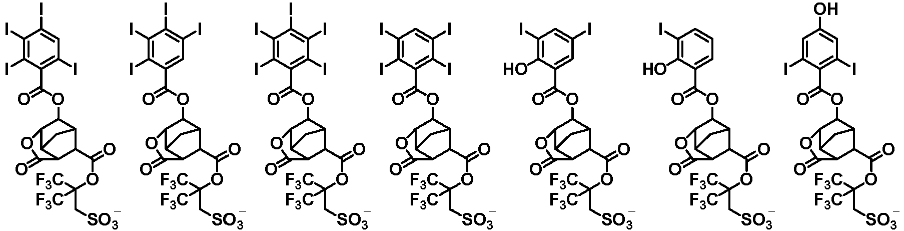

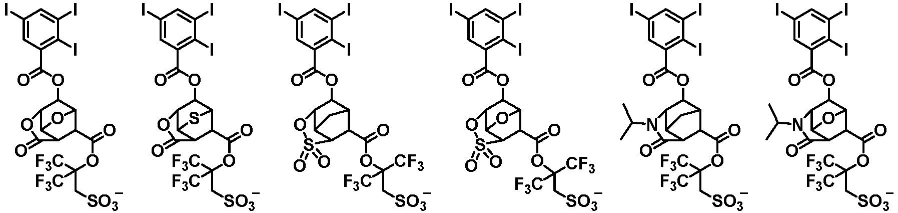

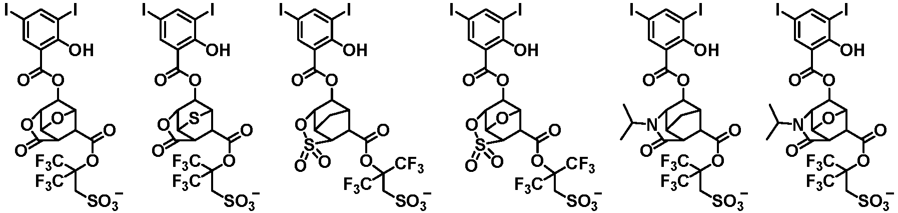

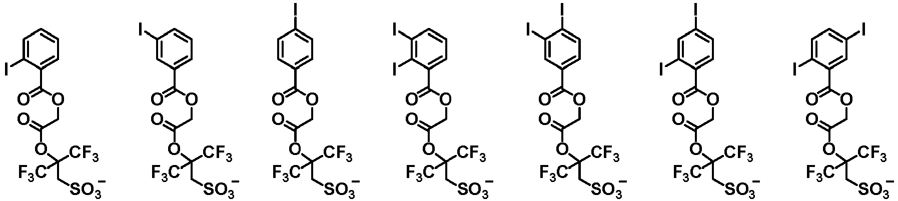

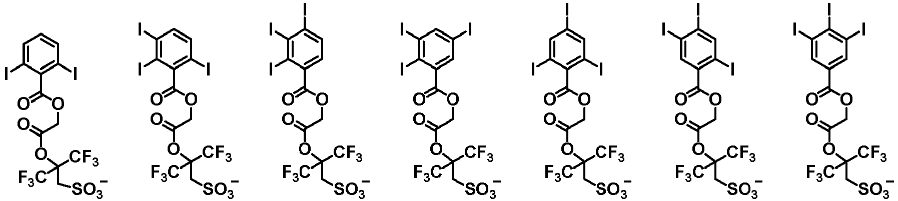

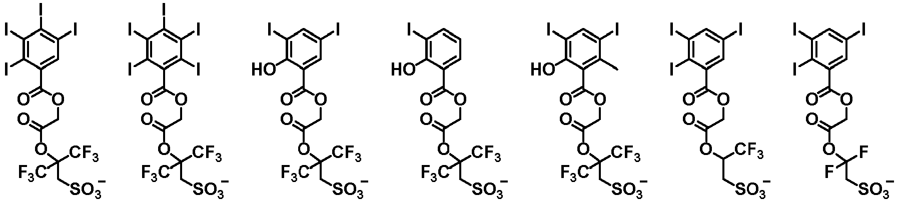

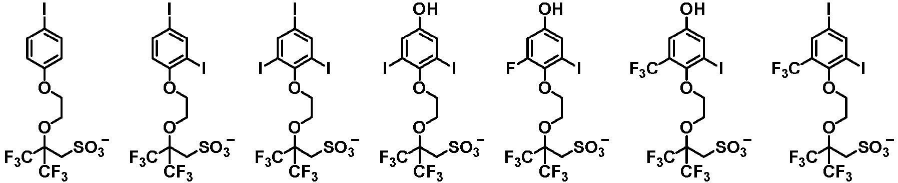

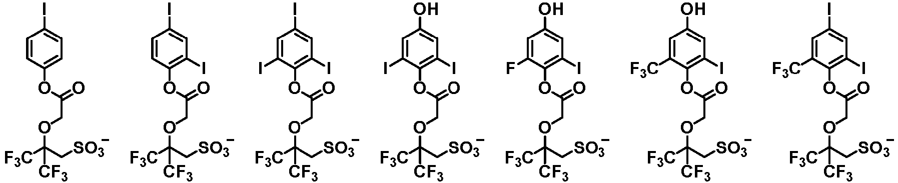

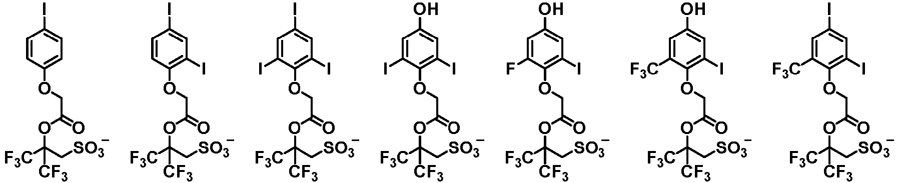

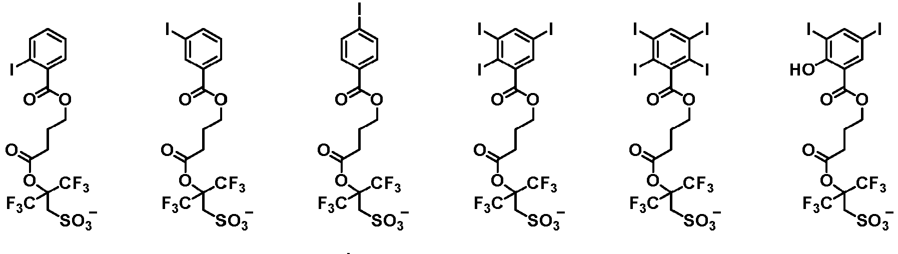

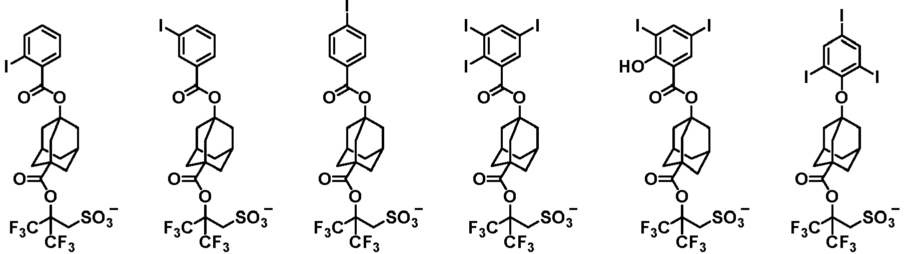

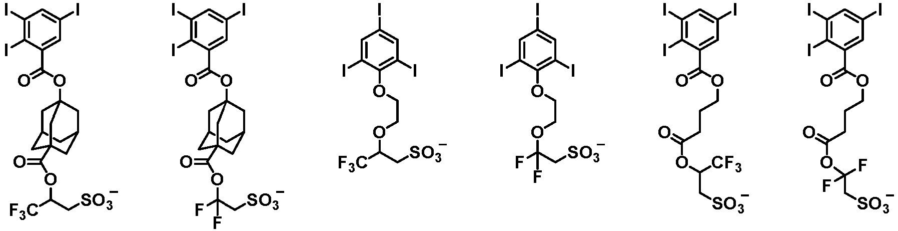

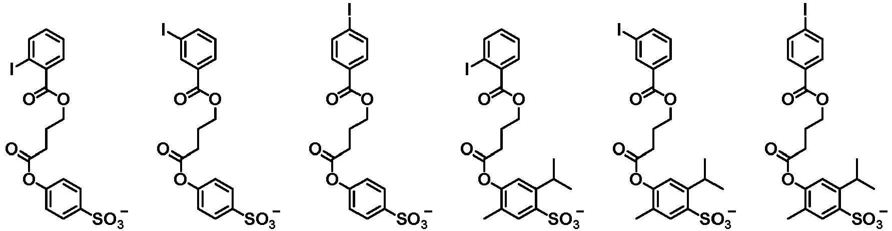

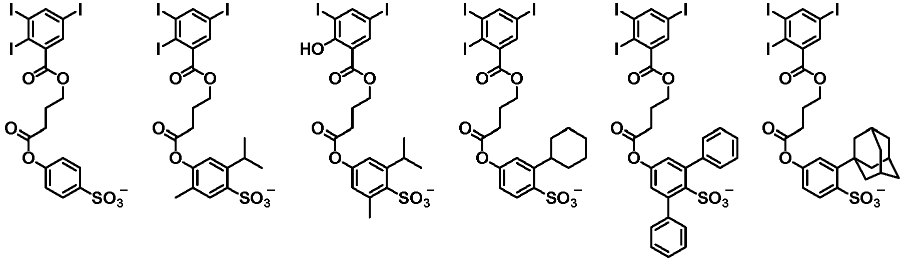

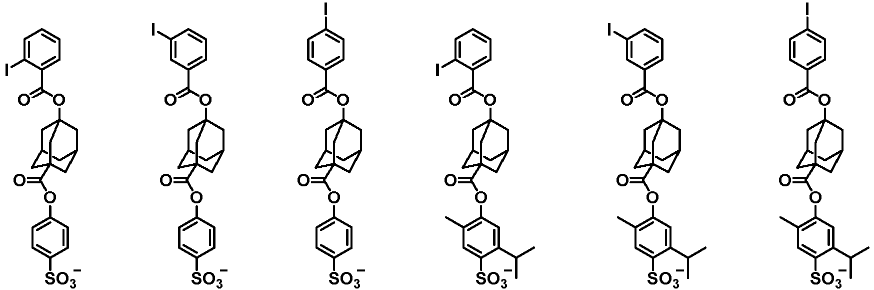

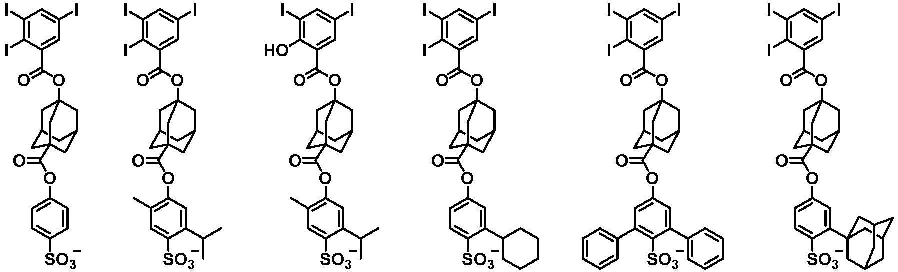

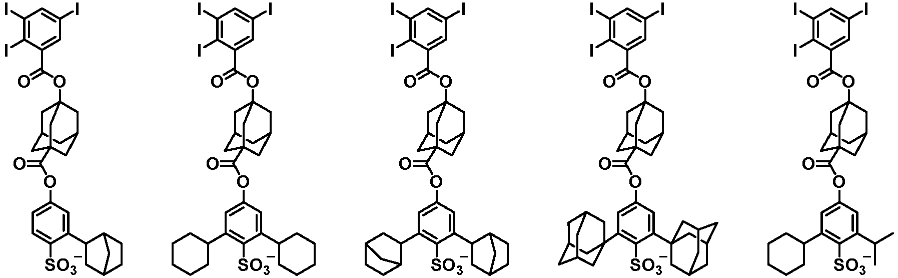

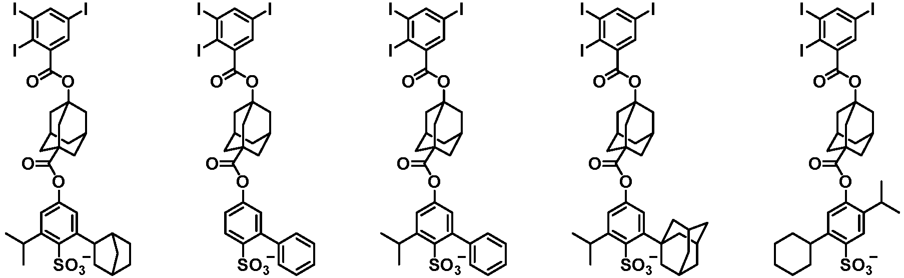

The cation that pairs with the anion of the onium salt is an onium cation. As the onium cation, sulfonium, iodonium and ammonium cations are preferred, with sulfonium and iodonium cations being more preferred. Preferred examples of the cation are described in JP-A 2023-091749, paragraphs [0154]-[0180]. The cations of the sulfonium salt having formula (A1) are especially preferred.

When the chemically amplified positive resist composition contains both the quencher (B) and the PAG (C), the weight ratio (C)/(B) of the PAG to the quencher is preferably less than 6/1, more preferably less than 4/1, even more preferably less than 2/1, further preferably less than 1/1. As long as the weight ratio of the PAG to the quencher is in the range, the resist composition is able to fully suppress acid diffusion, leading to improved resolution and dimensional uniformity.

### (D) Fluorinated polymer

The chemically amplified positive resist composition may further comprise a fluorinated polymer for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. The fluorinated polymer contains repeat units of at least one type selected from repeat units having the formula (D1), repeat units having the formula (D2), repeat units having the formula (D3), and repeat units having the formula (D4), and may contain repeat units of at least one type selected from repeat units having the formula (D5) and repeat units having the formula (D6). It is noted that repeat units having formulae (D1), (D2), (D3), (D4), (D5), and (D6) are also referred to as repeat units D1, D2, D3, D4, D5, and D6, respectively, hereinafter. Since the fluorinated polymer also has a surface active function, it can prevent insoluble residues from re-depositing onto the substrate during the development step and is thus effective for preventing development defects.

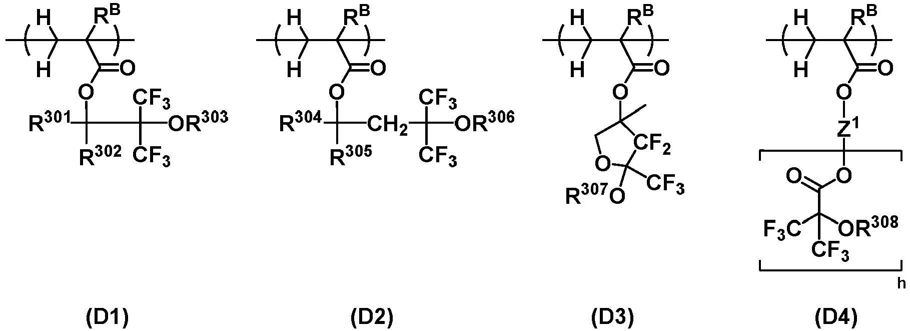

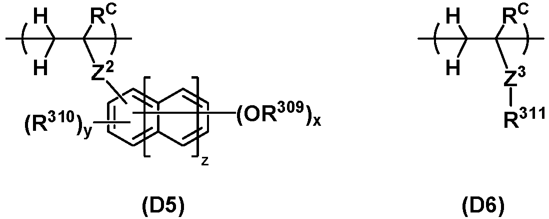

In formulae (D1) to (D6), x is an integer of 1 to 3, y is an integer meeting: 0 ≤ y ≤ 5+2z-x, z is 0 or 1, and h is an integer of 1 to 3. R^{B} is each independently hydrogen, fluorine, methyl or trifluoromethyl. R^{C} is each independently hydrogen or methyl. R³⁰¹, R³⁰², R³⁰⁴ and R³⁰⁵ are each independently hydrogen or a C₁-C₁₀ saturated hydrocarbyl group. R³⁰³, R³⁰⁶, R³⁰⁷ and R³⁰⁸ are each independently hydrogen, a C₁-C₁₅ hydrocarbyl group or fluorinated hydrocarbyl group, or an acid labile group, with the proviso that an ether bond or carbonyl moiety may intervene in a carbon-carbon bond in the hydrocarbyl or fluorinated hydrocarbyl groups represented by R³⁰³, R³⁰⁶, R³⁰⁷ and R³⁰⁸. R³⁰⁹ is hydrogen or a C₁-C₅ straight or branched hydrocarbyl group in which a heteroatom-containing moiety may intervene in a carbon-carbon bond. R³¹⁰ is a C₁-C₅ straight or branched hydrocarbyl group in which a heteroatom-containing moiety may intervene in a carbon-carbon bond. R³¹¹ is a C₁-C₂₀ saturated hydrocarbyl group in which at least one hydrogen is substituted by fluorine and some constituent -CH₂- may be replaced by an ester bond or ether bond. Z¹ is a C₁-C₂₀ (h+1)-valent hydrocarbon group or C₁-C₂₀ (h+1)-valent fluorinated hydrocarbon group. Z² is a single bond, *-C(=O)-O- or *-C(=O)-NH- wherein * designates a point of attachment to the carbon atom in the backbone. Z³ is a single bond, -O-, *-C(=O)-O-Z³¹-Z³²- or *-C(=O)-NH-Z³¹-Z³²-, wherein Z³¹ is a single bond or a C₁-C₁₀ saturated hydrocarbylene group, Z³² is a single bond, ester bond, ether bond or sulfonamide bond, and * designates a point of attachment to the carbon atom in the backbone.

In formulae (D1) and (D2), the C₁-C₁₀ saturated hydrocarbyl group represented by R³⁰¹, R³⁰², R³⁰⁴ and R³⁰⁵ may be straight, branched or cyclic and examples thereof include C₁-C₁₀ alkyl groups such as methyl, ethyl, n-propyl, isopropyl, n-butyl, isobutyl, sec-butyl, tert-butyl, n-pentyl, n-hexyl, n-heptyl, n-octyl, n-nonyl, and n-decyl, and C₃-C₁₀ cyclic saturated hydrocarbyl groups such as cyclopropyl, cyclobutyl, cyclopentyl, cyclohexyl, adamantyl, and norbornyl. Inter alia, C₁-C₆ saturated hydrocarbyl groups are preferred.

In formulae (D1) to (D4), the C₁-C₁₅ hydrocarbyl group represented by R³⁰³, R³⁰⁶, R³⁰⁷ and R³⁰⁸ may be straight, branched or cyclic and examples thereof include C₁-C₁₅ alkyl, C₂-C₁₅ alkenyl and C₂-C₁₅ alkynyl groups, with the alkyl groups being preferred. Suitable alkyl groups include methyl, ethyl, n-propyl, isopropyl, n-butyl, isobutyl, sec-butyl, n-pentyl, n-hexyl, n-heptyl, n-octyl, n-nonyl, n-decyl, n-undecyl, n-dodecyl, n-tridecyl, n-tetradecyl and n-pentadecyl. The fluorinated hydrocarbyl groups correspond to the foregoing hydrocarbyl groups in which some or all carbon-bonded hydrogen atoms are substituted by fluorine atoms.

In formula (D4), examples of the C₁-C₂₀ (h+1)-valent hydrocarbon group Z¹ include the foregoing C₁-C₂₀ alkyl groups and C₃-C₂₀ cyclic saturated hydrocarbyl groups, with h number of hydrogen atoms being eliminated. Examples of the C₁-C₂₀ (h+1)-valent fluorinated hydrocarbon group Z¹ include the foregoing (h+1)-valent hydrocarbon groups in which at least one hydrogen atom is substituted by fluorine.

Examples of the repeat units D1 to D4 are given below, but not limited thereto. Herein R^{B} is as defined above.

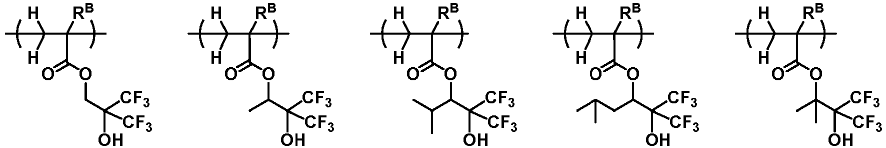

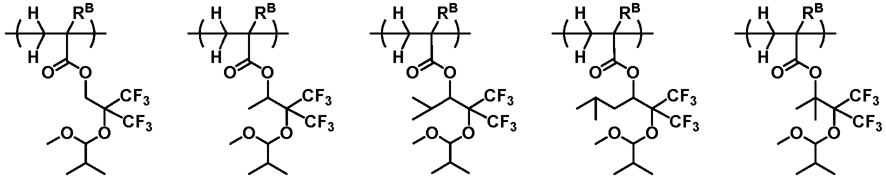

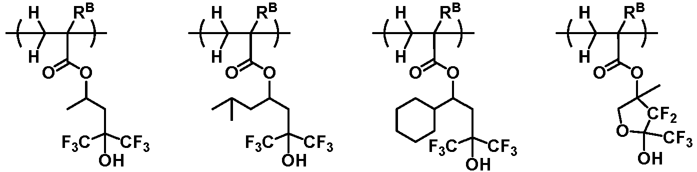

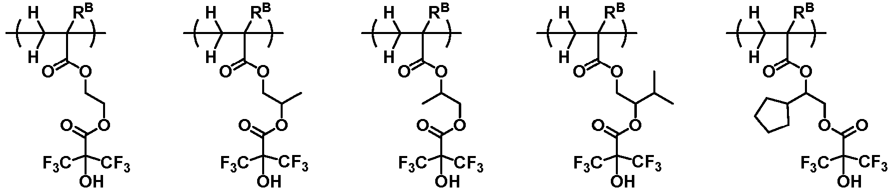

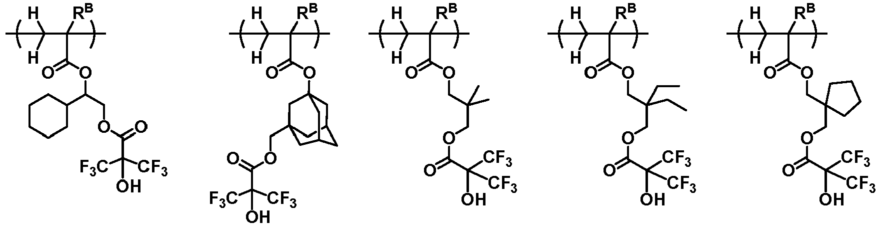

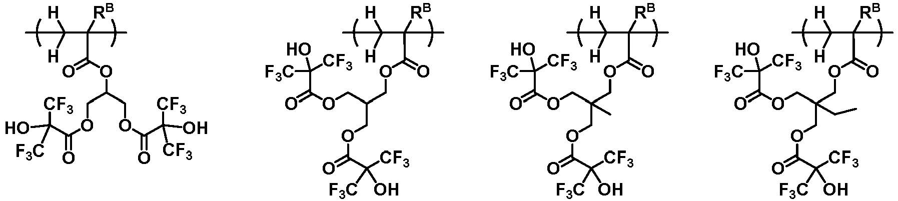

In formula (D5), examples of the C₁-C₅ hydrocarbyl groups R³⁰⁹ and R³¹⁰ include alkyl, alkenyl and alkynyl groups, with the alkyl groups being preferred. Suitable alkyl groups include methyl, ethyl, n-propyl, isopropyl, n-butyl, isobutyl, sec-butyl, and n-pentyl. In these groups, a moiety containing a heteroatom such as oxygen, sulfur or nitrogen may intervene in a carbon-carbon bond.

In formula (D5), -OR³⁰⁹ is preferably a hydrophilic group. In this case, R³⁰⁹ is preferably hydrogen or a C₁-C₅ alkyl group in which oxygen intervenes in a carbon-carbon bond.

In formula (D5), Z² is preferably *-C(=O)-O- or *-C(=O)-NH-. Also preferably R^{C} is methyl. The inclusion of carbonyl in Z² enhances the ability to trap the acid originating from the anti-charging film. A polymer wherein R^{C} is methyl is a robust polymer having a high glass transition temperature (Tg) which is effective for suppressing acid diffusion. As a result, the resist film is improved in stability with time, and neither resolution nor pattern profile is degraded.

Examples of the repeat unit D5 are given below, but not limited thereto. Herein R^{C} is as defined above.

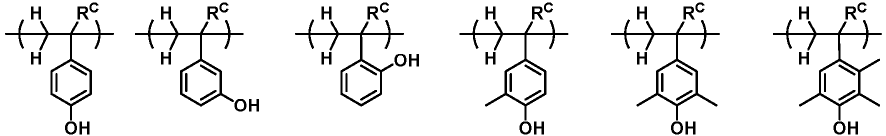

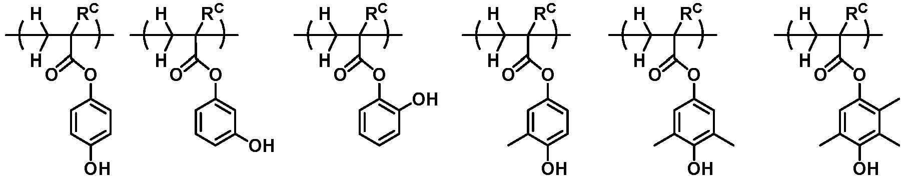

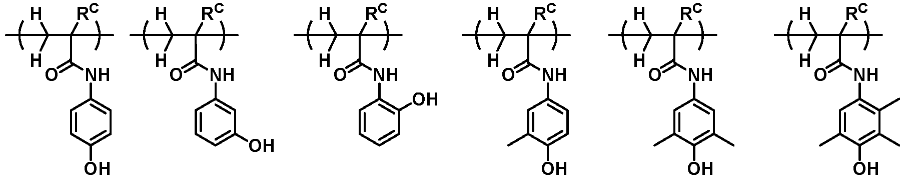

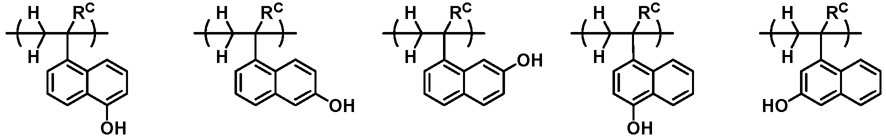

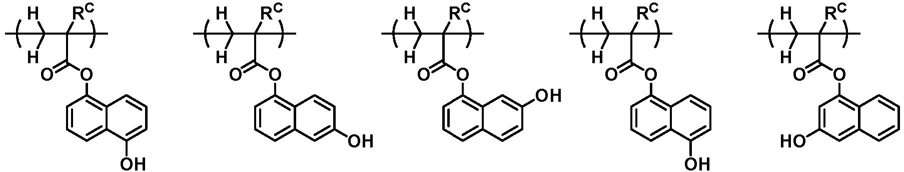

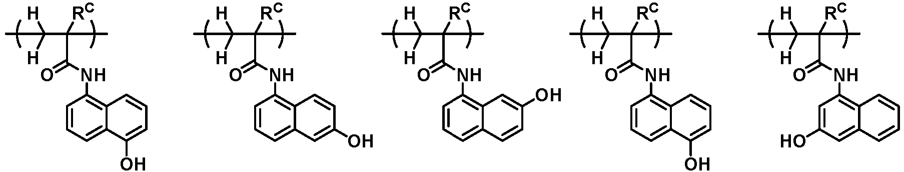

In formula (D6), the C₁-C₁₀ saturated hydrocarbylene group Z³ may be straight, branched or cyclic and examples thereof include methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, and 1,1-dimethylethane-1,2-diyl.

The C₁-C₂₀ saturated hydrocarbyl group having at least one hydrogen substituted by fluorine, represented by R³¹¹, may be straight, branched or cyclic and examples thereof include C₁-C₂₀ alkyl groups and C₃-C₂₀ cyclic saturated hydrocarbyl groups in which at least one hydrogen is substituted by fluorine.

Examples of the repeat unit D6 are given below, but not limited thereto. Herein R^{C} is as defined above.

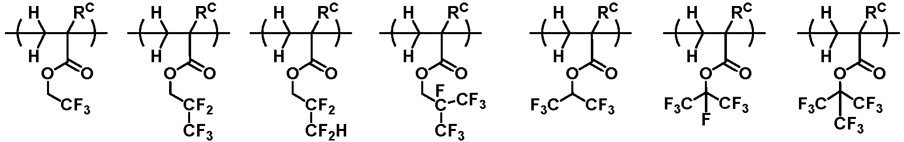

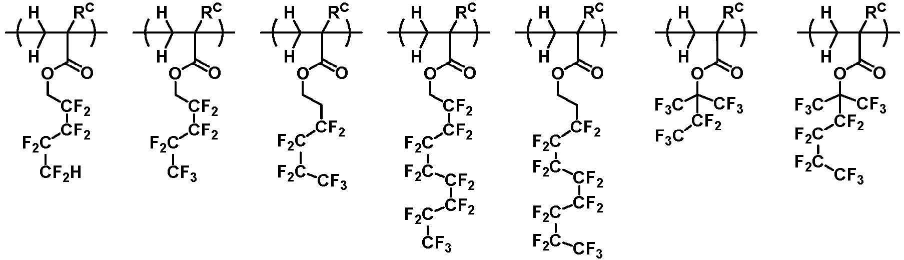

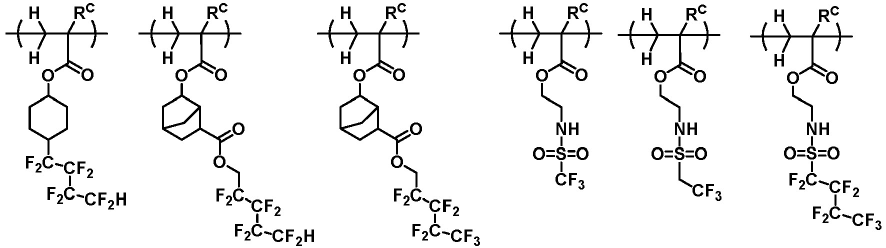

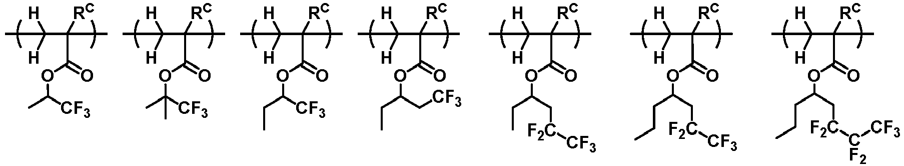

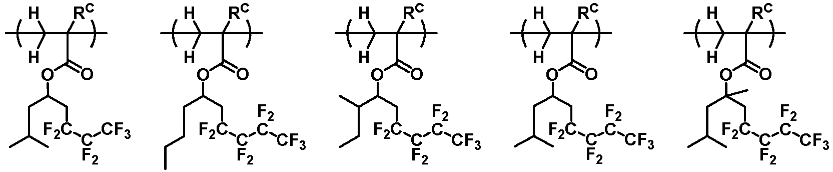

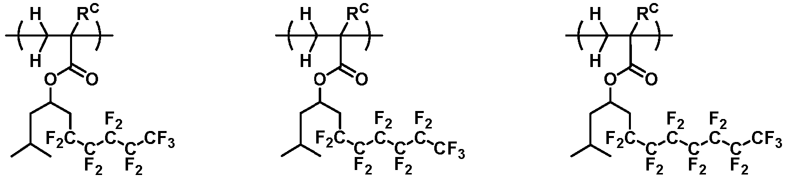

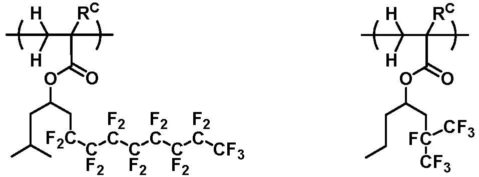

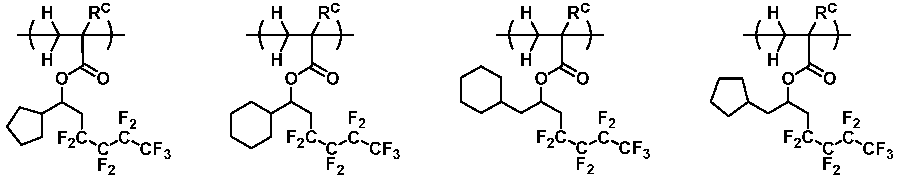

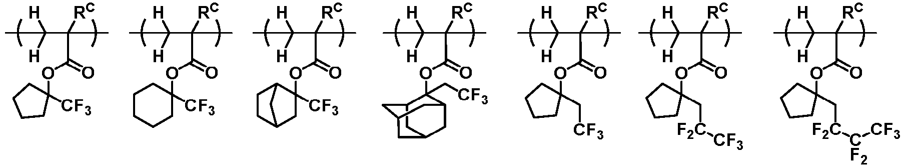

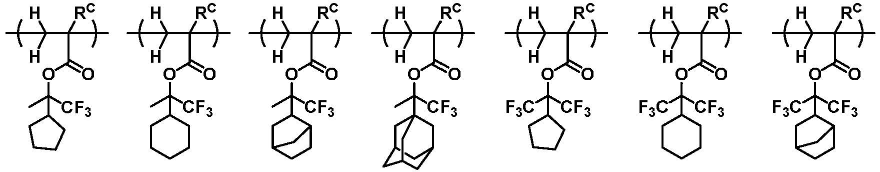

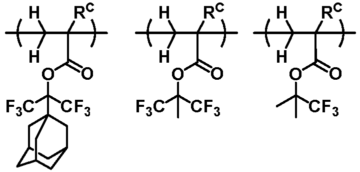

The content of repeat units D1 to D4 is preferably 15 to 95 mol%, more preferably 20 to 85 mol% based on the overall repeat units of the fluorinated polymer. The content of repeat unit D5 and/or D6 is preferably 5 to 85 mol%, more preferably 15 to 80 mol% based on the overall repeat units of the fluorinated polymer. Each of repeat units D1 to D6 may be used alone or in admixture.

The fluorinated polymer may comprise additional repeat units as well as the repeat units D1 to D6. Suitable additional repeat units include those described in USP 9,091,918 (JP-A 2014-177407, paragraphs [0046]-[0078]). When the fluorinated polymer comprises additional repeat units, their content is preferably up to 50 mol% based on the overall repeat units.

The fluorinated polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630.

The fluorinated polymer should preferably have a Mw of 2,000 to 50,000, and more preferably 3,000 to 20,000. A fluorinated polymer with a Mw of less than 2,000 may help acid diffusion, degrading resolution and detracting from age stability. A polymer with too high Mw may have a reduced solubility in solvent, with a risk of leaving coating defects. The fluorinated polymer preferably has a dispersity (Mw/Mn) of 1.0 to 2.2, more preferably 1.0 to 1.7.

In the resist composition, the fluorinated polymer (D) is preferably used in an amount of 0.01 to 30 parts by weight, more preferably 0.1 to 20 parts by weight, even more preferably 0.5 to 10 parts by weight per 80 parts by weight of the base polymer (A). The fluorinated polymer may be used alone or in admixture.

### (E) Organic solvent

The chemically amplified positive resist composition may further comprise an organic solvent as component (E). The organic solvent used herein is not particularly limited as long as the components are soluble therein. Examples of the organic solvent are described in JP-A 2008-111103, paragraphs [0144] to [0145] (USP 7,537,880). Specifically, exemplary solvents include ketones such as cyclohexanone, cyclopentanone, methyl-2-n-pentyl ketone and 2-heptanone; alcohols such as 3-methoxybutanol, 3-methyl-3-methoxybutanol, 1-methoxy-2-propanol, 1-ethoxy-2-propanol, and diacetone alcohol (DAA); ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate (EL), ethyl pyruvate, butyl acetate, methyl 3-methoxypropionate, ethyl 3-ethoxypropionate, t-butyl acetate, t-butyl propionate, and propylene glycol mono-t-butyl ether acetate; and lactones such as γ-butyrolactone (GBL), and mixtures thereof. Where an acid labile group of acetal form is used, a high boiling alcohol solvent such as diethylene glycol, propylene glycol, glycerol, 1,4-butanediol or 1,3-butanediol may be added to accelerate deprotection reaction of acetal.

Of the above organic solvents, it is recommended to use 1-ethoxy-2-propanol, PGMEA, PGME, cyclohexanone, EL, GBL, DAA, and mixtures thereof.

In the positive resist composition, the organic solvent (E) is preferably used in an amount of 200 to 10,000 parts, more preferably 400 to 5,000 parts by weight per 80 parts by weight of the base polymer (A). The organic solvent may be used alone or in admixture.

### (F) Surfactant

The positive resist composition may contain any conventional surfactants for facilitating to coat the composition to the substrate. A number of surfactants are known in the art as described in JP-A 2004-115630, and any suitable one may be chosen therefrom. The amount of the surfactant (F) added is preferably 0 to 5 parts by weight per 80 parts by weight of the base polymer (A). The surfactant may be used alone or in admixture.

### Process

A further embodiment of the invention is a resist pattern forming process comprising the steps of applying the chemically amplified positive resist composition defined above onto a substrate to form a resist film thereon, exposing the resist film patternwise to high-energy radiation, and developing the exposed resist film in an alkaline developer to form a resist pattern.

The substrate used herein may be selected from, for example, substrates for IC fabrication, e.g., Si, SiO, SiO₂, SiN, SiON, TiN, WSi, BPSG, SOG, and organic antireflective coating, and substrates for mask circuit fabrication, e.g., Cr, CrO, CrON, MoSi₂, Si, SiO, SiO₂, SiON, SiONC, CoTa, NiTa, TaBN, and SnO₂.

First the resist composition is applied onto a substrate by a suitable coating technique such as spin coating. The coating is prebaked on a hotplate preferably at a temperature of 60 to 150°C for 1 to 20 minutes, more preferably at 80 to 140°C for 1 to 10 minutes to form a resist film of 0.03 to 2 µm thick.

Then the resist film is exposed patternwise to high-energy radiation such as UV, deep-UV, excimer laser (KrF, ArF), EUV, x-ray, γ-ray, synchrotron radiation or EB. The resist composition of the invention is especially effective in the EUV or EB lithography.

On use of UV, deep-UV, EUV, excimer laser, x-ray, γ-ray or synchrotron radiation as the high-energy radiation, the resist film is exposed through a mask having a desired pattern, preferably in a dose of 1 to 500 mJ/cm², more preferably 10 to 400 mJ/cm². On use of EB, a pattern may be written directly in a dose of preferably 1 to 500 µC/cm², more preferably 10 to 450 µC/cm², even more preferably 100 to 400 µC/cm², most preferably 150 to 400 µC/cm².

The exposure may be performed by conventional lithography whereas the immersion lithography of holding a liquid, typically water between the mask and the resist film may be employed if desired. In the immersion lithography, a protective film which is insoluble in water may be used.

The resist film is then baked (PEB) on a hotplate preferably at 60 to 150°C for 1 to 20 minutes, more preferably at 80 to 140°C for 1 to 10 minutes.

Thereafter, the resist film is developed with a developer in the form of an aqueous base solution, for example, 0.1 to 5 wt%, preferably 2 to 3 wt% aqueous solution of tetramethylammonium hydroxide (TMAH) preferably for 0.1 to 3 minutes, more preferably 0.5 to 2 minutes by conventional techniques such as dip, puddle and spray techniques. In this way, a desired resist pattern is formed on the substrate.

From the positive resist composition, a pattern with a high resolution and reduced LER can be formed. The resist composition is effectively applicable to a substrate, specifically a substrate having a surface layer of material to which a resist film is less adherent and which is likely to invite pattern stripping or pattern collapse, and particularly a substrate having sputter deposited on its outermost surface metallic chromium or a chromium compound containing at least one light element selected from oxygen, nitrogen and carbon or a substrate having an outermost surface layer of SiO, SiOₓ, or a tantalum compound, molybdenum compound, cobalt compound, nickel compound, tungsten compound or tin compound. The substrate to which the positive resist composition is applied is most typically a photomask blank which may be either of transmission or reflection type.

The mask blank of transmission type is typically a photomask blank having a light-shielding film of chromium-based material. It may be either a photomask blank for binary masks or a photomask blank for phase shift masks. In the case of the binary mask-forming photomask blank, the light-shielding film may include an antireflection layer of chromium-based material and a light-shielding layer. In one example, the antireflection layer on the surface layer side is entirely composed of a chromium-based material. In an alternative example, only a surface side portion of the antireflection layer on the surface layer side is composed of a chromium-based material and the remaining portion is composed of a silicon compound-based material which may contain a transition metal. In the case of the phase shift mask-forming photomask blank, it may include a phase shift film and a chromium-based light-shielding film thereon.

Photomask blanks having an outermost layer of chromium base material are well known as described in JP-A 2008-026500 and JP-A 2007-302873 and the references cited therein. Although the detail description is omitted herein, the following layer construction may be employed when a light-shielding film including an antireflective layer and a light-shielding layer is composed of chromium base materials.

In the example where a light-shielding film including an antireflective layer and a light-shielding layer is composed of chromium base materials, layers may be stacked in the order of an antireflective layer and a light-shielding layer from the outer surface side, or layers may be stacked in the order of an antireflective layer, a light-shielding layer, and an antireflective layer from the outer surface side. Each of the antireflective layer and the light-shielding layer may be composed of multiple sub-layers. When the sub-layers have different compositions, the composition may be graded discontinuously or continuously from sub-layer to sub-layer. The chromium base material used herein may be metallic chromium or a material consisting of metallic chromium and a light element such as oxygen, nitrogen or carbon. Examples used herein include metallic chromium, chromium oxide, chromium nitride, chromium carbide, chromium oxynitride, chromium oxycarbide, chromium nitride carbide, and chromium oxide nitride carbide.

The mask blank of reflection type includes a substrate, a multilayer reflective film formed on one major surface (front surface) of the substrate, for example, a multilayer reflective film of reflecting exposure radiation such as EUV radiation, and an absorber film formed on the multilayer reflective film, for example, an absorber film of absorbing exposure radiation such as EUV radiation to reduce reflectivity. From the reflection type mask blank (reflection type mask blank for EUV lithography), a reflection type mask (reflection type mask for EUV lithography) having an absorber pattern (patterned absorber film) formed by patterning the absorber film is produced. The EUV radiation used in the EUV lithography has a wavelength of 13 to 14 nm, typically about 13.5 nm.

The multilayer reflective film is preferably formed contiguous to one major surface of a substrate. An underlay film may be disposed between the substrate and the multilayer reflective film as long as the benefits of the invention are not lost. The absorber film may be formed contiguous to the multilayer reflective film while a protective film (protective film for the multilayer reflective film) may be disposed between the multilayer reflective film and the absorber film, preferably contiguous to the multilayer reflective film, more preferably contiguous to the multilayer reflective film and the absorber film. The protective film is used for protecting the multilayer reflective film in a cleaning, tailoring or otherwise processing step. Also preferably, the protective film has an additional function of protecting the multilayer reflective film or preventing the multilayer reflective film from oxidation during the step of patterning the absorber film by etching. Besides, an electroconductive film, which is used for electrostatic chucking of the reflection type mask to an exposure tool, may be disposed below the other major surface (back side surface) which is opposed to the one major surface of the substrate, preferably contiguous to the other major surface. It is provided herein that a substrate has one major surface which is a front or upper side surface and another major surface which is a back or lower side surface. The terms "front and back" sides or "upper and lower" sides are used for the sake of convenience. One or another major surface may be either of the two major surfaces (film-bearing surfaces) of a substrate, and in this sense, front and back or upper and lower are exchangeable. Specifically, the multilayer reflective film may be formed by any of the methods of JP-A 2021-139970 and the references cited therein.

Even on a substrate (typically mask blank) whose outermost surface is made of a material tending to affect resist pattern profile such as a chromium, silicon or tantalum-containing material, the resist pattern forming process is successful in forming patterns of rectangular profile having a high resolution, reduced LER, and fidelity.

### EXAMPLES

Examples of the invention are given below by way of illustration and not by way of limitation. The abbreviation "pbw" is parts by weight. For copolymers, the compositional ratio is a molar ratio and Mw is determined by GPC versus polystyrene standards using DMF solvent. Analysis is made by IR, ¹H-NMR spectroscopy and time-of-flight mass spectrometry using analytic instruments as shown below.

| | |
|---|---|
| IR: | NICOLET 6700 by Thermo Fisher Scientific Inc. |
| ¹H-NMR: | ECA-500 by JEOL Ltd. |
| MALDI TOF-MS: | S3000 by JEOL Ltd. |

### Synthesis of onium salt monomers

### Example 1-1

### Synthesis of Monomer PM-1

### (1) Synthesis of Intermediate In-1

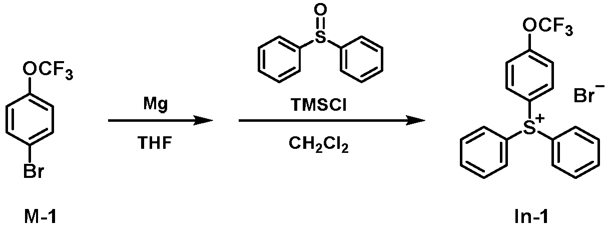

In nitrogen atmosphere, a Grignard reagent was prepared using 109.4 g of magnesium, 1084.6 g of reactant M-1, and 2250 g of THF. The reaction system was cooled below 10°C, after which a solution of 303.4 g of diphenyl sulfoxide in 1500 g of methylene chloride was added. Then 678.2 g of chlorotrimethylsilane was added dropwise while maintaining the internal temperature below 20°C. At the end of addition, the reaction solution was aged for 2 hours while maintaining the internal temperature below 20°C. The reaction system as aged was cooled, and a solution of 150 g of 36 wt% hydrochloric acid in 2250 g of water was added dropwise to quench the reaction. Thereafter, 2100 g of diisopropyl ether and 4500 g of water were added to the reaction solution, of which the water layer was taken out. The water layer was washed twice with 1950 g of diisopropyl ether. The water layer as washed was directly used in the subsequent step.

### (2) Synthesis of Monomer PM-1

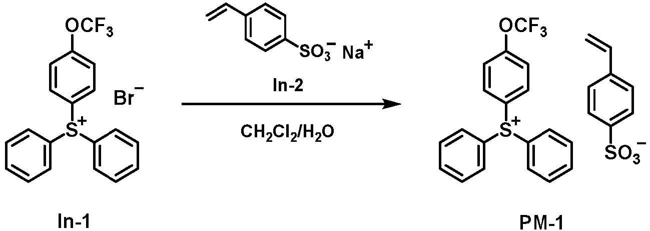

In nitrogen atmosphere, a reactor was charged with 537.0 g of the aqueous solution of Intermediate In-1, 45.3 g of Intermediate In-2, and 300 g of methylene chloride, which were stirred at room temperature for 30 minutes. The organic layer was taken out, washed with water, and concentrated under reduced pressure. There was obtained Monomer PM-1 as oily matter (amount 95.2 g, yield 90%).

Monomer PM-1 was analyzed by IR spectroscopy and TOF-MS, with the data shown below. FIG. 1 is the ¹H-NMR/DMSO-d₆ spectrum of PM-1.
IR (D-ATR): v = 3456, 3058, 1629, 1584, 1492, 1478, 1447, 1398, 1258, 1213, 1121, 1107, 1070, 1033, 1010, 924, 845, 809, 750, 733, 674, 581, 555, 530, 503 cm⁻¹
MALDI TOF-MS:
   positive M⁺ 347 (corresponding to C₁₉H₁₄F₃OS⁺)
   negative M⁻ 183 (corresponding to C₈H₇O₃S⁻)

### Synthesis of polymers

### Example 1-1

### Synthesis of Polymer P-1

In nitrogen atmosphere, a flask was charged with 52.2 g of 3-acetoxystyrene, 31.4 g of 4-(1-methyl-1-cyclopentyloxy)styrene, 16.5 g of PM-1, 11.4 g of V-601 (dimethyl 2,2'-azobis(2-methylpropionate) by Fujifilm Wako Pure Chemical Corp.), and 120 g of PGME to form a monomer/initiator solution. Another flask under nitrogen atmosphere was charged with 60 g of PGME, which was heated at 80°C with stirring. The monomer/initiator solution was added dropwise to the PGME over 4 hours. At the end of addition, the polymerization solution was continuously stirred for 18 hours while maintaining the temperature at 80°C. The polymerization solution was cooled to room temperature, after which it was added dropwise to 5,000 g of MeOH/H₂O mixture with stirring. The precipitate was collected by filtration. The precipitate was washed twice with 1,000 g of MeOH/H₂O mixture and vacuum dried at 40°C for 20 hours, obtaining 85.2 g of Polymer P-1 as white powder. Polymer P-1 was analyzed by ¹³C-NMR, ¹H-NMR and GPC.

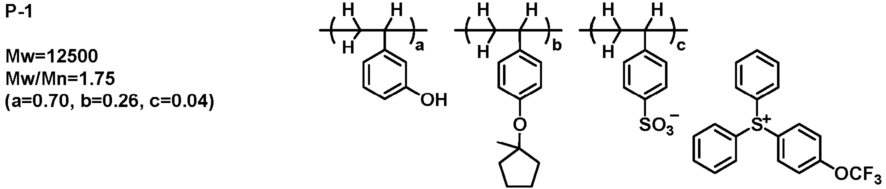

### Examples 1-2 to 1-75

### Synthesis of Polymers P-2 to P-75

Polymers P-2 to P-75 shown in Tables 1 to 3 were synthesized by the same procedure as in Example 1-2 except that the type and amount (blending ratio) of monomers were changed. In Tables 1 to 3, the incorporation ratio is on a molar basis. Repeat units PP-1 to PP-16 are polymer units corresponding to the monomers synthesized by the same method as Example 1-1.

**Table 1**

| | Unit 1 | Incorporation ratio (mol%) | Unit 2 | Incorporation ratio (mol%) | Unit 3 | Incorporation ratio (mol%) | Unit 4 | Incorporation ratio (mol%) | Unit 5 | Incorporation ratio (mol%) | Mw | Mw/Mn |
|---|---|---|---|---|---|---|---|---|---|---|---|---|
| P-1 | PP-1 | 4.0 | A-1 | 70.0 | C-1 | 26.0 | - | - | - | - | 12,500 | 1.74 |
| P-2 | PP-1 | 5.0 | A-1 | 65.0 | B-1 | 10.0 | C-1 | 20.0 | - | - | 16,000 | 1.72 |
| P-3 | PP-1 | 5.0 | A-1 | 65.0 | B-2 | 10.0 | C-1 | 20.0 | - | - | 12,600 | 1.72 |
| P-4 | PP-1 | 5.0 | A-1 | 65.0 | B-3 | 10.0 | C-1 | 20.0 | - | - | 12,400 | 1.73 |
| P-5 | PP-1 | 5.0 | A-1 | 65.0 | B-4 | 15.0 | C-1 | 20.0 | - | - | 12,400 | 1.71 |
| P-6 | PP-1 | 5.0 | A-1 | 63.0 | B-5 | 12.0 | C-1 | 20.0 | - | - | 15,000 | 1.72 |
| P-7 | PP-1 | 10.0 | A-1 | 56.0 | B-3 | 13.0 | C-1 | 21.0 | - | - | 12,800 | 1.72 |
| P-8 | PP-2 | 5.0 | A-1 | 65.0 | B-3 | 10.0 | C-1 | 20.0 | - | - | 12,600 | 1.73 |
| P-9 | PP-3 | 5.0 | A-1 | 65.0 | B-3 | 10.0 | C-1 | 20.0 | - | - | 12,700 | 1.74 |
| P-10 | PP-4 | 5.0 | A-1 | 65.0 | B-3 | 10.0 | C-1 | 20.0 | - | - | 12,400 | 1.75 |
| P-11 | PP-5 | 15.0 | A-1 | 55.0 | B-3 | 10.0 | C-1 | 20.0 | - | - | 12,600 | 1.74 |
| P-12 | PP-6 | 5.0 | A-1 | 65.0 | B-3 | 10.0 | C-1 | 20.0 | - | - | 12,600 | 1.76 |
| P-13 | PP-7 | 5.0 | A-1 | 65.0 | B-3 | 10.0 | C-1 | 20.0 | - | - | 12,300 | 1.72 |
| P-14 | PP-8 | 5.0 | A-1 | 65.0 | B-3 | 10.0 | C-1 | 20.0 | - | - | 12,400 | 1.74 |
| P-15 | PP-9 | 5.0 | A-1 | 65.0 | B-3 | 10.0 | C-1 | 20.0 | - | - | 12,500 | 1.75 |
| P-16 | PP-10 | 5.0 | A-1 | 65.0 | B-3 | 10.0 | C-1 | 20.0 | - | - | 12,400 | 1.73 |
| P-17 | PP-11 | 2.0 | A-1 | 68.0 | B-3 | 10.0 | C-1 | 20.0 | - | - | 12,300 | 1.75 |
| P-18 | PP-12 | 5.0 | A-1 | 65.0 | B-3 | 10.0 | C-1 | 20.0 | - | - | 12,400 | 1.72 |
| P-19 | PP-13 | 5.0 | A-1 | 65.0 | B-3 | 10.0 | C-1 | 20.0 | - | - | 12,600 | 1.74 |
| P-20 | PP-14 | 5.0 | A-1 | 65.0 | B-3 | 10.0 | C-1 | 20.0 | - | - | 12,100 | 1.76 |
| P-21 | PP-15 | 5.0 | A-1 | 65.0 | B-3 | 10.0 | C-1 | 20.0 | - | - | 12,200 | 1.76 |
| P-22 | PP-16 | 5.0 | A-1 | 65.0 | B-3 | 10.0 | C-1 | 20.0 | - | - | 12,100 | 1.75 |
| P-23 | PP-1 | 5.0 | A-1 | 65.0 | B-3 | 10.0 | C-2 | 20.0 | - | - | 12,300 | 1.74 |
| P-24 | PP-1 | 5.0 | A-1 | 65.0 | B-3 | 10.0 | C-3 | 20.0 | - | - | 12,900 | 1.74 |
| P-25 | PP-1 | 5.0 | A-1 | 65.0 | B-3 | 10.0 | C-4 | 20.0 | - | - | 12,800 | 1.75 |
| P-26 | PP-1 | 5.0 | A-1 | 65.0 | B-3 | 10.0 | C-5 | 20.0 | - | - | 12,700 | 1.76 |
| P-27 | PP-1 | 5.0 | A-1 | 65.0 | B-3 | 10.0 | C-6 | 20.0 | - | - | 12,600 | 1.76 |
| P-28 | PP-1 | 5.0 | A-1 | 65.0 | B-3 | 10.0 | C-7 | 20.0 | - | - | 12,500 | 1.74 |
| P-29 | PP-1 | 5.0 | A-1 | 65.0 | B-3 | 10.0 | C-8 | 20.0 | - | - | 12,200 | 1.73 |
| P-30 | PP-1 | 5.0 | A-1 | 65.0 | B-3 | 10.0 | C-9 | 20.0 | - | - | 12,300 | 1.72 |
| P-31 | PP-1 | 5.0 | A-1 | 65.0 | B-3 | 10.0 | C-10 | 20.0 | - | - | 12,400 | 1.72 |
| P-32 | PP-1 | 5.0 | A-1 | 65.0 | B-3 | 10.0 | C-11 | 20.0 | - | - | 12,600 | 1.73 |

**Table 2**

| | Unit 1 | Incorporation ratio (mol%) | Unit 2 | Incorporation ratio (mol%) | Unit 3 | Incorporation ratio (mol%) | Unit 4 | Incorporation ratio (mol%) | Unit 5 | Incorporation ratio (mol%) | Mw | Mw/Mn |
|---|---|---|---|---|---|---|---|---|---|---|---|---|
| P-33 | PP-1 | 5.0 | A-1 | 65.0 | B-3 | 10.0 | C-12 | 20.0 | - | - | 12,600 | 1.72 |
| P-34 | PP-1 | 5.0 | A-1 | 65.0 | B-3 | 10.0 | C-13 | 20.0 | - | - | 12,400 | 1.74 |
| P-35 | PP-1 | 5.0 | A-1 | 65.0 | B-3 | 10.0 | C-14 | 20.0 | - | - | 12,400 | 1.73 |
| P-36 | PP-1 | 5.0 | A-1 | 65.0 | B-3 | 10.0 | C-15 | 20.0 | - | - | 12,800 | 1.73 |
| P-37 | PP-1 | 5.0 | A-1 | 65.0 | B-3 | 10.0 | C-16 | 20.0 | - | - | 12,700 | 1.74 |
| P-38 | PP-1 | 5.0 | A-1 | 60.0 | B-3 | 10.0 | C-17 | 25.0 | - | - | 13,200 | 1.72 |
| P-39 | PP-1 | 5.0 | A-2 | 55.0 | B-2 | 12.0 | C-17 | 28.0 | - | - | 13,200 | 1.72 |
| P-40 | PP-1 | 5.0 | A-2 | 70.0 | B-3 | 10.0 | C-18 | 15.0 | - | - | 15,000 | 1.75 |
| P-41 | PP-1 | 5.0 | A-2 | 70.0 | B-3 | 10.0 | C-19 | 15.0 | - | - | 13,500 | 1.73 |
| P-42 | PP-1 | 5.0 | A-2 | 63.0 | B-3 | 10.0 | C-20 | 22.0 | - | - | 12,600 | 1.75 |
| P-43 | PP-1 | 5.0 | A-2 | 63.0 | B-3 | 10.0 | C-21 | 22.0 | - | - | 12,600 | 1.74 |
| P-44 | PP-1 | 5.0 | A-2 | 60.0 | B-3 | 10.0 | C-22 | 25.0 | - | - | 12,700 | 1.74 |
| P-45 | PP-1 | 5.0 | A-2 | 60.0 | B-3 | 10.0 | C-23 | 25.0 | - | - | 12,300 | 1.76 |
| P-46 | PP-1 | 5.0 | A-2 | 65.0 | B-3 | 10.0 | C-24 | 20.0 | - | - | 12,400 | 1.76 |
| P-47 | PP-1 | 5.0 | A-1 | 60.0 | B-3 | 10.0 | C-25 | 25.0 | - | - | 12,600 | 1.75 |
| P-48 | PP-1 | 5.0 | A-1 | 60.0 | B-3 | 10.0 | C-26 | 25.0 | - | - | 12,400 | 1.74 |
| P-49 | PP-1 | 5.0 | A-1 | 60.0 | B-3 | 10.0 | C-27 | 25.0 | - | - | 12,500 | 1.75 |
| P-50 | PP-1 | 5.0 | A-1 | 60.0 | B-3 | 10.0 | C-28 | 25.0 | - | - | 12,300 | 1.77 |
| P-51 | PP-1 | 5.0 | A-1 | 60.0 | B-3 | 10.0 | C-29 | 25.0 | - | - | 12,200 | 1.76 |
| P-52 | PP-1 | 5.0 | A-1 | 60.0 | B-3 | 10.0 | C-30 | 25.0 | - | - | 10,000 | 1.60 |
| P-53 | PP-1 | 5.0 | A-1 | 60.0 | B-3 | 10.0 | C-31 | 25.0 | - | - | 9,000 | 1.68 |
| P-54 | PP-1 | 5.0 | A-3 | 50.0 | B-3 | 15.0 | C-24 | 30.0 | - | - | 12,700 | 1.80 |
| P-55 | PP-1 | 5.0 | A-1 | 61.0 | B-2 | 10.0 | C-1 | 12.0 | C-27 | 12.0 | 12,600 | 1.76 |
| P-56 | PP-1 | 5.0 | A-1 | 61.0 | B-2 | 10.0 | C-7 | 12.0 | C-9 | 12.0 | 12,300 | 1.75 |
| P-57 | PP-1 | 5.0 | A-1 | 61.0 | B-2 | 10.0 | C-13 | 12.0 | C-15 | 12.0 | 11,500 | 1.72 |
| P-58 | PP-1 | 5.0 | A-2 | 60.0 | B-3 | 10.0 | C-1 | 25.0 | - | - | 14,700 | 1.73 |
| P-59 | PP-1 | 5.0 | A-2 | 60.0 | B-3 | 10.0 | C-7 | 25.0 | - | - | 14,500 | 1.72 |
| P-60 | PP-1 | 5.0 | A-2 | 60.0 | B-3 | 10.0 | C-9 | 25.0 | - | - | 14,600 | 1.76 |
| P-61 | PP-1 | 5.0 | A-2 | 60.0 | B-3 | 10.0 | C-17 | 25.0 | - | - | 13,600 | 1.75 |
| P-62 | PP-1 | 5.0 | A-2 | 60.0 | B-3 | 10.0 | C-32 | 25.0 | - | - | 10,000 | 1.73 |
| P-63 | PP-1 | 5.0 | A-2 | 72.0 | B-3 | 8.0 | C-33 | 15.0 | - | - | 11,500 | 1.72 |
| P-64 | PP-1 | 5.0 | A-2 | 72.0 | B-3 | 8.0 | C-34 | 18.0 | - | - | 11,000 | 1.73 |

**Table 3**

| | Unit 1 | Incorporation ratio (mol%) | Unit 2 | Incorporation ratio (mol%) | Unit 3 | Incorporation ratio (mol%) | Unit 4 | Incorporation ratio (mol%) | Unit 5 | Incorporation ratio (mol%) | Mw | Mw/Mn |
|---|---|---|---|---|---|---|---|---|---|---|---|---|
| P-65 | PP-1 | 5.0 | A-1 | 63.0 | B-3 | 12.0 | C-35 | 20.0 | - | - | 13,200 | 1.85 |
| P-66 | PP-1 | 5.0 | A-1 | 65.0 | C-36 | 30.0 | - | - | - | - | 12,800 | 1.93 |
| P-67 | PP-1 | 5.0 | A-1 | 63.0 | B-3 | 12.0 | C-37 | 20.0 | - | - | 12,200 | 1.79 |
| P-68 | PP-1 | 5.0 | A-1 | 68.0 | B-3 | 12.0 | C-38 | 15.0 | - | - | 13,400 | 1.81 |
| P-69 | PP-1 | 5.0 | A-1 | 75.0 | C-39 | 20.0 | - | - | - | - | 13,600 | 1.75 |
| P-70 | PP-1 | 5.0 | A-1 | 68.0 | B-3 | 12.0 | C-40 | 15.0 | - | - | 12,100 | 1.82 |
| P-71 | PP-1 | 5.0 | A-1 | 68.0 | B-3 | 12.0 | C-41 | 15.0 | - | - | 11,000 | 1.87 |
| P-72 | PP-1 | 5.0 | A-1 | 63.0 | B-3 | 12.0 | C-42 | 20.0 | - | - | 14,200 | 1.95 |
| P-73 | PP-1 | 5.0 | A-1 | 66.0 | B-3 | 12.0 | C-43 | 17.0 | - | - | 13,000 | 1.86 |
| P-74 | PP-1 | 5.0 | A-1 | 66.0 | B-3 | 12.0 | C-44 | 17.0 | - | - | 12,300 | 1.88 |
| P-75 | PP-1 | 5.0 | A-1 | 66.0 | B-3 | 12.0 | C-45 | 17.0 | - | - | 12,000 | 1.78 |

The repeat units in the polymers have the structure shown below.

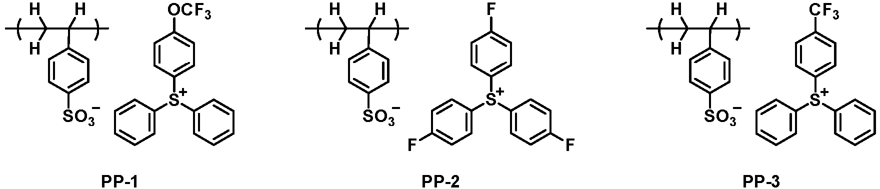

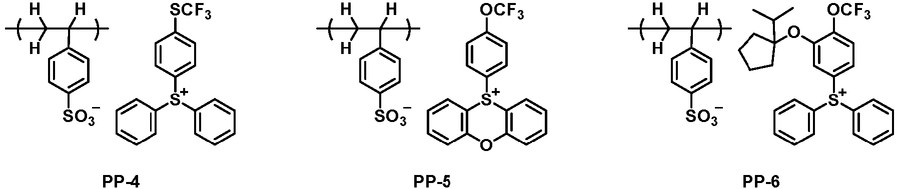

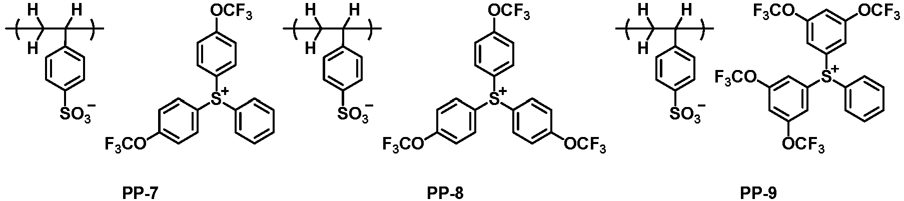

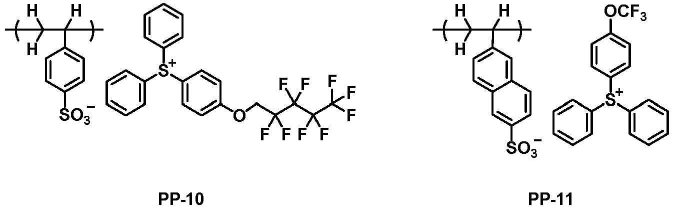

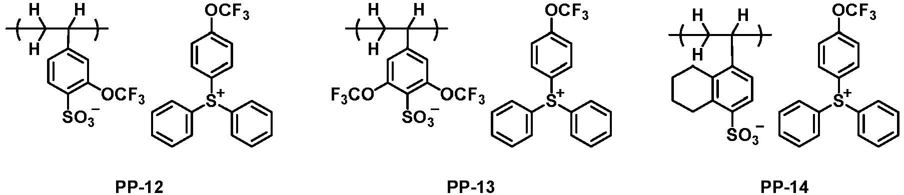

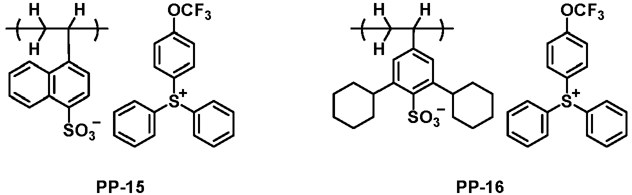

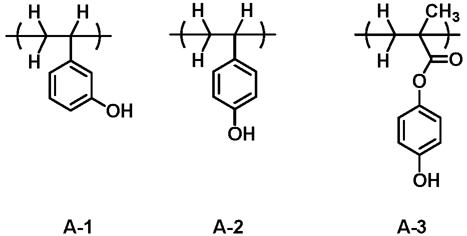

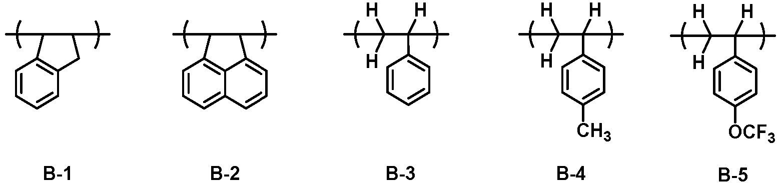

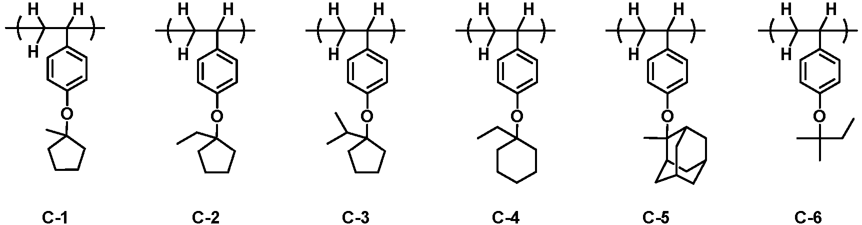

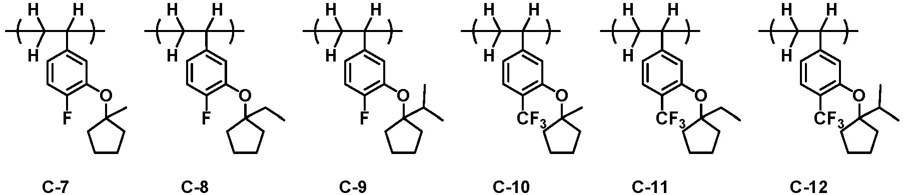

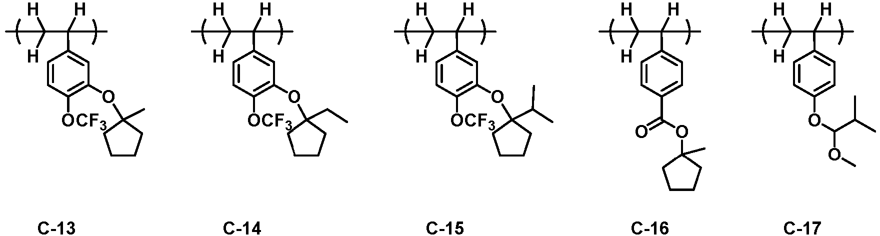

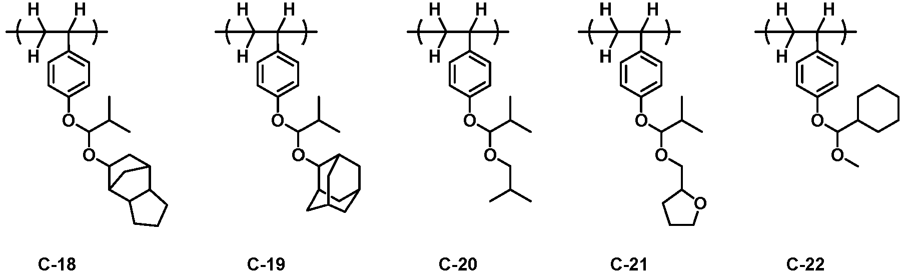

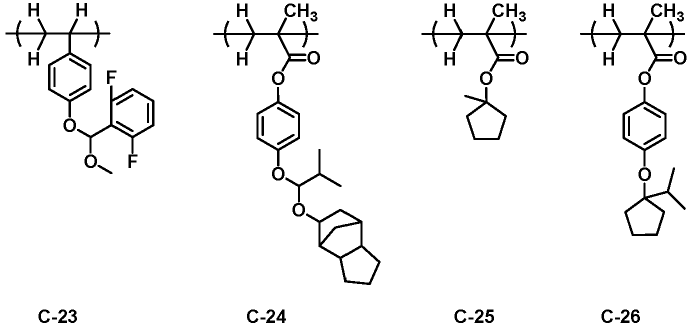

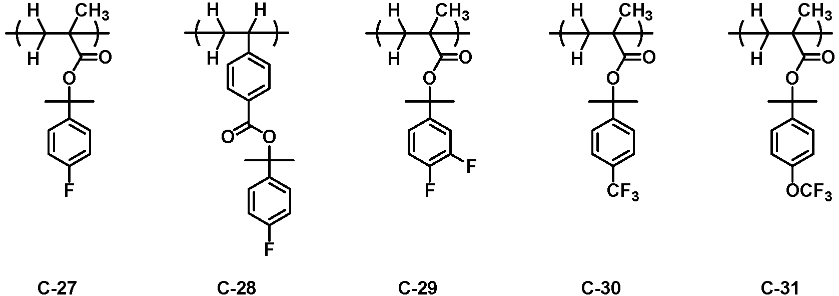

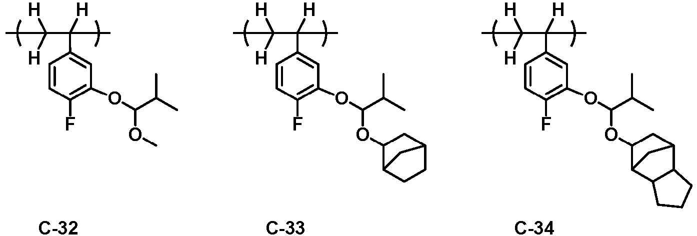

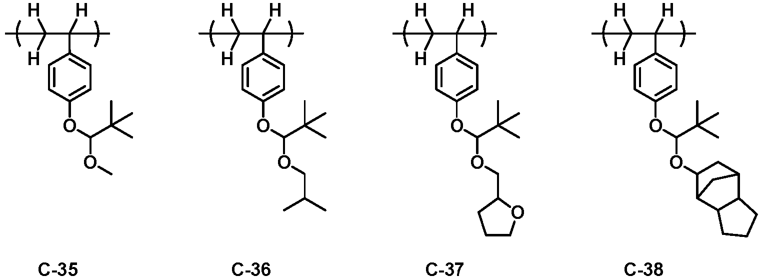

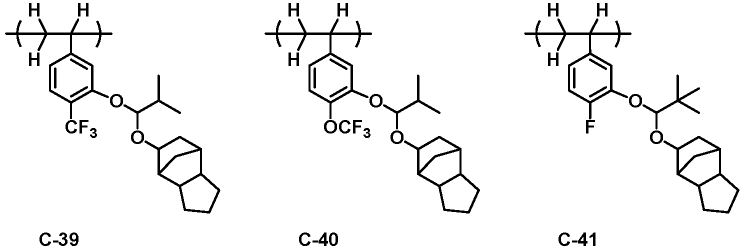

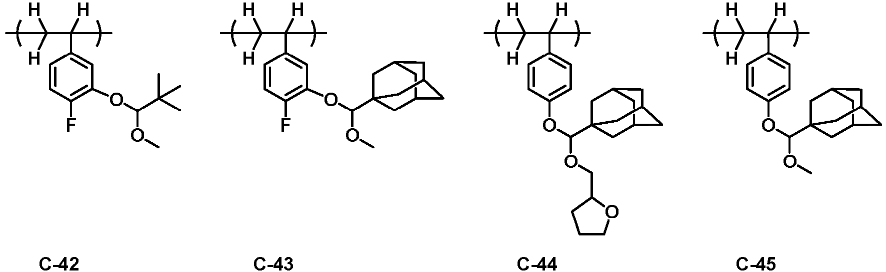

The dissolution rate of a polymer in alkaline developer was computed by spin coating a 16.7 wt% solution of a polymer in PGME solvent onto a 8-inch silicon wafer, baking at 100°C for 90 seconds to form a film of 1,000 nm thick, developing the film in a 2.38 wt% aqueous TMAH solution at 23°C for 100 seconds, and measuring a loss of film thickness. Polymers P-1 to P-75 had a dissolution rate of less than 10 nm/min.

### Synthesis Examples 1-1 to 1-16 and Comparative Examples 1-1 to 1-4

### Synthesis of Polymers AP-1 to AP-16 and Comparative Polymers cP-1 to cP-4

Polymers AP-1 to AP-16 and Comparative Polymers cP-1 to cP-4 were synthesized by the same procedure as in Example 1-2 except that the monomers were changed.

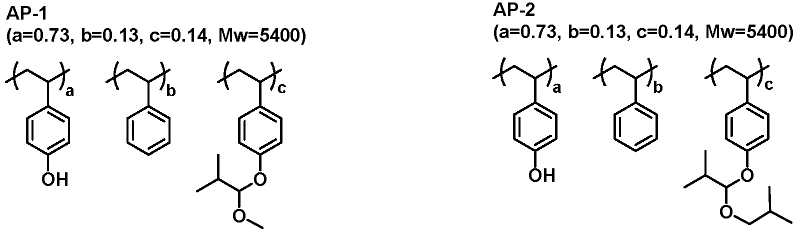

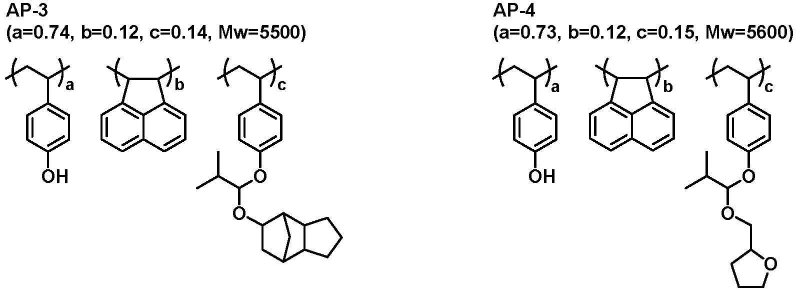

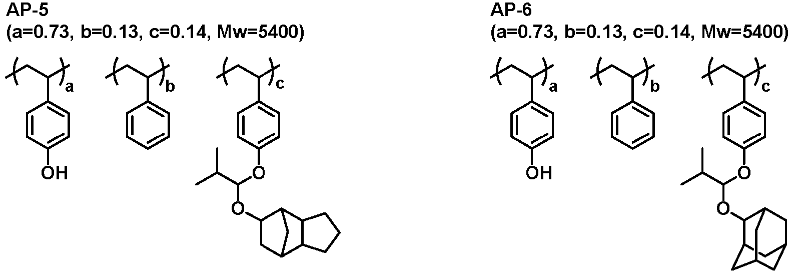

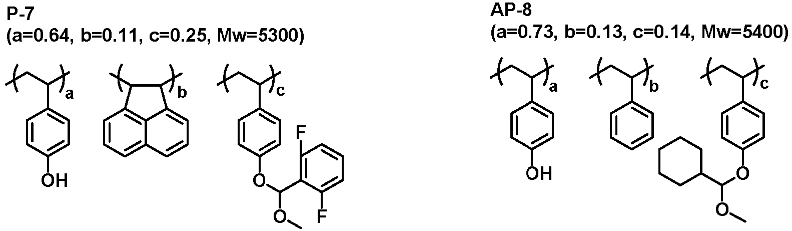

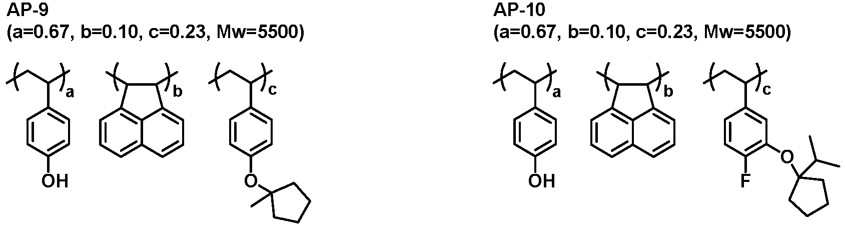

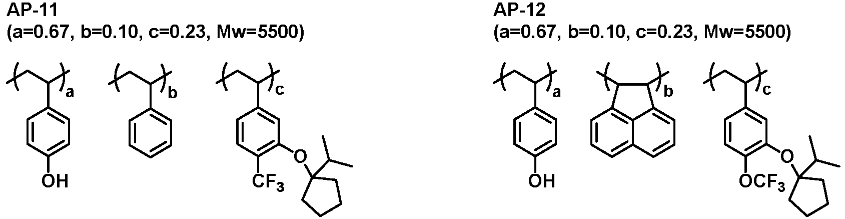

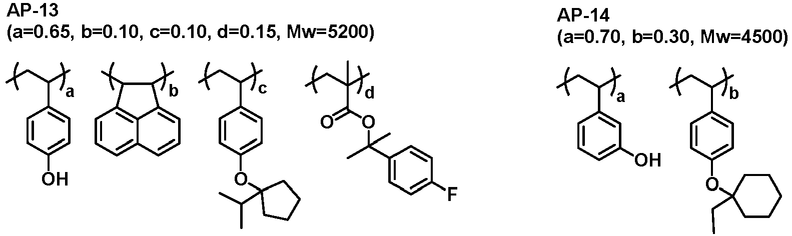

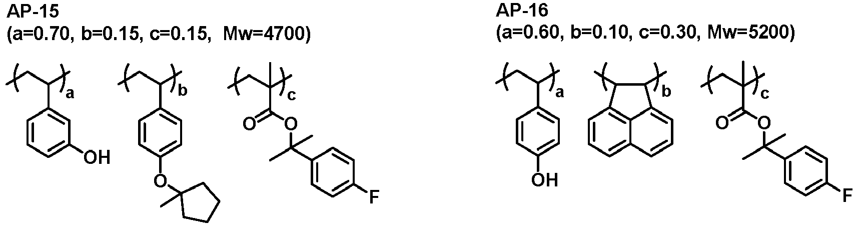

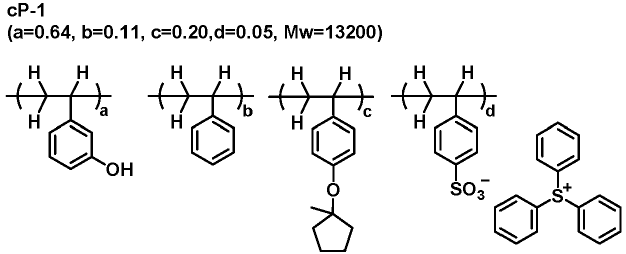

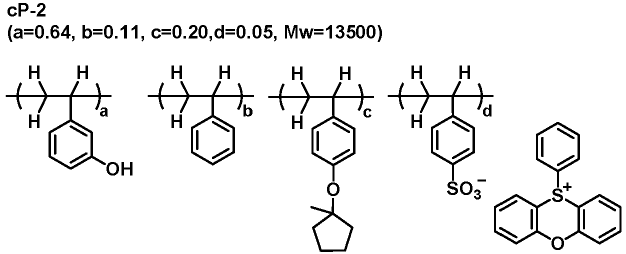

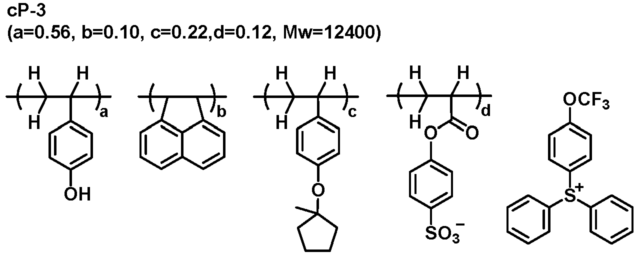

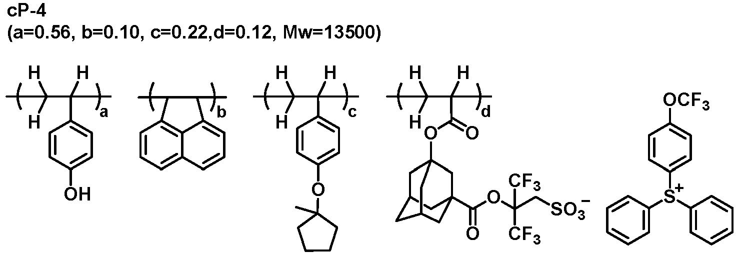

Polymers AP-1 to AP-16 and Comparative Polymers cP-1 to cP-4 had a dissolution rate of less than 10 nm/min.

### [2] Preparation of resist compositions

### Examples 2-1 to 2-111 and Comparative Examples 2-1 to 2-5

A chemically amplified positive resist composition (R-1 to R-111, CR-1 to CR-5) was prepared by dissolving selected components in an organic solvent in accordance with the formulation shown in Tables 4 to 8, and filtering the solution through a nylon filter with a pore size of 5 nm and a UPE filter with a sub-nanometer size. The organic solvent was a mixture of 920 parts by weight of PGMEA, 1,850 parts by weight of EL, 1,850 parts by weight of diacetone alcohol, and 1,850 parts by weight of PGME.

**Table 4**

| | | Resist composition | Polymer 1 (pbw) | Polymer 2 (pbw) | Photoacid generator (pbw) | Quencher (pbw) | Additive (pbw) |
|---|---|---|---|---|---|---|---|
| Example | 2-1 | R-1 | P-1 (80) | - | - | Q-1 (8.0) | - |
| | 2-2 | R-2 | P-1 (80) | - | - | Q-1 (8.0) | D-1 (1.5) |
| | 2-3 | R-3 | P-1 (80) | - | - | Q-1 (8.0) | D-2 (1.5) |
| | 2-4 | R-4 | P-1 (80) | - | - | Q-1 (8.0) | D-3 (3.0) |
| | 2-5 | R-5 | P-1 (80) | - | - | Q-1 (8.0) | D-4 (1.5) |
| | 2-6 | R-6 | P-1 (80) | - | - | Q-1 (8.0) | D-5 (1.5) |
| | 2-7 | R-7 | P-2 (80) | - | - | Q-1 (8.0) | D-1 (1.5) |
| | 2-8 | R-8 | P-3 (80) | - | - | Q-1 (8.0) | D-1 (1.5) |
| | 2-9 | R-9 | P-4 (80) | - | - | Q-1 (8.0) | D-1 (1.5) |
| | 2-10 | R-10 | P-5 (80) | - | - | Q-1 (8.0) | D-1 (1.5) |
| | 2-11 | R-11 | P-6 (80) | - | - | Q-1 (8.0) | D-1 (1.5) |
| | 2-12 | R-12 | P-7 (80) | - | - | Q-1 (10.0) | D-1 (1.5) |
| | 2-13 | R-13 | P-8 (80) | - | - | Q-1 (8.0) | D-1 (1.5) |
| | 2-14 | R-14 | P-9 (80) | - | - | Q-1 (8.0) | D-1 (1.5) |
| | 2-15 | R-15 | P-10 (80) | - | - | Q-1 (8.0) | D-1 (5.0) |
| | 2-16 | R-16 | P-11 (80) | - | - | Q-1 (12.0) | D-1 (1.5) |
| | 2-17 | R-17 | P-12 (80) | - | - | Q-1 (8.0) | D-1 (1.5) |
| | 2-18 | R-18 | P-13 (80) | - | - | Q-1 (8.0) | D-1 (1.5) |
| | 2-19 | R-19 | P-14 (80) | - | - | Q-1 (8.0) | D-1 (1.5) |
| | 2-20 | R-20 | P-15 (80) | - | - | Q-1 (8.0) | D-1 (1.5) |
| | 2-21 | R-21 | P-16 (80) | - | - | Q-1 (8.0) | D-1 (1.5) |
| | 2-22 | R-22 | P-17 (80) | - | - | Q-1 (8.0) | D-1 (1.5) |
| | 2-23 | R-23 | P-18 (80) | - | - | Q-1 (8.0) | D-1 (1.5) |
| | 2-24 | R-24 | P-19 (80) | - | - | Q-1 (8.0) | D-1 (1.5) |
| | 2-25 | R-25 | P-20 (80) | - | - | Q-1 (8.0) | D-1 (1.5) |
| | 2-26 | R-26 | P-21 (80) | - | - | Q-1 (8.0) | D-1 (1.5) |
| | 2-27 | R-27 | P-22 (80) | - | - | Q-1 (8.0) | D-1 (1.5) |
| | 2-28 | R-28 | P-23 (80) | - | - | Q-1 (8.0) | D-1 (1.5) |
| | 2-29 | R-29 | P-24 (80) | - | - | Q-1 (8.0) | D-1 (1.5) |
| | 2-30 | R-30 | P-25 (80) | - | - | Q-1 (8.0) | D-1 (1.5) |
| | 2-31 | R-31 | P-26 (80) | - | - | Q-1 (8.0) | D-1 (1.5) |
| | 2-32 | R-32 | P-27 (80) | - | - | Q-1 (8.0) | D-1 (1.5) |
| | 2-33 | R-33 | P-28 (80) | - | - | Q-1 (8.0) | D-1 (1.5) |
| | 2-34 | R-34 | P-29 (80) | - | - | Q-1 (8.0) | D-1 (1.5) |
| | 2-35 | R-35 | P-30 (80) | - | - | Q-1 (8.0) | D-1 (1.5) |

**Table 5**

| | | Resist composition | Polymer 1 (pbw) | Polymer 2 (pbw) | Photoacid generator (pbw) | Quencher (pbw) | Additive (pbw) |
|---|---|---|---|---|---|---|---|
| Example | 2-36 | R-36 | P-31 (80) | - | - | Q-1 (8.0) | D-1 (1.5) |
| | 2-37 | R-37 | P-32 (80) | - | - | Q-1 (8.0) | D-1 (1.5) |
| | 2-38 | R-38 | P-33 (80) | - | - | Q-1 (8.0) | D-1 (1.5) |
| | 2-39 | R-39 | P-34 (80) | - | - | Q-1 (8.0) | D-1 (1.5) |
| | 2-40 | R-40 | P-35 (80) | - | - | Q-1 (8.0) | D-1 (1.5) |
| | 2-41 | R-41 | P-36 (80) | - | - | Q-1 (8.0) | D-1 (1.5) |
| | 2-42 | R-42 | P-37 (80) | - | - | Q-1 (8.0) | D-1 (1.5) |
| | 2-43 | R-43 | P-38 (80) | - | - | Q-1 (8.0) | D-1 (1.5) |
| | 2-44 | R-44 | P-39 (80) | - | - | Q-1 (8.0) | D-1 (1.5) |
| | 2-45 | R-45 | P-40 (80) | - | - | Q-1 (8.0) | D-1 (1.5) |
| | 2-46 | R-46 | P-41 (80) | - | - | Q-1 (8.0) | D-1 (1.5) |
| | 2-47 | R-47 | P-42 (80) | - | - | Q-1 (8.0) | D-1 (1.5) |
| | 2-48 | R-48 | P-43 (80) | - | - | Q-1 (8.0) | D-1 (1.5) |
| | 2-49 | R-49 | P-44 (80) | - | - | Q-1 (8.0) | D-1 (1.5) |
| | 2-50 | R-50 | P-45 (80) | - | - | Q-1 (8.0) | D-1 (1.5) |
| | 2-51 | R-51 | P-46 (80) | - | - | Q-1 (8.0) | D-1 (1.5) |
| | 2-52 | R-52 | P-47 (80) | - | - | Q-1 (8.0) | D-1 (1.5) |
| | 2-53 | R-53 | P-48 (80) | - | - | Q-1 (8.0) | D-1 (1.5) |
| | 2-54 | R-54 | P-49 (80) | - | - | Q-1 (8.0) | D-1 (1.5) |
| | 2-55 | R-55 | P-50 (80) | - | - | Q-1 (8.0) | D-1 (1.5) |
| | 2-56 | R-56 | P-51 (80) | - | - | Q-1 (8.0) | D-1 (1.5) |
| | 2-57 | R-57 | P-52 (80) | - | - | Q-1 (8.0) | D-1 (1.5) |
| | 2-58 | R-58 | P-53 (80) | - | - | Q-1 (8.0) | D-1 (1.5) |
| | 2-59 | R-59 | P-54 (80) | - | - | Q-1 (8.0) | D-1 (1.5) |
| | 2-60 | R-60 | P-55 (80) | - | - | Q-1 (8.0) | D-1 (1.5) |
| | 2-61 | R-61 | P-56 (80) | - | - | Q-1 (8.0) | D-1 (1.5) |
| | 2-62 | R-62 | P-57 (80) | - | - | Q-1 (8.0) | D-1 (1.5) |
| | 2-63 | R-63 | P-4 (40) | AP-1 (40) | - | Q-1 (8.0) | D-1 (1.5) |
| | 2-64 | R-64 | P-4 (40) | AP-1 (40) | PAG-1 (5.0) | Q-1 (8.0) | D-1 (1.5) |
| | 2-65 | R-65 | P-4 (40) | AP-2 (40) | PAG-1 (5.0) | Q-1 (8.0) | D-1 (1.5) |
| | 2-66 | R-66 | P-4 (40) | AP-3 (40) | PAG-1 (5.0) | Q-1 (8.0) | D-1 (1.5) |
| | 2-67 | R-67 | P-4 (40) | AP-4 (40) | PAG-1 (5.0) | Q-1 (8.0) | D-1 (1.5) |
| | 2-68 | R-68 | P-4 (40) | AP-5 (40) | PAG-1 (5.0) | Q-1 (8.0) | D-1 (1.5) |
| | 2-69 | R-69 | P-4 (40) | AP-6 (40) | PAG-1 (5.0) | Q-1 (8.0) | D-1 (1.5) |
| | 2-70 | R-70 | P-4 (40) | AP-7 (40) | PAG-1 (5.0) | Q-1 (8.0) | D-1 (1.5) |

**Table 6**

| | | Resist composition | Polymer 1 (pbw) | Polymer 2 (pbw) | Photoacid generator (pbw) | Quencher (pbw) | Additive (pbw) |
|---|---|---|---|---|---|---|---|
| Example | 2-71 | R-71 | P-4 (40) | AP-8 (40) | PAG-1 (10.0) | Q-1 (10.0) | D-1 (1.5) |
| | 2-72 | R-72 | P-4 (40) | AP-9 (40) | PAG-1 (5.0) | Q-1 (8.0) | D-1 (1.5) |
| | 2-73 | R-73 | P-4 (40) | AP-10 (40) | PAG-1 (5.0) | Q-1 (8.0) | D-1 (1.5) |
| | 2-74 | R-74 | P-4 (40) | AP-11 (40) | PAG-1 (3.0) | Q-1 (8.0) | D-1 (1.5) |
| | | | | | PAG-6 (2.0) | | |
| | 2-75 | R-75 | P-4 (40) | AP-12 (40) | PAG-1 (5.0) | Q-1 (8.0) | D-1 (1.5) |
| | 2-76 | R-76 | P-4 (40) | AP-13 (40) | PAG-1 (5.0) | Q-1 (8.0) | D-1 (1.5) |
| | 2-77 | R-77 | P-4 (20) | AP-14 (60) | PAG-1 (8.0) | Q-1 (10.0) | D-1 (1.5) |
| | 2-78 | R-78 | P-4 (60) | AP-15 (20) | PAG-1 (2.0) | Q-1 (8.0) | D-1 (1.5) |
| | 2-79 | R-79 | P-4 (40) | AP-16 (40) | PAG-1 (5.0) | Q-1 (8.0) | D-1 (1.5) |
| | 2-80 | R-80 | P-38 (40) | AP-1 (40) | PAG-1 (5.0) | Q-1 (8.0) | D-1 (1.5) |
| | 2-81 | R-81 | P-38 (40) | AP-4 (40) | PAG-1 (5.0) | Q-1 (8.0) | D-1 (1.5) |
| | 2-82 | R-82 | P-4 (80) | - | - | Q-2 (8.0) | D-1 (1.5) |
| | 2-83 | R-83 | P-4 (80) | - | - | Q-3 (8.0) | D-1 (1.5) |
| | 2-84 | R-84 | P-4 (80) | - | - | Q-4 (8.0) | D-1 (1.5) |
| | 2-85 | R-85 | P-4 (80) | - | PAG-1 (5.0) | Q-1 (10.0) | D-1 (1.5) |
| | 2-86 | R-86 | P-4 (80) | - | PAG-2 (5.0) | Q-1 (10.0) | D-1 (1.5) |
| | 2-87 | R-87 | P-4 (80) | - | PAG-3 (8.0) | Q-1 (12.0) | D-1 (1.5) |
| | 2-88 | R-88 | P-4 (80) | - | PAG-4 (3.0) | Q-1 (10.0) | D-1 (1.5) |
| | | | | | PAG-6 (2.0) | | |
| | 2-89 | R-89 | P-4 (80) | - | PAG-5 (2.0) | Q-1 (10.0) | D-1 (1.5) |
| | 2-90 | R-90 | P-4 (80) | - | - | Q-1 (14.0) | D-1 (1.5) |
| | 2-91 | R-91 | P-4 (80) | - | - | Q-1 (5.0) | D-1 (1.5) |
| | 2-92 | R-92 | P-58 (80) | - | - | Q-1 (8.0) | D-1 (1.5) |
| | 2-93 | R-93 | P-59 (80) | - | - | Q-1 (8.0) | D-1 (1.5) |
| | 2-94 | R-94 | P-60 (80) | - | - | Q-1 (8.0) | D-1 (1.5) |
| | 2-95 | R-95 | P-61 (80) | - | - | Q-1 (8.0) | D-1 (1.5) |
| | 2-96 | R-96 | P-61 (80) | - | - | Q-5 (8.0) | D-1 (1.5) |
| | 2-97 | R-97 | P-61 (80) | - | - | Q-6 (8.0) | D-1 (1.5) |
| | 2-98 | R-98 | P-62 (80) | - | - | Q-6 (8.0) | D-1 (1.5) |
| | 2-99 | R-99 | P-63 (80) | - | - | Q-6 (8.0) | D-1 (1.5) |
| | 2-100 | R-100 | P-64 (80) | - | - | Q-6 (8.0) | D-1 (1.5) |

**Table 7**

| | | Resist composition | Polymer 1 (pbw) | Polymer 2 (pbw) | Photoacid generator (pbw) | Quencher (pbw) | Additive (pbw) |
|---|---|---|---|---|---|---|---|
| Example | 2-101 | R-101 | P-65 (80) | - | - | Q-1 (8.0) | D-1 (1.5) |
| | 2-102 | R-102 | P-66 (80) | - | - | Q-1 (8.0) | D-1 (1.5) |
| | 2-103 | R-103 | P-67 (80) | - | - | Q-1 (8.0) | D-1 (1.5) |
| | 2-104 | R-104 | P-68 (80) | - | - | Q-1 (8.0) | D-1 (1.5) |
| | 2-105 | R-105 | P-69 (80) | - | - | Q-1 (8.0) | D-1 (1.5) |
| | 2-106 | R-106 | P-70 (80) | - | PAG-3 (4.0) | Q-1 (8.0) | D-1 (1.5) |
| | 2-107 | R-107 | P-71 (80) | - | - | Q-1 (8.0) | D-1 (1.5) |
| | 2-108 | R-108 | P-72 (80) | - | - | Q-1 (8.0) | D-1 (1.5) |
| | 2-109 | R-109 | P-73 (80) | - | - | Q-1 (8.0) | D-1 (1.5) |
| | 2-110 | R-110 | P-74 (40) | AP-5 (40) | PAG-1 (6.0) | Q-1 (8.0) | D-1 (1.5) |
| | 2-111 | R-111 | P-75 (80) | - | - | Q-1 (8.0) | D-1 (1.5) |

**Table 8**

| | | Resist composition | Polymer 1 (pbw) | Polymer 2 (pbw) | Photoacid generator (pbw) | Quencher (pbw) | Additive (pbw) |
|---|---|---|---|---|---|---|---|
| Comparative Example | 2-1 | CR-1 | AP-9 (80) | - | PAG-1 (8) | Q-1 (7.0) | D-1 (1.5) |
| | | | | | PAG-6 (2) | | |
| | 2-2 | CR-2 | cP-1 (80) | - | - | Q-1 (8.0) | D-1 (1.5) |
| | 2-3 | CR-3 | cP-2 (80) | - | - | Q-1 (8.0) | D-1 (1.5) |
| | 2-4 | CR-4 | cP-3 (80) | - | - | Q-1 (8.0) | D-1 (1.5) |
| | 2-5 | CR-5 | cP-4 (80) | - | - | Q-1 (8.0) | D-1 (1.5) |

Quenchers Q-1 to Q-6, photoacid generators PAG-1 to PAG-6, and polymers D-1 to D-5 in Tables 4 to 8 are identified below.

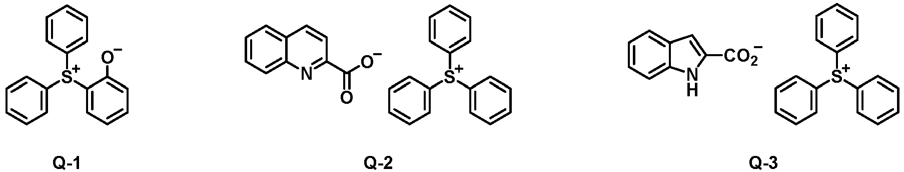

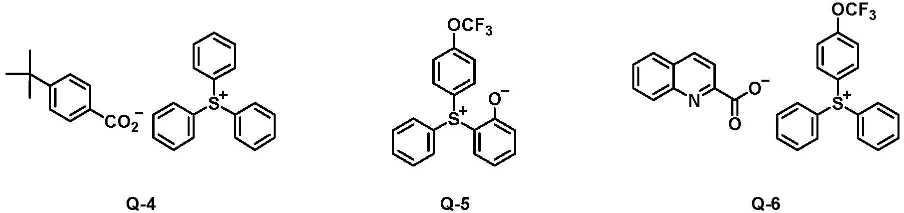

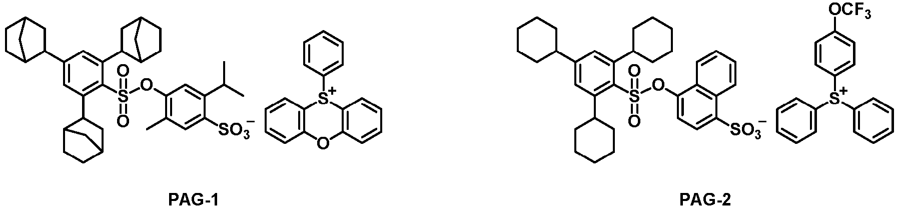

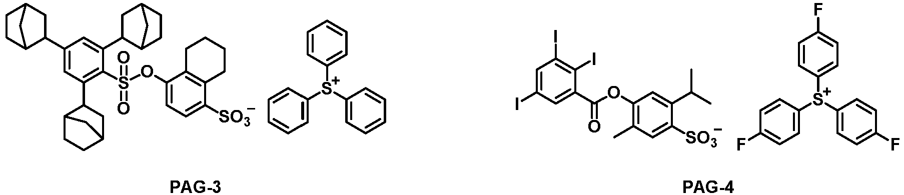

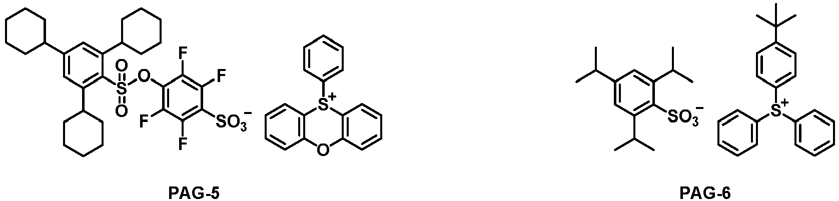

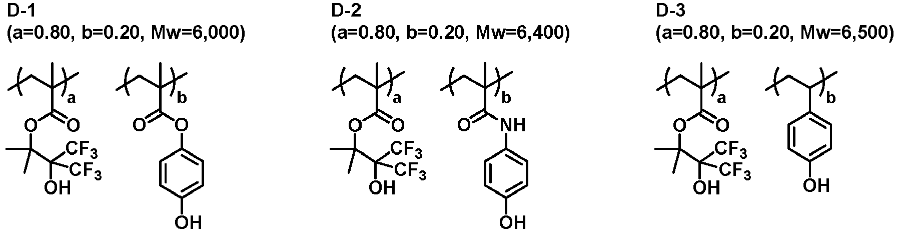

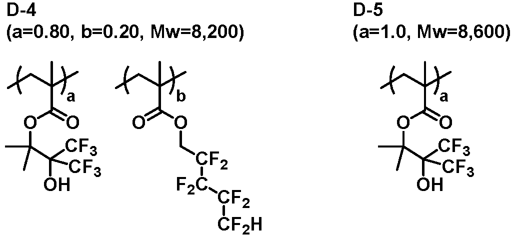

### EUV lithography test

### Examples 3-1 to 3-111 and Comparative Examples 3-1 to 3-5

Using a coater/developer system ACT-M (Tokyo Electron Ltd.), each of the positive resist compositions (R-1 to R-111, CR-1 to CR-5) was spin coated onto a reflection type mask blank (for EUV lithography mask, 152 mm square, outermost surface of chromium compound) and prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding an outer rim portion extending 10 mm inward from the periphery, and an average film thickness and a film thickness range were computed therefrom.

The resist film was exposed to EB using an EB writer system EBM-5000Plus (NuFlare Technology Inc., accelerating voltage 50 kV), then baked (PEB) at 110°C for 600 seconds, and developed in a 2.38 wt% TMAH aqueous solution, thereby yielding a positive pattern.

The resist pattern was evaluated as follows. The patterned mask blank was observed under a top-down scanning electron microscope (TD-SEM). The optimum dose (Eop) was defined as the exposure dose (µC/cm²) which provided a 1:1 resolution at the top and bottom of a 200-nm 1:1 line-and-space (LS) pattern. The resolution (or maximum resolution) of LS was defined as the minimum size at the optimum dose. The resolution (or maximum resolution) of isolated space (IS) was defined as the minimum size at the dose which provided a 9:1 resolution for a 200-nm 9:1 line-and-space pattern. Also, the pattern was visually observed to judge whether or not the profile was rectangular. The results are shown in Tables 9 to 13.

**Table 9**

| | | Resist composition | Eop (µC/cm²) | Maximum resolution of LS (nm) | Maximum resolution of IS (nm) | LER (nm) | Pattern profile |
|---|---|---|---|---|---|---|---|
| Example | 3-1 | R-1 | 220 | 40 | 24 | 3.8 | rectangular |
| | 3-2 | R-2 | 210 | 40 | 22 | 3.6 | rectangular |
| | 3-3 | R-3 | 210 | 40 | 22 | 3.7 | rectangular |
| | 3-4 | R-4 | 210 | 40 | 22 | 3.6 | rectangular |
| | 3-5 | R-5 | 210 | 40 | 22 | 3.8 | rectangular |
| | 3-6 | R-6 | 210 | 40 | 22 | 3.6 | rectangular |
| | 3-7 | R-7 | 210 | 35 | 20 | 3.7 | rectangular |
| | 3-8 | R-8 | 210 | 35 | 20 | 3.5 | rectangular |
| | 3-9 | R-9 | 210 | 35 | 20 | 3.5 | rectangular |
| | 3-10 | R-10 | 210 | 35 | 20 | 3.6 | rectangular |
| | 3-11 | R-11 | 210 | 35 | 20 | 3.6 | rectangular |
| | 3-12 | R-12 | 200 | 35 | 20 | 3.6 | rectangular |
| | 3-13 | R-13 | 210 | 35 | 20 | 3.6 | rectangular |
| | 3-14 | R-14 | 210 | 35 | 20 | 3.5 | rectangular |
| | 3-15 | R-15 | 210 | 35 | 20 | 3.6 | rectangular |
| | 3-16 | R-16 | 190 | 35 | 20 | 3.5 | rectangular |
| | 3-17 | R-17 | 210 | 35 | 20 | 3.5 | rectangular |
| | 3-18 | R-18 | 210 | 35 | 20 | 3.4 | rectangular |
| | 3-19 | R-19 | 210 | 35 | 20 | 3.5 | rectangular |
| | 3-20 | R-20 | 210 | 35 | 20 | 3.6 | rectangular |
| | 3-21 | R-21 | 210 | 35 | 20 | 3.7 | rectangular |
| | 3-22 | R-22 | 230 | 35 | 20 | 3.7 | rectangular |
| | 3-23 | R-23 | 210 | 35 | 20 | 3.5 | rectangular |
| | 3-24 | R-24 | 210 | 35 | 20 | 3.6 | rectangular |
| | 3-25 | R-25 | 210 | 35 | 20 | 3.7 | rectangular |
| | 3-26 | R-26 | 210 | 35 | 20 | 3.7 | rectangular |
| | 3-27 | R-27 | 210 | 35 | 20 | 3.6 | rectangular |
| | 3-28 | R-28 | 210 | 35 | 20 | 3.8 | rectangular |
| | 3-29 | R-29 | 210 | 35 | 20 | 3.5 | rectangular |
| | 3-30 | R-30 | 210 | 35 | 20 | 3.7 | rectangular |
| | 3-31 | R-31 | 210 | 35 | 20 | 3.5 | rectangular |
| | 3-32 | R-32 | 230 | 35 | 22 | 3.6 | rectangular |
| | 3-33 | R-33 | 210 | 35 | 20 | 3.4 | rectangular |
| | 3-34 | R-34 | 210 | 35 | 20 | 3.5 | rectangular |
| | 3-35 | R-35 | 210 | 35 | 20 | 3.4 | rectangular |

**Table 10**

| | | Resist composition | Eop (µC/cm²) | Maximum resolution of LS (nm) | Maximum resolution of IS (nm) | LER (nm) | Pattern profile |
|---|---|---|---|---|---|---|---|
| Example | 3-36 | R-36 | 210 | 35 | 20 | 3.5 | rectangular |
| | 3-37 | R-37 | 210 | 35 | 20 | 3.4 | rectangular |
| | 3-38 | R-38 | 210 | 35 | 20 | 3.6 | rectangular |
| | 3-39 | R-39 | 210 | 35 | 20 | 3.4 | rectangular |
| | 3-40 | R-40 | 210 | 35 | 20 | 3.5 | rectangular |
| | 3-41 | R-41 | 210 | 35 | 20 | 3.4 | rectangular |
| | 3-42 | R-42 | 210 | 35 | 20 | 3.4 | rectangular |
| | 3-43 | R-43 | 210 | 35 | 20 | 3.6 | rectangular |
| | 3-44 | R-44 | 210 | 35 | 20 | 3.5 | rectangular |
| | 3-45 | R-45 | 210 | 35 | 20 | 3.4 | rectangular |
| | 3-46 | R-46 | 210 | 35 | 20 | 3.5 | rectangular |
| | 3-47 | R-47 | 210 | 35 | 20 | 3.5 | rectangular |
| | 3-48 | R-48 | 210 | 35 | 20 | 3.4 | rectangular |
| | 3-49 | R-49 | 210 | 35 | 20 | 3.5 | rectangular |
| | 3-50 | R-50 | 210 | 35 | 20 | 3.6 | rectangular |
| | 3-51 | R-51 | 210 | 35 | 20 | 3.7 | rectangular |
| | 3-52 | R-52 | 220 | 40 | 22 | 3.3 | rectangular |
| | 3-53 | R-53 | 220 | 40 | 22 | 3.5 | rectangular |
| | 3-54 | R-54 | 210 | 35 | 20 | 3.6 | rectangular |
| | 3-55 | R-55 | 210 | 35 | 20 | 3.7 | rectangular |
| | 3-56 | R-56 | 210 | 35 | 20 | 3.7 | rectangular |
| | 3-57 | R-57 | 210 | 35 | 20 | 3.6 | rectangular |
| | 3-58 | R-58 | 210 | 35 | 20 | 3.6 | rectangular |
| | 3-59 | R-59 | 210 | 35 | 20 | 3.5 | rectangular |
| | 3-60 | R-60 | 210 | 35 | 20 | 3.5 | rectangular |
| | 3-61 | R-61 | 210 | 35 | 20 | 3.5 | rectangular |
| | 3-62 | R-62 | 230 | 35 | 22 | 3.6 | rectangular |
| | 3-63 | R-63 | 210 | 35 | 22 | 3.4 | rectangular |
| | 3-64 | R-64 | 210 | 35 | 20 | 3.5 | rectangular |
| | 3-65 | R-65 | 210 | 35 | 20 | 3.4 | rectangular |
| | 3-66 | R-66 | 210 | 35 | 20 | 3.5 | rectangular |
| | 3-67 | R-67 | 210 | 35 | 20 | 3.4 | rectangular |
| | 3-68 | R-68 | 210 | 35 | 20 | 3.6 | rectangular |
| | 3-69 | R-69 | 210 | 35 | 20 | 3.4 | rectangular |
| | 3-70 | R-70 | 210 | 35 | 20 | 3.5 | rectangular |

**Table 11**

| | | Resist composition | Eop (µC/cm²) | Maximum resolution of LS (nm) | Maximum resolution of IS (nm) | LER (nm) | Pattern profile |
|---|---|---|---|---|---|---|---|
| Example | 3-71 | R-71 | 210 | 35 | 20 | 3.4 | rectangular |
| | 3-72 | R-72 | 210 | 35 | 20 | 3.4 | rectangular |
| | 3-73 | R-73 | 210 | 35 | 20 | 3.6 | rectangular |
| | 3-74 | R-74 | 210 | 35 | 20 | 3.5 | rectangular |
| | 3-75 | R-75 | 210 | 35 | 20 | 3.4 | rectangular |
| | 3-76 | R-76 | 210 | 35 | 20 | 3.5 | rectangular |
| | 3-77 | R-77 | 210 | 35 | 20 | 3.5 | rectangular |
| | 3-78 | R-78 | 210 | 35 | 20 | 3.4 | rectangular |
| | 3-79 | R-79 | 210 | 35 | 20 | 3.5 | rectangular |
| | 3-80 | R-80 | 210 | 35 | 20 | 3.6 | rectangular |
| | 3-81 | R-81 | 210 | 35 | 20 | 3.7 | rectangular |
| | 3-82 | R-82 | 220 | 40 | 22 | 3.3 | rectangular |
| | 3-83 | R-83 | 220 | 40 | 22 | 3.5 | rectangular |
| | 3-84 | R-84 | 210 | 35 | 20 | 3.6 | rectangular |
| | 3-85 | R-85 | 170 | 35 | 20 | 3.7 | rectangular |
| | 3-86 | R-86 | 170 | 35 | 20 | 3.7 | rectangular |
| | 3-87 | R-87 | 170 | 35 | 20 | 3.6 | rectangular |
| | 3-88 | R-88 | 170 | 35 | 20 | 3.6 | rectangular |
| | 3-89 | R-89 | 150 | 35 | 20 | 3.5 | rectangular |
| | 3-90 | R-90 | 400 | 35 | 20 | 3.3 | rectangular |
| | 3-91 | R-91 | 100 | 35 | 20 | 4.0 | rectangular |
| | 3-92 | R-92 | 210 | 35 | 20 | 3.7 | rectangular |
| | 3-93 | R-93 | 210 | 35 | 20 | 3.6 | rectangular |
| | 3-94 | R-94 | 210 | 35 | 20 | 3.7 | rectangular |
| | 3-95 | R-95 | 210 | 35 | 20 | 3.5 | rectangular |
| | 3-96 | R-96 | 210 | 35 | 20 | 3.7 | rectangular |
| | 3-97 | R-97 | 210 | 35 | 20 | 3.6 | rectangular |
| | 3-98 | R-98 | 210 | 35 | 20 | 3.5 | rectangular |
| | 3-99 | R-99 | 210 | 35 | 20 | 3.6 | rectangular |
| | 3-100 | R-100 | 210 | 35 | 20 | 3.6 | rectangular |

**Table 12**

| | | Resist composition | Eop (µC/cm²) | Maximum resolution of LS (nm) | Maximum resolution of IS (nm) | LER (nm) | Pattern profile |
|---|---|---|---|---|---|---|---|
| Example | 3-101 | R-101 | 210 | 35 | 20 | 3.5 | rectangular |
| | 3-102 | R-102 | 210 | 35 | 20 | 3.6 | rectangular |
| | 3-103 | R-103 | 210 | 35 | 20 | 3.5 | rectangular |
| | 3-104 | R-104 | 210 | 35 | 20 | 3.6 | rectangular |
| | 3-105 | R-105 | 210 | 35 | 20 | 3.5 | rectangular |
| | 3-106 | R-106 | 210 | 35 | 20 | 3.6 | rectangular |
| | 3-107 | R-107 | 210 | 35 | 20 | 3.5 | rectangular |
| | 3-108 | R-108 | 210 | 35 | 20 | 3.6 | rectangular |
| | 3-109 | R-109 | 210 | 35 | 20 | 3.6 | rectangular |
| | 3-110 | R-110 | 210 | 35 | 20 | 3.5 | rectangular |
| | 3-111 | R-111 | 210 | 35 | 20 | 3.6 | rectangular |

**Table 13**

| | | Resist composition | Eop (µC/cm²) | Maximum resolution of LS (nm) | Maximum resolution of IS (nm) | LER (nm) | Pattern profile |
|---|---|---|---|---|---|---|---|
| Comparative Example | 3-1 | CR-1 | 210 | 45 | 30 | 4.6 | rounded top |
| | 3-2 | CR-2 | not dissolved in solvent, failed to prepare resist composition | | | | |
| | 3-3 | CR-3 | not dissolved in solvent, failed to prepare resist composition | | | | |
| | 3-4 | CR-4 | 210 | 40 | 30 | 4.7 | rounded top |
| | 3-5 | CR-5 | 210 | 45 | 30 | 4.8 | rounded top |

### Etch resistance test

### Examples 4-1 to 4-3 and Comparative Examples 4-1 and 4-2

Using a coater/developer system ACT-M (Tokyo Electron Ltd.), each of the positive resist compositions (R-8, R-9, CR-4, CR-5) was spin coated onto a reflection type mask blank (for EUV lithography mask, 152 mm square, outermost surface of chromium compound) and prebaked on a hotplate at 110°C for 600 seconds to form a resist film of 120 nm thick. The thickness of the resist film was measured by an optical film thickness measurement system Nanospec (Nanometrics Inc.). Measurement was made at 81 points in the plane of the blank substrate excluding an outer rim portion extending 10 mm inward from the periphery, and an average film thickness and a film thickness range were computed therefrom. Using a dry etching system UNAXIS G4, the coated substrate was dry etched under the conditions shown below. A rate of film thickness loss (A/sec) was computed from the initial thickness and the thickness of the remaining film after etching. The results are shown in Table 14.

| | |
|---|---|
| RF1 (RIE): | pulse 700 V |
| RF2 (ICP): | CW 400 W |
| Pressure: | 6 mTorr |
| Cl₂: | 185 sccm |
| O₂: | 55 sccm |
| He: | 9.25 sccm |
| etching time: | 75 sec |

**Table 14**

| | | Resist composition | Loss rate (Å/sec) |
|---|---|---|---|
| Example | 4-1 | R-8 | 6.7 |
| | 4-2 | R-63 | 6.6 |
| | 4-3 | R-64 | 6.6 |
| Comparative Example | 4-1 | CR-4 | 8.3 |
| | 4-2 | CR-5 | 8.4 |

All chemically amplified positive resist compositions (R-1 to R-111) within the scope of the invention were satisfactory in resolution, LER and pattern rectangularity. Of comparative resist compositions (CR-1 to CR-5), CR-2 and CR-3 were less soluble in the resist solvent, and a resist composition could not be prepared. CR-1, CR-4 and CR-5 were short in optimization of acid diffusion and the resulting patterns were poor in resolution, LER and/or rectangularity. In the dry etching test, the films of R-8, R-63 and R-64 showed better etch resistance than the films of CR-4 and CR-5. It is suggested that the incorporation of formula (A1) structure in the polymer is effective for mask processing.

It has been demonstrated that the resist pattern forming process using chemically amplified positive resist compositions within the scope of the invention are useful in the photolithography for the fabrication of semiconductor devices, especially the processing of photomask blanks of transmission and reflection types.

## Claims

1. A sulfonium salt monomer having the formula (A1): 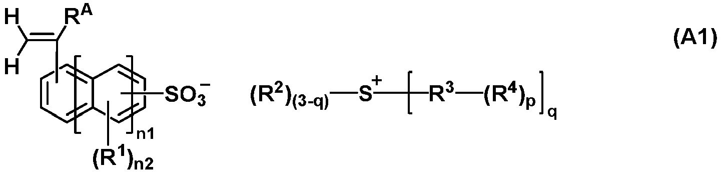 wherein n1 is an integer of 0 to 2, n2 is an integer meeting 0 ≤ n2 ≤ 5+2(n1)-1, p is an integer of 1 to 5, q is an integer of 1 to 3,
R^{A} is hydrogen, fluorine, methyl or trifluoromethyl,
R¹ is halogen, nitro, cyano, an optionally halogenated C₁-C₁₀ saturated hydrocarbyl group, optionally halogenated C₁-C₁₀ saturated hydrocarbyloxy group, optionally halogenated C₂-C₁₀ saturated hydrocarbylcarbonyloxy group, or C₁-C₁₀ fluorinated saturated hydrocarbylthio group,
R² is a C₁-C₃₀ hydrocarbyl group which may contain a heteroatom,
R³ is a C₁-C₃₀ (p+1)-valent hydrocarbon group which may contain a heteroatom,
R⁴ is fluorine, a C₁-C₅ fluorinated saturated hydrocarbyl group, C₁-C₅ fluorinated saturated hydrocarbyloxy group, C₂-C₅ fluorinated saturated hydrocarbylcarbonyloxy group, C₂-C₅ fluorinated saturated hydrocarbyloxycarbonyl group, or C₁-C₅ fluorinated saturated hydrocarbylthio group, in which some hydrogen may be substituted by at least one moiety selected from hydroxy, chlorine, bromine, iodine, nitro and cyano, and at least one moiety selected from ester bond, ether bond, sulfonate ester bond, carbonate bond and carbamate bond may intervene in a carbon-carbon bond,
when q is 1, any two of two R² and R³ may bond together to form a ring with the sulfur atom to which they are attached; when q is 2, any two of R² and two R³ may bond together to form a ring with the sulfur atom to which they are attached; when q is 3, any two of three R³ may bond together to form a ring with the sulfur atom to which they are attached.

2. The sulfonium salt monomer of claim 1, having the formula (A1-1): 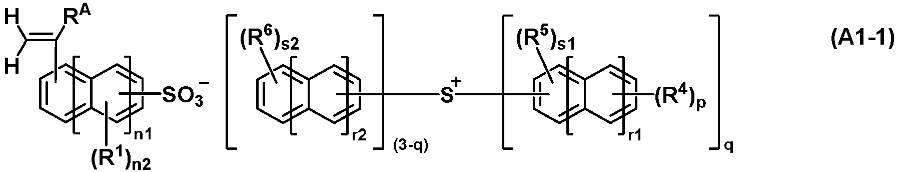 wherein n1, n2, p, q, R⁴, R¹ and R⁴ are as defined above,
R⁵ and R⁶ are each independently halogen exclusive of fluorine, nitro, cyano, or a C₁-C₂₀ hydrocarbyl group which may contain a heteroatom,
r1 and r2 are each independently an integer of 0 to 2, s1 is an integer meeting 0 ≤ s1 ≤ 2(r1)+4, s2 is an integer meeting 0 ≤ s2 ≤ 2(r2)+4.

3. A polymer comprising repeat units derived from the sulfonium salt monomer of claim 1 or 2 and adapted to increase its solubility in alkaline aqueous solution under the action of acid.

4. The polymer of claim 3, further comprising repeat units having the formula (A2): 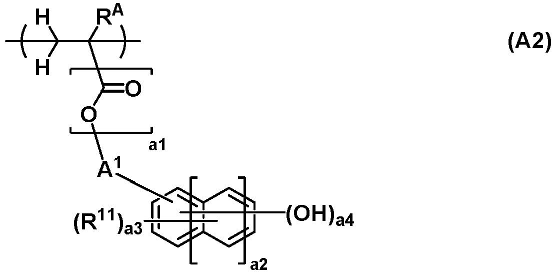 wherein a1 is 0 or 1, a2 is an integer of 0 to 2, a3 is an integer meeting 0 ≤ a3 ≤ 5+2(a2)-a4, a4 is an integer of 1 to 3,
R^{A} is hydrogen, fluorine, methyl or trifluoromethyl,
R¹¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, and
A¹ is a single bond or C₁-C₁₀ saturated hydrocarbylene group in which some -CH₂-may be replaced by -O-.

5. The polymer of claim 3 or 4, further comprising repeat units of at least one type selected from repeat units having the formula (A3-1) and repeat units having the formula (A3-2): 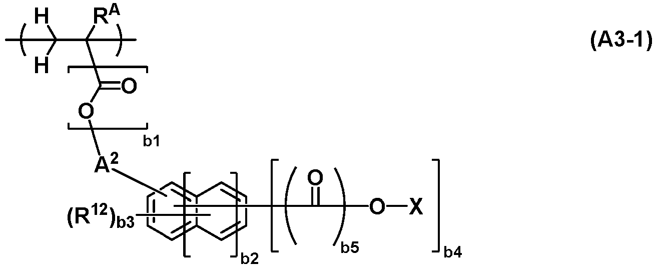 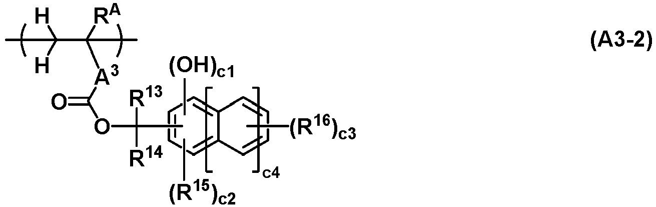 wherein b1 is 0 or 1, b2 is an integer of 0 to 2, b3 is an integer meeting 0 ≤ b3 ≤ 5+2(b2)-b4, b4 is an integer of 1 to 3, b5 is 0 or 1,
c1 is an integer of 0 to 2, c2 is an integer of 0 to 2, c3 is an integer of 0 to 5, c4 is an integer of 0 to 2,
R^{A} is hydrogen, fluorine, methyl or trifluoromethyl,
R¹² is nitro, cyano, halogen, an optionally halogenated C₁-C₆ saturated hydrocarbyl group, optionally halogenated C₁-C₆ saturated hydrocarbyloxy group, optionally halogenated C₁-C₆ saturated hydrocarbylthio group, C₂-C₈ saturated hydrocarbylcarbonyl group, or optionally halogenated C₂-C₈ saturated hydrocarbylcarbonyloxy group,
R¹³ and R¹⁴ are each independently a C₁-C₁₀ hydrocarbyl group which may contain a heteroatom, R¹³ and R¹⁴ may bond together to form a ring with the carbon atom to which they are attached,
R¹⁵ is each independently fluorine, a C₁-C₅ fluorinated alkyl group or C₁-C₅ fluorinated alkoxy group,
R¹⁶ is each independently a C₁-C₁₀ hydrocarbyl group which may contain a heteroatom,
A² is a single bond or C₁-C₁₀ saturated hydrocarbylene group in which some -CH₂-may be replaced by -O-,
X is an acid labile group when b4 is 1, and X is each independently hydrogen or an acid labile group, at least one X being an acid labile group, when b4 is 2 or 3,
A³ is a single bond, phenylene, naphthylene, or *-C(=O)-O-A³¹-, A³¹ is a C₁-C₂₀ aliphatic hydrocarbylene group which may contain at least one moiety selected from hydroxy, ether bond, ester bond and lactone ring, phenylene group, or naphthylene group, * designates a point of attachment to the carbon atom in the backbone.

6. The polymer of any one of claims 3 to 5, further comprising repeat units of at least one type selected from repeat units having the formula (A4), repeat units having the formula (A5), and repeat units having the formula (A6): 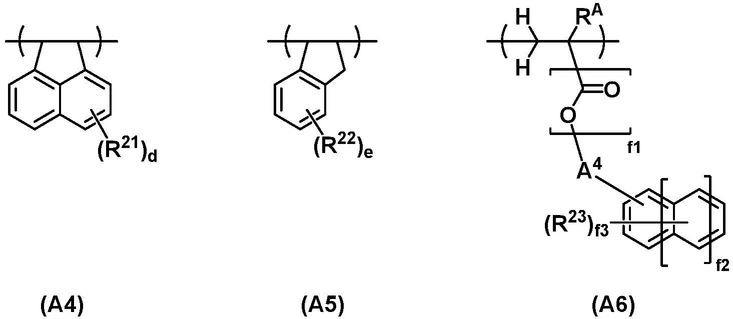 wherein d and e are each independently an integer of 0 to 4, f1 is 0 or 1, f2 is an integer of 0 to 2, f3 is an integer of 0 to 5,
R^{A} is hydrogen, fluorine, methyl or trifluoromethyl,
R²¹ and R²² are each independently hydroxy, halogen, an optionally halogenated C₁-C₈ saturated hydrocarbyl group, optionally halogenated C₁-C₈ saturated hydrocarbyloxy group, or optionally halogenated C₂-C₈ saturated hydrocarbylcarbonyloxy group,
R²³ is halogen, trifluoromethyl, trifluoromethoxy, nitro, cyano, a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, C₁-C₂₀ saturated hydrocarbylsulfinyl group, or C₁-C₂₀ saturated hydrocarbylsulfonyl group, and
A⁴ is a single bond or C₁-C₁₀ saturated hydrocarbylene group in which some -CH₂-may be replaced by -O-.

7. A chemically amplified positive resist composition comprising (A) a base polymer containing the polymer of any one of claims 3 to 6.

8. The resist composition of claim 7, further comprising (B) a quencher.

9. The resist composition of claim 7 or 8, further comprising (C) a photoacid generator.

10. The resist composition of any one of claims 7 to 9, further comprising (D) a fluorinated polymer comprising repeat units of at least one type selected from repeat units having the formula (D1), repeat units having the formula (D2), repeat units having the formula (D3) and repeat units having the formula (D4) and optionally repeat units of at least one type selected from repeat units having the formula (D5) and repeat units having the formula (D6): 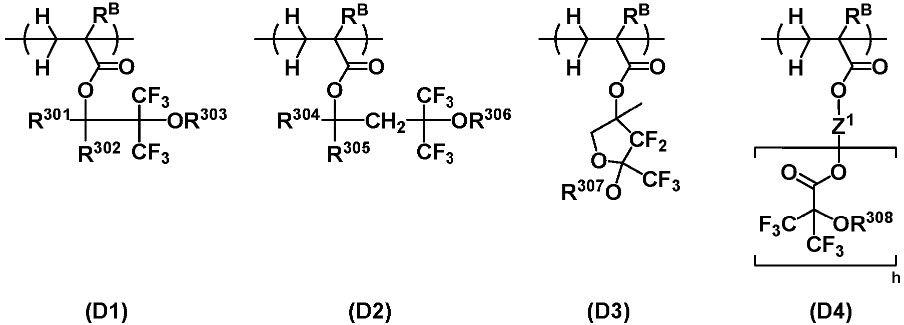 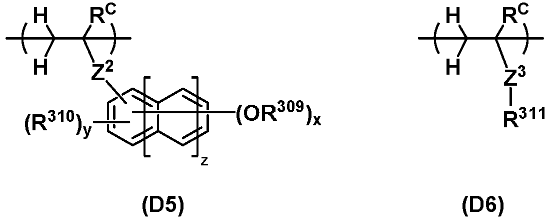 wherein x is an integer of 1 to 3, y is an integer meeting 0 ≤ y ≤ 5+2z-x, z is 0 or 1, h is an integer of 1 to 3,
R^{B} is each independently hydrogen, fluorine, methyl or trifluoromethyl,
R^{C} is each independently hydrogen or methyl,
R³⁰¹, R³⁰², R³⁰⁴ and R³⁰⁵ are each independently hydrogen or a C₁-C₁₀ saturated hydrocarbyl group,
R³⁰³, R³⁰⁶, R³⁰⁷ and R³⁰⁸ are each independently hydrogen, a C₁-C₁₅ hydrocarbyl group, C₁-C₁₅ fluorinated hydrocarbyl group, or acid labile group, and when R³⁰³, R³⁰⁶, R³⁰⁷ and R³⁰⁸ each are a hydrocarbyl or fluorinated hydrocarbyl group, an ether bond or carbonyl moiety may intervene in a carbon-carbon bond,
R³⁰⁹ is hydrogen or a C₁-C₅ straight or branched hydrocarbyl group in which a heteroatom-containing moiety may intervene in a carbon-carbon bond,
R³¹⁰ is a C₁-C₅ straight or branched hydrocarbyl group in which a heteroatom-containing moiety may intervene in a carbon-carbon bond,
R³¹¹ is a C₁-C₂₀ saturated hydrocarbyl group in which at least one hydrogen is substituted by fluorine, and in which some constituent -CH₂- may be replaced by an ester bond or ether bond,
Z¹ is a C₁-C₂₀ (h+1)-valent hydrocarbon group or C₁-C₂₀ (h+1)-valent fluorinated hydrocarbon group,
Z² is a single bond, *-C(=O)-O- or *-C(=O)-NH-, * designates a point of attachment to the carbon atom in the backbone,
Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.

11. The resist composition of any one of claims 7 to 10, further comprising (E) an organic solvent.

12. A resist pattern forming process comprising the steps of:
applying the chemically amplified positive resist composition of any one of claims 7 to 11 onto a substrate to form a resist film thereon,
exposing the resist film patternwise to high-energy radiation, and
developing the exposed resist film in an alkaline developer.

13. The process of claim 12 wherein the high-energy radiation is EUV or EB.

14. The process of claim 12 or 13 wherein the substrate is a mask blank of transmission or reflection type.

15. A mask blank of transmission or reflection type which is coated with the chemically amplified positive resist composition of any one of claims 7 to 11.
